# EUROPEAN PATENT APPLICATION

(11) **EP 4 328 310 A1**
(43) Date of publication of application: **28.02.2024**
(21) Application number: 22791121.1
(22) Date of filing: 22.04.2022
(51) Int. Cl.: C12N 15/113, A61K 31/713

(54) **SIRNA TARGETING 17B-HYDROXYSTEROID DEHYDROGENASE TYPE 13 AND SIRNA CONJUGATE**

(30) Priority: 22.04.2021 CN 202110435243
(71) Applicant: Tuojie Biotech (Shanghai) Co., Ltd., Shanghai 201203 (CN)
(72) Inventor: HUANG, Jinyu, Shanghai 201203 (CN); HUANG, Yanfen, Shanghai 201203 (CN); LUO, Min, Shanghai 201203 (CN); ZHANG, Fang, Shanghai 201203 (CN)
(74) Representative: Regimbeau
(86) International application number: PCT/CN2022/088351
(87) International publication number: WO 2022/223015

(57) **Abstract**

An siRNA targeting 17β-hydroxysteroid dehydrogenase type 13 and a siRNA conjugate. Also disclosed are a pharmaceutical composition, cell or kit containing the siRNA, and a method for using the siRNA for the treatment and/or prevention of subjects suffering from HSD17B13-related disorders (such as chronic fibroinflammatory liver disease).

## Description

The present disclosure claims priority to Chinese Patent Application No. CN202110435243.8 filed on Apr. 22, 2021, which is incorporated herein by reference in its entirety.

### TECHNICAL FIELD

The present disclosure relates to an siRNA targeting 17β-hydroxysteroid dehydrogenase type 13 (HSD17B13) and an siRNA conjugate, and use thereof.

### BACKGROUND

HSD17B13 is a steroid dehydrogenase that is a member of the 17β-hydroxysteroid dehydrogenase (17β-HSDs) family. Currently, 14 members of the 17β-HSDs family have been reported in mammals. These 14 members have different tissue distribution, subcellular localization, and functions, and are mainly involved in a series of physiological and pathophysiological processes of the body, such as reproduction, development, and obesity, as enzymes related to the metabolism of steroid hormones, prostaglandins, lipids, heterobiotins, and retinoids.

It is found that in human subjects suffering from simple steatosis, HSD17B13 protein expression is significantly upregulated in fatty liver lipid droplets (LDs) and is determined to be predominantly located on the surface of LDs. When the HSD17B13 protein is overexpressed, it results in an increase in the number and size of LDs in hepatocytes and a significant increase in hepatic lipogenesis and triglyceride (TG) content (Su W, Wang Y, Jia X, et al., Comparative proteomic study reveals 17β-HSD13 as a pathogenic protein in nonalcoholic fatty liver disease [J]. Proceedings of the National Academy of Sciences of the United States of America, 2014, 111 (31): 11437).

Recent studies have further shown that HSD17B13 gene expression plays an important role in the pathogenesis of nonalcoholic fatty liver disease (NAFLD) and nonalcoholic steatohepatitis (NASH). HSD17B13 (rs72613567:TA) loss-of-function mutant reduces levels of glutamic pyruvic transaminase (ALT) and glutamic oxaloacetic transaminase (AST) while reducing inflammation and liver injury in patients with fatty liver disease (Abul-Husn N S, Cheng X, Li AH, et al., A Protein-Truncating HSD17B13 Variant and Protection from Chronic Liver Disease. [J]. N Engl J Med, 2018, 378 (12): 1096-1106). NAFLD is a liver disease related to obesity, insulin resistance, type 2 diabetes, hypertension, hyperlipidemia, and metabolic syndrome. Patients with NAFLD account for approximately 25% of the total population in the united states, with nearly one-fourth of them progressing to NASH characterized by liver inflammation, hepatocyte injury, and liver fibrosis. It is found in HSD17B13 (rs72613567:TA) mutant carriers that expression levels of several key inflammatory factors are decreased, particularly the plasma concentration of interleukin (IL-6) is significantly lower than that in non-carriers; meanwhile, it is found in patients that serum ALT and pediatric NAFLD fibrosis index are both reduced, suggesting that the HSD17B13 loss-of-function mutant may reduce the risk of NASH and progressive liver injury (Luukkonen P K, Tukiainen T, Juuti A, et al., Hydroxysteroid 17-β dehydrogenase 13 variant increases phospholipids and protects against fibrosis in nonalcoholic fatty liver disease [J]. JCI Insight, 2020, 5 (5)). Currently, there is no clinically effective treatment for NASH. Therefore, the study results for HSD17B13 provide an early warning and a new therapeutic strategy for the treatment of NAFLD and related liver diseases.

RNA interference (siRNA) is an effective way to silence gene expression, which can specifically degrade mRNA of a target gene through a post-transcriptional regulatory mechanism.

Statistically, about more than 80% of the proteins related to diseases in humans are non-druggable proteins as they cannot be targeted by the conventional small-molecule drugs and biomacromolecule formulations. The overexpression of HSD17B13 during NAFLD progression induces steatosis, increases the accumulation of fat in the liver, and improves the level of liver TG. By designing an siRNA for the mRNA product transcribed by HSD17B13, the naturally occurring genetic variation in HSD17B13 that leads to loss of function can be mimicked, which prevents the NAFLD from further progressing to the NASH, and prevents the patient with the NASH from further developing the cirrhosis and the liver cancer, thereby achieving the purpose of treatment.

### SUMMARY

The present disclosure provides an siRNA targeting HSD17B13.

In some embodiments, provided is an siRNA targeting an HSD17B13 target gene (Genbank accession No. NM_178135.5).

In some embodiments, the present disclosure provides an siRNA comprising a sense strand and an antisense strand forming a double-stranded region; the sense strand comprises at least 15 contiguous nucleotides and differs from a nucleotide sequence of NM_178135.5 by no more than 3 nucleotides; the antisense strand comprises at least 15 contiguous nucleotide sequences and differs from a nucleotide sequence complementary to NM_178135.5 by no more than 3 nucleotides.

In some embodiments, the present disclosure provides an siRNA comprising a sense strand and an antisense strand forming a double-stranded region; the sense strand comprises at least 15 contiguous nucleotides and differs from a nucleotide sequence of SEQ ID NO: 1 by no more than 3 nucleotides; the antisense strand comprises at least 15 contiguous nucleotide sequences and differs from a nucleotide sequence of SEQ ID NO: 2 by no more than 3 nucleotides.

In some embodiments, the sense strand of the siRNA of the present disclosure comprises at least 15 (e.g., 16, 17, 18, 19, 20, 21, and 22) contiguous nucleotides and differs from any one of nucleotide sequences of SEQ ID NO: 3 to SEQ ID NO: 24 by no more than 3 nucleotides.

In some embodiments, the antisense strand of the siRNA of the present disclosure comprises at least 15 (e.g., 16, 17, 18, 19, 20, 21, 22, 23, and 24) contiguous nucleotide sequences and differs from any one of nucleotide sequences of SEQ ID NO: 25 to SEQ ID NO: 46 by no more than 3 nucleotides.

In some embodiments, the sense strand of the siRNA of the present disclosure comprises or is 19 contiguous nucleotides and differs from any one of nucleotide sequences of SEQ ID NO: 3 to SEQ ID NO: 24 by no more than 3 nucleotides, and in some embodiments, by no more than 1 nucleotide; in some embodiments, position 1 at the 3' end of the nucleotide sequence of the sense strand is identical to or different from that of any one of SEQ ID NO: 3 to SEQ ID NO: 24.

In some embodiments, the antisense strand of the siRNA of the present disclosure comprises or is 21 contiguous nucleotides and differs from any one of nucleotide sequences of SEQ ID NO: 25 to SEQ ID NO: 46 by no more than 3 nucleotides, and in some embodiments, by no more than 1 nucleotide; in some embodiments, position 1 at the 5' end of the nucleotide sequence of the antisense strand is identical to or different from that of any one of SEQ ID NO: 25 to SEQ ID NO: 46.

In some embodiments, the sense strand of the siRNA of the present disclosure comprises or is a nucleotide sequence of any one of SEQ ID NO: 3 to SEQ ID NO: 24.

In some embodiments, the antisense strand of the siRNA of the present disclosure comprises or is a nucleotide sequence of any one of SEQ ID NO: 25 to SEQ ID NO: 46. In some embodiments, the antisense strand comprises a chemical modification of formula (I) or a tautomeric modification thereof in at least one of nucleotide positions 2 to 8 (e.g., positions 2, 3, 4, 5, 6, 7, and 8) of the 5' region thereof: 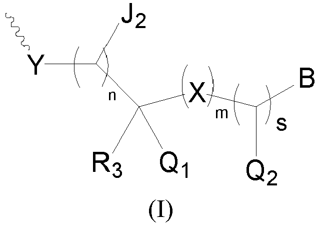 wherein: Y is selected from the group consisting of O, NH, and S;
each X is independently selected from the group consisting of CR₄(R₄'), S, NRs, and NH-CO, wherein R₄, R₄', and R₅ are each independently H or C₁-C₆ alkyl;
J₂ is H or C₁-C₆ alkyl;
n = 0, 1, or 2;
m = 0, 1, or 2;
s = 0 or 1;
R₃ is selected from the group consisting of H, OH, halogen, NH₂, C₁-C₆ alkyl, C₁-C₆ alkoxy, C₂-C₆ alkenyl, C₂-C₆ alkynyl, S-CH₃, NCH₃(CH₃), OCH₂CH₂OCH₃, -O-alkylamino, and (CH₂)ₚR₆, wherein R₆ is selected from the group consisting of OH, halogen, methoxy, ethoxy, N₃, C₂-C₆ alkenyl, and C₂-C₆ alkynyl, and p = 1, 2, or 3;
Q₁ is 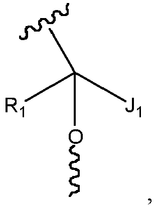 and Q₂ is R₂; or Q₁ is R₂, and Q₂ is 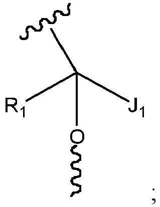 wherein:
   R₁ is selected from the group consisting of H, C₁-C₆ alkyl, C₁-C₆ alkoxy, C₂-C₆ alkenyl, C₂-C₆ alkynyl, and (CH₂)_{q}R₇, wherein R₇ is selected from the group consisting of OH, halogen, methoxy, ethoxy, N₃, C₂-C₆ alkenyl, and C₂-C₆ alkynyl, and q = 1, 2, or 3;
   Ji is H or C₁-C₆ alkyl;
   R₂ is selected from the group consisting of H, OH, halogen, NH₂, C₁-C₆ alkyl, C₁-C₆ alkoxy, C₂-C₆ alkenyl, C₂-C₆ alkynyl, S-CH₃, NCH₃(CH₃), OCH₂CH₂OCH₃, -O-alkylamino, and (CH₂)ᵣR₈, wherein R₈ is selected from the group consisting of OH, halogen, methoxy, ethoxy, N₃, C₂-C₆ alkenyl, and C₂-C₆ alkynyl, and r = 1, 2, or 3;
   optionally, R₁ and R₂ are directly connected to form a ring;
   B is a base;
   wherein: the chemical modification of formula (I) or the tautomeric modification thereof is not

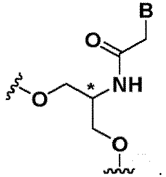

In some embodiments, when X is NH-CO, R₁ is not H.

In some embodiments, B is a base; for example, B is selected from the group consisting of those of purine bases, pyrimidine bases, indoles, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, each B is independently selected from the group consisting of natural bases at nucleotide positions 2 to 8 (e.g., positions 2, 3, 4, 5, 6, 7, and 8) containing the chemical modification of formula (I-1) of the 5' region of the antisense strand.

In some embodiments, the antisense strand comprises a chemical modification of formula (I-1) or a tautomeric modification thereof in at least one of nucleotide positions 2 to 8 (e.g., positions 2, 3, 4, 5, 6, 7, and 8) of the 5' region thereof: 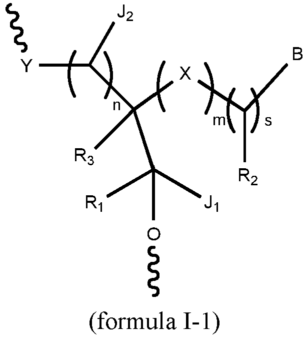 wherein: Y is selected from the group consisting of O, NH, and S;
each X is independently selected from the group consisting of CR₄(R₄'), S, NRs, and NH-CO, wherein R₄, R₄', and R₅ are each independently H or C₁-C₆ alkyl;
J₁ and J₂ are each independently H or C₁-C₆ alkyl;
n = 0, 1, or 2; m = 0, 1, or 2; s = 0 or 1;
R₃ is selected from the group consisting of H, OH, halogen, NH₂, C₁-C₆ alkyl, C₁-C₆ alkoxy, C₂-C₆ alkenyl, C₂-C₆ alkynyl, S-CH₃, NCH₃(CH₃), OCH₂CH₂OCH₃, -O-alkylamino, and (CH₂)ₚR₆, wherein R₆ is selected from the group consisting of OH, halogen, methoxy, ethoxy, N₃, C₂-C₆ alkenyl, and C₂-C₆ alkynyl, and p = 1, 2, or 3;
R₁ is selected from the group consisting of H, C₁-C₆ alkyl, C₁-C₆ alkoxy, C₂-C₆ alkenyl, C₂-C₆ alkynyl, and (CH₂)_{q}R₇, wherein R₇ is selected from the group consisting of OH, halogen, methoxy, ethoxy, N₃, C₂-C₆ alkenyl, and C₂-C₆ alkynyl, and q = 1, 2, or 3;
R₂ is selected from the group consisting of H, OH, halogen, NH₂, C₁-C₆ alkyl, C₁-C₆ alkoxy, C₂-C₆ alkenyl, C₂-C₆ alkynyl, S-CH₃, NCH₃(CH₃), OCH₂CH₂OCH₃, -O-alkylamino, and (CH₂)ᵣR₈, wherein R₈ is selected from the group consisting of OH, halogen, methoxy, ethoxy, N₃, C₂-C₆ alkenyl, and C₂-C₆ alkynyl, and r = 1, 2, or 3;
optionally, R₁ and R₂ are directly connected to form a ring;
B is as defined in formula (I).

In some embodiments, the antisense strand comprises a chemical modification of formula (I-2) or a tautomeric modification thereof in at least one of nucleotide positions 2 to 8 (e.g., positions 2, 3, 4, 5, 6, 7, and 8) of the 5' region thereof: 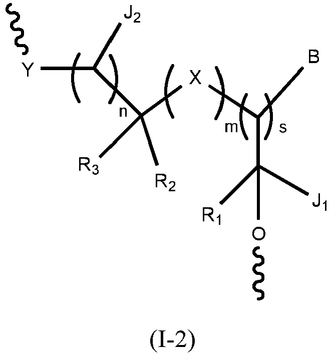 wherein Y is selected from the group consisting of O, NH, and S;
each X is independently selected from the group consisting of CR₄(R₄'), S, NRs, and NH-CO, wherein R₄, R₄', and R₅ are each independently H or C₁-C₆ alkyl;
n = 0, 1, or 2; m = 0, 1, or 2; s = 0 or 1;
J₁ and J₂ are each independently H or C₁-C₆ alkyl;
R₃ is selected from the group consisting of H, OH, halogen, NH₂, C₁-C₆ alkyl, C₁-C₆ alkoxy, C₂-C₆ alkenyl, C₂-C₆ alkynyl, S-CH₃, NCH₃(CH₃), OCH₂CH₂OCH₃, -O-alkylamino, and (CH₂)ₚR₆, wherein R₆ is selected from the group consisting of OH, halogen, methoxy, ethoxy, N₃, C₂-C₆ alkenyl, and C₂-C₆ alkynyl, and p = 1, 2, or 3;
R₁ is selected from the group consisting of H, C₁-C₆ alkyl, C₁-C₆ alkoxy, C₂-C₆ alkenyl, C₂-C₆ alkynyl, and (CH₂)_{q}R₇, wherein R₇ is selected from the group consisting of OH, halogen, methoxy, ethoxy, N₃, C₂-C₆ alkenyl, and C₂-C₆ alkynyl, and q = 1, 2, or 3;
R₂ is selected from the group consisting of H, C₁-C₆ alkyl, C₁-C₆ alkoxy, S-CH₃, NCH₃(CH₃), OCH₂CH₂OCH₃, -O-alkylamino, and (CH₂)ᵣR₈, wherein R₈ is selected from the group consisting of OH, halogen, methoxy, ethoxy, N₃, C₂-C₆ alkenyl, and C₂-C₆ alkynyl, and r = 1, 2, or 3;
optionally, R₁ and R₂ are directly connected to form a ring;
B is as defined in formula (I).

In some embodiments, B is a base; for example, B is selected from the group consisting of those of purine bases, pyrimidine bases, indoles, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, each B is independently selected from the group consisting of bases at nucleotide positions 2 to 8 (e.g., positions 2, 3, 4, 5, 6, 7, and 8) containing the chemical modification of formula (I-1) of the 5' region of the antisense strand. In some specific embodiments, each B is independently selected from the group consisting of natural bases at nucleotide positions 2 to 8 (e.g., positions 2, 3, 4, 5, 6, 7, and 8) containing the chemical modification of formula (I-1) of the 5' region of the antisense strand. In some embodiments, the chemical modification or the tautomeric modification thereof described above is not

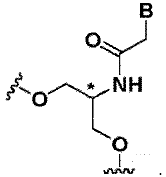

In some embodiments, each X is independently selected from the group consisting of CR₄(R₄'), S, NRs, and NH-CO, wherein R₄, R₄', and R₅ are each independently H or Ci-C₃ alkyl;
n = 0, 1, or 2; m = 0, 1, or 2; s = 0 or 1;
J₁ and J₂ are each independently H or C₁-C₃ alkyl;
R₃ is selected from the group consisting of H, OH, halogen, NH₂, C₁-C₃ alkyl, C₁-C₃ alkoxy, C₂-C₄ alkenyl, C₂-C₄ alkynyl, S-CH₃, NCH₃(CH₃), OCH₂CH₂OCH₃, -O-alkylamino, and (CH₂)ₚR₆, wherein R₆ is selected from the group consisting of OH, halogen, methoxy, ethoxy, N₃, C₂-C₆ alkenyl, and C₂-C₆ alkynyl, and p = 1, 2, or 3;
R₁ is selected from the group consisting of H, C₁-C₃ alkyl, C₁-C₃ alkoxy, C₂-C₄ alkenyl, C₂-C₄ alkynyl, and (CH₂)_{q}R₇, wherein R₇ is selected from the group consisting of OH, halogen, methoxy, ethoxy, N₃, C₂-C₄ alkenyl, and C₂-C₄ alkynyl, and q = 1, 2, or 3;
R₂ is selected from the group consisting of H, OH, halogen, NH₂, C₁-C₃ alkyl, C₁-C₃ alkoxy, C₂-C₄ alkenyl, C₂-C₄ alkynyl, S-CH₃, NCH₃(CH₃), OCH₂CH₂OCH₃, -O-alkylamino, and (CH₂)ᵣR₈, wherein R₈ is selected from the group consisting of OH, halogen, methoxy, ethoxy, N₃, C₂-C₄ alkenyl, and C₂-C₄ alkynyl, and r = 1, 2, or 3;
optionally, R₁ and R₂ are directly connected to form a ring.
B is as defined in formula (I).

In some embodiments, B is a base; for example, B is selected from the group consisting of those of purine bases, pyrimidine bases, indoles, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, each B is independently selected from the group consisting of bases at nucleotide positions 2 to 8 (e.g., positions 2, 3, 4, 5, 6, 7, and 8) containing the chemical modification of formula (I-1) of the 5' region of the antisense strand.

In some specific embodiments, each B is independently selected from the group consisting of natural bases at nucleotide positions 2 to 8 (e.g., positions 2, 3, 4, 5, 6, 7, and 8) containing the chemical modification of formula (I-1) of the 5' region of the antisense strand. In some embodiments, each X is independently selected from the group consisting of CR₄(R₄'), S, NRs, and NH-CO, wherein R₄, R₄', and R₅ are each independently H, methyl, ethyl, *n*-propyl, or isopropyl;
n = 0, 1, or 2; m = 0, 1, or 2; s = 0 or 1;
J₁ and J₂ are each independently H or methyl;
R₃ is selected from the group consisting of H, OH, F, Cl, NH₂, methyl, ethyl, n-propyl, isopropyl, methoxy, ethoxy, *n*-propoxy, isopropoxy, vinyl, allyl, ethynyl, propargyl, S-CH₃, NCH₃(CH₃), OCH₂CH₂OCH₃, -O-methylamino, -O-ethylamino, and (CH₂)ₚR₆, wherein R₆ is selected from the group consisting of OH, F, Cl, methoxy, ethoxy, N₃, vinyl, allyl, ethynyl, and propargyl, and p = 1 or 2;
R₁ is selected from the group consisting of H, methyl, ethyl, *n*-propyl, isopropyl, methoxy, ethoxy, *n*-propoxy, isopropoxy, vinyl, allyl, ethynyl, propargyl, and (CH₂)_{q}R₇, wherein R₇ is selected from the group consisting of OH, F, Cl, methoxy, ethoxy, N₃, vinyl, allyl, ethynyl, and propargyl, and q = 1 or 2;
R₂ is selected from the group consisting of H, OH, F, Cl, NH₂, methyl, ethyl, *n*-propyl, isopropyl, methoxy, ethoxy, *n*-propoxy, isopropoxy, vinyl, allyl, ethynyl, propargyl, S-CH₃, NCH₃(CH₃), OCH₂CH₂OCH₃, -O-methylamino, -O-ethylamino, and (CH₂)ᵣR₈, wherein R₈ is selected from the group consisting of OH, F, Cl, methoxy, ethoxy, N₃, vinyl, allyl, ethynyl, and propargyl, and r = 1 or 2;
optionally, R₁ and R₂ are directly connected to form a ring.
B is as defined in formula (I).

In some embodiments, B is a base; for example, B is selected from the group consisting of those of purine bases, pyrimidine bases, indoles, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, each B is independently selected from the group consisting of bases at nucleotide positions 2 to 8 (e.g., positions 2, 3, 4, 5, 6, 7, and 8) containing the chemical modification of formula (I-1) of the 5' region of the antisense strand.

In some specific embodiments, each B is independently selected from the group consisting of natural bases at nucleotide positions 2 to 8 (e.g., positions 2, 3, 4, 5, 6, 7, and 8) containing the chemical modification of formula (I-1) of the 5' region of the antisense strand. In some embodiments involving formula (I-1), Y is O or NH; each X is independently selected from the group consisting of NH-CO, CH₂, and NH;
n = 0 or1; m = 0 or1; s = 0 or 1;
J₁ and J₂ are each independently H;
R₁ is selected from the group consisting of H, methyl, and CH₂OH;
R₂ is selected from the group consisting of H, OH, NH₂, methyl, and CH₂OH;
R₃ is selected from the group consisting of H, OH, NH₂, methyl, and CH₂OH; optionally, R₁ and R₂ are directly connected to form a ring;
B is as defined in formula (I).

In some embodiments, B is a base; for example, B is selected from the group consisting of those of purine bases, pyrimidine bases, indoles, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, each B is independently selected from the group consisting of bases at nucleotide positions 2 to 8 (e.g., positions 2, 3, 4, 5, 6, 7, and 8) containing the chemical modification of formula (I-1) of the 5' region of the antisense strand.

In some specific embodiments, each B is independently selected from the group consisting of natural bases at nucleotide positions 2 to 8 (e.g., positions 2, 3, 4, 5, 6, 7, and 8) containing the chemical modification of formula (I-1) of the 5' region of the antisense strand. In some embodiments involving formula (I-2), Y is O or NH; each X is independently selected from the group consisting of NH-CO, CH₂, and NH;
n = 0 or1; m = 0 or1; s = 0 or 1;
J₁ and J₂ are each independently H;
R₁ is selected from the group consisting of H, methyl, and CH₂OH;
R₂ is selected from the group consisting of H, methyl, and CH₂OH;
R₃ is selected from the group consisting of H, OH, NH₂, methyl, and CH₂OH; optionally, R₁ and R₂ are directly connected to form a ring;

In some embodiments, B is a base; for example, B is selected from the group consisting of those of purine bases, pyrimidine bases, indoles, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, each B is independently selected from the group consisting of bases at nucleotide positions 2 to 8 (e.g., positions 2, 3, 4, 5, 6, 7, and 8) containing the chemical modification of formula (I-1) of the 5' region of the antisense strand.

In some specific embodiments, each B is independently selected from the group consisting of natural bases at nucleotide positions 2 to 8 (e.g., positions 2, 3, 4, 5, 6, 7, and 8) containing the chemical modification of formula (I-1) of the 5' region of the antisense strand. In some embodiments, the chemical modification of formula (I) or the tautomeric modification thereof is selected from the group consisting of: 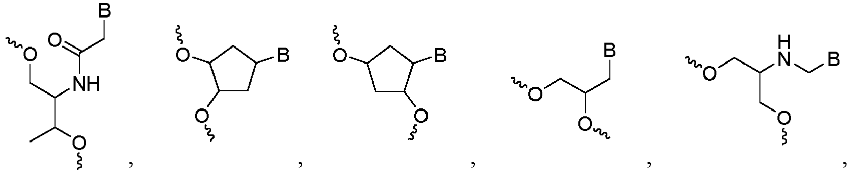 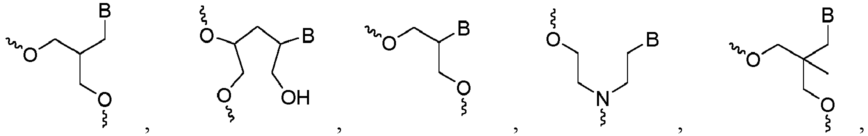 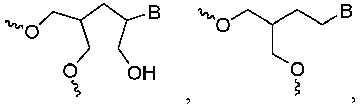 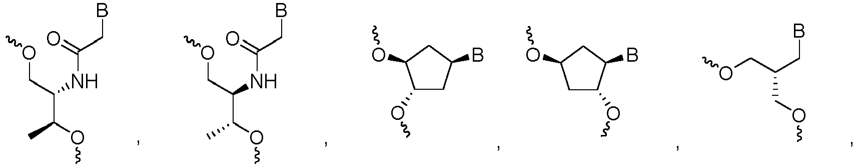 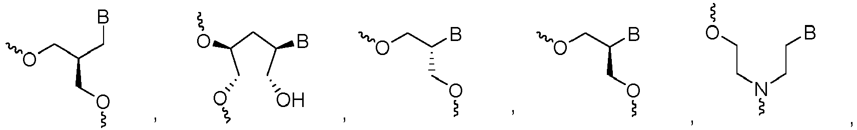 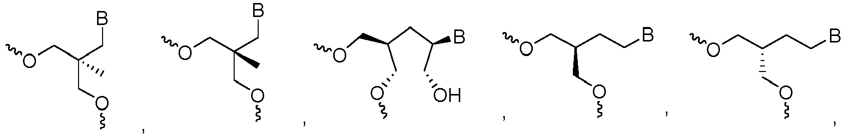 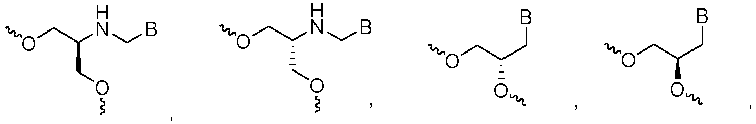 wherein: B is a base; for example, B is selected from the group consisting of those of purine bases, pyrimidine bases, indoles, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, each B is independently selected from the group consisting of bases at nucleotide positions 2 to 8 (e.g., positions 2, 3, 4, 5, 6, 7, and 8) containing the chemical modification of formula (I-1) of the 5' region of the antisense strand.

In some specific embodiments, each B is independently selected from the group consisting of natural bases at nucleotide positions 2 to 8 (e.g., positions 2, 3, 4, 5, 6, 7, and 8) containing the chemical modification of formula (I-1) of the 5' region of the antisense strand. In some embodiments, the chemical modification of formula (I) or the tautomeric modification thereof is selected from the group consisting of: 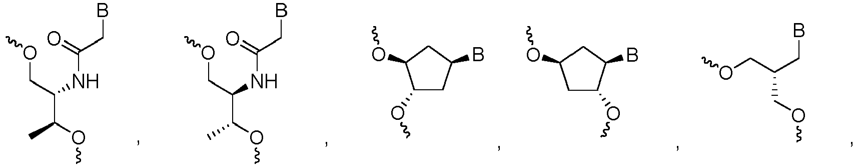 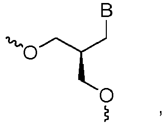 wherein: B is a base; for example, B is selected from the group consisting of those of purine bases, pyrimidine bases, indoles, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, each B is independently selected from the group consisting of bases at nucleotide positions 2 to 8 (e.g., positions 2, 3, 4, 5, 6, 7, and 8) containing the chemical modification of formula (I-1) of the 5' region of the antisense strand.

In some specific embodiments, each B is independently selected from the group consisting of natural bases at nucleotide positions 2 to 8 (e.g., positions 2, 3, 4, 5, 6, 7, and 8) containing the chemical modification of formula (I-1) of the 5' region of the antisense strand. The present disclosure provides an siRNA comprising a sense strand and an antisense strand forming a double-stranded region; the sense strand comprises at least 15 contiguous nucleotides and differs from a nucleotide sequence of SEQ ID NO: 1 by no more than 3 nucleotides; the antisense strand comprises at least 15 contiguous nucleotide sequences and differs from a nucleotide sequence of SEQ ID NO: 2 by no more than 3 nucleotides; wherein, the uppercase letters C, G, U, and A represent base components of a nucleotide.

In some embodiments, the antisense strand comprises a chemical modification of formula (I') or a tautomeric modification thereof in at least one of nucleotide positions 2 to 8 (e.g., positions 2, 3, 4, 5, 6, 7, and 8) of the 5' region thereof: 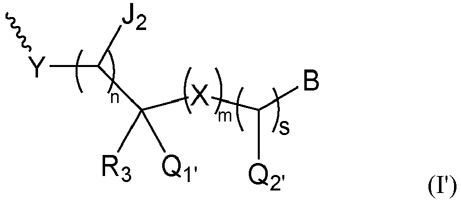 wherein: Y is selected from the group consisting of O, NH, and S;
each X is independently selected from the group consisting of CR₄(R₄'), S, NRs, and NH-CO, wherein R₄, R₄', and R₅ are each independently H or C₁-C₆ alkyl;
J₂ is H or C₁-C₆ alkyl;
n = 0, 1, or 2; m = 0, 1, or 2; s = 0 or 1;
R₃ is selected from the group consisting of H, OH, halogen, NH₂, C₁-C₆ alkyl, C₁-C₆ alkoxy, C₂-C₆ alkenyl, C₂-C₆ alkynyl, S-CH₃, NCH₃(CH₃), OCH₂CH₂OCH₃, -O-alkylamino, and (CH₂)ₚR₆, wherein R₆ is selected from the group consisting of OH, halogen, methoxy, ethoxy, N₃, C₂-C₆ alkenyl, and C₂-C₆ alkynyl, and p = 1, 2, or 3;
Q_{1'} is 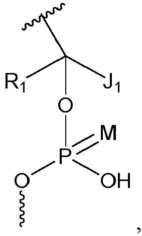 and Q_{2'} is R₂; or Q_{1'} is R₂, and Q_{2'} is 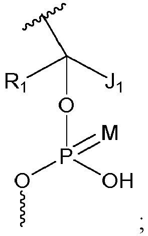 wherein:
   R₁ is selected from the group consisting of H, C₁-C₆ alkyl, C₁-C₆ alkoxy, C₂-C₆ alkenyl, C₂-C₆ alkynyl, and (CH₂)_{q}R₇, wherein R₇ is selected from the group consisting of OH, halogen, methoxy, ethoxy, N₃, C₂-C₆ alkenyl, and C₂-C₆ alkynyl, and q = 1, 2, or 3;
   Ji is H or C₁-C₆ alkyl;
   R₂ is selected from the group consisting of H, OH, halogen, NH₂, C₁-C₆ alkyl, C₁-C₆ alkoxy, C₂-C₆ alkenyl, C₂-C₆ alkynyl, S-CH₃, NCH₃(CH₃), OCH₂CH₂OCH₃, -O-alkylamino, and (CH₂)ᵣR₈, wherein R₈ is selected from the group consisting of OH, halogen, methoxy, ethoxy, N₃, C₂-C₆ alkenyl, and C₂-C₆ alkynyl, and r = 1, 2, or 3;
   optionally, R₁ and R₂ are directly connected to form a ring;
   B is a base;
   M is O or S;
   wherein: the chemical modification of formula (I') or the tautomeric modification thereof is not

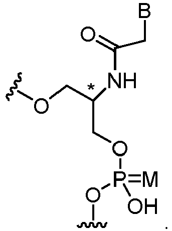

In some embodiments, when X is NH-CO, R₁ is not H.

In some embodiments, B is a base; for example, B is selected from the group consisting of those of purine bases, pyrimidine bases, indoles, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, each B is independently selected from the group consisting of bases at nucleotide positions 2 to 8 (e.g., positions 2, 3, 4, 5, 6, 7, and 8) containing the chemical modification of formula (I-1) of the 5' region of the antisense strand.

In some specific embodiments, each B is independently selected from the group consisting of natural bases at nucleotide positions 2 to 8 (e.g., positions 2, 3, 4, 5, 6, 7, and 8) containing the chemical modification of formula (I-1) of the 5' region of the antisense strand. In some embodiments, the antisense strand comprises a chemical modification of formula (I'-1) or a tautomeric modification thereof in at least one of nucleotide positions 2 to 8 (e.g., positions 2, 3, 4, 5, 6, 7, and 8) of the 5' region thereof: 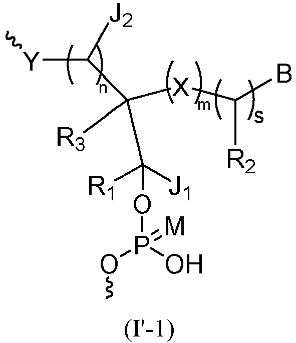 wherein: Y is selected from the group consisting of O, NH, and S;
each X is independently selected from the group consisting of CR₄(R₄'), S, NRs, and NH-CO, wherein R₄, R₄', and R₅ are each independently H or C₁-C₆ alkyl;
J₁ and J₂ are each independently H or C₁-C₆ alkyl;
n = 0, 1, or 2; m = 0, 1, or 2; s = 0 or 1;
R₃ is selected from the group consisting of H, OH, halogen, NH₂, C₁-C₆ alkyl, C₁-C₆ alkoxy, C₂-C₆ alkenyl, C₂-C₆ alkynyl, S-CH₃, NCH₃(CH₃), OCH₂CH₂OCH₃, -O-alkylamino, and (CH₂)ₚR₆, wherein R₆ is selected from the group consisting of OH, halogen, methoxy, ethoxy, N₃, C₂-C₆ alkenyl, and C₂-C₆ alkynyl, and p = 1, 2, or 3;
R₁ is selected from the group consisting of H, C₁-C₆ alkyl, C₁-C₆ alkoxy, C₂-C₆ alkenyl, C₂-C₆ alkynyl, and (CH₂)_{q}R₇, wherein R₇ is selected from the group consisting of OH, halogen, methoxy, ethoxy, N₃, C₂-C₆ alkenyl, and C₂-C₆ alkynyl, and q = 1, 2, or 3;
R₂ is selected from the group consisting of H, OH, halogen, NH₂, C₁-C₆ alkyl, C₁-C₆ alkoxy, C₂-C₆ alkenyl, C₂-C₆ alkynyl, S-CH₃, NCH₃(CH₃), OCH₂CH₂OCH₃, -O-alkylamino, and (CH₂)ᵣR₈, wherein R₈ is selected from the group consisting of OH, halogen, methoxy, ethoxy, N₃, C₂-C₆ alkenyl, and C₂-C₆ alkynyl, and r = 1, 2, or 3;
M is O or S;
optionally, R₁ and R₂ are directly connected to form a ring;
B is as defined in formula (I').

In some embodiments, B is a base; for example, B is selected from the group consisting of those of purine bases, pyrimidine bases, indoles, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, each B is independently selected from the group consisting of bases at nucleotide positions 2 to 8 (e.g., positions 2, 3, 4, 5, 6, 7, and 8) containing the chemical modification of formula (I-1) of the 5' region of the antisense strand.

In some specific embodiments, each B is independently selected from the group consisting of natural bases at nucleotide positions 2 to 8 (e.g., positions 2, 3, 4, 5, 6, 7, and 8) containing the chemical modification of formula (I-1) of the 5' region of the antisense strand. In some embodiments, the antisense strand comprises a chemical modification of formula (I'-2) or a tautomeric modification thereof in at least one of nucleotide positions 2 to 8 (e.g., positions 2, 3, 4, 5, 6, 7, and 8) of the 5' region thereof: 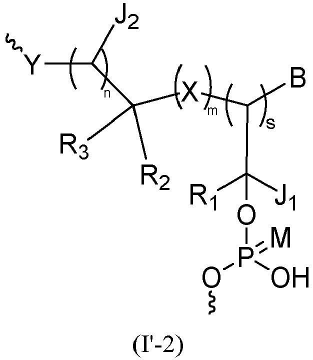 wherein Y is selected from the group consisting of O, NH, and S;
each X is independently selected from the group consisting of CR₄(R₄'), S, NRs, and NH-CO, wherein R₄, R₄', and R₅ are each independently H or C₁-C₆ alkyl;
n = 0, 1, or 2; m = 0, 1, or 2; s = 0 or 1;
J₁ and J₂ are each independently H or C₁-C₆ alkyl;
R₃ is selected from the group consisting of H, OH, halogen, NH₂, C₁-C₆ alkyl, C₁-C₆ alkoxy, C₂-C₆ alkenyl, C₂-C₆ alkynyl, S-CH₃, NCH₃(CH₃), OCH₂CH₂OCH₃, -O-alkylamino, and (CH₂)ₚR₆, wherein R₆ is selected from the group consisting of OH, halogen, methoxy, ethoxy, N₃, C₂-C₆ alkenyl, and C₂-C₆ alkynyl, and p = 1, 2, or 3;
R₁ is selected from the group consisting of H, C₁-C₆ alkyl, C₁-C₆ alkoxy, C₂-C₆ alkenyl, C₂-C₆ alkynyl, and (CH₂)_{q}R₇, wherein R₇ is selected from the group consisting of OH, halogen, methoxy, ethoxy, N₃, C₂-C₆ alkenyl, and C₂-C₆ alkynyl, and q = 1, 2, or 3;
R₂ is selected from the group consisting of H, C₁-C₆ alkyl, C₁-C₆ alkoxy, S-CH₃, NCH₃(CH₃), OCH₂CH₂OCH₃, -O-alkylamino, and (CH₂)ᵣR₈, wherein R₈ is selected from the group consisting of OH, halogen, methoxy, ethoxy, N₃, C₂-C₆ alkenyl, and C₂-C₆ alkynyl, and r = 1, 2, or 3;
optionally, R₁ and R₂ are directly connected to form a ring;
M is O or S;
B is as defined in formula (I').

In some embodiments, B is a base; for example, B is selected from the group consisting of those of purine bases, pyrimidine bases, indoles, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, each B is independently selected from the group consisting of bases at nucleotide positions 2 to 8 (e.g., positions 2, 3, 4, 5, 6, 7, and 8) containing the chemical modification of formula (I-1) of the 5' region of the antisense strand.

In some specific embodiments, each B is independently selected from the group consisting of natural bases at nucleotide positions 2 to 8 (e.g., positions 2, 3, 4, 5, 6, 7, and 8) containing the chemical modification of formula (I-1) of the 5' region of the antisense strand. In some embodiments, the chemical modification or the tautomeric modification thereof described above is not

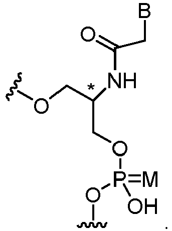

In some embodiments, when X is NH-CO, R₁ is not H.

In some embodiments, each X is independently selected from the group consisting of CR₄(R₄'), S, NRs, and NH-CO, wherein R₄, R₄', and R₅ are each independently H or Ci-C₃ alkyl;
n = 0, 1, or 2; m = 0, 1, or 2; s = 0 or 1;
J₁ and J₂ are each independently H or C₁-C₃ alkyl;
R₃ is selected from the group consisting of H, OH, halogen, NH₂, C₁-C₃ alkyl, C₁-C₃ alkoxy, C₂-C₄ alkenyl, C₂-C₄ alkynyl, S-CH₃, NCH₃(CH₃), OCH₂CH₂OCH₃, -O-alkylamino, and (CH₂)ₚR₆, wherein R₆ is selected from the group consisting of OH, halogen, methoxy, ethoxy, N₃, C₂-C₆ alkenyl, and C₂-C₆ alkynyl, and p = 1, 2, or 3;
R₁ is selected from the group consisting of H, C₁-C₃ alkyl, C₁-C₃ alkoxy, C₂-C₄ alkenyl, C₂-C₄ alkynyl, and (CH₂)_{q}R₇, wherein R₇ is selected from the group consisting of OH, halogen, methoxy, ethoxy, N₃, C₂-C₄ alkenyl, and C₂-C₄ alkynyl, and q = 1, 2, or 3;
R₂ is selected from the group consisting of H, OH, halogen, NH₂, C₁-C₃ alkyl, C₁-C₃ alkoxy, C₂-C₄ alkenyl, C₂-C₄ alkynyl, S-CH₃, NCH₃(CH₃), OCH₂CH₂OCH₃, -O-alkylamino, and (CH₂)ᵣR₈, wherein R₈ is selected from the group consisting of OH, halogen, methoxy, ethoxy, N₃, C₂-C₄ alkenyl, and C₂-C₄ alkynyl, and r = 1, 2, or 3;
optionally, R₁ and R₂ are directly connected to form a ring.

In some embodiments, each X is independently selected from the group consisting of CR₄(R₄'), S, NRs, and NH-CO, wherein R₄, R₄', and R₅ are each independently H, methyl, ethyl, n-propyl, or isopropyl;
n = 0, 1, or 2; m = 0, 1, or 2; s = 0 or 1;
J₁ and J₂ are each independently H or methyl;
R₃ is selected from the group consisting of H, OH, F, Cl, NH₂, methyl, ethyl, *n*-propyl, isopropyl, methoxy, ethoxy, *n*-propoxy, isopropoxy, vinyl, allyl, ethynyl, propargyl, S-CH₃, NCH₃(CH₃), OCH₂CH₂OCH₃, -O-methylamino, -O-ethylamino, and (CH₂)ₚR₆, wherein R₆ is selected from the group consisting of OH, F, Cl, methoxy, ethoxy, N₃, vinyl, allyl, ethynyl, and propargyl, and p = 1 or 2;
R₁ is selected from the group consisting of H, methyl, ethyl, *n*-propyl, isopropyl, methoxy, ethoxy, *n*-propoxy, isopropoxy, vinyl, allyl, ethynyl, propargyl, and (CH₂)_{q}R₇, wherein R₇ is selected from the group consisting of OH, F, Cl, methoxy, ethoxy, N₃, vinyl, allyl, ethynyl, and propargyl, and q = 1 or 2;
R₂ is selected from the group consisting of H, OH, F, Cl, NH₂, methyl, ethyl, n-propyl, isopropyl, methoxy, ethoxy, *n*-propoxy, isopropoxy, vinyl, allyl, ethynyl, propargyl, S-CH₃, NCH₃(CH₃), OCH₂CH₂OCH₃, -O-methylamino, -O-ethylamino, and (CH₂)ᵣR₈, wherein R₈ is selected from the group consisting of OH, F, Cl, methoxy, ethoxy, N₃, vinyl, allyl, ethynyl, and propargyl, and r = 1 or 2;
optionally, R₁ and R₂ are directly connected to form a ring.

In some embodiments, B is a base; for example, B is selected from the group consisting of those of purine bases, pyrimidine bases, indoles, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, each B is independently selected from the group consisting of bases at nucleotide positions 2 to 8 (e.g., positions 2, 3, 4, 5, 6, 7, and 8) containing the chemical modification of formula (I-1) of the 5' region of the antisense strand.

In some specific embodiments, each B is independently selected from the group consisting of natural bases at nucleotide positions 2 to 8 (e.g., positions 2, 3, 4, 5, 6, 7, and 8) containing the chemical modification of formula (I-1) of the 5' region of the antisense strand. In some embodiments involving formula (I'-1), Y is O or NH; each X is independently selected from the group consisting of NH-CO, CH₂, and NH;
n = 0 or1; m = 0 or1; s = 0 or 1;
J₁ and J₂ are each independently H;
R₁ is selected from the group consisting of H, methyl, and CH₂OH;
R₂ is selected from the group consisting of H, OH, NH₂, methyl, and CH₂OH;
R₃ is selected from the group consisting of H, OH, NH₂, methyl, and CH₂OH; optionally, R₁ and R₂ are directly connected to form a ring.

In some embodiments, B is a base; for example, B is selected from the group consisting of those of purine bases, pyrimidine bases, indoles, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, each B is independently selected from the group consisting of bases at nucleotide positions 2 to 8 (e.g., positions 2, 3, 4, 5, 6, 7, and 8) containing the chemical modification of formula (I-1) of the 5' region of the antisense strand.

In some specific embodiments, each B is independently selected from the group consisting of natural bases at nucleotide positions 2 to 8 (e.g., positions 2, 3, 4, 5, 6, 7, and 8) containing the chemical modification of formula (I-1) of the 5' region of the antisense strand. In some embodiments involving formula (I'-2), Y is O or NH; each X is independently selected from the group consisting of NH-CO, CH₂, and NH;
n = 0 or1; m = 0 or1; s = 0 or 1;
J₁ and J₂ are each independently H;
R₁ is selected from the group consisting of H, methyl, and CH₂OH;
R₂ is selected from the group consisting of H, methyl, and CH₂OH;
R₃ is selected from the group consisting of H, OH, NH₂, methyl, and CH₂OH; optionally, R₁ and R₂ are directly connected to form a ring.

In some embodiments, B is a base; for example, B is selected from the group consisting of those of purine bases, pyrimidine bases, indoles, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, each B is independently selected from the group consisting of bases at nucleotide positions 2 to 8 (e.g., positions 2, 3, 4, 5, 6, 7, and 8) containing the chemical modification of formula (I-1) of the 5' region of the antisense strand.

In some specific embodiments, each B is independently selected from the group consisting of natural bases at nucleotide positions 2 to 8 (e.g., positions 2, 3, 4, 5, 6, 7, and 8) containing the chemical modification of formula (I-1) of the 5' region of the antisense strand. In some embodiments, the chemical modification of formula (I') or the tautomeric modification thereof is selected from the group consisting of: 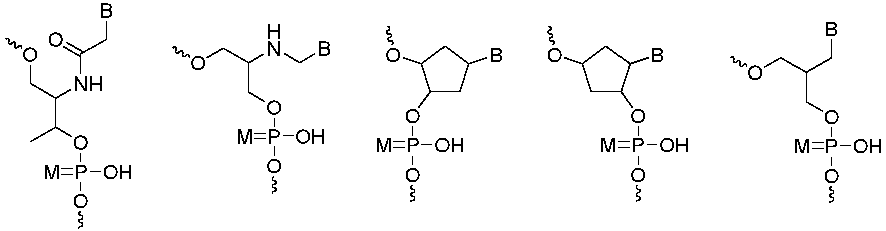 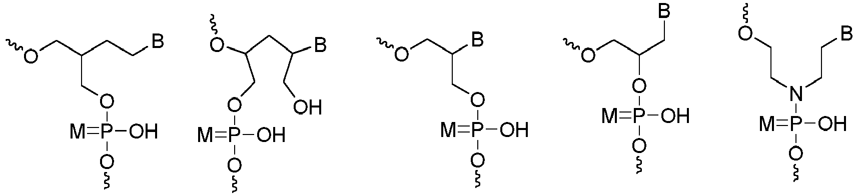 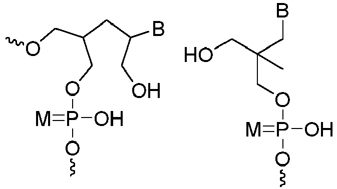 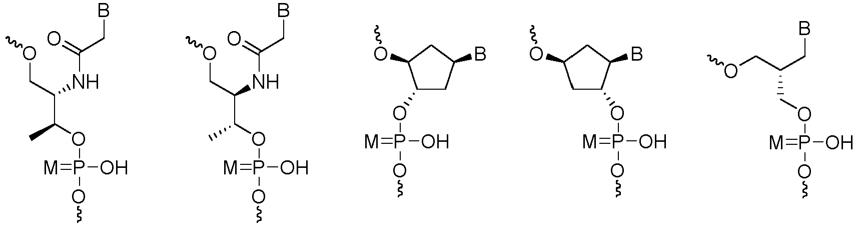 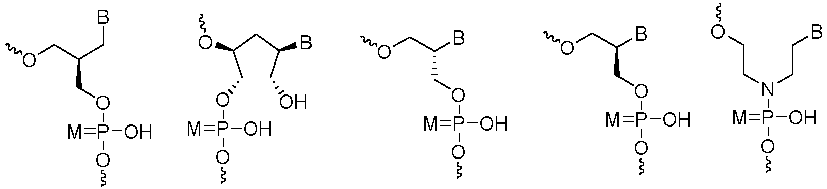 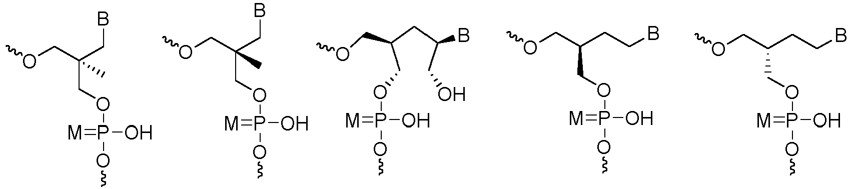 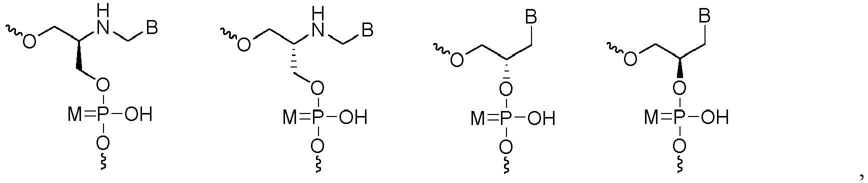 wherein M is O or S;
B is as defined in formula (I').

In some embodiments, B is a base; for example, B is selected from the group consisting of those of purine bases, pyrimidine bases, indoles, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, each B is independently selected from the group consisting of bases at nucleotide positions 2 to 8 (e.g., positions 2, 3, 4, 5, 6, 7, and 8) containing the chemical modification of formula (I-1) of the 5' region of the antisense strand.

In some specific embodiments, each B is independently selected from the group consisting of natural bases at nucleotide positions 2 to 8 (e.g., positions 2, 3, 4, 5, 6, 7, and 8) containing the chemical modification of formula (I-1) of the 5' region of the antisense strand. In some embodiments, the chemical modification of formula (I') or the tautomeric modification thereof is selected from the group consisting of: 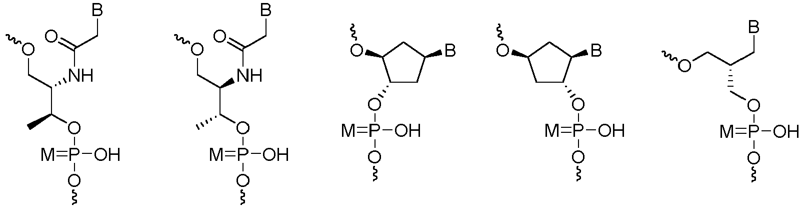 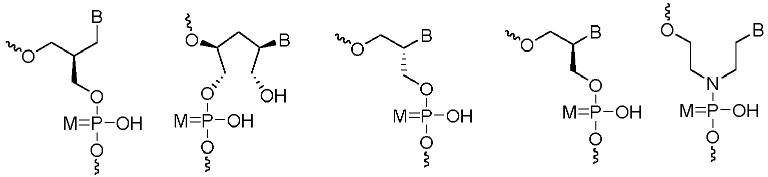 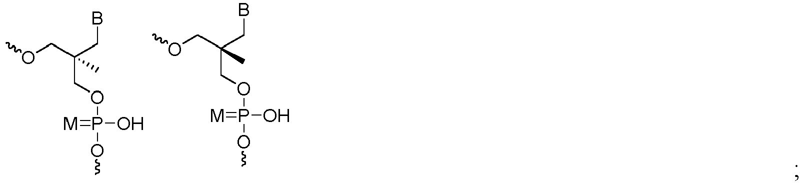 wherein: M is O or S;
B is as defined in formula (I').

In some embodiments, B is a base; for example, B is selected from the group consisting of those of purine bases, pyrimidine bases, indoles, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, each B is independently selected from the group consisting of bases at nucleotide positions 2 to 8 (e.g., positions 2, 3, 4, 5, 6, 7, and 8) containing the chemical modification of formula (I-1) of the 5' region of the antisense strand.

In some specific embodiments, each B is independently selected from the group consisting of natural bases at nucleotide positions 2 to 8 (e.g., positions 2, 3, 4, 5, 6, 7, and 8) containing the chemical modification of formula (I-1) of the 5' region of the antisense strand.

In some embodiments, the chemical modification of formula (I') or the tautomeric modification thereof is selected from the group consisting of: 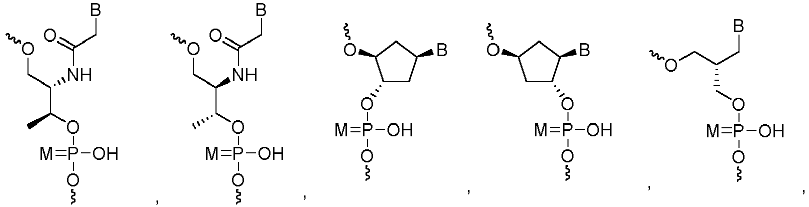 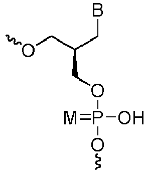 wherein: M is O or S;
B is as defined in formula (I').

In some embodiments, B is a base; for example, B is selected from the group consisting of those of purine bases, pyrimidine bases, indoles, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, each B is independently selected from the group consisting of bases at nucleotide positions 2 to 8 (e.g., positions 2, 3, 4, 5, 6, 7, and 8) containing the chemical modification of formula (I-1) of the 5' region of the antisense strand.

In some specific embodiments, each B is independently selected from the group consisting of natural bases at nucleotide positions 2 to 8 (e.g., positions 2, 3, 4, 5, 6, 7, and 8) containing the chemical modification of formula (I-1) of the 5' region of the antisense strand. In some embodiments, the chemical modification of formula (I') or the tautomeric modification thereof includes, but is not limited to: 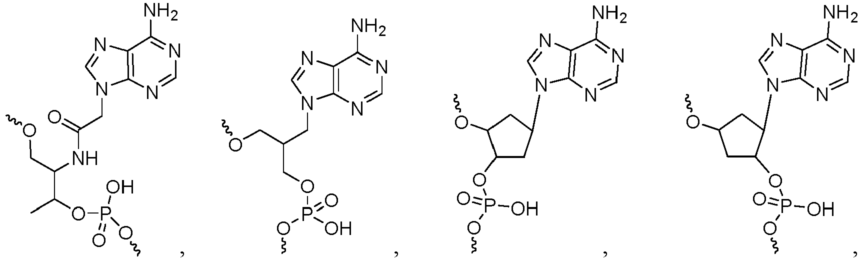 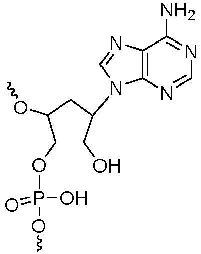 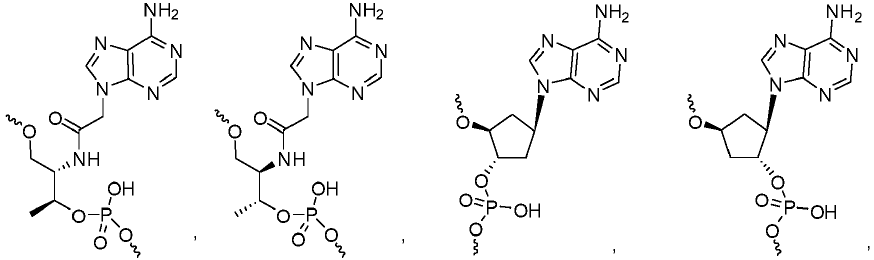 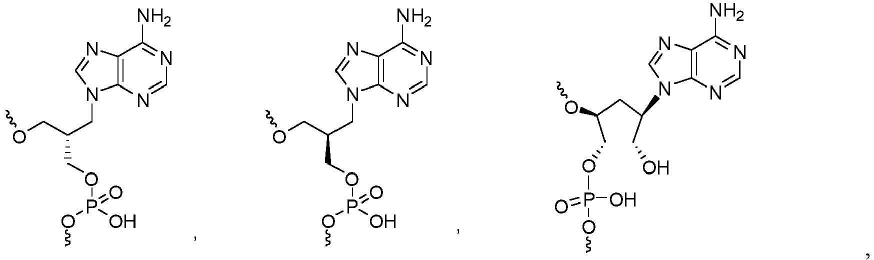 and those where adenine in the structure is replaced with guanine, cytosine, uracil, or thymine.

In some embodiments, the antisense strand comprises the chemical modification of formula (I) or formula (I') or the tautomeric modification thereof described above in at least one of nucleotide positions 2 to 8, 3 to 8, 4 to 8, 5 to 8, or 5 to 7 of the 5' region thereof.

In some embodiments, the antisense strand of the siRNA of the present disclosure comprises the chemical modification of formula (I) or formula (I') or the tautomeric modification thereof described above at position 5, 6, or 7 of the 5' region thereof.

In some specific embodiments, the chemical modification of formula (I) or formula (I') or the tautomeric modification thereof is at position 5 of the 5' region thereof; B is selected from the group consisting of adenine, guanine, 2,6-diaminopurine, 6-dimethylaminopurine, 2-aminopurine, cytosine, uracil, thymine, indole, 5-nitroindole, and 3-nitropyrrole; in some embodiments, B is selected from the group consisting of adenine, guanine, cytosine, and uracil.

In some specific embodiments, the chemical modification of formula (I) or formula (I') or the tautomeric modification thereof is at position 6 of the 5' region thereof; B is selected from the group consisting of adenine, guanine, 2,6-diaminopurine, 6-dimethylaminopurine, 2-aminopurine, cytosine, uracil, thymine, indole, 5-nitroindole, and 3-nitropyrrole; in some embodiments, B is selected from the group consisting of adenine, guanine, cytosine, and uracil.

In some specific embodiments, the chemical modification of formula (I) or formula (I') or the tautomeric modification thereof is at position 7 of the 5' region thereof; B is selected from the group consisting of adenine, guanine, 2,6-diaminopurine, 6-dimethylaminopurine, 2-aminopurine, cytosine, uracil, thymine, indole, 5-nitroindole, and 3-nitropyrrole; in some embodiments, B is selected from the group consisting of adenine, guanine, cytosine, and uracil.

In some embodiments, the sense strand and the antisense strand each independently have 16 to 35, 16 to 34, 17 to 34, 17 to 33, 18 to 33, 18 to 32, 18 to 31, 18 to 30, 18 to 29, 18 to 28, 18 to 27, 18 to 26, 18 to 25, 18 to 24, 18 to 23, 19 to 25, 19 to 24, or 19 to 23 nucleotides.

In some embodiments, the sense strand and the antisense strand are identical or different in length; the sense strand is 19-23 nucleotides in length, and the antisense strand is 19-26 nucleotides in length. As such, a ratio of the length of the sense strand to the length of the antisense strand of the siRNA provided by the present disclosure can be 19/20, 19/21, 19/22, 19/23, 19/24, 19/25, 19/26, 20/20, 20/21, 20/22, 20/23, 20/24, 20/25, 20/26, 21/20, 21/21, 21/22, 21/23, 21/24, 21/25, 21/26, 22/20, 22/21, 22/22, 22/23, 22/24, 22/25, 22/26, 23/20, 23/21, 23/22, 23/23, 23/24, 23/25, or 23/26. In some embodiments, a ratio of the length of the sense strand to the length of the antisense strand of the siRNA is 19/21, 21/23, or 23/25. In some embodiments, a ratio of the length of the sense strand to the length of the antisense strand of the siRNA is 19/21.

In some embodiments, the antisense strand is at least partially reverse complementary to the target sequence to mediate RNA interference. In some embodiments, there are no more than 5, no more than 4, no more than 3, no more than 2, or no more than 1 mismatch between the antisense strand and the target sequence. In some embodiments, the antisense strand is fully reverse complementary to the target sequence.

In some embodiments, the sense strand is at least partially reverse complementary to the antisense strand to form a double-stranded region. In some embodiments, there are no more than 5, no more than 4, no more than 3, no more than 2, or no more than 1 mismatch between the sense strand and the antisense strand. In some embodiments, the sense strand is fully reverse complementary to the antisense strand.

In some embodiments, the siRNA of the present disclosure comprises one or two blunt ends.

In some specific embodiments, the siRNA comprises an overhang having 1 to 4, e.g., 1, 2, 3, or 4, unpaired nucleotides.

In some embodiments, the siRNA of the present disclosure comprises an overhang at the 3' end of the antisense strand of the siRNA.

In some embodiments, the nucleotide sequence of the antisense strand of the siRNA of the present disclosure comprises or is a nucleotide sequence of any one of SEQ ID NO: 47 to SEQ ID NO: 68, wherein, W' represents a nucleotide comprising the chemical modification of formula (I) or formula (I') or the tautomeric modification thereof as defined herein.

In some specific embodiments, W' is selected from the group consisting of: 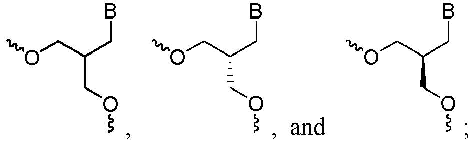 wherein: B is selected from the group consisting of guanine, adenine, cytosine, and uracil.

In some specific embodiments, W' is selected from the group consisting of: 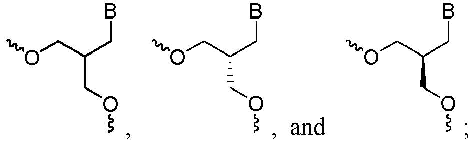 wherein: B is selected from the group consisting of bases at position 7 of the 5' regions of SEQ ID NO: 47 to SEQ ID NO: 68 and SEQ ID NO: 25 to SEQ ID NO: 46 in Table 12, wherein, for example, SEQ ID NO: 47 corresponds to SEQ ID NO: 25, SEQ ID NO: 68 corresponds to SEQ ID NO: 46, and SEQ ID NO: 52 corresponds to SEQ ID NO: 30.

In some specific embodiments, the nucleotide sequence of the antisense strand of the siRNA of the present disclosure comprises or is a nucleotide sequence of any one of SEQ ID NO: 47 to SEQ ID NO: 68, wherein, W' represents a nucleotide comprising the chemical modification of formula (I) or formula (I') or the tautomeric modification thereof as defined herein.

In some specific embodiments, W' is selected from the group consisting of: 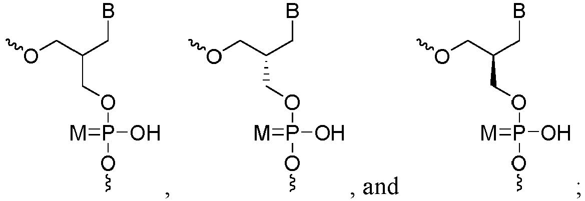 wherein: M is O or S; wherein: B is selected from the group consisting of guanine, adenine, cytosine, and uracil.

In some specific embodiments, M is S. In some specific embodiments, M is O.

In some specific embodiments, W' is selected from the group consisting of: 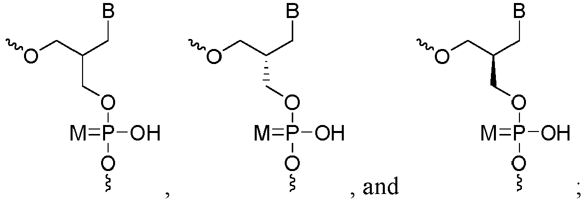 wherein: M is O or S; wherein: B is selected from the group consisting of bases at position 7 of the 5' regions of SEQ ID NO: 47 to SEQ ID NO: 68 and SEQ ID NO: 25 to SEQ ID NO: 46 in Table 12, wherein, for example, SEQ ID NO: 47 corresponds to SEQ ID NO: 25, SEQ ID NO: 68 corresponds to SEQ ID NO: 46, and SEQ ID NO: 52 corresponds to SEQ ID NO: 30.

In some specific embodiments, M is S. In some specific embodiments, M is O.

The present disclosure also provides an siRNA, which is the siRNA described above with modifications, wherein at least one additional nucleotide in the sense strand and/or the antisense strand is a modified nucleotide, in addition to the nucleotide with the chemical modification of formula (I) or formula (I') or the tautomeric modification thereof described above.

In some embodiments, all of the additional nucleotides are modified nucleotides, in addition to the nucleotide with the chemical modification of formula (I) or formula (I') or the tautomeric modification thereof described above.

In some embodiments, the additional modified nucleotides are each independently selected from the group consisting of a deoxy-nucleotide, a 3'-end deoxy-thymine (dT) nucleotide, a 2'-O-methyl-modified nucleotide, a 2'-fluoro-modified nucleotide, a 2'-deoxy-modified nucleotide, a locked nucleotide, an unlocked nucleotide, a conformationally constrained nucleotide, a constrained ethyl nucleotide, an abasic nucleotide, a 2'-amino-modified nucleotide, a 2'-O-allyl-modified nucleotide, a 2'-C-alkyl-modified nucleotide, a 2'-hydroxy-modified nucleotide, a 2'-methoxyethyl-modified nucleotide, a 2'-O-alkyl-modified nucleotide, a morpholino nucleotide, a phosphoramidate, a non-natural base-comprising nucleotide, a tetrahydropyran-modified nucleotide, a 1,5-anhydrohexitol-modified nucleotide, a cyclohexenyl-modified nucleotide, a phosphorothioate group-comprising nucleotide, a methylphosphonate group-comprising nucleotide, a 5'-phosphoester-comprising nucleotide, and a 5'-phosphoester mimic-comprising nucleotide.

In some embodiments, the modified nucleotides are each independently selected from the group consisting of a 2'-alkoxy-modified nucleotide, a 2'-substituted alkoxy-modified nucleotide, a 2'-alkyl-modified nucleotide, a 2'-substituted alkyl-modified nucleotide, a 2'-amino-modified nucleotide, a 2'-substituted amino-modified nucleotide, a 2'-fluoro-modified nucleotide, a 2'-deoxynucleotide, a 2'-deoxy-2'-fluoro-modified nucleotide, a 3'-deoxy-thymine (dT) nucleotide, an isonucleotide, LNA, ENA, cET, UNA, and GNA.

In some embodiments, the modified nucleotides are each independently selected from the group consisting of a 2'-methoxy-modified nucleotide, a 2'-fluoro-modified nucleotide, and a 2'-deoxy-modified nucleotide.

In the context of the present disclosure, the 2'-fluoro-modified nucleotide refers to a nucleotide in which the hydroxy group at 2'-position of the ribosyl group of the nucleotide is substituted with fluorine. In some embodiments, the 2'-alkoxy-modified nucleotide is a 2'-methoxy-modified nucleotide (2'-OMe). In some embodiments, the 2'-substituted alkoxy-modified nucleotide can be, for example, a 2'-O-methoxyethyl-modified nucleotide (2'-MOE).

In some embodiments, in a 5'-end to 3'-end direction, nucleotides at positions 2, 6, and 14 of the antisense strand are each independently a 2'-deoxynucleotide or a 2'-fluoro-modified nucleotide.

In some embodiments, in a 5'-end to 3'-end direction, nucleotides at positions 2, 6, 14, and 16 of the antisense strand are each independently a 2'-deoxynucleotide or a 2'-fluoro-modified nucleotide.

In some embodiments, in a 5'-end to 3'-end direction, nucleotides at positions 2, 6, 9, 12, and 14 of the antisense strand are each independently a 2'-deoxynucleotide or a 2'-fluoro-modified nucleotide.

In some embodiments, in a 5'-end to 3'-end direction, nucleotides at positions 2, 6, 10, 12, and 14 of the antisense strand are each independently a 2'-deoxynucleotide or a 2'-fluoro-modified nucleotide.

In some embodiments, in a 5'-end to 3'-end direction, nucleotides at positions 2, 4, 6, 9, 12, 14, and 18 of the antisense strand are each independently a 2'-deoxynucleotide or a 2'-fluoro-modified nucleotide.

In some embodiments, in a 5'-end to 3'-end direction, nucleotides at positions 2, 4, 6, 10, 12, 14, and 18 of the antisense strand are each independently a 2'-deoxynucleotide or a 2'-fluoro-modified nucleotide.

In some embodiments, in a 5'-end to 3'-end direction, nucleotides at positions 2, 4, 6, 9, 12, 14, 16, and 18 of the antisense strand are each independently a 2'-deoxynucleotide or a 2'-fluoro-modified nucleotide.

In some embodiments, in a 5'-end to 3'-end direction, nucleotides at positions 2, 4, 6, 10, 12, 14, 16, and 18 of the antisense strand are each independently a 2'-deoxynucleotide or a 2'-fluoro-modified nucleotide.

In some embodiments, in a 5'-end to 3'-end direction, nucleotides at positions 2, 4, 6, 9, 10 12, 14, 16, and 18 of the antisense strand are each independently a 2'-deoxynucleotide or a 2'-fluoro-modified nucleotide.

In some embodiments, in a 5'-end to 3'-end direction, nucleotides at positions 2, 6, and 14 of the antisense strand are each independently a 2'-fluoro-modified nucleotide.

In some embodiments, in a 5'-end to 3'-end direction, nucleotides at positions 2, 6, 14, and 16 of the antisense strand are each independently a 2'-fluoro-modified nucleotide. In some embodiments, in a 5'-end to 3'-end direction, nucleotides at positions 2, 6, 12, and 14 of the antisense strand are each independently a 2'-fluoro-modified nucleotide. In some embodiments, in a 5'-end to 3'-end direction, nucleotides at positions 2, 4, 6, 12, 14, 16, and 18 of the antisense strand are each independently a 2'-fluoro-modified nucleotide.

In some embodiments, in a 5'-end to 3'-end direction, nucleotides at positions 2, 4, 6, 9, 12, 14, 16, and 18 of the antisense strand are each independently a 2'-fluoro-modified nucleotide.

In some embodiments, in a 5'-end to 3'-end direction, nucleotides at positions 2, 4, 6, 10, 12, 14, 16, and 18 of the antisense strand are each independently a 2'-fluoro-modified nucleotide.

In some embodiments, the sense strand of the siRNA described herein has a nucleotide sequence of the formula shown below:

5'-NₐNₐNₐNₐXNₐN_{b}N_{b}N_{b}NₐNₐNₐNₐNₐNₐNₐNₐNₐNₐ-3'

wherein, Nₐ and N_{b} each independently represent a modified nucleotide or an unmodified nucleotide, wherein modifications on Nₐ and N_{b} are different; each X is independently Nₐ or N_{b}.

In some embodiments, the antisense strand of the siRNA described herein has a nucleotide sequence of the formula shown below:

5'-Nₐ'N_{b}'Nₐ'X'Nₐ'N_{b}'W'Nₐ'X'Y'Nₐ'X'Nₐ'N_{b}'Nₐ'X'Nₐ'X'Nₐ'Nₐ'Nₐ'-3';

wherein, Nₐ' and N_{b}' each independently represent a modified nucleotide or an unmodified nucleotide, wherein modifications on Nₐ' and N_{b}' are different; each X' is independently Nₐ' or N_{b}'; Y' is Nₐ' or N_{b}'; W' represents a nucleotide comprising any one of the chemical modifications of formula (I) or formula (I') or the tautomeric modifications thereof of the present disclosure.

In some embodiments, modifications on X' and Y' are different.

In some embodiments, Nₐ is a 2'-methoxy-modified nucleotide, and N_{b} is a 2'-fluoro-modified nucleotide or a 2'-deoxy-modified nucleotide.

In some embodiments, Nₐ' is a 2'-methoxy-modified nucleotide, and N_{b}' is a 2'-fluoro-modified nucleotide or a 2'-deoxy-modified nucleotide.

In some specific embodiments, Nₐ is a 2'-methoxy-modified nucleotide, and N_{b}' is a 2'-fluoro-modified nucleotide.

In some specific embodiments, Nₐ' is a 2'-methoxy-modified nucleotide, and N_{b}' is a 2'-fluoro-modified nucleotide.

In some embodiments, the antisense strand of the siRNA described herein has a nucleotide sequence of the formula shown below:

5'-Na'Nb'Na'Nb'Na'Nb'W'Na'X'Y'Na'Nb'Na'Nb'Na'Nb'Na'Nb'Na'Na'Na'-3';

wherein, each X' is independently Nₐ' or N_{b}', Y' is Nₐ' or N_{b}', and modifications on X' and Y' are different; Nₐ' is a 2'-methoxy-modified nucleotide, and N_{b}' is a 2'-fluoro-modified nucleotide; W' represents a nucleotide comprising any one of the chemical modifications of formula (I) or formula (I') or the tautomeric modifications thereof of the present disclosure.

In some embodiments, the sense strand of the siRNA described herein has a nucleotide sequence of the formula shown below:

5'-NₐNₐNₐNₐNₐNₐN_{b}N_{b}N_{b}NₐNₐNₐNₐNₐNₐNₐNₐNₐNₐ-3'; or,

5'-NₐNₐNₐNₐN_{b}NₐN_{b}N_{b}N_{b}NₐNₐNₐNₐNₐNₐNₐNₐNₐNₐ-3';

wherein, Nₐ is a 2'-methoxy-modified nucleotide, and N_{b} is a 2'-fluoro-modified nucleotide.

In some embodiments, the antisense strand of the siRNA described herein has a nucleotide sequence of the formula shown below:

5'-Nₐ'N_{b}'Nₐ'N_{b}'Nₐ'N_{b}'W'Nₐ'Nₐ'N_{b}'Nₐ'N_{b}'Nₐ'N_{b}'Nₐ'N_{b}'Nₐ'N_{b}'Nₐ'Nₐ'Nₐ'-3'; or,

5'-Nₐ'N_{b}'Nₐ'N_{b}'Nₐ'N_{b}'W'Nₐ'N_{b}'Nₐ'Nₐ'N_{b}'Nₐ'N_{b}'Nₐ'N_{b}'Nₐ'N_{b}'Nₐ'Nₐ'Nₐ'-3';

wherein, Nₐ is a 2'-methoxy-modified nucleotide, and N_{b} is a 2'-fluoro-modified nucleotide; and/or Nₐ' is a 2'-methoxy-modified nucleotide, and N_{b}' is a 2'-fluoro-modified nucleotide.

W' represents a nucleotide comprising any one of the chemical modifications of formula (I) or formula (I') or the tautomeric modifications thereof of the present disclosure.

In some specific embodiments, W' represents a nucleotide comprising a chemical modification or a tautomeric modification thereof; the chemical modification or the tautomeric modification thereof is selected from the group consisting of: 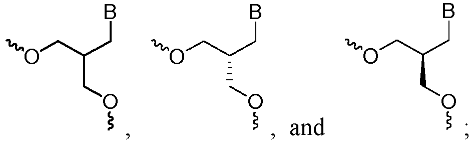 wherein: B is selected from the group consisting of guanine, adenine, cytosine, and uracil; in some specific embodiments, B is selected from the base at position 7 of the 5' region of the antisense strand.

In some specific embodiments, W' represents a nucleotide comprising a chemical modification or a tautomeric modification thereof; the chemical modification or the tautomeric modification thereof is selected from the group consisting of: 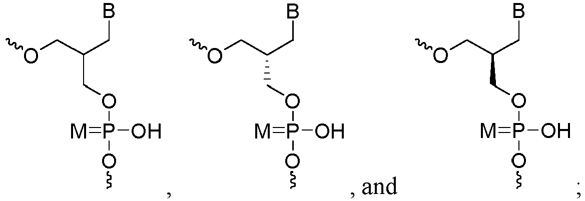 wherein: M is O or S; wherein: B is selected from the group consisting of guanine, adenine, cytosine, and uracil; in some specific embodiments, B is selected from the base at position 7 of the 5' region of the antisense strand.

In some specific embodiments, M is S. In some specific embodiments, M is O.

In some embodiments, at least one phosphoester group in the sense strand and/or the antisense strand is a phosphoester group with a modification that provides the siRNA with increased stability in a biological sample or environment. In some embodiments, the phosphoester group with a modification is a phosphorothioate group. Specifically, the phosphorothioate group refers to a phosphodiester group modified by replacing one non-bridging oxygen atom with a sulfur atom.

In some embodiments, the phosphorothioate group is present in at least one of the positions selected from the group consisting of:
a position between the 1st and 2nd nucleotides of the 5' end of the sense strand;
a position between the 2nd and 3rd nucleotides of the 5' end of the sense strand;
an end of the 1st nucleotide of the 3' end of the sense strand;
a position between the 1st and 2nd nucleotides of the 3' end of the sense strand;
a position between the 2nd and 3rd nucleotides of the 3' end of the sense strand;
a position between the 1st and 2nd nucleotides of the 5' end of the antisense strand;
a position between the 2nd and 3rd nucleotides of the 5' end of the antisense strand;
an end of the 1st nucleotide of the 3' end of the antisense strand;
a position between the 1st and 2nd nucleotides of the 3' end of the antisense strand; and
a position between the 2nd and 3rd nucleotides of the 3' end of the antisense strand.

In some embodiments, the sense strand and/or the antisense strand comprise a plurality of phosphorothioate groups that are present in:
a position between the 1st and 2nd nucleotides of the 5' end of the sense strand;
a position between the 2nd and 3rd nucleotides of the 5' end of the sense strand;
a position between the 1st and 2nd nucleotides of the 5' end of the antisense strand;
a position between the 2nd and 3rd nucleotides of the 5' end of the antisense strand;
a position between the 1st and 2nd nucleotides of the 3' end of the antisense strand;
a position between the 2nd and 3rd nucleotides of the 3' end of the antisense strand;
and optionally, an end of the 1st nucleotide of the 3' end of the sense strand; and/or
optionally, a position between the 1st and 2nd nucleotides of the 3' end of the sense strand.

In some embodiments, the sense strand is selected from a nucleotide sequence of the formula shown below:

5'-NmsNmsNmNmNfNmNfNfNfNmNmNmNmNmNmNmNmNmNm-3', or

5'-NmsNmsNmNmNmNmNfNfNfNmNmNmNmNmNmNmNmNmNm-3', or

5'-NmsNmsNmNmNfNmNfNfNfNmNmNmNmNmNmNmNmNmNms-3', or

5'-NmsNmsNmNmNmNmNfNfNfNmNmNmNmNmNmNmNmNmNms-3', or

5'-NmsNmsNmNmNfNmNfNfNfNmNmNmNmNmNmNmNmNmsNm-3', or

5'-NmsNmsNmNmNmNmNfNfNfNmNmNmNmNmNmNmNmNmsNm-3', or

5'-NmsNmsNmNmNfNmNfNfNfNmNmNmNmNmNmNmNmNmsNms-3', or

5'-NmsNmsNmNmNmNmNfNfNfNmNmNmNmNmNmNmNmNmsNms-3',

wherein, Nm represents any 2'-methoxy-modified nucleotide, such as 2'-methoxy-modified C, G, U, A, or T; Nf represents any 2'-fluoro-modified nucleotide, such as 2'-fluoro-modified C, G, U, A, or T;
the lowercase letter s indicates that the two nucleotides adjacent to either side of the letter s are linked by a phosphorothioate group; the lowercase letter s, when being the first at the 3' end, indicates that the upstream nucleotide adjacent to the letter s ends in a phosphorothioate group.

In some embodiments, the antisense strand has a nucleotide sequence of the formula shown below:

5'-Nm'sNf sNm'NfNm'NfW'Nm'Nm'NfNm'NfNm'NfNm'NfNm'NfNm'sNm'sNm'-3', or

5'-Nm'sNf sNm'NfNm'NfW'Nm'NfNm'Nm'NfNm'NfNm'NfNm'NfNm'sNm'sNm'-3', wherein, Nm' represents any 2'-methoxy-modified nucleotide, such as 2'-methoxy-modified C, G, U, A, or T; Nf represents any 2'-fluoro-modified nucleotide, such as 2'-fluoro-modified C, G, U, A, or T;
the lowercase letter s indicates that the two nucleotides adjacent to either side of the letter s are linked by a phosphorothioate group, and the lowercase letter s, when being the first at the 3' end, indicates that the upstream nucleotide adjacent to the letter s ends in a phosphorothioate group;
W' represents a nucleotide with a chemical modification or a tautomeric modification thereof; the chemical modification or the tautomeric modification thereof is selected from the group consisting of: 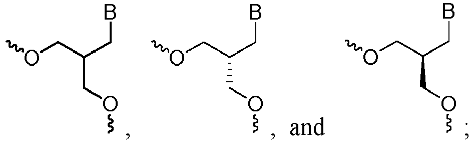 wherein: B is selected from the group consisting of guanine, adenine, cytosine, and uracil; in some embodiments, B is selected from the base at position 7 of the 5' region of the antisense strand.

In some specific embodiments, W' represents a nucleotide comprising a chemical modification or a tautomeric modification thereof; the chemical modification or the tautomeric modification thereof is selected from the group consisting of: 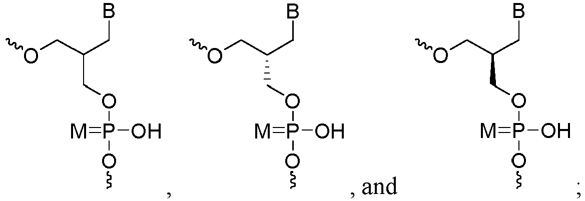 wherein: M is O or S; wherein: B is selected from the group consisting of guanine, adenine, cytosine, and uracil; in some specific embodiments, B is selected from the base at position 7 of the 5' region of the antisense strand.

In some specific embodiments, M is S. In some specific embodiments, M is O.

In some embodiments, the sense strand of the siRNA of the present disclosure comprises or is any one of SEQ ID NO: 69 to SEQ ID NO: 110.

In some embodiments, the antisense strand of the siRNA of the present disclosure comprises or is any one of SEQ ID NO: 111 to SEQ ID NO: 172.

The present disclosure also provides an siRNA conjugate comprising any one of the siRNAs described above and a targeting ligand linked to the siRNA.

In some embodiments, the siRNA and the targeting ligand are linked covalently or non-covalently.

In some embodiments, the targeting ligand is linked to the 3' end of the sense strand of the siRNA.

In some embodiments, to promote entry of the siRNA into a cell, a lipophilic group such as cholesterol can be introduced into an end of the sense strand of the siRNA, and the lipophilic group is covalently bonded to a small interfering nucleic acid; for example, cholesterol, lipoprotein, vitamin E, etc., are introduced into the end to facilitate going through the cell membrane consisting of a lipid bilayer and interacting with the mRNA in the cell. Meanwhile, the siRNA can also be modified by non-covalent bonding, for example, bonding to a phospholipid molecule, a polypeptide, a cationic polymer, etc., by a hydrophobic bond or an ionic bond to increase stability and biological activity.

In some embodiments, the targeting ligand is linked to an end of the siRNA by a phosphoester group, a phosphorothioate group, or a phosphonic acid group.

In some embodiments, the targeting ligand is indirectly linked to an end of the siRNA by a phosphoester group, a phosphorothioate group, or a phosphonic acid group.

In some embodiments, the targeting ligand is directly linked to an end of the siRNA by a phosphoester group, a phosphorothioate group, or a phosphonic acid group.

In some embodiments, the targeting ligand is directly linked to an end of the siRNA by a phosphoester group or a phosphorothioate group.

In some embodiments, the targeting ligand is directly linked to the 3' end of the sense strand of the siRNA by a phosphoester group or a phosphorothioate group.

In some embodiments, the targeting ligand has a structure of formula (II) shown below, 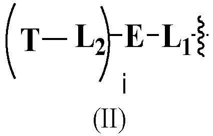 wherein T is a targeting moiety, E is a branching group, L₁ is a linker moiety, and L₂ is a tether moiety between the targeting moiety and the branching group, wherein i is selected from an integer from 1 to 10, e.g., 1, 2, 3, 4, 5, 6, 7, 8, 9, or 10.

In some embodiments, i is selected from an integer from 2 to 8.

In some embodiments, i is selected from an integer from 3 to 5.

In some embodiments, L₁ is 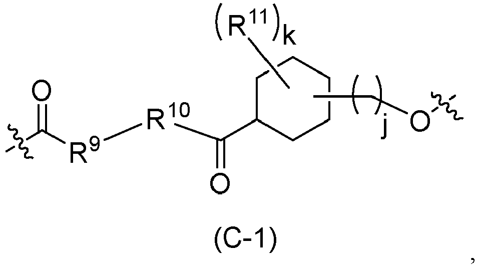
wherein R⁹ and R¹⁰ are each independently selected from the group consisting of -S-, - NH-, -O-, -C(O)-, -OC(O)-, -C(O)O-, -NHC(O)-, -C(O)NH-, -CH₂-, -CH₂NH-, -CH₂O-, -NH-C(O)-CH₂-, -C(O)-CH₂-NH-, -NH(CO)NH-, and 3- to 12-membered heterocyclyl, wherein the -CH₂- is optionally substituted with a substituent selected from the group consisting of halogen, alkyl, alkoxy, and alkylamino, and the alkyl is optionally further substituted with a substituent selected from the group consisting of hydroxy, amino, and halogen;
R¹¹ is selected from the group consisting of deuterium, halogen, alkyl, amino, cyano, nitro, alkenyl, alkynyl, carboxyl, hydroxy, sulfhydryl, alkylsulfhydryl, alkoxy, alkylamino, -C(O)-alkyl, -C(O)-O-alkyl, -CONH₂, -CONH-alkyl, -OC(O)-alkyl, -NH-C(O)-alkyl, -S(O)O-alkyl, -S(O)ONH₂, and -S(O)ONH-alkyl, wherein the alkyl, alkenyl, alkynyl, alkylsulfhydryl, alkoxy, -C(O)-alkyl, -C(O)-O-alkyl, -CONH-alkyl, -OC(O)-alkyl, -NH-C(O)-alkyl, -S(O)O-alkyl, and -S(O)ONH-alkyl are optionally further substituted with substituents selected from the group consisting of halogen, hydroxy, amino, and sulfhydryl;
k is selected from the group consisting of 0, 1, 2, 3, and 4;
j is selected from an integer from 1 to 20 (e.g., 0, 1, 2, 3, 4, 5, 6, 7, 8, 9, 10, 11, 12, 13, 14, 15, 16, 17, 18, 19, or 20).

In some embodiments, L₁ is 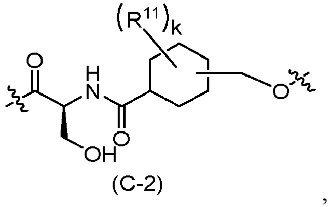 wherein R¹¹ is selected from the group consisting of deuterium, halogen, alkyl, amino, cyano, nitro, alkenyl, alkynyl, carboxyl, hydroxy, sulfhydryl, alkyl sulfhydryl, alkoxy, alkylamino, -C(O)-alkyl, -C(O)-O-alkyl, -CONH₂, - CONH-alkyl, -OC(O)-alkyl, -NH-C(O)-alkyl, -S(O)O-alkyl, -S(O)ONH₂, and - S(O)ONH-alkyl, wherein the alkyl, alkenyl, alkynyl, carboxyl, alkylsulfhydryl, alkoxy, - C(O)-alkyl, -C(O)-O-alkyl, -CONH-alkyl, -OC(O)-alkyl, -NH-C(O)-alkyl, -S(O)O-alkyl, and -S(O)ONH-alkyl are optionally further substituted with substituents selected from the group consisting of halogen, hydroxy, amino, and sulfhydryl;
k is selected from the group consisting of 0, 1, 2, 3, and 4.

In some embodiments, L₁ is 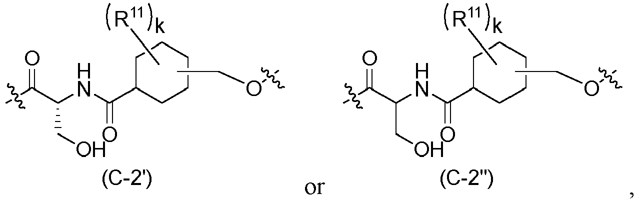 wherein R¹¹ is selected from the group consisting of deuterium, halogen, alkyl, amino, cyano, nitro, alkenyl, alkynyl, carboxyl, hydroxy, sulfhydryl, alkylsulfhydryl, alkoxy, alkylamino, -C(O)-alkyl, -C(O)-O-alkyl, -CONH₂, -CONH-alkyl, -OC(O)-alkyl, -NH-C(O)-alkyl, -S(O)O-alkyl, - S(O)ONH₂, and -S(O)ONH-alkyl, wherein the alkyl, alkenyl, alkynyl, carboxyl, alkylsulfhydryl, alkoxy, -C(O)-alkyl, -C(O)-O-alkyl, -CONH-alkyl, -OC(O)-alkyl, -NH-C(O)-alkyl, -S(O)O-alkyl, and -S(O)ONH-alkyl are optionally further substituted with substituents selected from the group consisting of halogen, hydroxy, amino, and sulfhydryl; k is selected from the group consisting of 0, 1, 2, 3, and 4.

In some embodiments, L₁ is

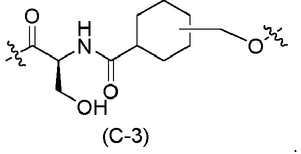

In some embodiments, L₁ is

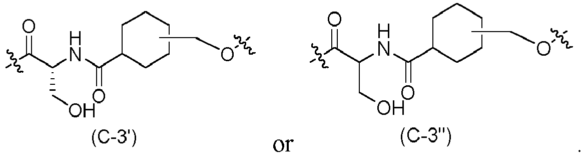

In some embodiments, L₁ is

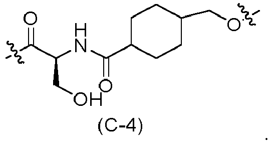

In some embodiments, L₁ is

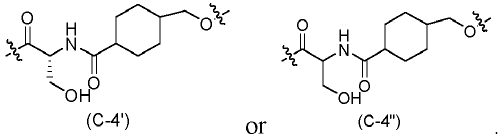

In some embodiments, L₁ is

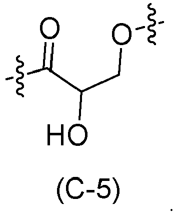

In some embodiments, E in the targeting ligand is 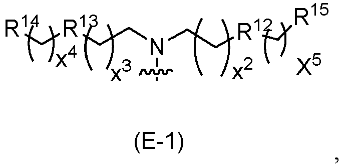 wherein R¹², R¹³, R¹⁴, and R¹⁵ are each independently selected from the group consisting of -C(O)NH- and -C(O)-, wherein the carbonyl is optionally further substituted with alkyl, and the alkyl is optionally further substituted with a group selected from the group consisting of alkyl, hydroxy, -C(O)O-, -C(O)O-alkyl-, and -C(O)NH-;
X², X³, X⁴, and X⁵ are each independently selected from an integer from 0 to 10 (e.g., 0, 1, 2, 3, 4, 5, 6, 7, 8, 9, or 10).

In some embodiments, E in the targeting ligand is 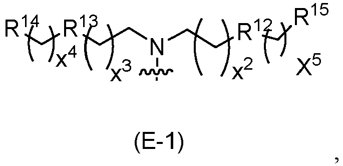 wherein R¹², R¹³, R¹⁴, and R¹⁵ are each independently selected from the group consisting of -C(O)NH- and -C(O)-, wherein the -C(O)NH- or -C(O)- is optionally further substituted with alkyl, and the alkyl is optionally further substituted with a group selected from the group consisting of alkyl, hydroxy, -C(O)O-, -C(O)O-alkyl-, and -C(O)NH-; X², X³, X⁴, and X⁵ are each independently selected from an integer from 0 to 10 (e.g., 0, 1, 2, 3, 4, 5, 6, 7, 8, 9, or 10).

In some embodiments, E in the targeting ligand is 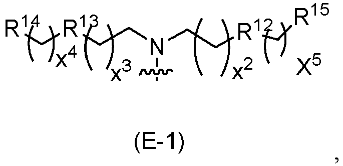 wherein R¹², R¹³, R¹⁴, and R¹⁵ are each independently selected from the group consisting of -C(O)NH- and -C(O)-, wherein the -C(O)NH- or -C(O)- is further substituted with a substituent selected from the group consisting of 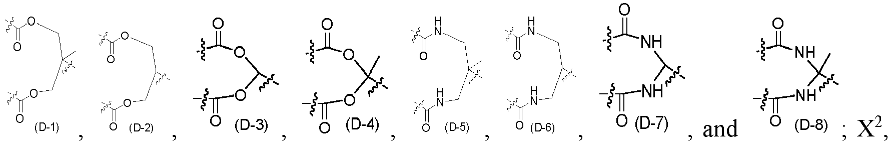 X³, X⁴, and X⁵ are each independently selected from an integer from 0 to 10 (e.g., 0, 1, 2, 3, 4, 5, 6, 7, 8, 9, or 10).

In some embodiments, E in the targeting ligand is

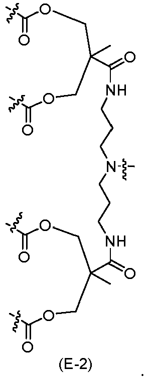

In some embodiments, E in the targeting ligand is selected from the group consisting of:

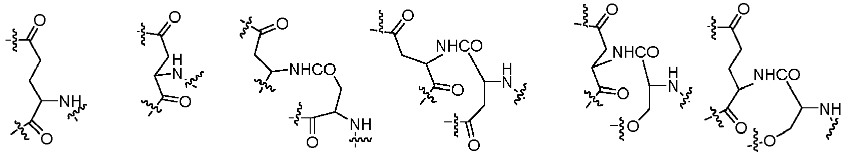

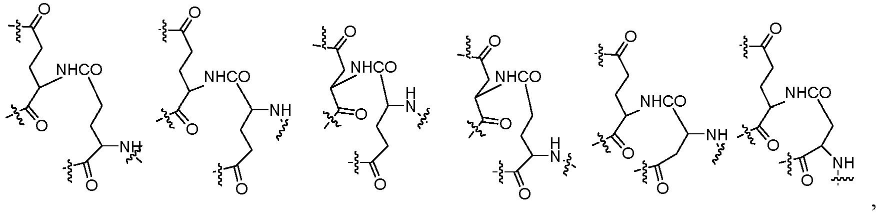

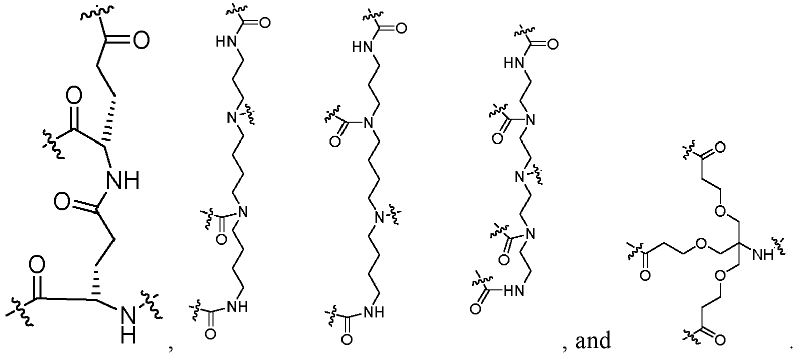

In some embodiments, E in the targeting ligand is selected from

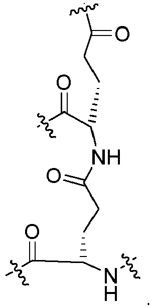

In some embodiments, E in the targeting ligand is selected from

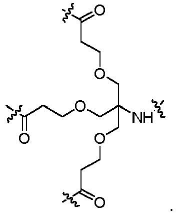

In some embodiments, E in the targeting ligand is 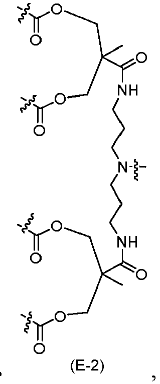 and L₁ is selected from the group consisting of the following structures: 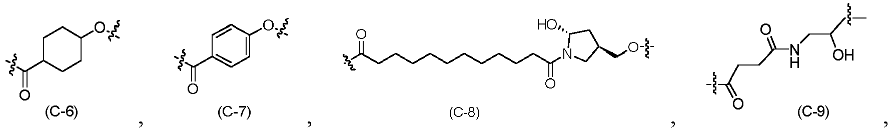 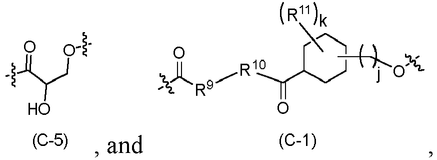 wherein R⁹ and R¹⁰ are each independently selected from the group consisting of -S-, - NH-, -O-, -S-, -C(O)-, -OC(O)-, -C(O)O-, -NHC(O)-, -C(O)NH-, -CH₂-, -CH₂NH-, - CH₂O-, -NH-C(O)-CH₂-, -C(O)-CH₂-NH-, -NH(CO)NH-, and 3- to 12-membered heterocyclyl, wherein the -CH₂- is optionally substituted with a substituent selected from the group consisting of halogen, alkyl, alkoxy, and alkylamino, and the alkyl is optionally further substituted with a substituent selected from the group consisting of hydroxy, amino, and halogen;
R¹¹ is selected from the group consisting of deuterium, halogen, alkyl, amino, cyano, nitro, alkenyl, alkynyl, carboxyl, hydroxy, sulfhydryl, alkylsulfhydryl, alkoxy, alkylamino, -C(O)-alkyl, -C(O)-O-alkyl, -CONH₂, -CONH-alkyl, -OC(O)-alkyl, -NH-C(O)-alkyl, -S(O)O-alkyl, -S(O)ONH₂, and -S(O)ONH-alkyl, wherein the alkyl, alkenyl, alkynyl, alkylsulfhydryl, alkoxy, -C(O)-alkyl, -C(O)-O-alkyl, -CONH-alkyl, -OC(O)-alkyl, -NH-C(O)-alkyl, -S(O)O-alkyl, and -S(O)ONH-alkyl are optionally further substituted with substituents selected from the group consisting of halogen, hydroxy, amino, and sulfhydryl;
k is selected from the group consisting of 0, 1, 2, 3, and 4;
j is selected from the group consisting of 0, 1, 2, 3, 4, 5, 6, 7, 8, 9, 10, 11, 12, 13, 14, 15, 16, 17, 18, 19, and 20.

In some embodiments, E in the targeting ligand is selected from the group consisting of: 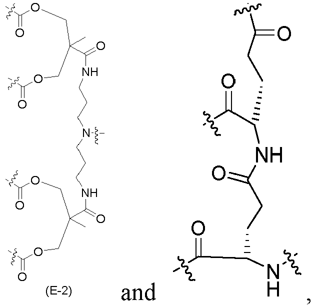 and Li is selected from the group consisting of:

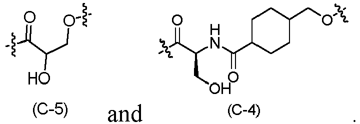

In some embodiments, E in the targeting ligand is selected from 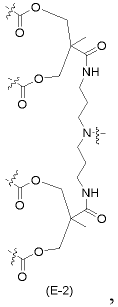 and L₁ is selected from 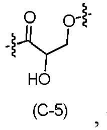 that is, E-L₁ is

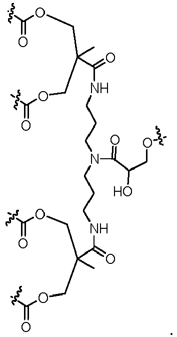

In some embodiments, E in the targeting ligand is selected from 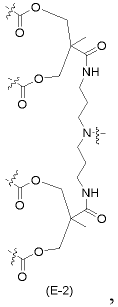 and L₁ is selected from 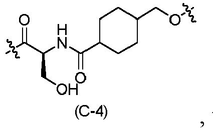 that is, E-L₁ is

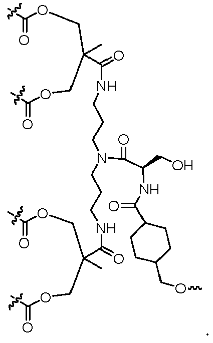

In some embodiments, E in the targeting ligand is selected from 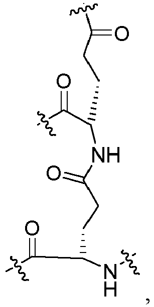 and L₁ is selected from 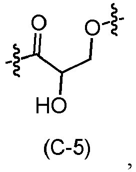 that is, E-L₁ is

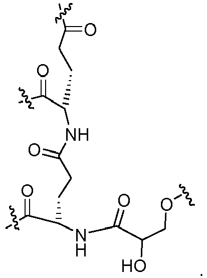

In some embodiments, E in the targeting ligand is selected from 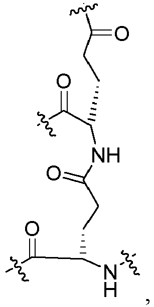 and L₁ is selected from 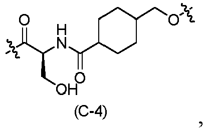 that is, E-L₁ is

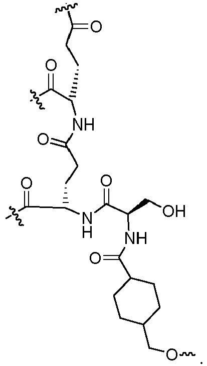

In the present disclosure, L₂ is a tether moiety between the targeting moiety and the branching group, and L₂ links and spaces the targeting moiety and the branching group. In some embodiments, one end of L₂ is directly linked to the targeting ligand and the other end is directly linked to the branching group E.

In some embodiments, one end of L₂ is directly linked to the targeting ligand and the other end is indirectly linked to the branching group E.

In some embodiments, one end of L₂ is indirectly linked to the targeting ligand and the other end is indirectly linked to the branching group E.

In some embodiments, the targeting ligand disclosed herein comprises two L₂ and two targeting moieties.

In some embodiments, the targeting ligand disclosed herein comprises three L₂ and three targeting moieties.

In some embodiments, the targeting ligand disclosed herein comprises four L₂ and four targeting moieties.

In some embodiments, the targeting ligand disclosed herein comprises a plurality of L₂ and a plurality of targeting moieties.

In some embodiments, L₂ in the present disclosure is optionally selected from a combination of 1 to 20 (e.g., 1, 2, 3, 4, 5, 6, 7, 8, 9, 10, 11, 12, 13, 14, 15, 16, 17, 18, 19, or 20) groups optionally selected from the following groups covalently linked: 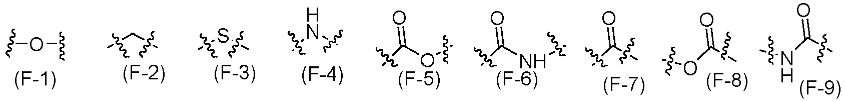 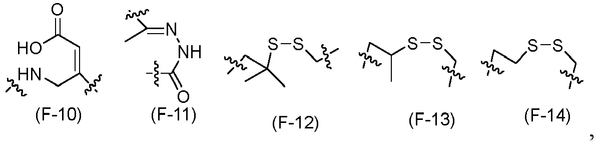 substituted or unsubstituted cycloalkyl (e.g., cyclohexyl, cyclopropyl, cyclobutyl, cyclopentyl, cycloheptyl, or cyclooctyl), substituted or unsubstituted cycloalkenyl (e.g., cyclohexenyl, cyclobutenyl, cyclopentenyl, cycloheptenyl, cyclooctenyl, cyclohexadienyl, cyclopentadienyl, cycloheptadienyl, or cyclooctadienyl), substituted or unsubstituted aryl (e.g., phenyl, naphthyl, binaphthyl, or anthracenyl), substituted or unsubstituted heteroaryl (e.g., pyridyl, pyrimidinyl, pyrrole, imidazole, furan, benzofuran, or indole), and substituted or unsubstituted heterocyclyl (e.g., tetrahydrofuran, tetrahydropyran, piperidine, or pyrrolidine).

In some embodiments, L₂ in the present disclosure is a combination of 1 to 20 (e.g., 1, 2, 3, 4, 5, 6, 7, 8, 9, 10, 11, 12, 13, 14, 15, 16, 17, 18, 19, or 20) groups optionally selected from the group consisting of the following groups covalently linked:

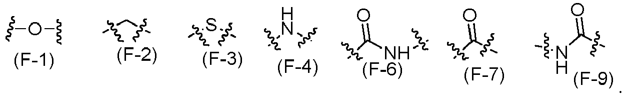

In some embodiments, the targeting ligand comprises L₂ having the structure shown below, 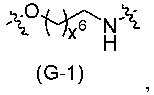 wherein x⁶ is an integer from 1 to 20 (e.g., 1, 2, 3, 4, 5, 6, 7, 8, 9, 10, 11, 12, 13, 14, 15, 16, 17, 18, 19, or 20).

In some embodiments, the targeting ligand comprises L₂ having the structure shown below,

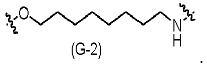

In some embodiments, the targeting ligand comprises L₂ having the structure shown below,

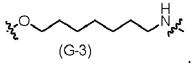

In some embodiments, the targeting ligand comprises L₂ having the structure shown below,

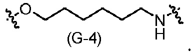

In some embodiments, the targeting ligand comprises L₂ having the structure shown below,

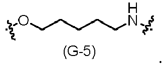

In some embodiments, the targeting ligand comprises L₂ having the structure shown below,

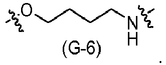

In some embodiments, the targeting ligand comprises L₂ having the structure shown below, 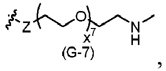 wherein, x⁷ is an integer from 1 to 20 (e.g., 1, 2, 3, 4, 5, 6, 7, 8, 9, 10, 11, 12, 13, 14, 15, 16, 17, 18, 19, or 20), and Z is 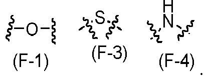 In some embodiments, the targeting ligand comprises L₂ having the structure shown below,

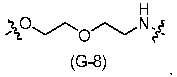

In some embodiments, the targeting ligand comprises L₂ having the structure shown below, 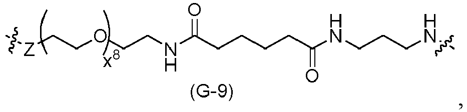 wherein, x⁸ is an integer from 1 to 20 (e.g., 1, 2, 3, 4, 5, 6, 7, 8, 9, 10, 11, 12, 13, 14, 15, 16, 17, 18, 19, or 20), and Z is

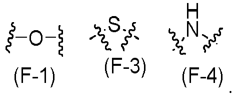

In some embodiments, the targeting ligand comprises L₂ having the structure shown below, 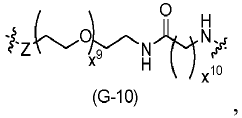 wherein x⁹ and X¹⁰ are each independently selected from an integer from 1 to 20 (e.g., 1, 2, 3, 4, 5, 6, 7, 8, 9, 10, 11, 12, 13, 14, 15, 16, 17, 18, 19, or 20), and Z is

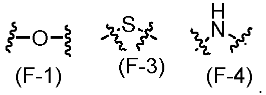

In some embodiments, the targeting ligand comprises L₂ having the structure shown below,

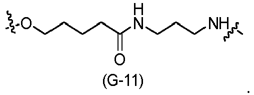

In some embodiments, the targeting ligand comprises L₂ having the structure shown below, 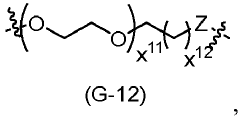 wherein, x⁷ and X⁸ are each independently selected from an integer from 1 to 20 (e.g., 1, 2, 3, 4, 5, 6, 7, 8, 9, 10, 11, 12, 13, 14, 15, 16, 17, 18, 19, or 20), and Z is

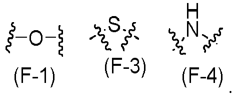

In some specific embodiments, the targeting ligand has the structure shown below:

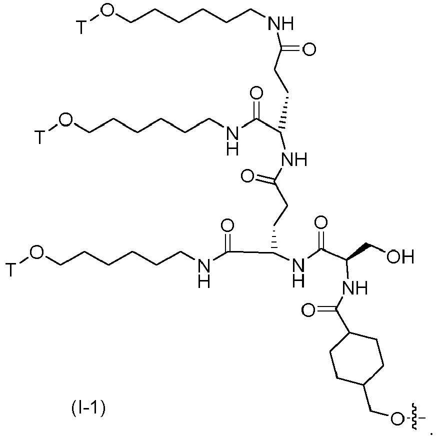

In some specific embodiments, the targeting ligand has the structure shown below:

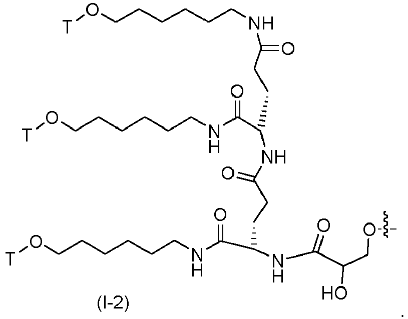

In some specific embodiments, the targeting ligand has the structure shown below:

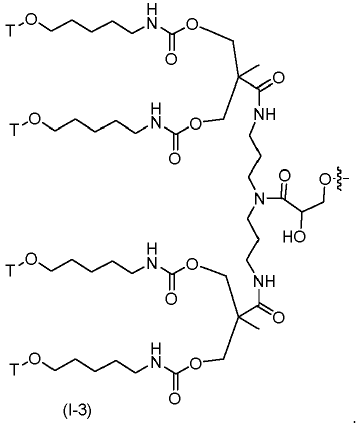

In some specific embodiments, the targeting ligand has the structure shown below:

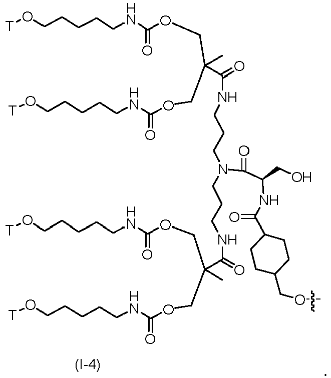

In some embodiments, the targeting moiety T of the targeting ligand consists of one or more targeting moieties, and the targeting ligand assists in directing the delivery of the therapeutic agent linked thereto to the desired target position. In some cases, the targeting moiety can bind to a cell or cellular receptor and initiate endocytosis to promote entry of the therapeutic agent into the cell. The targeting moiety can comprise a compound with affinity for a cellular receptor or a cell surface molecule or an antibody. Various targeting ligands comprising targeting moieties can be linked to therapeutic agents and other compounds to target the agents at cells and particular cellular receptors.

In some embodiments, the types of the targeting moiety T include carbohydrates, cholesterol and cholesterol groups, or steroids. Targeting moieties that can bind to cellular receptors include saccharides such as galactose, galactose derivatives (e.g., *N*-acetyl-galactosamine, *N*-trifluoroacetylgalactosamine, *N*-propionylgalactosamine, *N-n-*butyrylgalactosamine, and *N*-isobutyrylgalactosamine), mannose, and mannose derivatives.

It is known that targeting moieties that bind to asialoglycoprotein receptors (ASGPR) can be particularly used for directing the delivery of oligomeric compounds (e.g., siRNA) to the liver. Asialoglycoprotein receptors are highly expressed on liver cells (hepatocytes).

The targeting moieties of cellular receptors targeting ASGPR include galactose and galactose derivatives. Specifically, clusters of galactose derivatives, including clusters consisting of 2, 3, 4, or more than 4 *N*-acetyl-galactosamines (GalNAc or NAG), can promote the uptake of certain compounds in hepatocytes. The GalNAc cluster coupled to the oligomeric compound is used for directing the composition to the liver where the *N-*acetyl-galactosamine saccharide can bind to the asialoglycoprotein receptors on the liver cell surface. It is believed that the binding to the asialoglycoprotein receptors will initiate receptor-mediated endocytosis, thereby promoting entry of the compound into the interior of the cell.

In some embodiments, the targeting ligand can comprise 2, 3, 4, or more than 4 targeting moieties. In some embodiments, the targeting ligand disclosed herein can comprise 1, 2, 3, 4, or more than 4 targeting moieties linked to the branching group by L₂.

In some embodiments, the targeting ligand is in the form of a galactose cluster.

In some embodiments, each of the targeting moieties comprises a galactosamine derivative, which is *N*-acetyl-galactosamine. Other saccharides that can be used as targeting moieties and have affinity for asialoglycoprotein receptors can be selected from the group consisting of galactose, galactosamine, *N*-formyl-galactosamine, *N*-acetyl-galactosamine, *N*-propionyl-galactosamine, *N-n*-butyryl-galactosamine, *N*-isobutyrylgalactosamine, etc.

In some embodiments, the targeting ligand in the present disclosure comprises *N-*acetylgalactosamine as a targeting moiety,

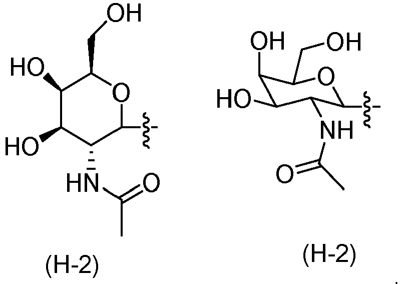

In some embodiments, the targeting ligand comprises three terminal galactosamines or galactosamine derivatives (such as *N*-acetyl-galactosamine), each of which has affinity for asialoglycoprotein receptors. In some embodiments, the targeting ligand comprises three terminal *N-*acetyl-galactosamine (GalNAc or NAG) as targeting moieties.

In some embodiments, the targeting ligand comprises four terminal galactosamines or galactosamine derivatives (such as *N*-acetyl-galactosamine), each of which has affinity for asialoglycoprotein receptors. In some embodiments, the targeting ligand comprises four terminal N acetyl-galactosamine (GalNAc or NAG) as targeting moieties.

The term commonly used in the art when referring to the three terminal *N*-acetyl-galactosamine includes tri-antennary, tri-valent, and trimer.

The term commonly used in the art when referring to the four terminal *N*-acetyl-galactosamine includes tetra-antennary, tetra-valent, and tetramer.

In some specific embodiments, the targeting ligand provided by the present disclosure has the structure shown below,

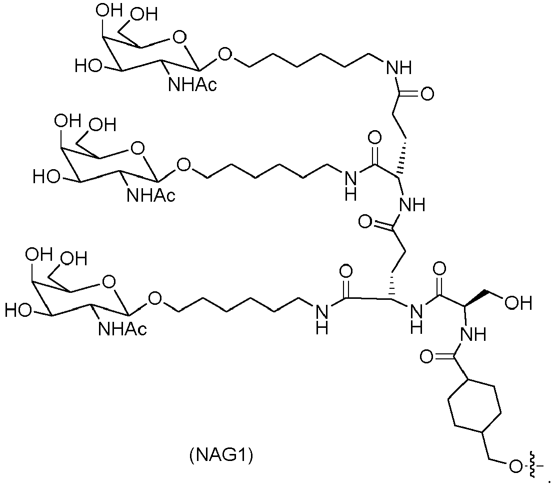

In some specific embodiments, the targeting ligand provided by the present disclosure has the structure shown below,

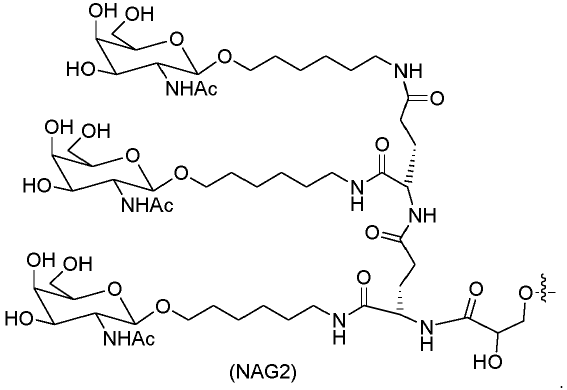

In some specific embodiments, the targeting ligand provided by the present disclosure has the structure shown below,

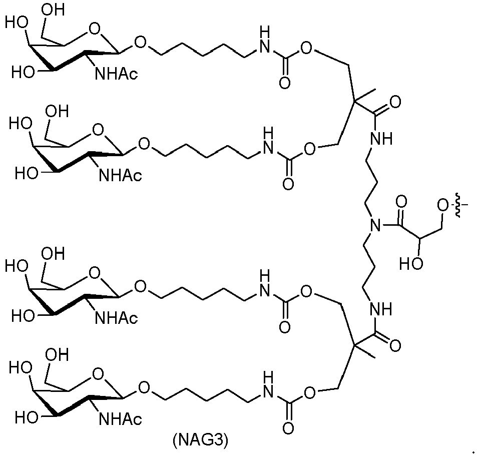

In some specific embodiments, the targeting ligand provided by the present disclosure has the structure shown below,

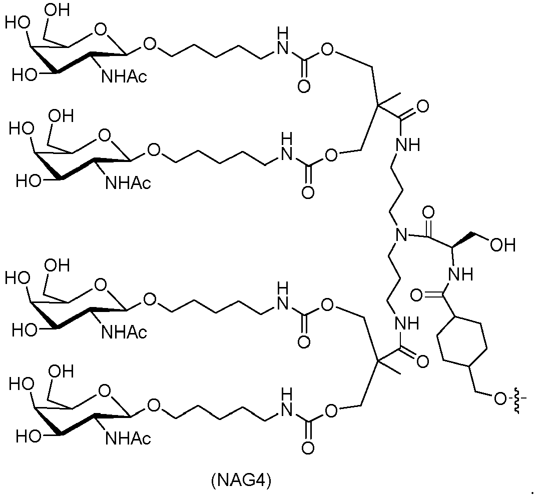

In some embodiments, the siRNA of the present disclosure is linked to the targeting ligand of the present disclosure, forming an siRNA conjugate shown below, 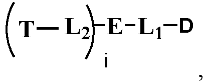 wherein T is a targeting moiety, E is a branching group, L₁ is a linker moiety, and L₂ is a tether moiety between the targeting moiety and the branching group, wherein x is selected from an integer from 1 to 10, and D is an siRNA targeting HSD17B13.

In some embodiments, D is an siRNA targeting HSD17B13.

In some embodiments, D is any one of the siRNAs of the present disclosure. In some embodiments, L₁ is linked to the 3' end of the sense strand of the siRNA.

In some embodiments, the targeting ligand is linked to an end of the siRNA by a phosphoester group, a phosphorothioate group, or a phosphonic acid group.

In some embodiments, the targeting ligand is indirectly linked to an end of the siRNA by a phosphoester group, a phosphorothioate group, or a phosphonic acid group.

In some embodiments, the targeting ligand is directly linked to an end of the siRNA by a phosphoester group, a phosphorothioate group, or a phosphonic acid group.

In some embodiments, the targeting ligand is directly linked to an end of the siRNA by a phosphoester group or a phosphorothioate group.

In some embodiments, the targeting ligand is directly linked to the 3' end of the sense strand of the siRNA by a phosphoester group or a phosphorothioate group.

In some embodiments, the sense strand of the siRNA of the present disclosure comprises or is any one of SEQ ID NO: 194 to SEQ ID NO: 212.

In some embodiments, the antisense strand of the siRNA of the present disclosure comprises or is any one of SEQ ID NO: 111 to SEQ ID NO: 172.

Another aspect of the present disclosure provides a composition, which comprises the conjugate described above, and one or more pharmaceutically acceptable excipients, such as carriers, vehicles, diluents, and/or delivery polymers.

Another aspect of the present disclosure provides use of the conjugate or the composition comprising the conjugate described above in preparing a medicament for treating a disease in a subject; in some embodiments, the disease is selected from a hepatic disease. Another aspect of the present disclosure provides a method for treating a disease in a subject, which comprises administering to the subject the conjugate or the composition described above.

Another aspect of the present disclosure provides a method for inhibiting mRNA expression in a subject, which comprises administering to the subject the conjugate or the composition described above.

Another aspect of the present disclosure provides a method for delivering an siRNA, an siRNA conjugate, or a pharmaceutical composition to the liver *in vivo,* which comprises administering to a subject the conjugate or the composition described above.

The conjugate, the composition, and the method disclosed herein can reduce the level of a target mRNA in a cell, a cell population, tissue, or a subject, which comprises: administering to the subject a therapeutically effective amount of the siRNA, the siRNA conjugate, or the pharmaceutical composition described herein linked to a targeting ligand, thereby inhibiting expression of the target mRNA in the subject.

In some embodiments, the subject has been identified as having pathogenic upregulation of the target gene in the targeted cell or tissue.

The subject described herein refers to a subject having a disease or condition that would benefit from reduction or inhibition of the target mRNA.

Delivery can be accomplished by topical administration (e.g., direct injection, implantation, or topical application), systemic administration, or through subcutaneous, intravenous, intraperitoneal, or parenteral routes, including intracranial (e.g., intraventricular, intraparenchymal, and intrathecal), intramuscular, transdermal, airway (aerosol), nasal, oral, rectal, or topical (including buccal and sublingual) administration. In optional embodiments, the siRNA, the siRNA conjugate, or the pharmaceutical composition provided by the present disclosure can be administered by injection, for example, by intravenous, intramuscular, intradermal, subcutaneous, intraduodenal, or intraperitoneal injection.

Various delivery systems are known and can be used for the siRNA, the siRNA conjugate, or the pharmaceutical composition of the present disclosure, e.g., encapsulation in liposomes, microparticles, microcapsules, recombinant cells capable of expressing the compound, receptor-mediated endocytosis, and construction of a nucleic acid as part of a retroviral or other vectors.

In optional embodiments, after the targeting ligand and siRNA are linked to form a conjugate, the conjugate can be packaged in a kit.

The present disclosure also provides a pharmaceutical composition, which comprises the siRNA or the siRNA conjugate of the present disclosure.

In some embodiments, the pharmaceutical composition can further comprise a pharmaceutically acceptable auxiliary material and/or adjuvant; the auxiliary material can be one or more of various formulations or compounds conventionally used in the art. For example, the pharmaceutically acceptable auxiliary material can include at least one of a pH buffer, a protective agent, and an osmotic pressure regulator.

In some embodiments, the siRNA, the siRNA conjugate, or the pharmaceutical composition described above, when in contact with a target gene-expressing cell, inhibits expression of the target gene by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99%, as determined by, for example, psiCHECK activity screening and luciferase reporter gene assay, and other methods such as PCR or branched DNA (bDNA)-based methods.

In some embodiments, the siRNA, the siRNA conjugate, or the pharmaceutical composition described above, when in contact with a target gene-expressing cell, results in a percentage of residual expression of target gene's mRNA of no more than 99%, no more than 95%, no more than 90%, no more than 85%, no more than 80%, no more than 75%, no more than 70%, no more than 65%, no more than 60%, no more than 55%, no more than 50%, no more than 45%, no more than 40%, no more than 35%, no more than 30%, no more than 25%, no more than 20%, no more than 15%, or no more than 10%, as measured by, for example, psiCHECK activity screening and luciferase reporter gene assay, and other methods such as PCR or branched DNA (bDNA)-based methods.

In some embodiments, when the siRNA, the siRNA conjugate, or the pharmaceutical composition is in contact with a target gene-expressing cell, the siRNA or the siRNA conjugate reduces off-target activity by at least 20%, at least 25%, at least 30%, at least 35%, at least 40%, at least 45%, at least 50%, at least 55%, at least 60%, at least 65%, at least 70%, or at least 75%, while maintaining on-target activity, as measured by, for example, psiCHECK activity screening and luciferase reporter gene assay, and other methods such as PCR or branched DNA (bDNA)-based methods.

In some embodiments, the siRNA, the siRNA conjugate, or the pharmaceutical composition, when in contact with a target gene-expressing cell, reduces off-target activity by at least 20%, at least 25%, at least 30%, at least 35%, at least 40%, at least 45%, at least 50%, at least 55%, at least 60%, at least 65%, at least 70%, or at least 75%, while reducing on-target activity by at most 20%, at most 19%, at most 15%, at most 10%, at most 5%, or more than 1%, as measured by, for example, psiCHECK activity screening and luciferase reporter gene assay, and other methods such as PCR or branched DNA (bDNA)-based methods.

In some embodiments, when the siRNA, the siRNA conjugate, or the pharmaceutical composition is in contact with a target gene-expressing cell, the siRNA or the siRNA conjugate reduces off-target activity by at least 20%, at least 25%, at least 30%, at least 35%, at least 40%, at least 45%, at least 50%, at least 55%, at least 60%, at least 65%, at least 70%, or at least 75%, while increasing on-target activity by at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, or at least 80%, as measured by, for example, psiCHECK activity screening and luciferase reporter gene assay, and other methods such as PCR or branched DNA (bDNA)-based methods.

The present disclosure also provides a cell, which comprises the siRNA or the siRNA conjugate of the present disclosure. The cells cannot develop into complete animal and plant individuals.

The present disclosure also provides a kit, which comprises the siRNA or the siRNA conjugate of the present disclosure.

The present disclosure also provides a method for silencing a target gene or mRNA of the target gene in a cell, which comprises the step of introducing the siRNA, the siRNA conjugate, and/or the pharmaceutical composition according to the present disclosure into the cell.

The present disclosure also provides a method for silencing a target gene or mRNA of the target gene in a cell *in vivo* or *in vitro,* which comprises the step of introducing the siRNA, the siRNA conjugate, and/or the pharmaceutical composition according to the present disclosure into the cell.

The present disclosure also provides a method for inhibiting expression of a target gene or mRNA of the target gene, which comprises administering to a subject in need thereof an effective amount or dose of the siRNA, the siRNA conjugate, and/or the pharmaceutical composition according to the present disclosure.

In some embodiments, administration is carried out through routes of administration including intramuscular, intrabronchial, intrapleural, intraperitoneal, intra-arterial, lymphatic, intravenous, subcutaneous, or cerebrospinal administration, or combinations thereof.

In some embodiments, the effective amount or dose of the siRNA, the siRNA conjugate, and/or the pharmaceutical composition is from about 0.001 mg/kg body weight to about 200 mg/kg body weight, from about 0.01 mg/kg body weight to about 100 mg/kg body weight, or from about 0.5 mg/kg body weight to about 50 mg/kg body weight.

In some embodiments, the target gene is an HSD17B13 gene.

The present disclosure provides the siRNA and/or the pharmaceutical composition and/or the siRNA conjugate described above for use in treating and/or preventing a disease related to HSD17B13 gene expression in a subject. In some embodiments, the disease related to HSD17B13 gene expression is chronic fibro-inflammatory liver disease. In some embodiments, the chronic fibro-inflammatory liver disease is related to the accumulation and/or expansion of lipid droplets in the liver.

The present disclosure provides the siRNA and/or the pharmaceutical composition and/or the siRNA conjugate described above for use in treating and/or preventing a disease selected from the group consisting of hepatitis, liver fibrosis, nonalcoholic steatohepatitis (NASH), nonalcoholic fatty liver disease (NAFLD), cirrhosis, alcoholic steatohepatitis (ASH), alcoholic fatty liver disease (ALD), HCV-associated cirrhosis, drug-induced liver injury, and hepatic necrosis. In some embodiments, the disease is related to HSD17B13 gene expression.

The present disclosure provides the siRNA and/or the pharmaceutical composition and/or the siRNA conjugate described above for use in reducing the risk of developing chronic liver disease in an individual with steatosis, and/or for use in inhibiting the progression of steatosis to steatohepatitis in an individual with steatosis, and/or for use in inhibiting the accumulation of lipid droplets in the liver.

The present disclosure provides use of the siRNA and/or the pharmaceutical composition and/or the siRNA conjugate described above in preparing a medicament for treating and/or preventing a disease related to HSD17B13 gene expression in a subject. In some embodiments, the disease related to HSD17B13 gene expression is chronic fibro-inflammatory liver disease. In some embodiments, the chronic fibro-inflammatory liver disease is related to the accumulation and/or expansion of lipid droplets in the liver. The present disclosure provides use of the siRNA and/or the pharmaceutical composition and/or the siRNA conjugate in preparing a medicament for treating and/or preventing a disease selected from the group consisting of hepatitis, liver fibrosis, nonalcoholic steatohepatitis (NASH), nonalcoholic fatty liver disease (NAFLD), cirrhosis, alcoholic steatohepatitis (ASH), alcoholic fatty liver disease (ALD), HCV-associated cirrhosis, drug-induced liver injury, and hepatic necrosis. In some embodiments, the disease is related to HSD17B13 gene expression.

The present disclosure provides use of the siRNA and/or the pharmaceutical composition and/or the siRNA conjugate described above in preparing a medicament for reducing the risk of developing chronic liver disease in an individual with steatosis, and/or for inhibiting the progression of steatosis to steatohepatitis in an individual with steatosis, and/or for inhibiting the accumulation of lipid droplets in the liver.

The present disclosure provides a method for inhibiting expression of a 17β-hydroxysteroid dehydrogenase type 13 (HSD17B13) gene, which comprises administering to a subject an effective amount or dose of the siRNA and/or the pharmaceutical composition and/or the siRNA conjugate described above.

The present disclosure provides a method for treating and/or preventing a disease related to HSD17B13 gene expression in a subject, which comprises administering to the subject an effective amount or dose of the siRNA and/or the pharmaceutical composition and/or the siRNA conjugate described above. In some embodiments, the disease related to HSD17B13 gene expression is chronic fibro-inflammatory liver disease. In some embodiments, the chronic fibro-inflammatory liver disease is related to the accumulation and/or expansion of lipid droplets in the liver.

The present disclosure provides a method for treating and/or preventing a disease selected from the group consisting of hepatitis, liver fibrosis, nonalcoholic steatohepatitis (NASH), nonalcoholic fatty liver disease (NAFLD), cirrhosis, alcoholic steatohepatitis (ASH), alcoholic fatty liver disease (ALD), HCV-associated cirrhosis, drug-induced liver injury, and hepatic necrosis, which comprises administering to a subject an effective amount or dose of the siRNA and/or the pharmaceutical composition and/or the siRNA conjugate described above. In some embodiments, the disease is related to HSD17B13 gene expression.

The present disclosure provides a method for reducing the risk of developing chronic liver disease in an individual with steatosis, and/or for inhibiting the progression of steatosis to steatohepatitis in an individual with steatosis, and/or for inhibiting the accumulation of lipid droplets in the liver, which comprises administering to a subject an effective amount or effective dose of the siRNA and/or the pharmaceutical composition and/or the siRNA conjugate described above.

The present disclosure provides a method for delivering siRNA that inhibits the expression and/or replication of an HSD17B 13 gene to the liver *in vivo,* which comprises administering to a subject an effective amount or effective dose of the pharmaceutical composition and/or the siRNA conjugate described above.

The present disclosure also provides an siRNA or an siRNA conjugate, wherein the chemical modification of formula (I) or formula (I') or the tautomeric modification thereof in the antisense strand of any one of the siRNAs or siRNA conjugates of the present disclosure is replaced with a 2'-methoxy modification.

The present disclosure also provides an siRNA or an siRNA conjugate, wherein the chemical modification of formula (I) or formula (I') in the antisense strand of any one of the siRNAs or the siRNA conjugates of the present disclosure is a 2'-methoxy modification.

The present disclosure also provides an siRNA or an siRNA conjugate, wherein one or more bases U, e.g., 1, 2, 3, 3, 5, 6, 7, 8, 9, or 10 bases U, of any one of the siRNAs or the siRNA conjugates of the present disclosure are replaced with bases T.

The pharmaceutically acceptable salts of the compounds described herein are selected from the group consisting of inorganic salts and organic salts. The compounds described herein can react with acidic or basic substances to form corresponding salts.

Unless otherwise specified, the "siRNA", "siRNA conjugate", "chemical modification", and "targeting ligand" of the present disclosure can each independently exist in the form of a salt, a mixed salt, or a non-salt (e.g., free acid or free base). When existing as a salt or a mixed salt, it may be a pharmaceutically acceptable salt.

The pharmaceutically acceptable salts of the compounds described herein are selected from the group consisting of inorganic salts and organic salts. The compounds described herein can react with acidic or basic substances to form corresponding salts.

"Corresponding salt formed by reacting with an acidic substance" refers to a salt that is capable of retaining the bioavailability of a free base without having any side effects and that are formed with an inorganic acid or organic acid. Inorganic acid salts include, but are not limited to, hydrochlorides, hydrobromides, sulfates, nitrates, phosphates, etc.; organic acid salts include, but are not limited to, formates, acetates, 2,2-dichloroacetates, trifluoroacetates, propionates, caproates, caprylates, caprates, undecenates, glycolates, gluconates, lactates, sebacates, adipates, glutarates, malonates, oxalates, maleates, succinates, fumarates, tartrates, citrates, palmitates, stearates, oleates, cinnamates, laurates, malates, glutamates, pyroglutamates, aspartates, benzoates, methanesulfonates, benzenesulfonates, *p*-toluenesulfonates, alginates, ascorbates, salicylates, 4-aminosalicylates, napadisylates, etc. These salts can be prepared using methods known in the art.

"Corresponding salt formed by reacting with a basic substance" refers to a salt that is capable of retaining the bioavailability of a free base without having any side effects and that is formed with an inorganic base or organic base. Salts derived from inorganic bases include, but are not limited to, sodium salts, potassium salts, lithium salts, ammonium salts, calcium salts, magnesium salts, iron salts, zinc salts, copper salts, manganese salts, aluminum salts, etc. Preferred inorganic salts are ammonium salts, sodium salts, potassium salts, calcium salts and magnesium salts; sodium salts are preferred. Salts derived from organic bases include, but are not limited to, salts of the following: primary, secondary and tertiary amines, substituted amines including naturally occurring substituted amines, cyclic amines and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine, piperazine, piperidine, *N*-ethylpiperidine, polyamine resins, etc. Preferred organic bases include isopropylamine, diethylamine, ethanolamine, trimethylamine, dicyclohexylamine, choline, and caffeine. These salts can be prepared using methods known in the art.

In another aspect, the compounds of the present disclosure may exist in specific geometric or stereoisomeric forms. The present disclosure encompasses all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which are within the scope of the present disclosure. Additional asymmetric carbon atoms may be present in substituents such as an alkyl group. All such isomers and mixtures thereof are included within the scope of the present disclosure.

The compounds and intermediates of the present disclosure may also exist in different tautomeric forms, and all such forms are included within the scope of the present disclosure. The term "tautomer" or "tautomeric form" refers to structural isomers of different energies that can interconvert via a low energy barrier. For example, proton tautomers (also known as proton transfer tautomers) include interconversion via proton migration, such as keto-enol and imine-enamine, lactam-lactim isomerization. An example of a lactam-lactim equilibrium is present between A and B as shown below.

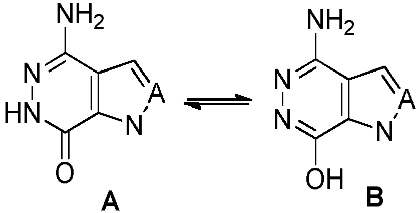

All compounds in the present disclosure can be drawn as form A or form B. All tautomeric forms are within the scope of the present disclosure. The nomenclature of the compounds does not exclude any tautomers.

The compounds of the present disclosure may be asymmetric; for example, the compounds have one or more stereoisomers. Unless otherwise specified, all stereoisomers include, for example, enantiomers and diastereomers. The compounds of the present disclosure containing asymmetric carbon atoms can be isolated in optically active pure form or in racemic form. The optically active pure form can be isolated from a racemic mixture or synthesized using chiral starting materials or chiral reagents.

Optically active (R)- and (S)-enantiomers, and D- and L-isomers can be prepared by chiral synthesis, chiral reagents or other conventional techniques. If one enantiomer of a certain compound of the disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, followed by separation of diastereomers by conventional methods known in the art, and the pure enantiomers are obtained by recovery. In addition, separation of enantiomers and diastereomers is generally accomplished by chromatography using a chiral stationary phase, optionally in combination with chemical derivatization (e.g., carbamate formation from amines). The present disclosure also includes isotopically-labeled compounds which are identical to those recited herein but have one or more atoms replaced with an atom having an atomic mass or mass number different from the atomic mass or mass number usually found in nature. Examples of isotopes that can be incorporated into the compound of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl.

Unless otherwise specified, when a position is specifically assigned deuterium (D), the position should be construed as deuterium with an abundance that is at least 1000 times greater than the natural abundance of deuterium (which is 0.015%) (i.e., at least 10% deuterium incorporation). The compounds of examples comprise deuterium having an abundance that is at least 1000 times greater than the natural abundance, at least 2000 times greater than the natural abundance, at least 3000 times greater than the natural abundance, at least 4000 times greater than the natural abundance, at least 5000 times greater than the natural abundance, at least 6000 times greater than the natural abundance, or higher times greater than the natural abundance. The present disclosure also includes various deuterated forms of the compounds of formula I and formula II. Each available hydrogen atom connected to a carbon atom may be independently replaced with a deuterium atom. Those skilled in the art are able to synthesize the deuterated forms of the compounds of formula I and formula II with reference to the relevant literature. Commercially available deuterated starting materials can be used in preparing the deuterated forms of the compounds of formula I and formula II, or they can be synthesized using conventional techniques with deuterated reagents including, but not limited to, deuterated borane, tri-deuterated borane in tetrahydrofuran, deuterated lithium aluminum hydride, deuterated iodoethane, deuterated iodomethane, and the like.

"Optionally" or "optional" means that the event or circumstance subsequently described may, but does not necessarily, occur and that the description includes instances where the event or circumstance occurs or does not occur. For example, "C₁₋₆ alkyl that is optionally substituted with a halogen or cyano" means that the halogen or cyano may, but does not necessarily, exist, and the description includes the instance where alkyl is substituted with a halogen or cyano and the instance where alkyl is not substituted with a halogen or cyano. In the chemical structure of the compound of the present disclosure, a bond " 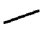 " represents an unspecified configuration; that is, if chiral isomers exist in the chemical structure, the bond " 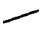 " may be " 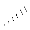 " or " 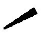 ", or contains both the configurations of " 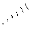 " and " 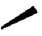 ". Although all of the above structural formulae are drawn as certain isomeric forms for the sake of simplicity, the present disclosure may include all isomers, such as tautomers, rotamers, geometric isomers, diastereomers, racemates and enantiomers. In the chemical structure of the compound of the present disclosure, a bond " 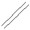 " does not specify a configuration; that is, the configuration for the bond " 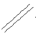 " can be an E configuration or a Z configuration, or includes both the E configuration and the Z configuration.

In the present disclosure, the reference sequence of NM_178135.5 of a target gene HSD17B13, i.e., a sequence of SEQ ID NO: 1 (5'-3'), is:

ACACAAGGACUGAACCAGAAGGAAGAGGACAGAGCAAAGCCAUGAACAU
CAUCCUAGAAAUCCUUCUGCUUCUGAUCACCAUCAUCUACUCCUACUUGG
AGUCGUUGGUGAAGUUUUUCAUUCCUCAGAGGAGAAAAUCUGUGGCUGG
GGAGAUUGUUCUCAUUACUGGAGCUGGGCAUGGAAUAGGCAGGCAGACU
ACUUAUGAAUUUGCAAAACGACAGAGCAUAUUGGUUCUGUGGGAUAUUA
AUAAGCGCGGUGUGGAGGAAACUGCAGCUGAGUGCCGAAAACUAGGCGU
CACUGCGCAUGCGUAUGUGGUAGACUGCAGCAACAGAGAAGAGAUCUAU
CGCUCUCUAAAUCAGGUGAAGAAAGAAGUGGGUGAUGUAACAAUCGUGG
UGAAUAAUGCUGGGACAGUAUAUCCAGCCGAUCUUCUCAGCACCAAGGA
UGAAGAGAUUACCAAGACAUUUGAGGUCAACAUCCUAGGACAUUUUUGG
AUCACAAAAGCACUUCUUCCAUCGAUGAUGGAGAGAAAUCAUGGCCACA
UCGUCACAGUGGCUUCAGUGUGCGGCCACGAAGGGAUUCCUUACCUCAUC
CCAUAUUGUUCCAGCAAAUUUGCCGCUGUUGGCUUUCACAGAGGUCUGAC
AUCAGAACUUCAGGCCUUGGGAAAAACUGGUAUCAAAACCUCAUGUCUC
UGCCCAGUUUUUGUGAAUACUGGGUUCACCAAAAAUCCAAGCACAAGAU
UAUGGCCUGUAUUGGAGACAGAUGAAGUCGUAAGAAGUCUGAUAGAUGG
AAUACUUACCAAUAAGAAAAUGAUUUUUGUUCCAUCGUAUAUCAAUAUC
UUUCUGAGACUACAGAAGUUCUUCCUGAACGCGCCUCAGCGAUUUUAA
AUCGUAUGCAGAAUAUUCAAUUUGAAGCAGUGGUUGGCCACAAAAUCAA
AAUGAAAUGAAUAAAUAAGCUCCAGCCAGAGAUGUAUGCAUGAUAAUGA
UAUGAAUAGUUUCGAAUCAAUGCUGCAAAGCUUUAUUUCACAUUUUUUC
AGUCCUGAUAAUAUUAAAAACAUUGGUUUGGCACUAGCAGCAGUCAAAC
GAACAAGAUUAAUUACCUGUCUUCCUGUUUCUCAAGAAUAUUUACGUAG
UUUUUCAUAGGUCUGUUUUUCCUUUCAUGCCUCUUAAAAACUUCUGUGC
UUACAUAAACAUACUUAAAAGGUUUUCUUUAAGAUAUUUUAUUUUUCCA
UUUAAAGGUGGACAAAAGCUACCUCCCUAAAAGUAAAUACAAAGAGAAC
UUAUUUACACAGGGAAGGUUUAAGACUGUUCAAGUAGCAUUCCAAUCUG
UAGCCAUGCCACAGAAUAUCAACAAGAACACAGAAUGAGUGCACAGCUA
AGAGAUCAAGUUUCAGCAGGCAGCUUUAUCUCAACCUGGACAUAUUUUA
AGAUUCAGCAUUUGAAAGAUUUCCCUAGCCUCUUCCUUUUUCAUUAGCCC
AAAACGGUGCAACUCUAUUCUGGACUUUAUUACUUGAUUCUGUCUUCUG
UAUAACUCUGAAGUCCACCAAAAGUGGACCCUCUAUAUUUCCUCCCUUUU
UAUAGUCUUAUAAGAUACAUUAUGAAAGGUGACCGACUCUAUUUUAAAU
CUCAGAAUUUUAAGUUCUAGCCCCAUGAUAACCUUUUUCUUUGUAAUUU
AUGCUUUCAUAUAUCCUUGGUCCCAGAGAUGUUUAGACAAUUUUAGGCU
CAAAAAUUAAAGCUAACACAGGAAAAGGAACUGUACUGGCUAUUACAUA
AGAAACAAUGGACCCAAGAGAAGAAAAGGAAGAAAGAAAGGUUUUUUGG

UUUUGUUUUGUUUUGUUUUGUUUUUGUUUUUUGAGAUGGAGUCUCA
CUCUUUCGCCCAGGCUGGAGUGCAGUGGUAUGAUCUCAGCUCACUGCAAG
CUCCACCUCCCGGGUUCACGCCAUUCUCCUGCCUCAGCCUCCUGAGUAGC
UGGGACUACAGGCGCCCGCCACCACACCCGGCUAAUUUUUGUAUUUUU
GUAGAGACGGGGUUUCACCAUGUUAGCCAAGAUGGUCUCGAUCUCCUGA
CCUCGUGAUCCACCUGCCUCGGCCUCCCAAAGUGCUGGGAUUACGGGUGU
GAGCCACCGUGCCCAGCCUUUUUUUUUUUAAUAGAAAAAAUAAUCCGAC
UCCCACUACAUCAAGACUAAUCUUGUUUUGUGUGUUUUUCACAUGUAUU
AUAGAAUGCUUUUGCAUGGACUAUCCUCUUGUUUUUAUUAAAAACAAAU
GAUUUUUUUAAAAGUCACAAAAACAAUUCACUAAAAAUAAAUAUGUCAU
UGUGCUUUAAAAAAAUAACCUCUUGUAGUUAUAAAAUAAAACGUUUGAC
UUCUAAA;

In the present disclosure, a sequence of SEQ ID NO: 2 (5'-3') is:

UUUAGAAGUCAAACGUUUAUUUAUAACUACAAGAGGUUAUUUUUUA
AAGCACAAUGACAUAUUUAUUUUAGUGAAUUGUUUUGUGACUUUAA
AAAAAUCAUUUGUUUUAAUAAAAACAAGAGGAUAGUCCAUGCAAAAGC
AUUCUAUAAUACAUGUGAAAAACACACAAAACAAGAUUAGUCUUGAUGU
AGUGGGAGUCGGAUUAUUUUUCUAUUAAAAAAAAAAAGGCUGGGCACG
GUGGCUCACACCCGUAAUCCCAGCACUUUGGGAGGCCGAGGCAGGUGGAU
CACGAGGUCAGGAGAUCGAGACCAUCUUGGCUAACAUGGUGAAACCCCGU
CUCUACAAAAAUACAAAAAUUAGCCGGGUGUGGUGGCGGGCGCCUGU
AGUCCCAGCUACUCAGGAGGCUGAGGCAGGAGAAUGGCGUGAACCCGGG
AGGUGGAGCUUGCAGUGAGCUGAGAUCAUACCACUGCACUCCAGCCUGGG
CGAAAGAGUGAGACUCCAUCUCAAAAAAACAAAAAACAAAACAAAACAA
AACAAAAACCAAAAAACCUUUCUUUCUUCCUUUUCUUCUCUUGGGUCCAU
UGUUUCUUAUGUAAUAGCCAGUACAGUUCCUUUUCCUGUGUUAGCUUUA
AUUUUUGAGCCUAAAAUUGUCUAAACAUCUCUGGGACCAAGGAUAUAUG
AAAGCAUAAAUUACAAAGAAAAAGGUUAUCAUGGGGCUAGAACUUAAAA
UUCUGAGAUUUAAAAUAGAGUCGGUCACCUUUCAUAAUGUAUCUUAUAA
GACUAUAAAAAGGGAGGAAAUAUAGAGGGUCCACUUUUGGUGGACUUCA
GAGUUAUACAGAAGACAGAAUCAAGUAAUAAAGUCCAGAAUAGAGUUGC
ACCGUUUUGGGCUAAUGAAAAAGGAAGAGGCUAGGGAAAUCUUUCAAAU
GCUGAAUCUUAAAAUAUGUCCAGGUUGAGAUAAAGCUGCCUGCUGAAAC
UUGAUCUCUUAGCUGUGCACUCAUUCUGUGUUCUUGUUGAUAUUCUGUG
GCAUGGCUACAGAUUGGAAUGCUACUUGAACAGUCUUAAACCUUCCCUG
UGUAAAUAAGUUCUCUUUGUAUUUACUUUUAGGGAGGUAGCUUUUGUCC
ACCUUUAAAUGGAAAAAUAAAAUAUCUUAAAGAAAACCUUUUAAGUAUG
UUUAUGUAAGCACAGAAGUUUUUAAGAGGCAUGAAAGGAAAAACAGACC
UAUGAAAAACUACGUAAAUAUUCUUGAGAAACAGGAAGACAGGUAAUUA
AUCUUGUUCGUUUGACUGCUGCUAGUGCCAAACCAAUGUUUUUAAUAUU
AUCAGGACUGAAAAAAUGUGAAAUAAAGCUUUGCAGCAUUGAUUCGAAA
CUAUUCAUAUCAUUAUCAUGCAUACAUCUCUGGCUGGAGCUUAUUUAUU
CAUUUCAUUUUGAUUUUGUGGCCAACCACUGCUUCAAAUUGAAUAUUCU
GCAUACGAUUUAAAAUCGCUGAGGCGCGUUCAGGAAGAAACUUCUGUAG
UCUCAGAAAGAUAUUGAUAUACGAUGGAACAAAAAUCAUUUCUUAUUG
GUAAGUAUUCCAUCUAUCAGACUUCUUACGACUUCAUCUGUCUCCAAUAC
AGGCCAUAAUCUUGUGCUUGGAUUUUUGGUGAACCCAGUAUUCACAAAA
ACUGGGCAGAGACAUGAGGUUUGAUACCAGUUUUCCCAAGGCCUGAA
GUUCUGAUGUCAGACCUCUGUGAAAGCCAACAGCGGCAAAUUUGCUGGA
ACAAUAUGGGAUGAGGUAAGGAAUCCCUUCGUGGCCGCACACUGAAGCC

ACUGUGACGAUGUGGCCAUGAUUUCUCUCCAUCAUCGAUGGAAGAAGUG
CUUUUGUGAUCCAAAAAUGUCCUAGGAUGUUGACCUCAAAUGUCUUGGU
AAUCUCUUCAUCCUUGGUGCUGAGAAGAUCGGCUGGAUAUACUGUCCCA
GCAUUAUUCACCACGAUUGUUACAUCACCCACUUCUUUCUUCACCUGAUU
UAGAGAGCGAUAGAUCUCUUCUCUGUUGCUGCAGUCUACCACAUACGCAU
GCGCAGUGACGCCUAGUUUUCGGCACUCAGCUGCAGUUCCUCCACACCG
CGCUUAUUAAUAUCCCACAGAACCAAUAUGCUCUGUCGUUUUGCAAAUUC
AUAAGUAGUCUGCCUGCCUAUUCCAUGCCCAGCUCCAGUAAUGAGAACAA
UCUCCCCAGCCACAGAUUUUCUCCUCUGAGGAAUGAAAAACUUCACCAAC
GACUCCAAGUAGGAGUAGAUGAUGGUGAUCAGAAGCAGAAGGAUUUCUA
GGAUGAUGUUCAUGGCUUUGCUCUGUCCUCUUCCUUCUGGUUCAGUCCUU
GUGU.

### BRIEF DESCRIPTION OF THE DRAWINGS

FIG. 1 shows the inhibitory activity of GalNAc-conjugated siRNAs against murine primary hepatocytes mTTR.
FIG. 2. shows the *in vivo* inhibitory activity of GalNAc-conjugated siRNAs against the murine mTTR gene.

### DETAILED DESCRIPTION

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure belongs. As used herein, in the context of RNA-mediated gene silencing, the sense strand (also referred to as SS or SS strand) of an siRNA refers to a strand comprising a sequence identical or substantially identical to a target mRNA sequence; the antisense strand (also referred to as AS or AS strand) of an siRNA refers to a strand having a sequence complementary to a target mRNA sequence.

The terms "complementary" and "reverse complementary" are used interchangeably and have the meaning well known to those skilled in the art, that is, in a double-stranded nucleic acid molecule, the bases of one strand are paired with the bases of the other strand in a complementary manner. In DNA, the purine base adenine (A) is paired with the pyrimidine base thymine (T) (or uracil (U) in RNA), and the purine base guanine (C) is paired with the pyrimidine base cytosine (G). Each base pair comprises a purine and a pyrimidine. When adenines of one strand are paired with thymines (or uracils) of the other strand and guanines are always paired with cytosines, the two strands are considered complementary to each other, and the sequences of the strands can be deduced from the sequences of their complementary strands. Accordingly, "mismatch" in the art means that in a double-stranded nucleic acid, the bases in the corresponding positions are not paired in a complementary manner.

The term "base" includes any known DNA and RNA bases and base analogs such as purines or pyrimidines, and also include natural compounds adenine, thymine, guanine, cytosine, uracil, and inosine and natural analogs.

The term "base analog" refers to a heterocyclic moiety located at 1'-position of a nucleotide sugar moiety in a modified nucleotide that may be incorporated into a nucleic acid duplex (or the equivalent position of a nucleotide sugar moiety substitution that may be incorporated into a nucleic acid duplex). In the present disclosure, base analogs are generally purine or pyrimidine bases, excluding common bases: guanine (G), cytosine (C), adenine (A), thymine (T), and uracil (U). Non-limiting examples of bases include hypoxanthine (I), xanthine (X), 3β-D-ribofuranosyl-(2,6-diaminopyrimidine) (K), 3-β-D-ribofuranosyl-(1-methyl-pyrazolo[4,3-d]pyrimidine-5,7(4*H*,6*H*)-dione) (P), isocytosine (iso-C), isoguanine (iso-G), 1-β-D-ribofuranosyl-(5-nitroindole), 1-β-D-ribofuranosyl-(3-nitropyrrole), 5-bromouracil, 2-aminopurine, 4-thio-dT, 7-(2-thienyl)-imidazo[4,5-b]pyridine (Ds) and pyrrole-2-carbaldehyde (Pa), 2-amino-6-(2-thienyl)purine (S), 2-oxopyridine (Y), difluorotolyl, 4-fluoro-6-methylbenzimidazole, 4-methylbenzimidazole, 3-methylhydroxyisoquinolyl, 5-methylhydroxyisoquinolyl and 3-methyl-7-propynyl hydroxyisoquinolyl, 7-azaindolyl, 6-methyl-7-azaindolyl, imidazopyridinyl, 9-methyl-imidazopyridinyl, pyrrolopyrazinyl, hydroxyisoquinolyl, 7-propynyl hydroxyisoquinolyl, propynyl-7-azaindolyl, 2,4,5-trimethylphenyl, 4-methylindolyl, 4,6-dimethylindolyl, phenyl, naphthyl, anthracenyl, phenanthrenyl, pyrenyl, stilbenzyl, tetracenyl, and pentacenyl and structural derivatives thereof. Base analogs can also be universal bases.

The term "universal base" refers to a heterocyclic moiety located at 1'-position of a nucleotide sugar moiety in a modified nucleotide, or the equivalent position in a nucleotide sugar moiety substitution, and the heterocyclic moiety, when present in a nucleic acid duplex, can be paired with one or more types of bases without altering the double helical structure (e.g., the structure of the phosphate backbone). In addition, the universal base does not destroy the ability of the single-stranded nucleic acid in which it resides to form a duplex with a target nucleic acid. The ability of a single-stranded nucleic acid containing a universal base to form a duplex with a target nucleic acid can be determined using methods apparent to those skilled in the art (e.g., UV absorbance, circular dichroism, gel shift, single-stranded nuclease sensitivity, etc.). In addition, conditions under which duplex formation is observed can be changed to determine duplex stability or formation, e.g., temperature, such as melting temperature (Tm), related to the stability of nucleic acid duplexes. Compared to a reference single-stranded nucleic acid that is exactly complementary to a target nucleic acid, the single-stranded nucleic acid containing the universal base forms a duplex with the target nucleic acid that has a lower Tm than a duplex formed with the complementary nucleic acid. However, compared to a reference single-stranded nucleic acid in which the universal base has been replaced with a base to generate a single mismatch, the single-stranded nucleic acid containing the universal base forms a duplex with the target nucleic acid that has a higher Tm than a duplex formed with the nucleic acid having the mismatched base.

Some universal bases are capable of base pairing by forming hydrogen bonds between the universal base and all of the bases guanine (G), cytosine (C), adenine (A), thymine (T), and uracil (U) under base pairing conditions. A universal base is not a base that forms a base pair with only one single complementary base. In a duplex, a universal base can form no hydrogen bond, one hydrogen bond, or one or more hydrogen bonds with each of G, C, A, T, and U opposite thereto on the opposite strand of the duplex. In some embodiments, the universal base does not interact with the base opposite thereto on the opposing strand of the duplex. In a duplex, base pairing with a universal base will not alter the double helical structure of the phosphate backbone. A universal base may also interact with bases in adjacent nucleotides on the same nucleic acid strand by stacking interactions. Such stacking interactions can stabilize the duplex, particularly in cases where the universal base does not form any hydrogen bond with the base opposite thereto on the opposite strand of the duplex. Non-limiting examples of universal binding nucleotides include inosine, 1-β-D-ribofuranosyl-5-nitroindole, and/or 1-β-D-ribofuranosyl-3-nitropyrrole.

The terms "blunt end" and "blunt terminus" are used interchangeably to refer to a given end of the siRNA that has no unpaired nucleotides or nucleotide analogs, i.e., no nucleotide overhangs. In most cases, an siRNA with both ends being blunt ends will be double-stranded over its entire length.

In the present disclosure, the "5' region", i.e., "5' end" and "5' terminus" of the sense strand or the antisense strand, are used interchangeably. For example, nucleotides at positions 2 to 8 of the 5' region of the antisense strand may be replaced with nucleotides at positions 2 to 8 of the 5' end of the antisense strand. Similarly, the "3' region", "3' terminus", and "3' end" of the sense or antisense strand are also used interchangeably. In the context of the present disclosure, the term "chemical modification" or "modification" includes all changes made by chemical means, such as the addition or removal of a chemical moiety, or the substitution of one chemical moiety for another. The term "2'-fluoro-modified nucleotide" refers to a nucleotide in which the hydroxy group at 2'-position of the ribosyl group of the nucleotide is substituted with fluorine. "Non-fluorinated modified nucleotide" refers to a nucleotide or a nucleotide analog in which the hydroxy group at 2'-position of the ribosyl group of the nucleotide is substituted with a non-fluorine group. "Nucleotide analog" refers to a group that can replace a nucleotide in a nucleic acid but has a structure different from adenine ribonucleotide, guanine ribonucleotide, cytosine ribonucleotide, uracil ribonucleotide, or thymine deoxyribonucleotide, e.g., an isonucleotide, a bridged nucleic acid (BNA for short), or an acyclic nucleotide. The methoxy-modified nucleotide refers to a nucleotide in which the 2'-hydroxy group of the ribosyl group is substituted with a methoxy group. An isonucleotide refers to a compound formed by changing the position of a base on the ribose ring in a nucleotide. In some embodiments, the isonucleotide may be a compound formed by moving a base from the 1'-position to the 2'-position or 3'-position of the ribose ring. BNA refers to a constrained or inaccessible nucleotide. BNA may contain five-membered, six-membered, or seven-membered ring bridged structure with a "fixed" C3'-endo sugar puckering. The bridge is generally incorporated at the 2'- and 4'-positions of the ribose to afford a 2',4'-BNA nucleotide. In some embodiments, BNA may be LNA, ENA, cET BNA, etc. Acyclic nucleotides are a class of nucleotides in which the sugar ring of the nucleotide is opened. In some embodiments, the acyclic nucleotide may be an unlocked nucleic acid (UNA) or a glycerol nucleic acid (GNA).

The term "inhibit" is used interchangeably with "decrease", "silence", "down-regulate", "repress" and other similar terms, and includes any level of inhibition. Inhibition can be assessed in terms of a decrease in the absolute or relative level of one or more of these variables relative to a control level. The control level may be any type of control level used in the art, such as a pre-dose baseline level or a level determined from an untreated or control (e.g., only buffer control or inert agent control) treated subject, cell, or sample. For example, the residual expression level of mRNA can be used to characterize the degree of inhibition of target gene expression by the siRNA; for example, the residual expression level of mRNA is not greater than 99%, not greater than 95%, not greater than 90%, not greater than 85%, not greater than 80%, not greater than 75%, not greater than 70%, not greater than 65%, not greater than 60%, not greater than 55%, not greater than 50%, not greater than 45%, not greater than 40%, not greater than 35%, not greater than 30%, not greater than 25%, not greater than 20%, not greater than 15%, or not greater than 10%. The inhibition rate of target gene expression can be determined using Dual-Glo^{®} Luciferase Assay System: the Firefly chemiluminescence value (Fir) and the Renilla chemiluminescence value (Ren) are each read, and the relative value Ratio = Ren/Fir and inhibition rate (%) = 1 - (Ratio + siRNA/Ratioreporter only) × 100% are calculated; in the present disclosure, the proportion of residual expression level of mRNA (or residual activity %) = 100% - inhibition (%).

The term "effective amount" or "effective dose" refers to the amount of a drug, a compound or a pharmaceutical composition necessary to obtain any one or more beneficial or desired therapeutic results. For preventive use, the beneficial or desired results include elimination or reduction of risk, reduction of severity, or delay of the onset of a disorder, including the biochemistry, histology and/or behavioral symptoms of the disorder, complications thereof and intermediate pathological phenotypes that appear during the progression of the disorder. For therapeutic applications, the beneficial or desired results include clinical results, such as reducing the incidence rate of various disorders related to the target gene, the target mRNA, or the target protein of the present disclosure or alleviating one or more symptoms of the disorders, reducing the dosage of other agents required to treat the disorders, enhancing the therapeutic effect of another agent, and/or delaying the progression of disorders related to the target gene, the target mRNA, or the target protein of the present disclosure in a patient.

The terms "patient", "subject", and "individual" are used interchangeably and include human or non-human animals, e.g., mammals, e.g., humans or monkeys.

The siRNA provided by the present disclosure can be obtained by a preparation method conventional in the art (e.g., solid-phase synthesis and liquid-phase synthesis). Solid phase synthesis has been commercially available as customization service. A modified nucleotide group can be introduced into the siRNA described herein using a nucleoside monomer with a corresponding modification. Methods of preparing a nucleoside monomer with a corresponding modification and introducing a modified nucleotide group into an siRNA are also well known to those skilled in the art.

The term "pharmaceutical composition" refers to a mixture containing one or more of the compounds or the physiologically pharmaceutically acceptable salts or pro-drugs thereof described herein, and other chemical components, for example, physiologically pharmaceutically acceptable carriers and excipients. The pharmaceutical composition is intended to promote the administration to an organism, which facilitates the absorption of the active ingredient, thereby exerting biological activity.

The term "pharmaceutically acceptable excipient" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent or emulsifier that has been approved by the FDA as acceptable for use in humans or livestock animals.

The term "effective amount" or "therapeutically effective amount" includes an amount sufficient to ameliorate or prevent a symptom or disorder of a medical disorder. An effective amount also refers to an amount sufficient to allow or facilitate diagnosis. The effective amount for a particular subject or veterinary subject may vary depending on the factors such as the disorder to be treated, the general health of the subject, the method and route and dosage of administration, and the severity of side effects. An effective amount may be the maximum dose or administration regimen to avoid significant side effects or toxic effects.

The term "alkyl" refers to a saturated aliphatic hydrocarbon group which is a linear or branched group containing 1 to 20 carbon atoms. In some embodiments, the alkyl is selected from the group consisting of alkyl groups containing 1 to 12 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, and 2,2-diethylhexyl and various branched isomers thereof, and the like. In some embodiments, the alkyl is selected from the group consisting of alkyl groups containing 1 to 6 carbon atoms; non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n-*hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. The alkyl may be substituted or unsubstituted, and when it is substituted, the substituent may be substituted at any accessible connection site, and the substituent, in some embodiments, is selected from the group consisting of one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl, and carboxylate group.

The term "alkoxy" refers to -O-(alkyl) and -O-(unsubstituted cycloalkyl), wherein the alkyl is as defined above. Non-limiting examples of alkoxy include: methoxy, ethoxy, propoxy, butoxy, cyclopropyloxy, cyclobutoxy, cyclopentyloxy, and cyclohexyloxy. The alkoxy may be optionally substituted or unsubstituted, and when it is substituted, the substituent, in some embodiments, is selected from the group consisting of one or more of the following groups independently selected from the group consisting of halogen, deuterium, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl are optionally substituted with one or more groups selected from the group consisting of halogen, deuterium, hydroxy, oxo, nitro, and cyano. Similarly, "alkynyloxy", "alkenyloxy", "cycloalkoxy", "heterocycloalkoxy", and "cycloalkenyloxy" are as defined above for "alkoxy".

The term "alkenyl" refers to a linear or branched non-aromatic hydrocarbon group containing at least one carbon-carbon double bond and having 2-10 carbon atoms. Up to 5 carbon-carbon double bonds may be present in such groups. For example, "C₂-C₆" alkenyl is defined as an alkenyl group having 2-6 carbon atoms. Examples of the alkenyl include, but are not limited to: ethenyl, propenyl, butenyl, and cyclohexenyl. The linear, branched, or cyclic moiety of the alkenyl can contain a double bond and is optionally mono-, di-, tri-, tetra-, or penta-substituted at any position as permitted by normal valency. The term "cycloalkenyl" refers to a monocyclic hydrocarbon group having the specified number of carbon atoms and at least one carbon-carbon double bond.

The term "alkynyl" refers to a linear or branched hydrocarbon group containing 2-10 carbon atoms and containing at least one carbon-carbon triple bond. Up to 5 carbon-carbon triple bonds may be present. Thus, "C₂-C₆ alkynyl" refers to an alkynyl group having 2-6 carbon atoms. Examples of the alkynyl group include, but are not limited to: ethynyl, 2-propynyl, and 2-butynyl. The linear or branched moiety of the alkynyl can contain triple bonds as permitted by normal valency, and is optionally mono-, di-, tri-, tetra-, or penta-substituted at any position as permitted by normal valency.

The term "ketone" refers to any alkyl, alkenyl, alkynyl, cycloalkyl, cycloalkenyl, heterocyclyl, heteroaryl, or aryl group described herein linked through a carbonyl bridge. Examples of the ketone groups include, but are not limited to: alkanoyl (e.g., acetyl, propionyl, butyryl, pentanoyl, and hexanoyl), enoyl (e.g., acryloyl), alkynoyl (e.g., ethynylacyl, propynoyl, butynoyl, pentynoyl, and hexynoyl), aroyl (e.g., benzoyl), and heteroaroyl (e.g., pyrroyl, imidazoloyl, quinolinoyl, and picolinoyl).

The term "alkoxycarbonyl" refers to any alkoxy group defined above linked through a carbonyl bridge (i.e., -C(O)O-alkyl). Examples of the alkoxycarbonyl include, but are not limited to: methoxycarbonyl, ethoxycarbonyl, isopropoxycarbonyl, *n*-propoxycarbonyl, *tert*-butoxycarbonyl, benzyloxycarbonyl, or *n*-pentoxycarbonyl.

The term "aryloxycarbonyl" refers to any aryl group defined above linked through an oxycarbonyl bridge (i.e., -C(O)O-aryl). Examples of the aryloxycarbonyl include, but are not limited to: phenoxycarbonyl and naphthyloxycarbonyl.

The term "heteroaryloxycarbonyl" refers to any heteroaryl group defined above linked through an oxycarbonyl bridge (i.e., -C(O)O-heteroaryl). Examples of the heteroaryloxycarbonyl include, but are not limited to: 2-pyridyloxycarbonyl, 2-oxazolyloxycarbonyl, 4-thiazolyloxycarbonyl, or pyrimidyloxycarbonyl.

The term "cycloalkyl" or "carbocycle" refers to a saturated or partially unsaturated monocyclic or polycyclic cyclic hydrocarbon substituent, and the cycloalkyl ring contains from 3 to 20 carbon atoms. In some embodiments, the cycloalkyl is selected from the group consisting of cycloalkyl groups containing 3 to 7 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, and the like. The polycyclic cycloalkyl includes spiro cycloalkyl, fused cycloalkyl, and bridged cycloalkyl. The cycloalkyl may be substituted or unsubstituted, and when it is substituted, the substituent may be substituted at any accessible connection site; in some embodiments, the substituent is selected from the group consisting of one or more of the following groups independently selected from the group consisting of halogen, deuterium, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl are optionally substituted with one or more groups selected from the group consisting of halogen, deuterium, hydroxy, oxo, nitro, and cyano.

The cycloalkyl ring may be fused to an aryl or heteroaryl ring, wherein the ring attached to the parent structure is cycloalkyl. Non-limiting examples of cycloalkyl ring include indanyl, tetrahydronaphthyl, benzocycloheptyl, etc. The cycloalkyl may be optionally substituted or unsubstituted, and when it is substituted, the substituent, in some embodiments, is selected from the group consisting of one or more of the following groups independently selected from the group consisting of halogen, deuterium, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl are optionally substituted with one or more groups selected from the group consisting of halogen, deuterium, hydroxy, oxo, nitro, and cyano.

The term "heterocycloalkyl", "heterocycle", or "heterocyclyl" refers to a saturated or partially unsaturated monocyclic or polycyclic cyclohydrocarbon substituent containing 3 to 20 ring atoms, wherein one or more of the ring atoms are heteroatoms selected from the group consisting of nitrogen, oxygen, and S(O)m (wherein m is an integer from 0 to 2), excluding a cyclic moiety of -O-O-, -O-S-, or -S-S-, and the remaining ring atoms are carbon atoms. In some embodiments, the heterocycloalkyl is selected from the group consisting of heterocycloalkyl groups containing 3 to 12 ring atoms, of which 1 to 4 are heteroatoms. In some embodiments, the heterocycloalkyl is selected from the group consisting of heterocycloalkyl groups containing 3 to 7 ring atoms. Non-limiting examples of monocyclic heterocycloalkyl include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, and the like. The polycyclic heterocycloalkyl includes spiro heterocyclyl, fused heterocyclyl, and bridged heterocycloalkyl. Non-limiting examples of "heterocycloalkyl" include: 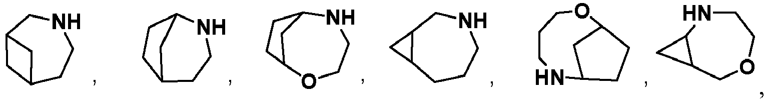 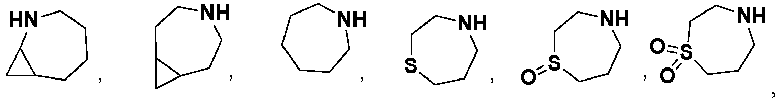 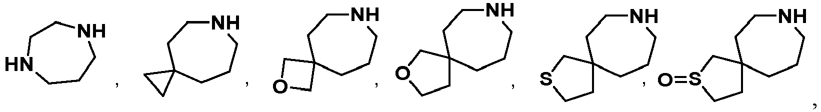 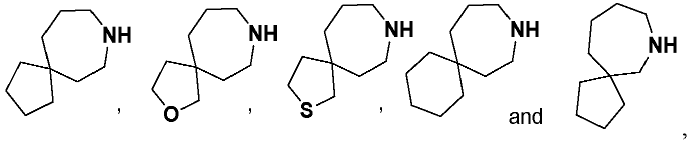 and the like.

The heterocycloalkyl ring may be fused to an aryl or heteroaryl ring, wherein the ring attached to the parent structure is heterocycloalkyl. Non-limiting examples of the heterocycloalkyl ring include, but are not limited to: 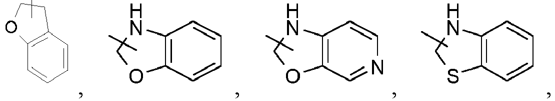 and the like.

The heterocycloalkyl may be optionally substituted or unsubstituted, and when it is substituted, the substituent, in some embodiments, is selected from the group consisting of one or more of the following groups independently selected from the group consisting of halogen, deuterium, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl are optionally substituted with one or more groups selected from the group consisting of halogen, deuterium, hydroxy, oxo, nitro, and cyano.

The term "aryl" refers to a 6- to 14-membered all-carbon monocyclic or fused polycyclic (i.e., rings that share a pair of adjacent carbon atoms) group having a conjugated π-electron system. In some embodiments, the aryl is selected from the group consisting of 6- to 12-membered aryl groups, e.g., phenyl and naphthyl. The aryl ring may be fused to a heteroaryl, heterocycloalkyl or cycloalkyl ring, wherein the ring attached to the parent structure is the aryl ring. Non-limiting examples include, but are not limited to: 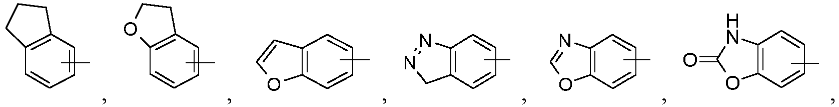 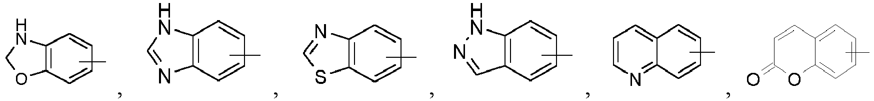 and

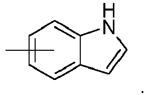

The aryl may be substituted or unsubstituted, and when it is substituted, the substituent, in some embodiments, is selected from the group consisting of one or more of the following groups independently selected from the group consisting of halogen, deuterium, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl are optionally substituted with one or more groups selected from the group consisting of halogen, deuterium, hydroxy, oxo, nitro, and cyano.

The term "heteroaryl" refers to a heteroaromatic system containing 1 to 4 heteroatoms and 5 to 14 ring atoms, wherein the heteroatoms are selected from the group consisting of oxygen, sulfur, and nitrogen. The heteroaryl, in some embodiments, is selected from the group consisting of 6- to 12-membered heteroaryl groups, and in further embodiments, is selected from the group consisting of 5- and 6-membered heteroaryl groups. Non-limiting examples include: imidazolyl, furyl, thienyl, thiazolyl, pyrazolyl, oxazolyl, isoxazolyl, pyrrolyl, tetrazolyl, pyridyl, pyrimidinyl, thiadiazole, pyrazinyl, triazolyl, indazolyl, benzimidazolyl, 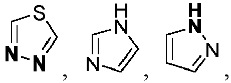 and the like.

The heteroaryl ring may be fused to an aryl, heterocycloalkyl, or cycloalkyl ring, wherein the ring attached to the parent structure is a heteroaryl ring. Non-limiting examples of the heteroaryl ring include:

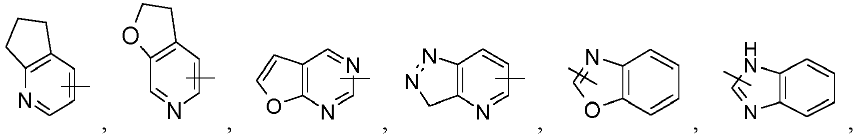

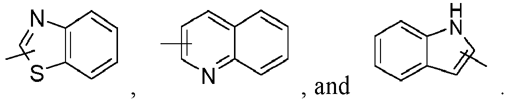

The heteroaryl may be optionally substituted or unsubstituted, and when it is substituted, the substituent, in some embodiments, is selected from the group consisting of one or more of the following groups independently selected from the group consisting of halogen, deuterium, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl are optionally substituted with one or more groups selected from the group consisting of halogen, deuterium, hydroxy, oxo, nitro and cyano.

The term "hydroxy" refers to the -OH group.

The term "halogen" refers to fluorine, chlorine, bromine, or iodine.

The term "haloalkyl" refers to an alkyl group substituted with halogen, wherein the alkyl group is as defined above.

The term "cyano" refers to -CN.

The term "nitro" refers to -NO₂.

The term "oxo" refers to the =O group. For example, a carbon atom is connected to an oxygen atom by a double bond to form a ketone or aldehyde group.

The term "amino" refers to -NH₂.

The term "carboxyl" refers to -C(O)OH.

The term "aldehyde" refers to -CHO.

In the chemical structural formulas of the present disclosure, " 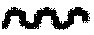 " or 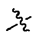 may link any one or more groups according to the scope of the present disclosure described herein; the asterisks "*" represents chiral centers.

In the present disclosure, the term "comprising" may be replaced with "consisting of ...". In the present disclosure, "phosphate group", "phosphoester group", and "phosphoester bond" are used interchangeably and include a phosphomonoester group, a phosphodiester group, or a phosphotriester group. Unless otherwise specified, the natural internucleotide linkage phosphate group is a phosphodiester group.

In the present disclosure, a phosphorothioate group refers to a phosphodiester group modified by replacing one non-bridged oxygen atom with a sulfur atom, and is used interchangeably with 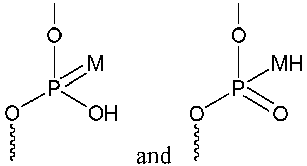 (wherein M is an S atom).

In the context of the present disclosure, the moiety 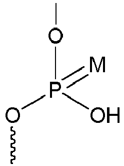 in the group 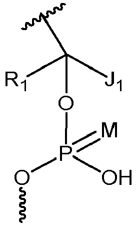 can be replaced with any group capable of linking to an adjacent nucleotide.

The term "link", when referring to a relationship between two molecules, means that the two molecules are linked by a covalent bond or that the two molecules are associated via a non-covalent bond (e.g., a hydrogen bond or an ionic bond).

The term "directly linked" means that a first compound or group is linked to a second compound or group without any atom or group of atoms interposed between.

The term "indirectly linked" means that a first compound or group is linked to a second compound or group by an intermediate group, a compound, or a molecule (e.g., a linking group).

The term "substituted" means that any one or more hydrogen atoms on the designated atom (generally carbon, oxygen, or nitrogen atom) are replaced with any group as defined herein, provided that the normal valency of the designated atom is not exceeded and the substitution results in a stable compound. Non-limiting examples of substituents include C1-C6 alkyl, C2-C6 alkenyl, C2-C6 alkynyl, cyano, hydroxy, oxo, carboxyl, cycloalkyl, cycloalkenyl, heterocyclyl, heteroaryl, aryl, ketone, alkoxycarbonyl, aryloxycarbonyl, heteroaryloxycarbonyl, and halogen (e.g., F, Cl, Br, or I). When the substituent is ketone or oxo (i.e., =O), two (2) hydrogens on the atom are replaced.

"Substituted with one or more ..." means that it may be substituted with a single substituent or multiple substituents. In the case of substitution with multiple substituents, there may be a plurality of identical substituents, or one combination of or a plurality of combinations of different substituents.

Some abbreviations in the present disclosure are defined as follows:
DCE: dichloroethane;
Sc(OTf)₃: scandium trifluoromethanesulfonate;
TFH: tetrahydrofuran;
Pd/C: palladium on carbon;
TFA: trifluoroacetic acid;
DMF: dimethylformamide;
DIPEA: N-ethyldiisopropylamine;
HoBt: 1-hydroxybenzotriazole;
EDCI: 1-ethyl-(3-dimethylaminopropyl)carbonyldiimine hydrochloride;
DMTrCl: 4,4'-dimethoxytrityl chloride;
DIEA: *N*,*N*-diisopropylethylamine;
HATU: *O*-(7-azabenzotriazol-1-yl)-*N*,*N*,*N*',*N*'-tetramethyluronium hexafluorophosphate;
LiOH: lithium hydroxide;
DMAP: 4-dimethylaminopyridine;
HBTU: *O*-(benzotriazol-1-yl)-*N,N,N',N'*-tetramethyluronium hexafluorophosphate;
DMTrCl: 1-[chloro(4-methoxyphenyl)benzyl]-4-methoxybenzene;
CF₃SO₃H: trifluoromethanesulfonic acid;
BnBr: benzyl bromide;
DEPBT: 3-(diethoxyphosphoryloxy)-1,2,3-benzotriazin-4-one;
Bz: a benzoyl protecting group;
MMTr: methoxyphenyl diphenylmethyl; and
DMTr: a dimethoxytrityl protecting group.

### Examples

The present disclosure is further described below with reference to examples, which, however, are not intended to limit the present disclosure. The experimental methods in the examples of the present disclosure without specific conditions indicated are generally performed under conventional conditions such as Antibodies: A Laboratory Manual and Molecular Cloning: A Laboratory Manual by Cold Spring Harbor Laboratory, or under conditions recommended by the manufacturer of the starting materials or the goods. Reagents without specific origins indicated are commercially available conventional reagents.

### I. Preparation and Activity Evaluation of Chemical Modifications of Formula (I)

### Example 1. Preparation of Chemical Modifications

### 1.1 Synthesis of compound 1-1a and compound 1-1b

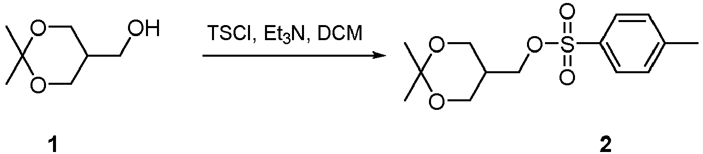

Compound 1 (500 mg, 3.42 mmol) and triethylamine (Et₃N, 692 mg, 6.84 mmol, 0.95 mL) were dissolved in dichloromethane (DCM, 10 mL). A solution of 4-toluenesulfonyl chloride (TsCl, 717 mg, 3.76 mmol) in dichloromethane (10 mL) was added dropwise in an ice bath. After the dropwise addition, the mixture was stirred at room temperature overnight. After the reaction was completed, the reaction mixture was quenched with water. The aqueous phase was extracted three times with dichloromethane (15 mL). The combined organic phase was first washed with a saturated aqueous sodium bicarbonate solution (10 mL) and then with saturated brine (20 mL), and then concentrated under reduced pressure to evaporate the solvent to give a crude product **2** (820 mg, 80%), which was directly used in the next step. MS m/z: C₁₄H₂₁O₅S, [M+H]⁺ calculated: 301.10, found: 301.2.

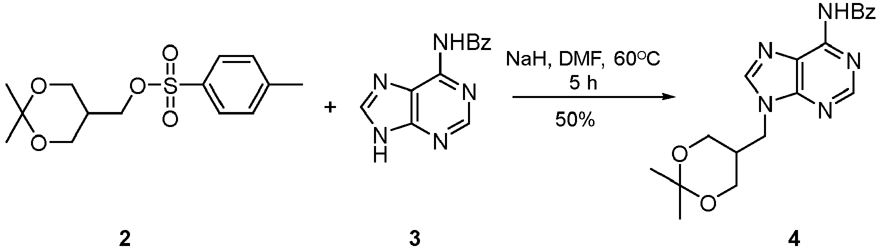

Compound **3** (239 mg, 1.22 mmol) was dissolved in dimethylformamide (DMF, 10 mL). A solution of NaH (60% in mineral oil, 93 mg, 2.33 mmol) was added in an ice bath. The mixture was stirred for 30 min, and then compound **2** (350 mg, 1.16 mmol) was added dropwise. After the dropwise addition, the mixture was stirred at 60 °C for 5 h. After the reaction was completed, the reaction mixture was quenched with water. The aqueous phase was extracted three times with ethyl acetate (15 mL). The combined organic phase was first washed with water (10 mL) three times and then with saturated brine (10 mL), and then concentrated under reduced pressure to evaporate the solvent, purified by reversed-phase preparative HPLC (C¹⁸, conditions: 5%-50% (A: HzO, B: CH₃CN), flow rate: 70 mL/min), and lyophilized to give compound **4** (220 mg). MS m/z: C₁₉H₂₁N₅O₃Na, [M+Na]⁺ calculated: 390.16, found: 390.3.

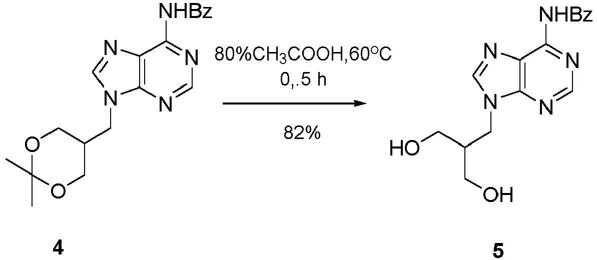

Compound **4** (1.50 g, 4.08 mmol) was dissolved in 20 mL of a mixed solution of acetic acid and water (4:1) at room temperature. The mixture was stirred at 60 °C for 30 min. After the reaction was completed, the reaction mixture was concentrated under reduced pressure to evaporate the solvent, purified by reversed-phase preparative HPLC (C¹⁸, conditions: 5%-25% (A: H₂O, B: CH₃CN), flow rate: 70 mL/min), and lyophilized to give compound 5 (1.10 g). MS m/z: C₁₆H₁₈N₅O₃, [M+H]⁺ calculated: 328.13, found: 328.4.

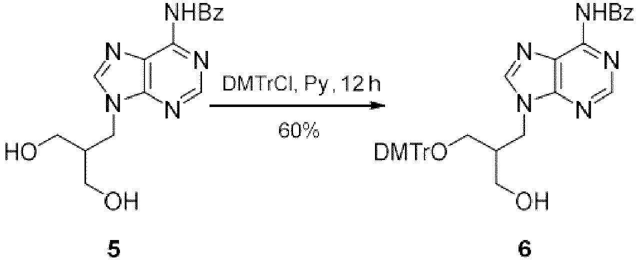

Compound **5** (1.00 g, 3.05 mmol) was dissolved in pyridine (Py, 10 mL). A solution of 4,4'-dimethoxytrityl chloride (DMTrCl, 1.50 g, 4.58 mmol) in pyridine (5 mL) was added dropwise in an ice bath. After the dropwise addition, the mixture was stirred at room temperature overnight. After the reaction was completed, the reaction mixture was quenched with water, concentrated under reduced pressure to evaporate the solvent, purified by reversed-phase preparative HPLC (C¹⁸, conditions: 5%-80% (A: H₂O, B: CH₃CN), flow rate: 70 mL/min), and lyophilized to give compound **6** (1.00 g). MS m/z: C₃₇H₃₆N₅O₅, [M-H]⁺ calculated: 630.26, found: 630.5. The racemate compound **6** was resolved using a chiral column (Daicel CHIRALPAK^{®} IE 250 × 4.6 mm, 5 µm, A: *n-*hexane, B: ethanol) into 6A(-) (410 mg) and 6B(+) (435 mg).

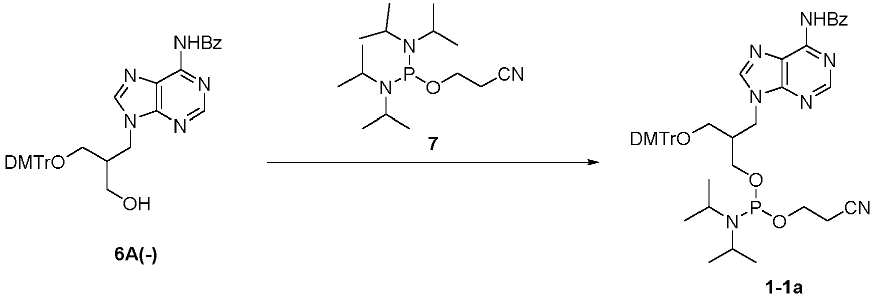

Compound **6A(-)** (200 mg, 0.32 mmol), tetrazole (11 mg, 0.16 mmol), *N*-methylimidazole (5 mg, 0.06 mmol), and 3A molecular sieves (500 mg) were dissolved in 10 mL of acetonitrile. Compound 7 (144 mg, 0.48 mmol) was added at room temperature. The mixture was stirred at room temperature overnight. After the reaction was completed, the molecular sieves were filtered out, and dichloromethane (30 mL) was added. The mixture was washed with a saturated aqueous sodium bicarbonate solution (10 mL) three times and then with saturated brine (20 mL). The filtrate was concentrated to dryness by rotary evaporation, purified by reversed-phase preparative HPLC (C¹⁸, conditions: 5%-100% (A: water, B: CH₃CN), flow rate: 70 mL/min), and lyophilized to give compound **1-1a** (200 mg). MS m/z: C₄₀H₃₉N₆O₇P, [M-diisopropyl + OH]⁺ calculated: 747.26, found: 747.6.

1H NMR (400 MHz, acetonitrile-*d₃*) δ 7.56, 7.54 (2s, 1H), 7.36-7.27 (m, 2H), 7.24-7.21 (m, 7H), 6.83-6.80 (m, 4H), 4.12-4.10 (m, 2H), 3.75-3.68 (m, 10H), 3.20-2.80 (m, 2H), 2.68-2.54 (m, 4H), 1.22-1.04 (m, 18H).

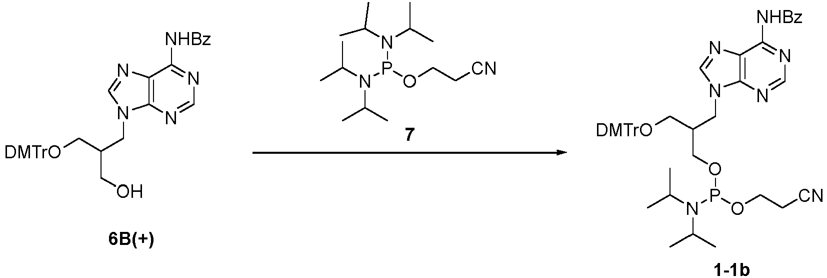

Compound **6B(+)** (200 mg, 0.32 mmol), tetrazole (11 mg, 0.16 mmol), *N-*methylimidazole (5 mg, 0.06 mmol), and 3A molecular sieves (500 mg) were dissolved in 10 mL of acetonitrile. Compound 7 (144 mg, 0.48 mmol) was added at room temperature. The mixture was stirred at room temperature overnight. After the reaction was completed, the molecular sieves were filtered out, and dichloromethane (30 mL) was added. The mixture was washed with a saturated aqueous sodium bicarbonate solution (10 mL) three times and then with saturated brine (20 mL). The filtrate was concentrated to dryness by rotary evaporation, purified by reversed-phase preparative HPLC (C¹⁸, conditions: 5%-100% (A: water, B: CH₃CN), flow rate: 70 mL/min), and lyophilized to give compound **1-1b** (200 mg). MS m/z: C₄₀H₃₉N6O₇P, [M-diisopropyl + OH]⁺ calculated: 747.26, found: 747.5.

### 1.2 Synthesis of compound 1-2

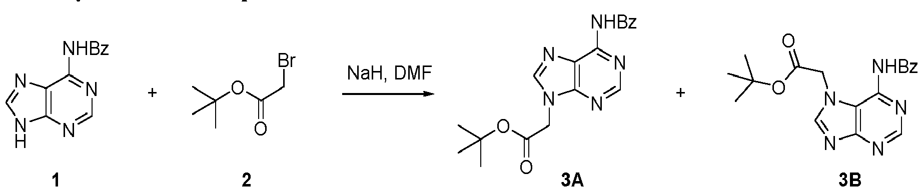

Compound **1** (2 g, 8.36 mmol) was dissolved in DMF (20 mL), and NaH (0.37 g, 9.2 mmol, 60% in mineral oil) was slowly added under argon atmosphere at room temperature. After 2 h of stirring at room temperature, compound **2** (3.3 g, 16.72 mmol) was added to the reaction mixture. After 12 h of stirring at room temperature, the reaction mixture was concentrated. The residue was recrystallized from ethanol (EtOH, 50 mL) to give the target product 3**A** (1.3 g, yield: 44.0%) (dichloromethane:ethyl acetate = 2:1, Rf = 0.2) and the target product **3B** (0.6 g, a mixture of compound 1) (dichloromethane:ethyl acetate = 2:1, Rf = 0.18).

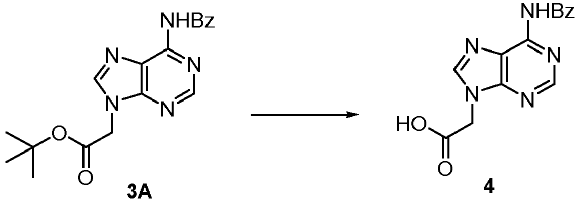

Compound **3A** (1.3 g, 3.68 mmol) was dissolved in a mixture of trifluoroacetic acid (TFA, 4 mL) and DCM (20 mL), and then the mixture was stirred at room temperature for 12 h.

After the reaction mixture was concentrated, the resulting residue was purified using a reversed-phase column (C¹⁸, H₂O + acetonitrile) to give the target product **4** (1 g, yield: 91.44%). MS m/z: C₃₉H₃₈N₆O₆, [M+H]⁺: 687.5.

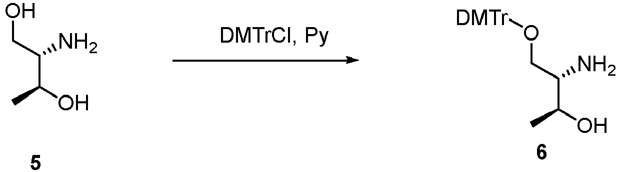

The compound (D-Threoninol **5,** 1.2 g, 11.4 mmol) was dissolved in pyridine (10 mL), and then a solution of DMTrCl (4.64 g, 13.70 mmol) in pyridine (15 mL) was slowly added. After 16 h of stirring at room temperature, the reaction mixture was quenched with H₂O (10 mL) and concentrated. After the reaction mixture was concentrated, the resulting residue was purified using a reversed-phase column (C¹⁸, H₂O + acetonitrile) to give the target product **6** (4.0 g, yield: 86.0%). MS m/z: C₂₅H₂₉NO₄, [M+Na]⁺: 430.4.

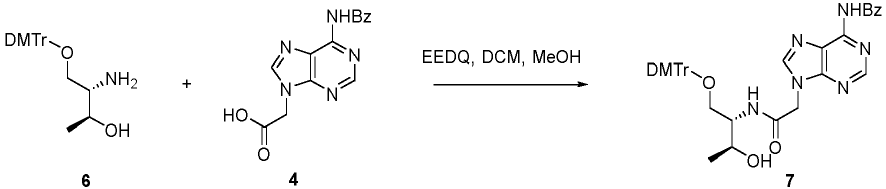

Compound **6** (600 mg, 2.02 mmol), compound **4** (822.5 mg, 2.02 mmol), and dihydroquinoline (EEDQ, 998.2 mg, 4.04 mmol) were dissolved in DCM (10 mL) and methanol (MeOH, 5 mL). After the mixture was stirred at room temperature for 16 h, the solid was filtered out and the filtrate was diluted with DCM (100 mL). The organic phase was washed three times with H₂O (30 mL), dried over anhydrous Na₂SO₄, filtered, and concentrated. The resulting residue was purified using a reversed-phase column (C¹⁸, H₂O + acetonitrile) to give the target product 7 (780 mg, yield: 56.3%). MS m/z: C₃₉H₃₈N₆O₆, [M+H]⁺: 687.5.

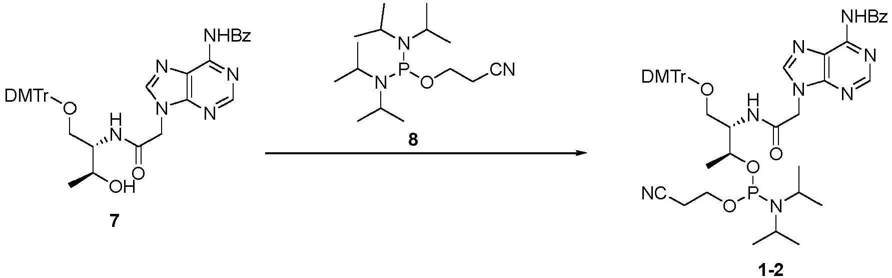

Compound **7** (780 mg, 1.13 mmol), tetrazole (39.8 mg, 0.57 mmol), and *N-*methylimidazole (18.7 mg, 0.23 mmol) were dissolved in CH₃CN (10 mL), and 3A molecular sieves (700 mg) were added. After 5 min of stirring at room temperature under argon atmosphere, compound **8** (513.5 g, 1.70 mmol) was added. After 1 h of stirring at room temperature, the molecular sieves were filtered out, and the solid was rinsed three times with DCM (30 mL). The filtrate was washed sequentially with a saturated aqueous NaHCOs solution (30 mL × 4) and H₂O (30 mL × 4). The organic phase was concentrated at 30 °C. The resulting residue was purified using a reversed-phase column (C¹⁸, H₂O + acetonitrile, acetonitrile 90%) and lyophilized to give the target compound **1-2** (700 mg, yield: 69.5%). MS m/z: C₄₈H₅₅N₈O₇P, [M-cyanoethyl-diisopropyl + OH]⁻: 749.3.

### 1.3 Synthesis of compound 1-3

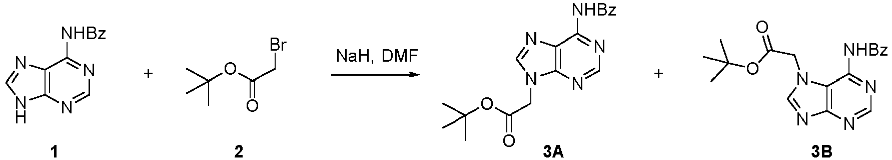

Compound **1** (2 g, 8.36 mmol) was dissolved in DMF (20 mL), and NaH (0.37 g, 9.2 mmol, 60% in mineral oil) was slowly added under argon atmosphere at room temperature. After 2 h of stirring at room temperature, compound **2** (3.3 g, 16.72 mmol) was added to the reaction mixture. After 12 h of stirring at room temperature, the reaction mixture was concentrated. The residue was recrystallized from EtOH (50 mL) to give the target product **3A** (1.3 g, yield: 44.0%) (dichloromethane:ethyl acetate = 2:1, Rf = 0.2) and the target product **3B** (0.6 g, a mixture of compound 1) (dichloromethane:ethyl acetate = 2:1, Rf = 0.18).

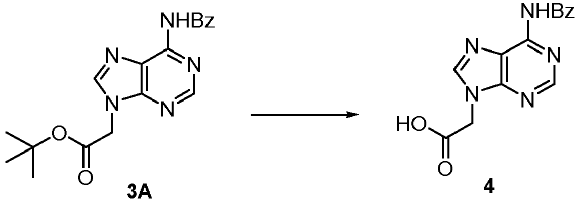

Compound **3A** (1.3 g, 3.68 mmol) was dissolved in a mixture of TFA (4 mL) and DCM (20 mL) and then the mixture was stirred at room temperature for 12 h. After the reaction mixture was concentrated, the resulting residue was purified using a reversed-phase column (C¹⁸, H₂O + acetonitrile) to give the target product **4** (1 g, yield: 91.44%). MS m/z: C₃₉H₃₈N₆O₆, [M+H]⁺: 687.5.

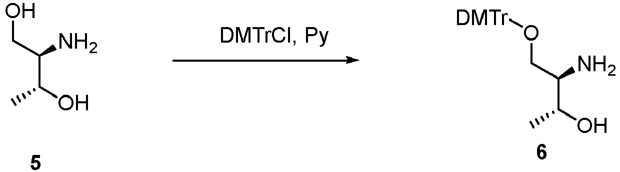

The compound L-Threoninol 5 (1.2 g, 11.4 mmol) was dissolved in pyridine (10 mL), and then a solution of DMTrCl (4.64 g, 13.70 mmol) in pyridine (15 mL) was slowly added. After 16 h of stirring at room temperature, the reaction mixture was quenched with H₂O (10 mL) and concentrated. After the reaction mixture was concentrated, the resulting residue was purified using a reversed-phase column (C¹⁸, H₂O + acetonitrile) to give the target product **6** (4.0 g, yield: 86.0%). MS m/z: C₂₅H₂₉NO₄, [M+Na]⁺: 430.4.

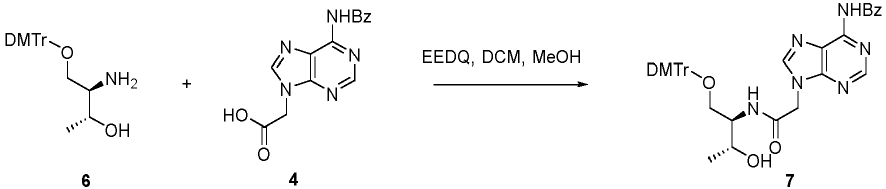

Compound **6** (600 mg, 2.02 mmol), compound **4** (822.5 mg, 2.02 mmol), tetramethyluronium hexafluorophosphate (HATU, 1.15 g, 3.03 mmol), and diisopropylethylamine (DIEA, 1 mL, 6.05 mmol) were dissolved in DMF (10 mL). After 16 h of stirring at room temperature, the reaction mixture was filtered and the filtrate was diluted with DCM (100 mL). The organic phase was washed three times with H₂O (30 mL), dried over anhydrous Na₂SO₄, filtered, and concentrated. The resulting residue was purified using a reversed-phase column (C¹⁸, H₂O + acetonitrile, acetonitrile 60%) and lyophilized to give the target compound 7 (1.0 g, yield: 72.1%). MS m/z: C₃₉H₃₈N₆O₆, [M+H]⁺: 687.5.

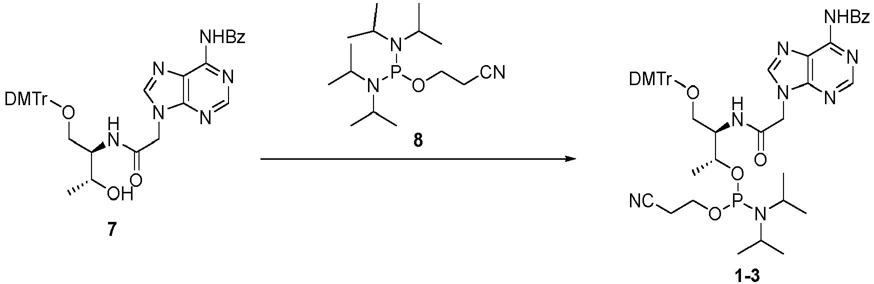

Compound **7** (1.2 g, 1.75 mmol), tetrazole (61.2 mg, 0.87 mmol), and *N*-methylimidazole (28.7 mg, 0.35 mmol) were dissolved in CH₃CN (10 mL), and 3A molecular sieves (700 mg) were added. After 5 min of stirring at room temperature under argon atmosphere, compound **8** (0.79 g, 2.62 mmol) was added. After 1 h of stirring at room temperature, the molecular sieves were filtered out, and the solid was rinsed three times with DCM (30 mL). The filtrate was washed sequentially with a saturated aqueous NaHCOs solution (30 mL × 4) and H₂O (30 mL × 4). The organic phase was concentrated at 30 °C. The resulting residue was purified using a reversed-phase column (C¹⁸, H₂O + acetonitrile, acetonitrile 90%) and lyophilized to give the target compound **1-3** (1.2 g, yield: 77.4%). MS m/z: C₄₈H₅₅N₈O₇P, [M-cyanoethyl-diisopropyl + OH]⁻: 749.3.

### 1.4 Synthesis of compound 1-4a and compound 1-4b

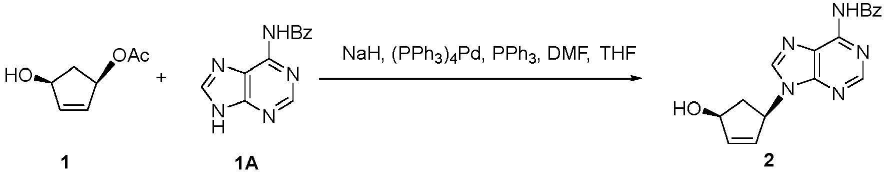

Compound **1A** (6.73 g, 28.14 mmol) was dissolved in dry DMF (80 mL), and NaH (60%, 1.24 g, 30.95 mmol) was slowly added under argon atmosphere. After the mixture was stirred at room temperature for 30 min, the reaction mixture was added to a solution of tetrakis(triphenylphosphine)palladium(0) (Pd(PPh₃)₄, 1.95 g, 1.69 mmol), triphenylphosphine (PPh₃, 0.74 g, 2.81 mmol), and compound 1 (4.0 g, 28.14 mmol) in tetrahydrofuran (THF, 60 mL). After the reaction mixture was stirred at 55 °C for 16 h, the solid was filtered out and washed three times with DCM (60 mL). The filtrate was concentrated. The resulting residue was purified using a normal phase column (elution first with ethyl acetate and then with ethyl acetate: methanol (12:1)) to give the target product **2** (7 g, crude product).

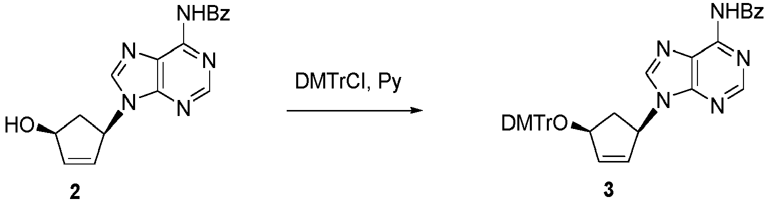

Compound **2** (8 g, crude product) and DMTrCl (12.65 g, 37.34 mmol) were dissolved in pyridine (10 mL). After the mixture was stirred at room temperature for 16 h, the reaction mixture was quenched with water (80 mL) and concentrated. The resulting residue was purified using a reversed-phase column (C¹⁸, water + acetonitrile) and lyophilized to give the target compound **3** (13 g, yield: 83.7%).

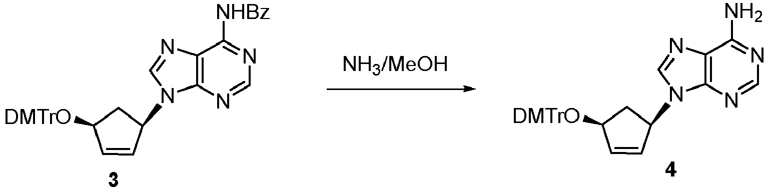

Compound **3** (5 g, 8.02 mmol) was dissolved in methanol (MeOH, 20 mL) and ammonia water (6 mL). After the mixture was stirred at room temperature for 16 h, the reaction mixture was concentrated. The resulting residue was purified using a normal phase column (DCM:MeOH = 20:1) to give the target compound **4** (4 g, yield: 96.0%).

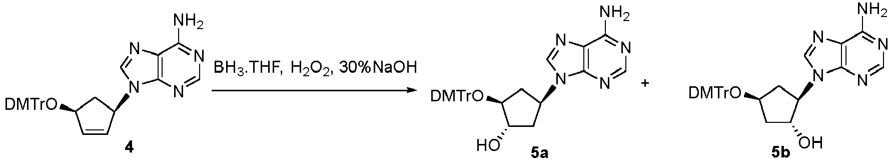

A solution of borane (BH₃) in tetrahydrofuran (1.0 M in THF, 38.54 mL, 38.54 mmol) was added dropwise to a solution of compound **4** (4.00 g, 7.71 mmol) in THF (12 mL) at 0 °C under argon atmosphere. After the compound was stirred at 0 °C under argon atmosphere for 6 h, H₂O (27 mL) was added dropwise. Then, after 3 M aqueous NaOH solution (52 mL, 156 mmol) was added dropwise to the reaction mixture at 0 °C, 30% aqueous H₂O₂ (106 mL) was added dropwise to the reaction mixture, and EtOH (10 mL) was added. After the reaction mixture was stirred at room temperature for 48 h, a saturated Na₂S₂O₃ was added slowly at 0 °C until no bubbles were formed. H₂O (300 mL) was added to the reaction mixture, and the mixture was extracted with DCM (4 × 200 mL). The organic phase was dried over anhydrous Na₂SO₄, filtered, and concentrated. The resulting residue was purified using a reversed-phase column (C¹⁸, acetonitrile + H2O, 50%) and lyophilized to give the target product **5a** (730 mg, yield: 17.6%) and the target product **5b** (1.1 g, 26.6%).

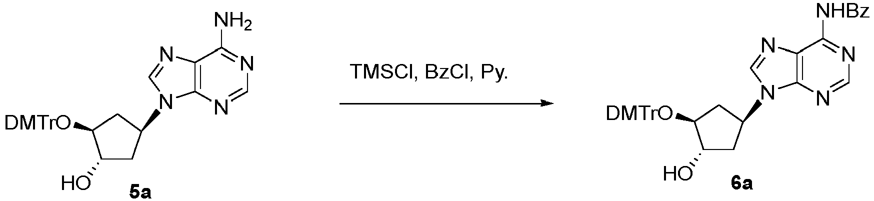

Compound **5a** (730 mg, 1.36 mmol) was dissolved in pyridine (8 mL), and TMSCl (0.67 g, 6.14 mmol) was added at room temperature under argon atmosphere. After 1 h of stirring at room temperature, BzCl (0.29 mL, 2.46 mmol) was added to the reaction mixture. After 16 h of stirring at room temperature, the reaction mixture was quenched with H₂O (10 mL) and concentrated. The resulting residue was dissolved in THF (30 mL), and tetrabutylammonium fluoride (TBAF, 1 mL) was added. After 1 h of stirring at room temperature, ammonia water (0.5 mL) was added. The mixture was stirred at room temperature for 5 h. The reaction mixture was diluted with ethanol (EA, 100 mL) and washed five times with saturated brine (30 mL). The organic phase was concentrated. The resulting residue was purified using a reversed-phase column (C18, H₂O + acetonitrile, acetonitrile 60%) and lyophilized to give the target product **6a** (480 mg, yield: 74.8%). MS m/z: C₃₈H₃₅N₅O₅, [M+H]⁺: 642.6.

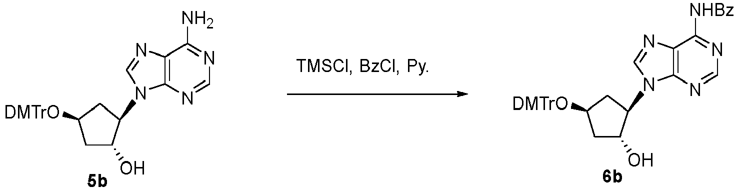

Compound **5b** (1.1 g, 2.05 mmol) was dissolved in pyridine (20 mL), and TMSCl (1.34 g, 1.28 mmol) was added at room temperature under argon atmosphere. After 1 h of stirring at room temperature, benzoyl chloride (BzCl, 0.59 mL, 5.92 mmol) was added to the reaction mixture. After 16 h of stirring at room temperature, the reaction mixture was quenched with H₂O (10 mL) and concentrated. The resulting residue was dissolved in THF (30 mL), and TBAF (2 mL) was added. After 1 h of stirring at room temperature, ammonia water (0.5 mL) was added. The mixture was stirred at room temperature for 5 h. The reaction mixture was diluted with EA (100 mL) and washed five times with saturated brine (30 mL). The organic phase was concentrated. The resulting residue was purified using a reversed-phase column (C18, H₂O + acetonitrile, acetonitrile 60%) and lyophilized to give the target product **6b** (1.4 g, yield: 82.1%). MS m/z: C₃₈H₃₅N₅O₅, [M+H]⁺: 642.5.

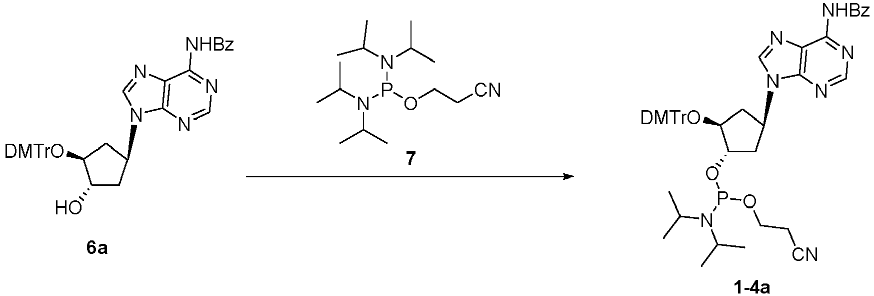

Compound **6a** (700 mg, 1.04 mmol), tetrazole (26.2 mg, 0.37 mmol), and *N-*methylimidazole were dissolved in CH₃CN (10 mL), and 3A molecular sieves (500 mg) were added. After 5 min of stirring at room temperature under argon atmosphere, compound 7 (470.4 mg, 1.56 mmol) was added. After 1 h of stirring at room temperature, the molecular sieves were filtered out, and the solid was rinsed three times with DCM (50 mL). The filtrate was washed sequentially with a saturated aqueous NaHCOs solution (50 mL × 4) and H₂O (50 mL × 4). The organic phase was concentrated at 30 °C. The resulting residue was purified using a reversed-phase column (C¹⁸, H₂O + acetonitrile, acetonitrile 90%) and lyophilized to give the target compound **1-4a** (600 mg, yield: 66.1%). MS m/z: C₄₇H₅₂N₇O₆P, [M-cyanoethyl-diisopropyl + OH]⁻: 704.3.

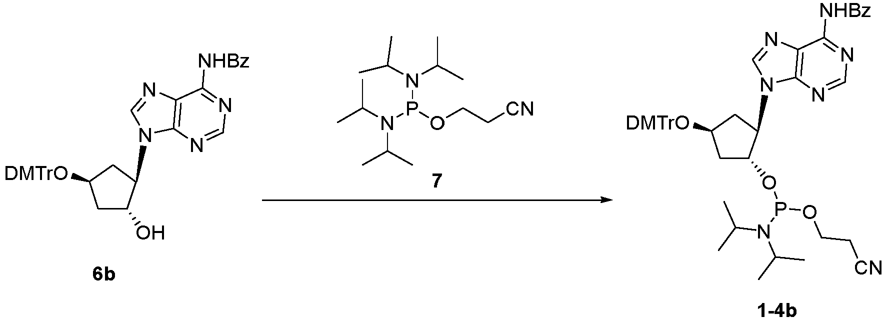

Compound **6b** (1.3 g, 2.03 mmol), tetrazole (71.0 mg, 1.01 mmol), and *N-*methylimidazole (33.3 mg, 0.41 mmol) were dissolved in CH₃CN (20 mL), and 3A molecular sieves (700 mg) were added. After 5 min of stirring at room temperature under argon atmosphere, compound 7 (0.92 g, 3.04 mmol) was added. After 1 h of stirring at room temperature, the molecular sieves were filtered out, and the solid was rinsed three times with DCM (50 mL). The filtrate was washed sequentially with a saturated aqueous NaHCOs solution (50 mL × 4) and H₂O (50 mL × 4). The organic phase was concentrated at 30 °C. The resulting residue was purified using a reversed-phase column (C¹⁸, H₂O + acetonitrile, acetonitrile 90%) and lyophilized to give the target compound **1-4b** (1.4 g, yield: 82.1%). MS m/z: C₄₇H₅₂N₇O₆P, [M-cyanoethyl-diisopropyl]⁻: 704.3.

### 1.5 Synthesis of compound 1-5

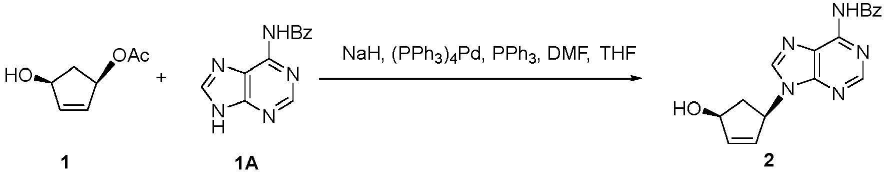

Compound **1A** (6.73 g, 28.14 mmol) was dissolved in dry DMF (80 mL), and NaH (60%, 1.24 g, 30.95 mmol) was slowly added under argon atmosphere. After the mixture was stirred at room temperature for 30 min, the reaction mixture was added to a solution of tetrakis(triphenylphosphine)palladium(0) (Pd(PPh₃)₄, 1.95 g, 1.69 mmol), triphenylphosphine (PPh₃, 0.74 g, 2.81 mmol), and compound **1** (4.0 g, 28.14 mmol) in THF (60 mL). After the reaction mixture was stirred at 55 °C for 16 h, the solid was filtered out and washed three times with DCM (60 mL). The filtrate was concentrated. The resulting residue was purified using a normal phase column (elution first with ethyl acetate and then with ethyl acetate:methanol (12:1)) to give the target solid **2** (7 g, crude product).

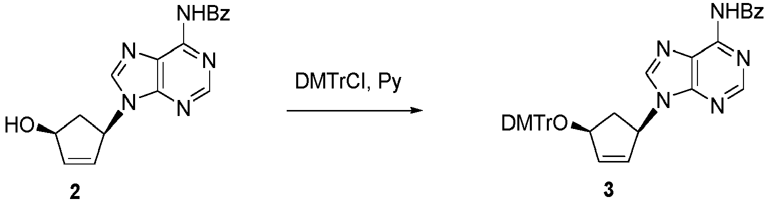

Compound **2** (8 g, crude product) and DMTrCl (12.65 g, 37.34 mmol) were dissolved in pyridine (10 mL). After the mixture was stirred at room temperature for 16 h, the reaction mixture was quenched with water (80 mL) and concentrated. The resulting residue was purified using a reversed-phase column (C¹⁸, water + acetonitrile) and lyophilized to give the target compound **3** (13 g, yield: 83.7%).

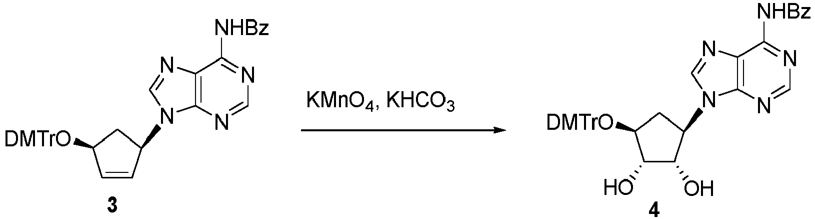

Compound **3** (1 g, 1.60 mmol), KHCO₃ (0.48 g, 4.81 mmol) and ethylene glycol (0.40 g, 6.41 mmol) were dissolved in acetone (50 mL), and KMnO₄ (40% in water, 0.67 g, 1.68 mmol) was slowly added at -30 °C. After 1 h of stirring at -30 °C, the reaction mixture was quenched with a saturated aqueous sodium thiosulfate solution (30 mL). The mixture was extracted four times with DCM (30 mL). The organic phase was dried over anhydrous Na₂SO₄, filtered, and concentrated. The residue was purified using a reversed-phase column (C18, H₂O + acetonitrile, acetonitrile 60%) and lyophilized to give the target product **4** (600 mg, yield: 56.9%). MS m/z: C₃₈H₃₅N₅O₆, [M+H]⁺: 658.5.

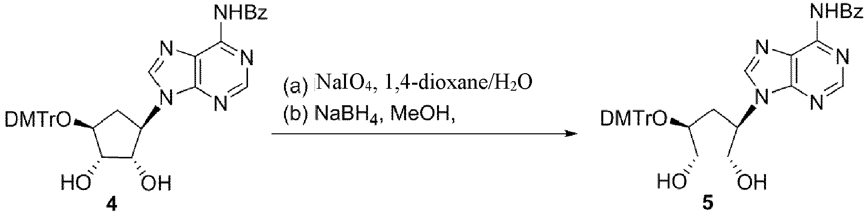

To a 250 mL round-bottom flask were added reactant **4** (5.0 g, 7.601 mmol), NaIO₄, and 1,4-dioxane/water (50 mL/5 mL), and the mixture was reacted at room temperature for 2 h. The reaction mixture was concentrated under reduced pressure to remove the solvent to give a white solid (6.0 g). Then, the solid was dissolved in methanol (50 mL), and sodium borohydride (1.62 g, 38 mmol) was added. After the mixture was stirred at room temperature for 2 h, a 10% ammonium chloride solution (10 mL) was added. The reaction mixture was concentrated under reduced pressure to remove the solvent. The residue was purified by C18 column chromatography (water/acetonitrile: 5%-95%) to give the product P1 as a colorless oil 5 (2.0 g, 3.0315 mmol, 39%), LCMS, MS+, [M+H]+: 660.

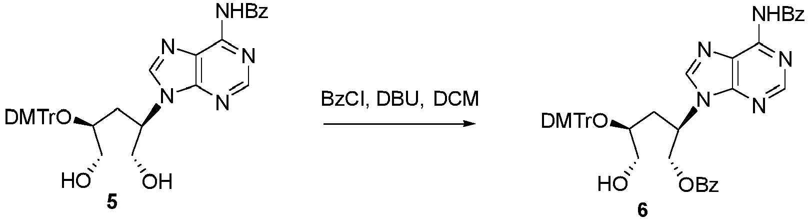

Compound **5** (1.7 g, 2.58 mmol) and DBU (0.77 mL, 5.15 mmol) were dissolved in DCM (20 mL), and BzCl (0.5 M in DCM, 0.8 mL) was added dropwise to the reaction mixture at -70 °C under argon atmosphere. The reaction mixture was left to stand at -70 °C for 1 h and quenched with ethanol (5 mL). The quenched reaction mixture was diluted with DCM (100 mL) and washed three times with water (30 mL). The organic phase was dried over anhydrous Na₂SO₄, filtered, and concentrated. The resulting residue was purified using a normal phase column (DCM:EA = 1:1) to give **6** as a white solid (80 mg, yield: 4.14%). MS m/z: C₄₅H₄₁N₅O₇, [M+H]⁺: 764.5.

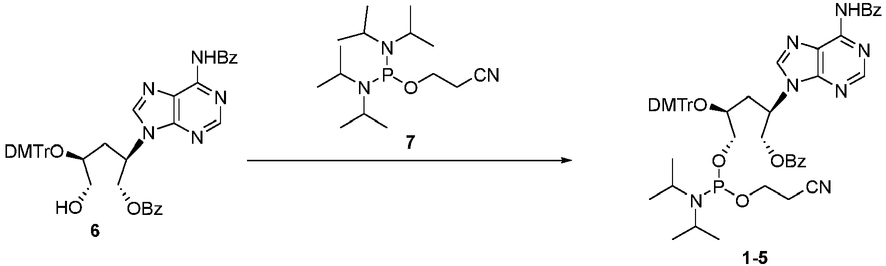

Compound **6** (380 mg, 0.50 mmol), tetrazole (17.43 mg, 0.25 mmol), and *N-*methylimidazole (8.17 mg, 0.10 mmol) were dissolved in CH₃CN (10 mL), and 3A molecular sieves (500 mg) were added. After 5 min of stirring at room temperature under argon atmosphere, compound 7 (224.95 mg, 0.75 mmol) was added. After 1 h of stirring at room temperature, the molecular sieves were filtered out, and the solid was rinsed three times with DCM (50 mL). The filtrate was washed sequentially with a saturated aqueous NaHCOs solution (50 mL × 4) and H₂O (50 mL × 4). The organic phase was concentrated at 30 °C. The resulting residue was purified using a reversed-phase column (C¹⁸, H₂O + acetonitrile, acetonitrile 90%) and lyophilized to give the target product **1-5** (330 mg, yield: 68.8%). MS m/z: C₅₄H₅₈N₇O₈P, [M-cyanoethyl-diisopropyl]⁻: 826.3.

### 1.6 Synthesis of compound 1-6a

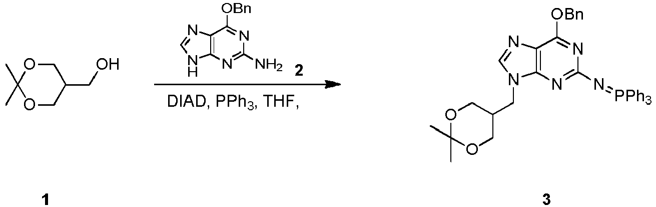

Compound 1 (10 g, 68.404 mmol), compound 2 (15 g, 62.186 mmol), and triphenylphosphine (32.62 g, 124.371 mmol) were dissolved in dry THF (30 mL), and DIAD (24.656 mL, 124.371 mmol) was slowly added dropwise at 0 °C. The mixture was reacted at 25 °C for 12 h. After LCMS showed that the reaction was completed, the reaction mixture was extracted with ethyl acetate (200 mL) and water (200 mL). The organic phase was dried. The filtrate was concentrated. The resulting residue was purified using a normal phase column (DCM/MeOH = 10/1) to give the target product 3 (20 g).

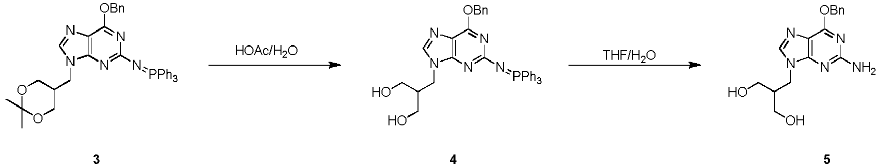

Compound **3** (20 g, 28.585 mmol) was dissolved in acetic acid (24 mL, 426.016 mmol) and H₂O (12 mL), and the mixture was stirred at 60 °C for 1 h. Then, the reaction mixture was concentrated to dryness by rotary evaporation, and THF (12 mL) and H₂O (12 mL) were added. The mixture was stirred at 80 °C for 7 h. After LCMS showed that the reaction was completed, the reaction mixture was extracted with ethyl acetate (200 mL) and water (100 mL). Solid sodium carbonate was added to the aqueous phase until a large amount of solid was precipitated out of the aqueous phase. The solid was collected by filtration and washed with water. The filter cake was dried with an oil pump to give the target compound **5** (9 g).

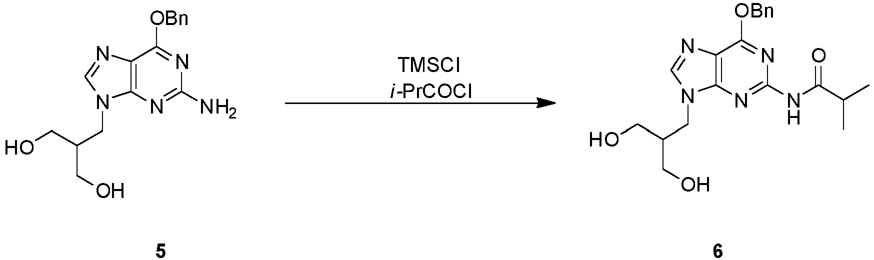

Compound 5 (6.8 g, 18.581 mmol) was dissolved in pyridine (80 mL) under nitrogen atmosphere, and TMSCl (14.250 mL, 111.489 mmol) was slowly added at 0 °C. The mixture was stirred for 2 h. Then, isobutyryl chloride (2.044 mL, 19.511 mmol) was added at 0 °C. The mixture was stirred at 25 °C for 1 h. After LCMS showed that the reaction was completed, the reaction mixture was extracted with dichloromethane (200 mL) and water (200 mL). The organic phase was dried and concentrated to dryness by rotary evaporation, and a sample to be purified was prepared. The sample was purified using a normal phase column (elution with DCM:MeOH = 10:1, peak at 4.8%) to give the target compound 6 (12 g).

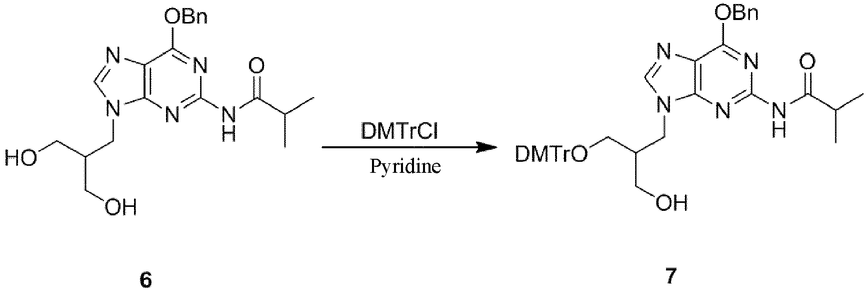

Compound 6 (5.5 g, 12.392 mmol) was dissolved in pyridine (30 mL) under nitrogen atmosphere. MOLECULAR SIEVE 4A 1/16 (7 g, 12.392 mmol) was added, and then solid DMTrCl (5.04 g, 14.870 mmol) was added in batches at 0 °C. The mixture was reacted at 25 °C for 2 h. After TLC (PE:EtOAc = 1: 1, Rf = 0.69) showed that the reaction was completed, the reaction mixture and TJN200879-040-P1 were combined and treated together. The reaction mixture was extracted with ethyl acetate (200 mL) and water (200 mL). The organic phase was dried and concentrated to dryness by rotary evaporation, and a sample to be purified was prepared. The sample was purified using a normal phase column (elution with PE:EtOAc, peak at 84%) to give the target compound 7 (12 g).

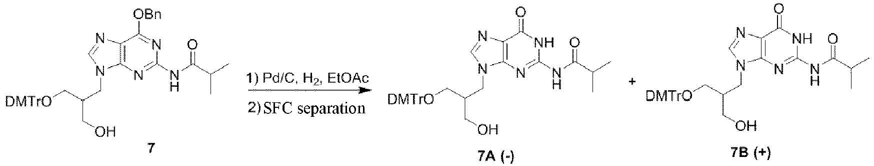

Compound 7 (12 g, 15.389 mmol) was dissolved in EtOAc (140 mL), and wet palladium on carbon Pd/C (7 g, 15.389 mmol) was added. The mixture was reacted at 25 °C for 2 h under hydrogen atmosphere (15 Psi). After TLC (PE:EtOAc = 0: 1, Rf = 0.09) showed that the reaction was completed, the reaction mixture was filtered. The filter cake was rinsed three times with ethyl acetate (30 mL), and the filtrate was collected. The filtrate was concentrated to dryness by rotary evaporation, and 50 mL of dichloromethane and 2 mL of triethylamine were added to prepare a sample to be purified. The sample was purified using a normal phase column (elution with DCM:MeOH = 10: 1, peak at 0.5%) to give 9 g (yellow foamy solid). The resulting racemic compound was separated by SFC to give the target compound 7A(-) (3.9 g) and the target compound 7B(+) (3.8 g).

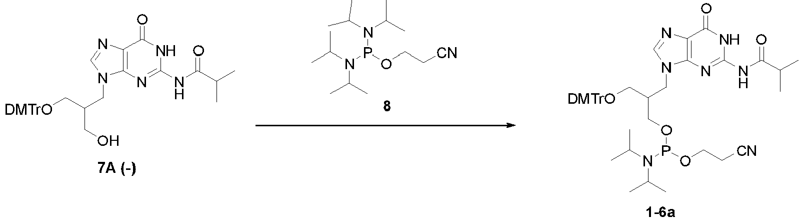

Compound 7A(-) (3.30 g, 5.40 mmol), tetrazole (190 mg, 2.70 mmol), 1-methylimidazole (90 mg, 1.10 mmol), and 3A molecular sieves (500 mg) were dissolved in 30 mL of acetonitrile, and compound 8 (2.50 g, 8.10 mmol) was added at room temperature. The mixture was stirred at room temperature for 2 h. After the reaction was completed, the molecular sieves were filtered out, and DCM (150 mL) was added. The mixture was washed with a saturated aqueous sodium bicarbonate solution (30 mL × 3) and then with saturated brine (30 mL). The filtrate was concentrated to dryness by rotary evaporation, purified by reversed-phase preparative HPLC (C18, conditions: 5%-100% (A: water, B: CH₃CN), flow rate: 70 mL/min), and lyophilized to give compound **1-6a** (2.9 g, 66%). MS m/z: C43H55N7O7P [M+H]+, calculated: 812.38, found: 812.5. 1H NMR (400 MHz, acetonitrile-d3) δ 7.56, 7.54 (2s, 1H), 7.36-7.27 (m, 2H), 7.24-7.21 (m, 7H), 6.83-6.80 (m, 4H), 4.12-4.10 (m, 2H), 3.75-3.68 (m, 10H), 3.20-2.80 (m, 2H), 2.68-2.54 (m, 4H), 1.22-1.04 (m, 18H).

### 1.7 Synthesis of compound 1-7a

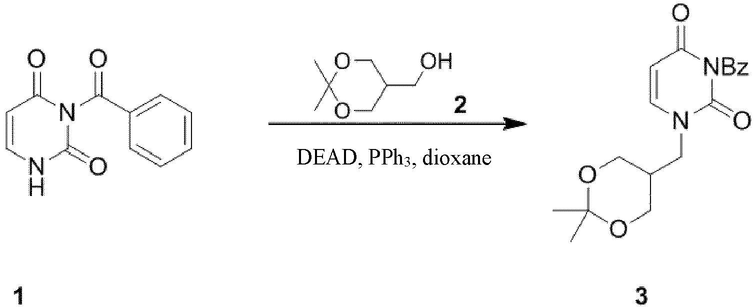

Compound 1 (5 g, 23.1272 mmol), compound 2 (6.76 g, 46.254 mmol), and triphenylphosphine (7.28 g, 27.753 mmol) were dissolved in 30 mL of dioxane under nitrogen atmosphere. DEAD (5.502 mL, 27.753 mmol) was slowly added dropwise at 0 °C. After the dropwise addition, the reaction mixture was slowly heated to 25 °C and reacted for 1 h. The reaction mixture was extracted with 100 mL of H₂O and 100 mL of EtOAc. The organic phases were combined, dried, filtered, and concentrated, and a sample to be purified was prepared. The sample was purified using a normal phase column (elution with PE:EtOAc = 1: 1) to give the target product (4 g).

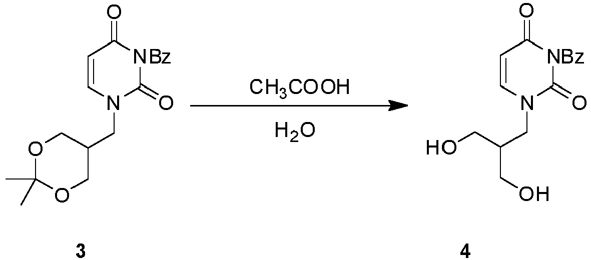

Compound 3 (3.3 g) was dissolved in HOAc (16 mL) and H₂O (4 mL). The mixture was heated at 60 °C for 0.5 h in an oil bath. The reaction mixture was concentrated to dryness by rotary evaporation. The resulting residue was purified using a normal phase column (elution with PE:EtOAc = 0:1) to give the target product 4 (3 g).

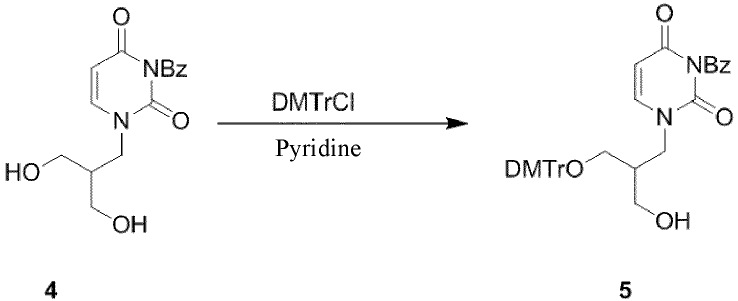

Compound 4 (3 g, 8.873 mmol) was dissolved in 5 mL of pyridine, and a solution of DMTrCl (3.91 g, 11.535 mmol) in 10 mL of pyridine was slowly added dropwise at 0 °C under nitrogen atmosphere. After the dropwise addition, the reaction mixture was heated to 25 °C and reacted for 1 h. The reaction mixture was extracted with 50 mL of water and 100 mL of ethyl acetate. The aqueous phase was extracted three times with 100 mL of ethyl acetate. The organic phases were combined, dried, filtered, concentrated, and purified using a normal phase column (with PE:EtOAc = 2:1) to give the target product 5 (4 g).

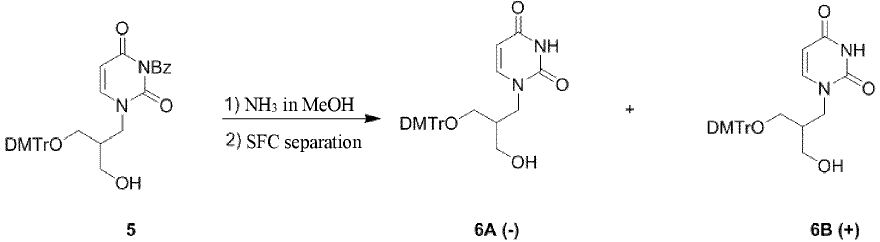

Compound 5 (4 g, 5.769 mmol) was dissolved in methanol (10 mL), and a saturated solution of NH₃ in methanol (40 mL) was added. The mixture was reacted at 0 °C for 6 h. The reaction mixture was concentrated to dryness by rotary evaporation and purified using a normal phase column (PE:EtOAc = 0:1) to give a racemic compound (2.4 g). The compound was separated by SFC to give the target product 6A (750 mg, 100% purity) and the target product 6B (400 mg, 99.16% purity).

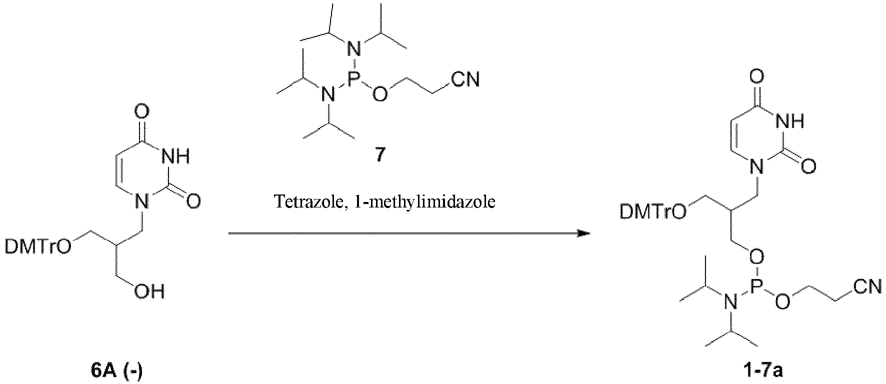

Compound 6A(-) (700 mg, 1.40 mmol), tetrazole (50 mg, 0.70 mmol), 1-methylimidazole (23 mg, 0.28 mmol), and 3A molecular sieves (500 mg) were dissolved in 10 mL of acetonitrile, and compound 7 (630 mg, 2.10 mmol) was added at room temperature. The mixture was stirred at room temperature for 2 h. After the reaction was completed, the molecular sieves were filtered out, and DCM (50 mL) was added. The mixture was washed with a saturated aqueous sodium bicarbonate solution (10 mL × 3) and then with saturated brine (20 mL). The filtrate was concentrated to dryness by rotary evaporation, purified by reversed-phase preparative HPLC (C18, conditions: 5%-100% (A: water, B: CH₃CN), flow rate: 70 mL/min), and lyophilized to give compound **1-7a** (700 mg, 72%). MS m/z: C38H47N4O7PNa [M+Na]+, calculated: 725.32, found: 725.5.

### 1.8 Synthesis of compound 1-8a

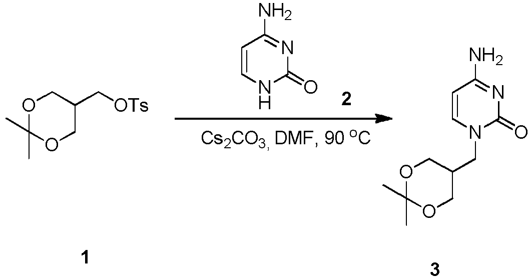

Compound 1 (8.5 g, 76.508 mmol) and compound 2 (30.64 g, 91.809 mmol) were dissolved in DMF (150 mL), and CS₂CO₃ (29.91 g, 91.809 mmol) was added. The mixture was reacted at 90 °C for 12 h under nitrogen atmosphere. After the reaction was completed as detected by LCMS, the reaction mixture was filtered, concentrated to dryness by rotary evaporation with an oil pump, and separated and purified using a normal phase column (80 g, DCM/MeOH = 10/1 to 5/1) to give the target product 3 (13.5 g, 80% purity).

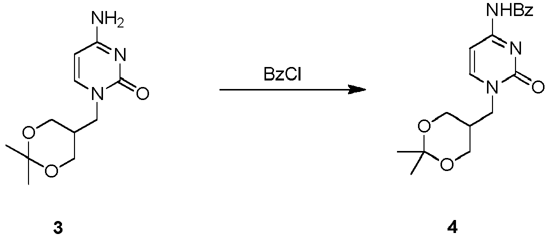

Compound 3 (10.5 g, 35.105 mmol) was dissolved in pyridine (65 mL) and CH₃CN (65 mL), and BzCl (4.894 mL, 42.126 mmol) was added dropwise to the solution. The mixture was reacted at 25 °C for 2 h. After the starting materials were mostly reacted as detected by LCMS, the reaction mixture was quenched with H₂O (100 mL), extracted with EtOAc (100 mL × 3), concentrated to dryness by rotary evaporation, and separated (combined with TJN200872-101) and purified by column chromatography (80 g, PE/EtOAc = 10/1 to 0/1, DCM/MeOH = 10/1) to give the target product **4** (14 g, 90% purity).

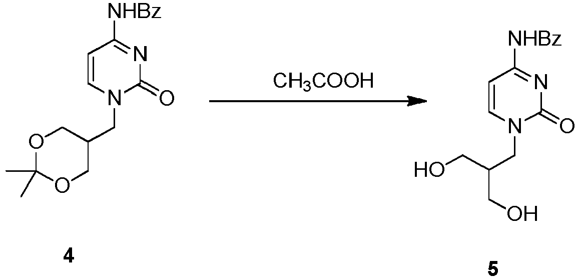

Compound 4 (14 g, 36.694 mmol) was dissolved in HOAc (56 mL, 314.796 mmol) and H₂O (14 mL). The mixture was reacted at 60 °C for 2 h. After LCMS showed that the reaction was completed, the reaction mixture was concentrated with an oil pump and separated using a normal phase column (40 g, DCM/MeOH = 1/0 to 5/1) to give the target product **5** (8.4 g, 90% purity & 2.4 g, 80% purity).

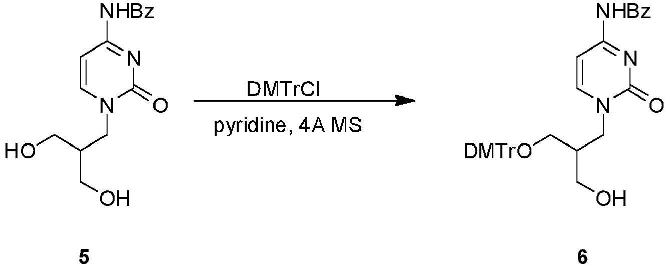

Compound 5 (7.4 g, 21.957 mmol), DMAP (0.54 g, 4.391 mmol), and MOLECULAR SIEVE 4A (11.1 g, 2.967 mmol) were dissolved in pyridine (60 mL). The mixture was stirred for 10 min in an ice bath, and then DMTrCl (8.93 g, 26.348 mmol) was added. The reaction mixture was stirred for 1.8 h. After about 19% of the starting material remained was detected by LCMS, about 60% of target MS was obtained. The mixture was combined with TJN200872-105&106 and purified together. H₂O (50 mL) was added to the reaction mixture. The mixture was extracted with DCM (50 mL × 3), dried, concentrated to dryness by rotary evaporation, and separated by column chromatography (120 g, PE/(EA:DCM:TEA = 1:1:0.05) = 1/0 to 0/1 to DCM/MeOH = 10/1) to give the target product 6 (11 g, 89% purity, TJN200872-105&106&107). The starting material (3.0 g, 70% purity) was recovered.

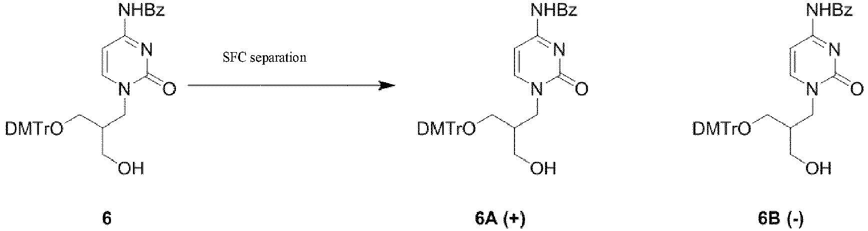

Compound 6 (15 g, 22.041 mmol) was separated by SFC (DAICEL CHIRALPAK AD (250 mm × 50 mm,10 µm); 0.1% NH₃H₂O·EtOH, B: 45%-45%; 200 mL/min) to give the target product 6A (5.33 g, 94.29% purity) and the target product 6B (6.14 g, 97.91% purity), and 1.0 g of compound 6 was recovered.

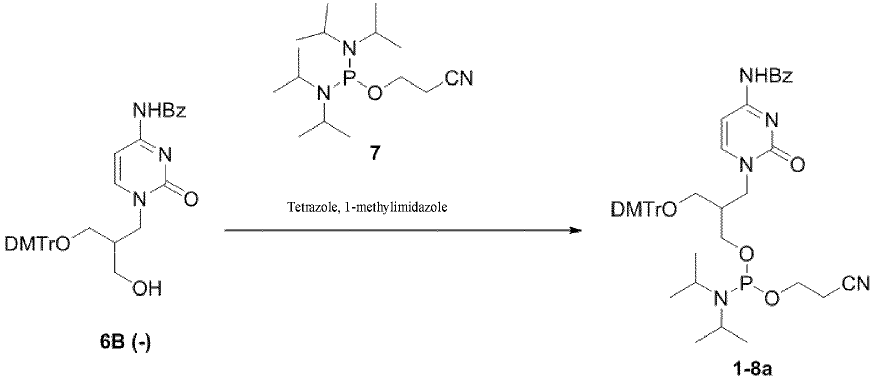

Compound 6B(-) (5.4 g, 8.92 mmol), tetrazole (312 mg, 4.46 mmol), 1-methylimidazole (146 mg, 1.78 mmol), and 3A molecular sieves (500 mg) were dissolved in 40 mL of acetonitrile, and compound 7 (4 g, 13.4 mmol) was added at room temperature. The mixture was stirred at room temperature for 2 h. After the reaction was completed, the molecular sieves were filtered out, and DCM (200 mL) was added. The mixture was washed with a saturated aqueous sodium bicarbonate solution (30 mL × 3) and then with saturated brine (50 mL). The filtrate was concentrated to dryness by rotary evaporation, purified by reversed-phase preparative HPLC (C18, conditions: 5%-100% (A: water, B: CH₃CN), flow rate: 70 mL/min), and lyophilized to give compound **1-8a** (5.8 g, 80%). MS m/z: C45H51N5O7P, [M+H]+, calculated: 804.36, found: 804.4.

### Example 2. Synthesis of siRNAs

The synthesis of siRNAs was the same as the conventional phosphoramidite solid-phase synthesis, except that in the synthesis of a nucleotide with a modification at position 7 of the 5' end of the AS strand, the original nucleotide of the parent sequence was replaced with the phosphoramidite monomer synthesized above.

The synthesis process was briefly described as follows: Nucleoside phosphoramidite monomers were linked one by one according to the synthesis program on a Dr. Oligo48 synthesizer (Biolytic) starting at a Universal CPG support. Other than the phosphoramidite monomer at position 7 of the 5' end of the AS strand described above, the other nucleoside monomer starting materials 2'-F RNA, 2'-O-methyl RNA, and other nucleoside phosphoramidite monomers were purchased from Hongene, Shanghai or Genepharma, Suzhou. 5-Ethylthio-1*H*-tetrazole (ETT) was used as an activator (a 0.6 M solution in acetonitrile), a 0.22 M solution of PADS in acetonitrile and collidine in a 1:1 volume ratio (Kroma, Suzhou) was used as a sulfurizing agent, and an iodopyridine/water solution (Kroma) was used as an oxidant.

After the solid-phase synthesis was completed, oligoribonucleotides were cleaved from the solid support and soaked in a solution of 28% ammonia water and ethanol (3:1) at 50 °C for 16 h. The mixture was centrifuged, and the supernatant was transferred to another centrifuge tube. After the supernatant was concentrated to dryness by evaporation, the residue was purified by C18 reversed-phase chromatography using 0.1 M TEAA and acetonitrile as the mobile phase, and DMTr was removed using a 3% trifluoroacetic acid solution. The target oligonucleotides were collected, lyophilized, identified as the target products by LC-MS, and quantified by UV (260 nm).

The resulting single-stranded oligonucleotides were paired in an equimolar ratio in a complementary manner and annealed. The final double-stranded siRNA was dissolved in 1× PBS, and the solution was adjusted to the concentration required for the experiment for later use.

### Example 3. psiCHECK Activity Screening

Huh7 cells were cultured in a DMEM high-glucose medium containing 10% fetal bovine serum at 37 °C with 5% CO₂. 18 h before transfection, the Huh7 cells were seeded into a 96-well plate at a density of 10,000 cells/well with 100 µL of medium each well. Before transfection, the DMEM high-glucose medium containing 10% fetal bovine serum in the wells was discarded by pipetting and replaced with 80 µL of Opti-MEM for cell starvation for 1.5 h. Then, the cells were co-transfected with siRNA and the corresponding plasmid using Lipofectamine2000 (ThermoFisher, 11668019) according to the instructions. 20 µL of Opti-MEM containing 0.2 µL of Lipofectamine2000, 20 ng of plasmids, and 2.2 µL of siRNA (maximum concentration: 40 nM, 3-fold gradient dilution, 11 concentration points in total, duplicate wells for each concentration) was added to each well of the 96-well plate. After incubation in an incubator at 37 °C for 4 h, a DMEM high-glucose medium containing 20% fetal bovine serum was added. After further culturing for 24 h, the luciferase activity was assayed according to the experimental protocol of the Dual-Glo^{®} Luciferase Assay System (Promega Cat. # E2940) assay kit. The relative value (Ratio = Ren/Fir (renilla/firefly ratio)) and inhibition rate (%) (1 - (Ratio + siRNA/Ratio_{reporter only}) × 100%) were calculated from the detected signals. In the present disclosure, the residual activity % (also referred to as residual expression level of mRNA % or residual expression proportion of mRNA) = 100% - inhibition rate (%). IC₅₀ values were calculated by analysis using Graphpad Prism software (four parameter logistic equations).

### Example 4. On-Target and Off-Target Activity Experiments of siRNAs Comprising Different Chemical Modifications

The siRNAs in Table 1 were synthesized by the method of Example 2 using the compounds of Example 1, and the on-target activity and off-target activity of the siRNAs were verified by the method of Example 3. The siRNAs had identical sense strands and comprised the following modified nucleotides/chemical modifications, respectively, at position 7 of the 5' end of the antisense strand as follows: 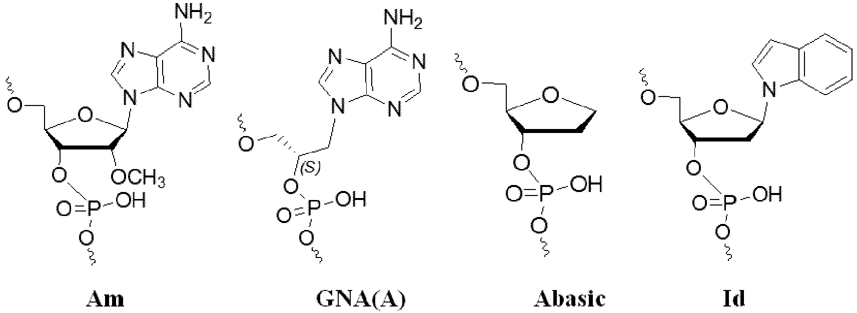 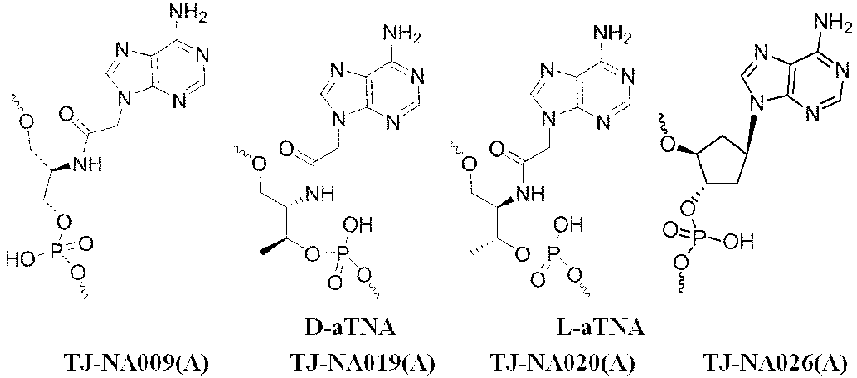 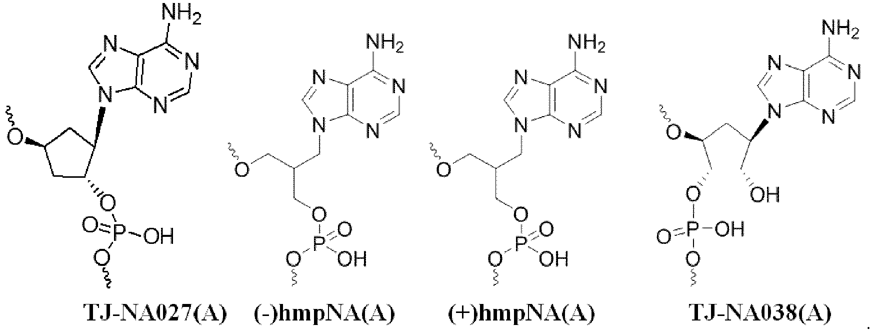 wherein: the nucleotide synthesized using 2-hydroxymethyl-1,3-propanediol as the starting material was defined as hmpNA;
TJ-NA019(A) was obtained by solid-phase synthesis using the nucleoside phosphoramidite monomer 1-2 of example section 1.1;
TJ-NA020(A) was obtained by solid-phase synthesis using the nucleoside phosphoramidite monomer 1-3 of example section 1.1;
TJ-NA026(A) was obtained by solid-phase synthesis using the nucleoside phosphoramidite monomer 1-4a of example section 1.1;
TJ-NA027(A) was obtained by solid-phase synthesis using the nucleoside phosphoramidite monomer 1-4b of example section 1.1;
(+)hmpNA(A) was obtained by solid-phase synthesis using the nucleoside phosphoramidite monomer 1-1b of example section 1.1;
(-)hmpNA(A) was obtained by solid-phase synthesis using the nucleoside phosphoramidite monomer 1-1a of example section 1.1;
TJ-NA038(A) was obtained by solid-phase synthesis using the nucleoside phosphoramidite monomer 1-5 of example section 1.1;
(+)hmpNA(A) was obtained by solid-phase synthesis using the nucleoside phosphoramidite monomer 1-1b of example section 1.1, and its absolute configuration was (S)-hmpNA(A);
(-)hmpNA(A) was obtained by solid-phase synthesis using the nucleoside phosphoramidite monomer 1-1a of example section 1.1, and its absolute configuration was (R)-hmpNA(A).

Similarly, the following structures were obtained by solid-phase synthesis by changing the base species of hmpNA, and their absolute configurations were determined as follows:
(+)hmpNA(G), with the absolute configuration of (S)-hmpNA(G);
(-)hmpNA(G), with the absolute configuration of (R)-hmpNA(G);
(+)hmpNA(C), with the absolute configuration of (S)-hmpNA(C);
(-)hmpNA(C), with the absolute configuration of (R)-hmpNA(C);
(+)hmpNA(U), with the absolute configuration of (R)-hmpNA(U); and
(-)hmpNA(U), with the absolute configuration of (S)-hmpNA(U).

The absolute configurations (S)-hmpNA(G), (R)-hmpNA(G), (S)-hmpNA(C), (R)-hmpNA(C), (S)-hmpNA(U), and (R)-hmpNA(U) are determined from their intermediates or derivatives by X-Ray diffraction.

The structures of the intermediates or derivatives were as follows:

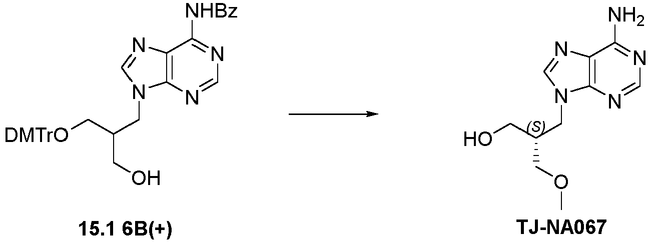

**TJ-NA067:** determined as a colorless massive crystal (0.30 × 0.10 × 0.04 mm³), belonging to the monoclinic crystal system with a P21 space group. Lattice parameter a = 16.0496(5) Å, b = 4.86260(10) Å, c = 16.4686(5) Å, *α* = 90°, *β* = 118.015(4)°, *γ* = 90°, V = 1134.65(7) Å3, Z = 4. Calculated density Dc = 1.389 g/cm³; the number of electrons in a unit cell F(000) = 504.0; linear absorption coefficient of a unit cell *µ* (Cu Kα) = 0.840 mm⁻¹; diffraction experiment temperature T = 150.00(11) K.

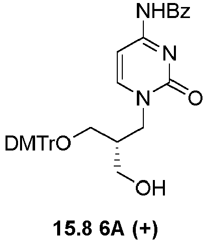

6A(+): determined as a colorless massive crystal (0.30 × 0.20 × 0.10 mm³), belonging to the monoclinic crystal system with a P21 space group. Lattice parameter a = 22.6688(7)Å, b = 8.5595(2) Å, c = 23.3578(5) Å, *α* = 90°, *β* = 113.876(3) °, *γ* = 90 °, V = 4144.3(2) Å3, Z = 2. Calculated density Dc = 0.999 g/cm³; the number of electrons in a unit cell F(000) = 1318.0; linear absorption coefficient of a unit cell *µ* (Cu Kα) = 0.570 mm⁻¹; diffraction experiment temperature T = 100.01(18) K.

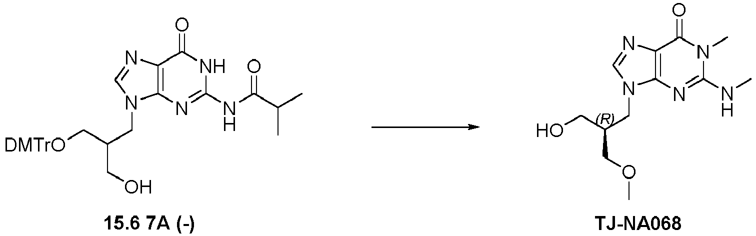

**TJ-NA048:** determined as a colorless acicular crystal (0.30 × 0.04 × 0.04 mm³), belonging to the monoclinic crystal system with a P1 space group. Lattice parameter a = 7.6165(4)Å, b = 11.3423(5)Å, c = 17.3991(8) Å, *α* = 85.007(4)°, *β* = 88.052 (4)°, *γ* = 70.532 (4)°, V = 1411.75(12) Å3, Z = 2. Calculated density Dc = 1.366 g/cm³; the number of electrons in a unit cell F(000) = 620.0; linear absorption coefficient of a unit cell *µ* (Cu Kα) = 0.856mm⁻¹; diffraction experiment temperature T = 150.00(13) K.

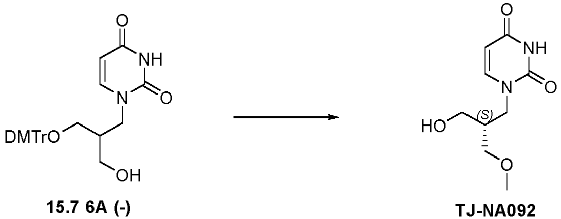

**TJ-NA092:** determined as a colorless prismatic crystal (0.30 × 0.10 × 0.10 mm³), belonging to the triclinic crystal system with a P1 space group. Lattice parameter a = 5.17960(10) Å, b = 8.0667(2) Å, c = 12.4077(2) Å, *α* = 93.146(2)°, *β* = 101.266(2)°, *γ* = 96.134(2)°, V = 503.993(18) Å3, Z = 2. Calculated density Dc = 1.412 g/cm³; the number of electrons in a unit cell F(000) = 228.0; linear absorption coefficient of a unit cell *µ* (Cu Kα) = 0.945 mm⁻¹; diffraction experiment temperature T = 100.00(10) K.

**Table 1. HBV-S-targeting siRNA sequences and modifications**

| | SEQ ID NO: | SS strand 5'-3' |
|---|---|---|
| | 213 | UmsGmsAmCmAfAmGfAfAfUmCmCmUmCmAmCmAmAmUm |
| Double strand code | | AS strand 5'-3' |
| TRD4389 Parent sequence | 214 | AmsUfsUmGmUmGfAmGmGmAmUmUmCmUfUmGfUmCmAmsAms Cm |
| TRD5252 | 215 | AmsUfsUmGmUmGfGNA(A)GmGmAmUmUmCmUfUmGfUmCmAm sAmsCm |
| TRD5812 | 216 | AmsUfsUmGmUmGf**Abasic**GmGmAmUmUmCmUfUmGfUmCmAms AmsCm |
| TRD5813 | 217 | AmsUfsUmGmUmGf**Id**GmGmAmUmUmCmUfUmGfUmCmAmsAmsC m |
| TRD5816 | 218 | AmsUfsUmGmUmGfTJ- NA009(A)GmGmAmUmUmCmUfUmGfUmCmAmsAmsCm |
| TRD5817 | 219 | AmsUfsUmGmUmGf**TJ-** **NA019(A)**GmGmAmUmUmCmUfUmGfUmCmAmsAmsCm |
| TRD5818 | 220 | AmsUfsUmGmUmGfTJ- NA020(A)GmGmAmUmUmCmUfUmGfUmCmAmsAmsCm |
| TRD5821 | 221 | AmsUfsUmGmUmGfTJ- NA027(A)GmGmAmUmUmCmUfUmGfUmCmAmsAmsCm |
| TRD5822 | 222 | AmsUfsUmGmUmGf(+)hmpNA(A)GmGmAmUmUmCmUfUmGfUmC mAmsAmsCm |
| TRD5823 | 223 | AmsUfsUmGmUmGf(-)hmpNA(A)GmGmAmUmUmCmUfUmGfUmC mAmsAmsCm |
| TRD5825 | 224 | AmsUfsUmGmUmGf**TJ-** **NA038(A)**GmGmAmUmUmCmUfUmGfUmCmAmsAmsCm |

The experimental results for on-target activity are shown in Table 2, and the experimental results for off-target activity are shown in Table 3. The test sequences with the compounds of the current experiment all showed activity comparable to or slightly better than that of the parent sequence, indicating that the modifications did not affect on-target activity. The siRNAs comprising GNA/Abasic/Id, TJ-NA019(A), TJ-NA020(A), (+)hmpNA(A), and (-)hmpNA(A) had the best activity. In addition, the parent sequence had significant off-target activity, and all the modifications showed significant inhibitory effects against off-target activity. Particularly, in the siRNAs comprising TJ-NA027(A), (+)hmpNA(A), and (-)hmpNA(A), no off-target activity was observed.

**Table 2. Results for on-target activity ofHBV-S-targeting siRNAs**

| Double strand code | Percentage of residual expression of target gene's mRNA (on-target activity) (mean) | | | | | | | | | | | |
|---|---|---|---|---|---|---|---|---|---|---|---|---|
| | 40nM | 13.3 nM | 4.44 nM | 1.48 nM | 0.493 nM | 0.16 4nM | 0.05 4nM | 0.018 2nM | 0.006 09nM | 0.002 03nM | 0.000 67nM | IC50 (nM) |
| TRD 4389 | 5.4% | 4.1% | 4.8% | 4.8% | 8.4% | 21.7% | 53.0% | 82.5% | 104.9% | 99.4% | 95.2% | 0.0589 |
| TRD 5252 | 3.4% | 3.1% | 3.1% | 3.6% | 5.7% | 11.1% | 22.1% | 44.7% | 72.8% | 92.2% | 86.6% | 0.0162 |
| TRD 5812 | 3.8% | 3.0% | 3.4% | 3.6% | 7.3% | 9.8% | 23.5% | 44.8% | 63.9% | 90.4% | 81.4% | 0.0158 |
| TRD 5813 | 5.1% | 3.8% | 4.4% | 4.3% | 6.1% | 13.2% | 33.5% | 53.8% | 74.5% | 80.8% | 96.4% | 0.0214 |
| TRD 5816 | 3.9% | 3.8% | 3.4% | 4.9% | 6.9% | 16.1% | 39.8% | 71.8% | 96.3% | 92.9% | 108.1% | 0.0389 |
| TRD 5817 | 4.8% | 4.2% | 4.5% | 3.7% | 6.6% | 13.7% | 31.0% | 61.0% | 81.8% | 92.9% | 103.7% | 0.0251 |
| TRD 5818 | 3.7% | 3.3% | 3.1% | 3.7% | 6.1% | 10.9% | 26.3% | 55.8% | 69.1% | 87.4% | 88.8% | 0.0195 |
| TRD 5821 | 6.8% | 5.2% | 5.7% | 6.1% | 8.7% | 19.7% | 39.3% | 69.9% | 102.8% | 92.9% | 97.9% | 0.0398 |
| TRD 5822 | 4.4% | 4.5% | 4.1% | 3.7% | 5.3% | 13.2% | 24.6% | 51.2% | 82.3% | 84.9% | 101.9% | 0.0200 |
| TRD 5823 | 3.6% | 3.8% | 3.4% | 3.4% | 5.2% | 11.0% | 29.7% | 58.3% | 71.4% | 84.7% | 100.7% | 0.0200 |
| TRD 5825 | 4.3% | 3.6% | 3.4% | 4.2% | 7.1% | 18.0% | 32.7% | 66.0% | 88.7% | 93.8% | 103.2% | 0.0302 |

**Table 3. Results for off-target activity of HBV-S-targeting siRNAs**

| Double strand code | Percentage of residual expression of target gene's mRNA (off-target activity) (mean) | | | | | | | | | | |
|---|---|---|---|---|---|---|---|---|---|---|---|
| | 40nM | 13.3 nM | 4.44 nM | 1.48 nM | 0.493 nM | 0.164 nM | 0.054 nM | 0.0182 nM | 0.006 09nM | 0.002 03nM | 0.000 67nM |
| TRD 4389 | 57.4% | 55.9% | 65.5% | 73.3% | 89.2% | 92.8% | 105.3% | 102.4% | 107.6% | 96.0% | 101.2% |
| TRD 5252 | 97.3% | 100.4% | 104.0% | 108.1% | 107.3% | 102.9% | 108.7% | 94.9% | 101.2% | 101.8% | 97.7% |
| TRD 5812 | 98.2% | 107.0% | 99.1% | 100.7% | 110.1% | 125.2% | 113.7% | 105.3% | 105.5% | 99.5% | 93.8% |
| TRD 5813 | 100.5% | 105.2% | 95.9% | 112.1% | 102.3% | 104.3% | 101.5% | 97.2% | 110.7% | 100.6% | 93.6% |
| TRD 5816 | 108.3% | 101.5% | 97.2% | 109.5% | 116.7% | 122.8% | 108.5% | 113.2% | 121.6% | 112.9% | 106.8% |
| TRD 5817 | 104.5% | 106.7% | 110.0% | 109.3% | 119.4% | 120.9% | 127.3% | 113.6% | 117.7% | 112.2% | 105.0% |
| TRD 5818 | 83.7% | 89.7% | 83.0% | 91.0% | 117.5% | 79.4% | 99.1% | 103.4% | 89.2% | 92.9% | 98.7% |
| TRD 5821 | 102.9% | 99.3% | 98.3% | 99.6% | 106.8% | 106.4% | 108.7% | 108.1% | 104.5% | 95.4% | 107.8% |
| TRD 5822 | 106.1% | 93.8% | 81.6% | 100.4% | 100.4% | 96.9% | 105.3% | 101.9% | 94.6% | 101.4% | 94.0% |
| TRD 5823 | 91.8% | 89.1% | 92.9% | 99.8% | 97.8% | 101.1% | 90.7% | 92.6% | 97.9% | 95.9% | 87.1% |
| TRD 5825 | 84.9% | 89.7% | 97.7% | 106.7% | 103.9% | 104.7% | 100.0% | 100.9% | 90.2% | 112.7% | 98.3% |

### Example 5. Sequence-Dependence Experiment of siRNAs Comprising Different Chemical Modifications

The Abasic modification is known to be siRNA sequence-dependent, so the inventors tested the experimental compounds of the present disclosure on multiple different sequences. siRNAs targeting mRNAs of different genes (HBV-S and HBV-X) (their sequences are shown in Table 4) were used and modified at position 7 of the 5' end of the AS strand with the compounds of Example 1 (the sequences are shown in Table 5): TJ-NA020(A), TJ-NA027(A), (+)hmpNA(A), (-)hmpNA(A), GNA(A) (as a control), and Id compound, and then were compared to the parent sequence with respect of on-target activity and off-target activity.

**Table 4. Sequences of siRNAs targeting different genes**

| siRNA target gene | SS strand 5'-3' | AS strand 5'-3' |
|---|---|---|
| HBV-S (siRNA2) | CmsCmsAmUmUfUmGfUfUfCm AmGmUmGmGmUmUmCmsGm (SEQ ID NO: 225) | UmsGfsAmAmCmCfAmCmUmGmAmA mCmAfAmAfUmGmGmsCmsAm(SEQ ID NO: 226) |
| HBV-X (siRNA3) | CmsAmsCmCmUfCmUfGfCfAm CmGmUmCmGmCmAmUmsGm (SEQ ID NO: 227) | UmsAfsUmGfCmGfAmCmGfUmGmCf AmGfAmGfGmUfGmsAmsAm(SEQ ID NO: 228) |

**Table 5. Sequences of siRNAs targeting different genes and comprising chemical modifications**

| Target mRNA | siRNA | AS strand modification |
|---|---|---|
| HBV-S | TRD5847 | UmsGfsAmAmCmCfAmCmUmGmAmAmCmAfAmAfUmGmGmsCmsA m (SEQ ID NO: 229) |
| | TRD5848 | UmsGfsAmAmCmCfGNA(A)CmUmGmAmAmCmAfAmAfUmGmGms CmsAm (SEQ ID NO: 230) |
| | TRD5849 | UmsGfsAmAmCmCfIdCmUmGmAmAmCmAfAmAfUmGmGmsCmsA m (SEQ ID NO: 231) |
| | TRD5850 | UmsGfsAmAmCmCfTJ- 020(A)CmUmGmAmAmCmAfAmAfUmGmGmsCmsAm (SEQ ID NO: 232) |
| | TRD5851 | UmsGfsAmAmCmCfTJ- 027(A)CmUmGmAmAmCmAfAmAfUmGmGmsCmsAm (SEQ ID NO: 233) |
| | TRD5852 | UmsGfsAmAmCmCf(+)hmpNA(A)CmUmGmAmAmCmAfAmAfUmGm GmsCmsAm (SEQ ID NO: 234) |
| | TRD5853 | UmsGfsAmAmCmCf(-)hmpNA(A)CmUmGmAmAmCmAfAmAfUmGm GmsCmsAm (SEQ ID NO: 235) |
| HBV-X | TRD5854 | UmsAfsUmGfCmGfAmCmGfUmGmCfAmGfAmGfGmUfGmsAmsAm (SEQ ID NO: 236) |
| | TRD5855 | UmsAfsUmGfCmGfGNA(A)CmGfUmGmCfAmGfAmGfGmUfGmsAms Am (SEQ ID NO: 237) |
| | TRD5856 | UmsAfsUmGfCmGfIdCmGfUmGmCfAmGfAmGfGmUfGmsAmsAm (SEQ ID NO: 238) |
| | TRD5857 | UmsAfsUmGfCmGfTJ- 020(A)CmGfUmGmCfAmGfAmGfGmUfGmsAmsAm (SEQ ID NO: 239) |
| | TRD5858 | UmsAfsUmGfCmGfTJ- 027(A)CmGfUmGmCfAmGfAmGfGmUfGmsAmsAm (SEQ ID NO: 240) |
| | TRD5859 | UmsAfsUmGfCmGf(+)hmpNA(A)CmGfUmGmCfAmGfAmGfGmUfGm sAmsAm (SEQ ID NO: 241) |
| | TRD5860 | UmsAfsUmGfCmGf(-)hmpNA(A)CmGfUmGmCfAmGfAmGfGmUfGms AmsAm (SEQ ID NO: 242) |

The experimental results for the on-target activity are shown in Table 6. GNA**(A)** showed significant sequence dependence, and different sequences had significantly different on-target activity. The experimental compounds of the present disclosure did not show significant sequence dependence, indicating that they were more universally applicable.

**Table 6. Results for on-target activity of siRNAs for different target sequences**

| Double strand code | Percentage of residual expression of target gene's mRNA (on-target activity) (mean) | | | | | | | | | | | |
|---|---|---|---|---|---|---|---|---|---|---|---|---|
| | 40nM | 13.3 nM | 4.44 nM | 1.48 nM | 0.493 nM | 0.164 nM | 0.054 nM | 0.0182 nM | 0.006 09nM | 0.002 03nM | 0.000 67nM | IC₅₀ value (nM) |
| TRD 5847 | 9.3% | 7.2% | 6.3% | 8.5% | 17.9% | 47.2% | 80.6% | 94.7% | 100.5% | 106.1% | 110.6% | 0.1380 |
| TRD 5848 | 46.5% | 35.1% | 26.6% | 36.0% | 67.3% | 76.3% | 88.4% | 104.1% | 91.6% | 95.1% | 98.1% | 0.7943 |
| TRD 5849 | 24.8% | 16.7% | 13.7% | 20.9% | 41.0% | 71.6% | 95.5% | 98.2% | 93.1% | 104.3% | 113.3% | 0.3311 |
| TRD 5850 | 19.7% | 14.2% | 12.8% | 15.5% | 29.3% | 54.3% | 84.2% | 87.6% | 86.6% | 90.0% | 95.2% | 0.2042 |
| TRD 5851 | 22.9% | 15.5% | 12.6% | 20.2% | 38.6% | 70.0% | 88.4% | 102.3% | 106.6% | 101.0% | 101.9% | 0.3020 |
| TRD 5852 | 24.7% | 17.5% | 13.1% | 21.1% | 40.5% | 64.1% | 84.3% | 94.5% | 88.4% | 100.2% | 95.1% | 0.2951 |
| TRD 5853 | 17.5% | 11.5% | 9.9% | 13.5% | 30.3% | 54.5% | 74.6% | 86.3% | 90.3% | 91.0% | 84.1% | 0.1905 |
| TRD 5854 | 37.9% | 32.4% | 35.3% | 50.3% | 70.6% | 89.7% | 98.8% | 101.1% | 106.1% | 99.6% | 114.7% | 1.3804 |
| TRD 5855 | 41.3% | 40.7% | 36.9% | 73.6% | 71.7% | 87.0% | 89.0% | 85.8% | 94.9% | 104.4% | 101.6% | 4.2658 |
| TRD 5856 | 38.6% | 37.8% | 35.8% | 59.5% | 72.7% | 92.3% | 92.5% | 85.2% | 102.1% | 93.1% | 102.1% | 2.0417 |
| TRD 5857 | 38.5% | 34.4% | 35.6% | 45.6% | 66.8% | 81.4% | 82.7% | 84.7% | 85.6% | 95.0% | 103.3% | 1.1749 |
| TRD 5858 | 25.0% | 24.3% | 26.0% | 38.1% | 59.3% | 75.4% | 86.5% | 104.8% | 93.8% | 92.4% | 94.7% | 0.7244 |
| TRD 5860 | 43.5% | 37.1% | 34.1% | 50.8% | 77.6% | 88.5% | 86.6% | 100.0% | 95.1% | 97.8% | 110.8% | 1.5488 |

The experimental results for the off-target activity of siRNA2 and siRNA3 are shown in Table 7. It can be seen that the experimental compounds of the present disclosure significantly reduced the off-target activity of siRNA relative to the parent sequence.

**Table 7. Results for off-target activity of siRNAs for different target sequences**

| Double strand code | Percentage of residual expression of target gene's mRNA (off-target activity) (mean) | | | | | | | | | | |
|---|---|---|---|---|---|---|---|---|---|---|---|
| | 40nM | 13.3 nM | 4.44 nM | 1.48 nM | 0.493 nM | 0.164 nM | 0.054 nM | 0.0182 nM | 0.006 09nM | 0.002 03nM | 0.000 67nM |
| TRD 5847 | 51.2% | 47.6% | 47.5% | 66.7% | 77.8% | 81.8% | 93.2% | 93.3% | 93.1% | 96.5% | 85.7% |
| TRD 5848 | 99.9% | 96.7% | 101.6% | 100.6% | 91.6% | 107.0% | 96.7% | 100.7% | 95.4% | 101.9% | 113.0% |
| TRD 5849 | 77.3% | 77.6% | 69.3% | 87.2% | 90.7% | 83.1% | 85.4% | 95.2% | 94.1% | 94.0% | 108.0% |
| TRD 5850 | 86.3% | 90.2% | 92.1% | 92.9% | 89.8% | 99.3% | 98.6% | 96.0% | 95.8% | 98.0% | 103.5% |
| TRD 5851 | 84.9% | 85.0% | 87.7% | 84.8% | 86.8% | 88.7% | 92.1% | 83.2% | 91.5% | 84.8% | 104.1% |
| TRD 5852 | 81.8% | 83.1% | 79.0% | 89.9% | 91.3% | 98.2% | 99.3% | 96.7% | 109.6% | 94.0% | 99.8% |
| TRD 5853 | 86.4% | 87.2% | 91.4% | 92.9% | 91.9% | 99.7% | 87.0% | 81.0% | 89.0% | 86.8% | 91.3% |
| TRD 5854 | 36.9% | 32.7% | 36.1% | 39.8% | 62.9% | 81.3% | 87.6% | 87.0% | 95.8% | 93.6% | 99.8% |
| TRD 5855 | 71.1% | 78.2% | 81.6% | 92.0% | 91.0% | 94.1% | 87.3% | 93.6% | 99.4% | 119.9% | 96.6% |
| TRD 5856 | 89.7% | 100.1% | 96.5% | 106.1% | 112.7% | 124.4% | 117.5% | 122.3% | 117.5% | 120.1% | 112.6% |
| TRD 5857 | 84.9% | 69.5% | 86.0% | 79.6% | 87.1% | 91.1% | 96.1% | 87.8% | 104.8% | 95.1% | 95.2% |
| TRD 5858 | 73.9% | 82.8% | 92.5% | 95.4% | 107.5% | 97.5% | 99.1% | 96.1% | 94.1% | 101.8% | 99.8% |
| TRD 5859 | 79.8% | 81.0% | 86.0% | 96.4% | 101.9% | 98.8% | 99.8% | 118.4% | 101.3% | 93.3% | 103.2% |
| TRD 5860 | 78.4% | 75.6% | 80.6% | 86.1% | 83.2% | 95.9% | 91.6% | 91.5% | 95.6% | 97.3% | 98.6% |

### II. Preparation and Activity Evaluation of Targeting Ligands

**Table 8. Main instrument models and sources of starting materials for preparing targeting ligands**

| **Main instrument models and sources of starting materials** | | |
|---|---|---|
| **Name** | **Company** | **Catalog number/model** |
| Solid-phase synthesizer | Dr.Oligo 48 | Biolytic |
| HPLC | Agilent 1260 Infinity II | Agilent |
| Mass spectrometer | Waters Acquity UPLC | Waters |
| Nucleoside phosphoramidite monomer starting material | | Hongene Biotech |

### Example 6. Galactosamine Compound 1-t Linked to Solid-Phase Support

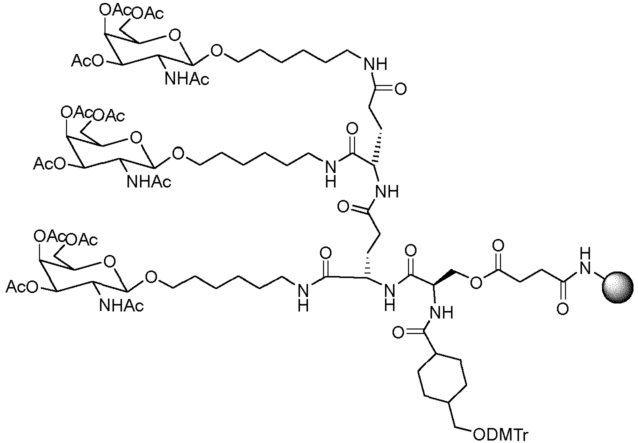

The synthetic routes were as follows:

### 1) Synthetic route of compound 1-g

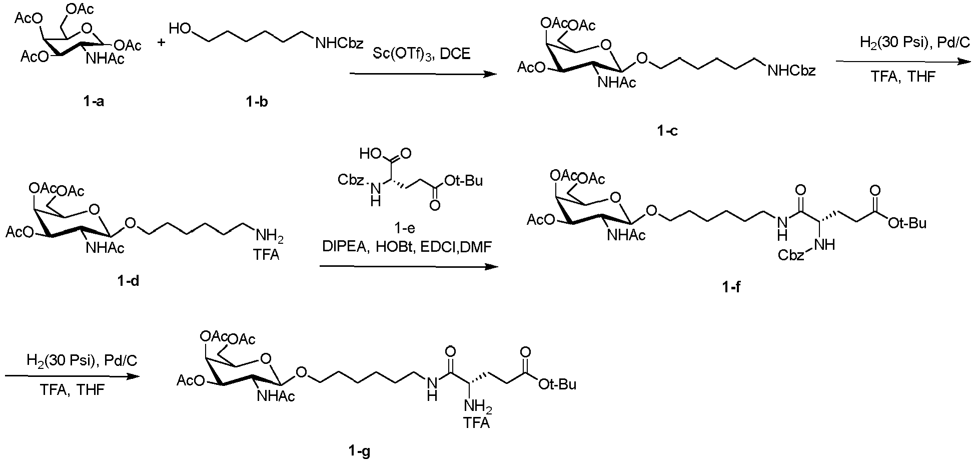

### 2) Synthetic route of compound 1-h

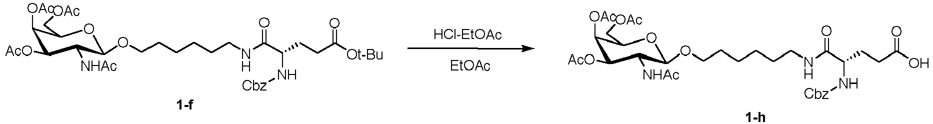

### 3) Synthetic route of compound 1-1

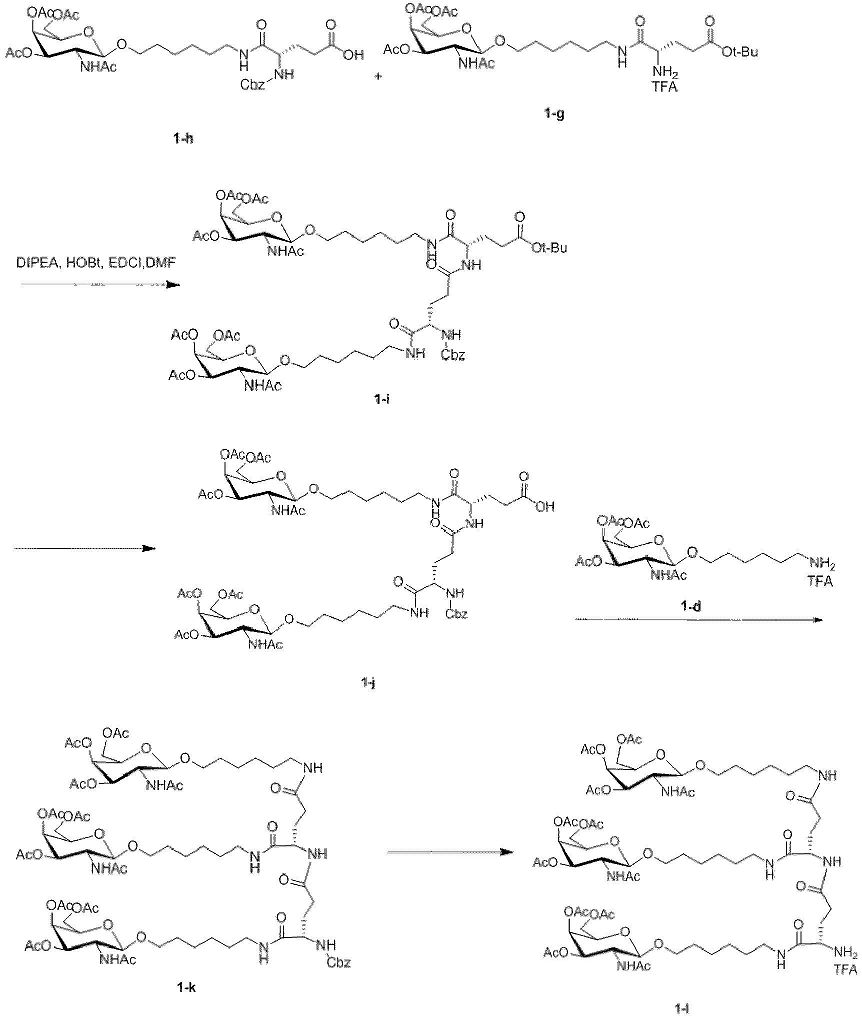

### 4) Synthetic route of compound 1-q

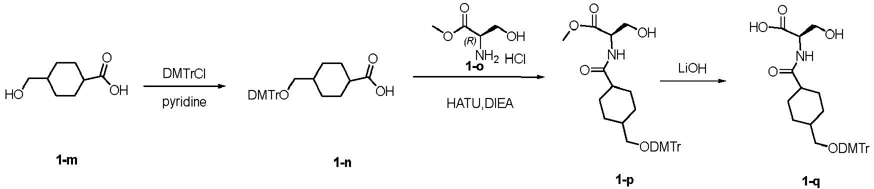

### 5) Synthesis of galactosamine compound 1-t linked to a solid-phase support

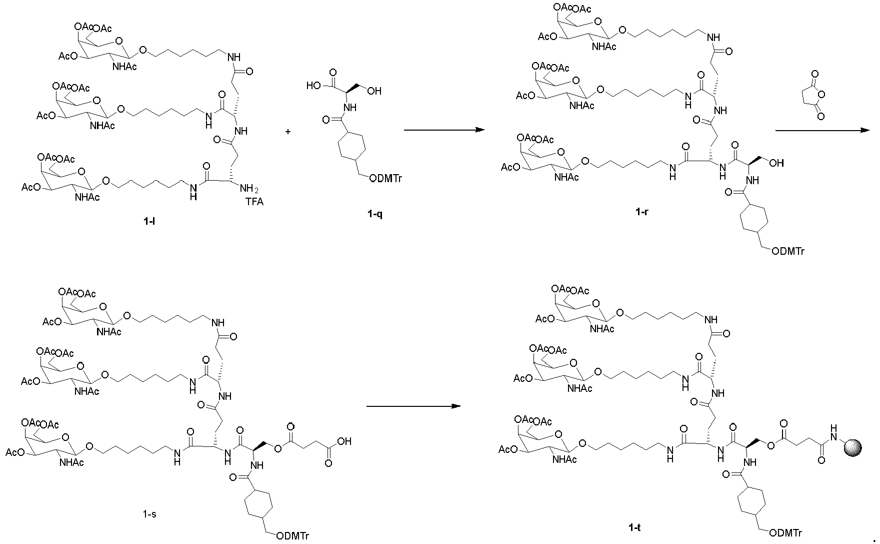

### Step 1

The starting material **1-a** (297 g, 763 mmol) and the starting material **1-b** (160 g, 636 mmol) were dissolved in 960 mL of DCE, and Sc(OTf)₃ (15.6 g, 31.8 mmol) was added at 15 °C. Then, the mixture was heated to 85 °C and stirred for 2 h. After the reaction was completed, 1.5 L of saturated NaHCO₃ was added to stop the reaction. The organic phase was isolated, washed with 1.5 L of saturated brine, dried over anhydrous Na₂SO₄, and filtered. The filtrate was distilled under reduced pressure and purified by silica gel column chromatography (petroleum ether:ethyl acetate = 5:1 to 0:1) to give the product **1-c** (328 g, 544 mmol, yield: 85.5%, purity: 96.4%) as a light yellow oil.

¹HNMR: (400 MHz, CDCl₃) δ 7.44-7.29 (m, 5H), 5.83 (d, J = 8.8 Hz, 1H), 5.40-5.23 (m, 2H), 5.18-5.06 (m, 2H), 4.86 (s, 1H), 4.66 (d, J = 8.4 Hz, 1H), 4.21-4.07 (m, 2H), 4.04-3.77 (m, 3H), 3.51-3.45 (m, 1H), 3.31-3.11 (m, 2H), 2.18 (d, J = 2.0 Hz, 1H), 2.14 (s, 3H), 2.06 (s, 3H), 2.03-1.99 (m, 3H), 1.95 (s, 3H), 1.64-1.46 (m, 4H), 1.43-1.29 (m, 4H). MS, C₂₈H₄₀N₂O¹¹, found: M⁺ 581.3.

### Step 2

The compound obtained in step 1 was divided into two parts for parallel reactions, each of which was carried out as follows: Compound **1-c** (72.0 g, 124 mmol) was added to 432 mL of THF. Pd/C (20.0 g, 10% purity) was added under argon atmosphere, and then TFA (14.1 g, 124 mmol, 9.18 mL) was added. Hydrogen gas was introduced into the reaction solution, and the gas pressure was maintained at 30 Psi. The mixture was heated to 30 °C and stirred for 16 h. After the reaction was completed, the reaction mixtures from the two parallel reactions were combined and filtered, and the filtrate was concentrated under reduced pressure. The residue was diluted with dichloromethane and concentrated under reduced pressure; the process was repeated three times. The residue was dried under reduced pressure to give the target compound **1-d** (139 g).

¹HNMR(400 MHz, DMSO-*d*₆) δ 7.85 (d, *J* = 9.2 Hz, 1H), 7.74 (s, 3H), 5.21 (d, *J* = 3.6 Hz, 1H), 4.97 (dd, *J* = 2.8, 10.8 Hz, 1H), 4.48 (d, *J* = 8.8 Hz, 1H), 4.06-3.98 (m, 3H), 3.93-3.82 (m, 1H), 3.73-3.68 (m, 1H), 3.63-3.56 (m, 1H), 3.43-3.38 (m, 1H), 2.82-2.71 (m, 2H), 2.13-2.09 (m, 3H), 2.01-1.97 (m, 3H), 1.91-1.87 (m, 3H), 1.77 (s, 3H), 1.76-1.73 (m, 1H), 1.52-1.44 (m, 4H), 1.28 (s, 4H).

### Step 3

Compound **1-d** (139 g, 247 mmol) and compound **1-e** (75.3 g, 223 mmol) were added to a DMF solution (834 mL), and then DIPEA (41.6 g, 322 mmol, 56.1 mL), HOBt (36.8 g, 272 mmol), and EDCI (52.2 g, 272 mmol) were added at 0 °C. The mixture was stirred at 15 °C for 16 h. After the reaction was completed, the reaction mixture was diluted with dichloromethane (400 mL) and then washed successively with a saturated ammonium chloride solution (1 L), saturated NaHCO₃ (1.00 L), and saturated brine. The organic phase was isolated, dried over anhydrous sodium sulfate, filtered, and distilled under reduced pressure to remove the solvent. The residue was purified by silica gel column chromatography (petroleum ether:ethyl acetate = 5:1 to 0:1) to give the target compound **1-f** (108 g, yield: 56.8%).

¹HNMR (400 MHz, DMSO-*d*₆)δ 7.89-7.78 (m, 2H), 7.41-7.27 (m, 6H), 5.21 (d, *J* = 3.2 Hz, 1H), 5.08-4.92 (m, 3H), 4.48 (d, *J* = 8.4 Hz, 1H), 4.07-3.99 (m, 3H), 3.97-3.81 (m, 2H), 3.75-3.64 (m, 1H), 3.42-3.37 (m, 1H), 3.13-2.93 (m, 2H), 2.20 (t, *J* = 8.0 Hz, 2H), 2.10 (s, 3H), 1.99 (s, 3H), 1.89 (s, 3H), 1.87-1.79 (m, 1H), 1.76 (s, 3H), 1.74-1.64 (m, 1H), 1.48-1.41 (m, 2H), 1.38 (s, 12H), 1.29-1.20 (m, 4H), 1.19-1.14 (m, 1H).

MS, C₃₇H₅₅N₃O₁₄, found: M⁺766.4.

### Step 4

The compound **1-f** obtained above was divided into two parts for parallel reactions, each of which was carried out as follows: Compound 6 (47.0 g, 61.3 mmol) was added to 280 mL of THF. Pd/C (15.0 g, 10% purity) was added under argon atmosphere, and then TFA (7.00 g, 61.3 mmol, 4.54 mL) was added. Hydrogen gas was introduced into the reaction solution, and the gas pressure was maintained at 30 Psi. The mixture was heated to 30 °C and stirred for 16 h. After the reaction was completed, the reaction mixtures from the two parallel reactions were combined and filtered, and the filtrate was concentrated under reduced pressure. The residue was diluted with dichloromethane and concentrated under reduced pressure; the process was repeated three times. The residue was dried under reduced pressure to give the target compound **1-g** (94.0 g, crude product).

¹HNMR (400 MHz, DMSO-d6) δ 8.38 (s, 1H), 8.10 (s, 3H), 7.83 (d, J = 9.2 Hz, 1H), 5.21 (d, J = 3.2 Hz, 1H), 4.96 (dd, J = 3.6, 11.2 Hz, 1H), 4.47 (d, J = 8.4 Hz, 1H), 4.06-3.98 (m, 3H), 3.92-3.82 (m, 1H), 3.75-3.67 (m, 2H), 3.60 (s, 1H), 3.43-3.37 (m, 1H), 3.18-3.04 (m, 2H), 2.30-2.24 (m, 2H), 2.10 (s, 3H), 2.00 (s, 3H), 1.95-1.90 (m, 2H), 1.89 (s, 3H), 1.78-1.75 (m, 3H), 1.49-1.41 (m, 3H), 1.40 (s, 9H), 1.26 (s, 4H).

### Step 5

The compound **1-f** obtained above was divided into two parts for parallel reactions, each of which was carried out as follows: Compound **1-f** (46.0 g, 60 mmol) was added to HCl-EtOAc (2.00 M, 276 mL). The mixture was stirred at 15 °C for 16 h. After the reaction was completed, the reaction solutions from the two reactions were combined, distilled under reduced pressure, and concentrated. The residue was diluted with dichloromethane and concentrated under reduced pressure; the process was repeated three times. The residue was dried under reduced pressure to give a light red compound **1-h** (91.0 g, crude product).

¹HNMR (400 MHz, DMSO-*d*₆) δ 7.91-7.80 (m, 2H), 7.42-7.26 (m, 6H), 5.21 (d, *J* = 3.2 Hz, 1H), 5.07-4.92 (m, 4H), 4.48 (d, *J* = 8.4 Hz, 1H), 4.06-3.98 (m, 3H), 3.98-3.82 (m, 3H), 3.73-3.65 (m, 1H), 3.44-3.35 (m, 1H), 3.12-2.94 (m, 2H), 2.22 (t, J = 8.0 Hz, 2H), 2.10 (s, 3H), 2.01-1.97 (m, 4H), 1.94-1.90 (m, 1H), 1.89 (s, 3H), 1.87-1.79 (m, 2H), 1.76 (s, 3H), 1.74-1.67 (m, 1H), 1.49-1.40 (m, 2H), 1.40-1.32 (m, 2H), 1.24 (d, *J* = 4.0 Hz, 4H), 1.19-1.13 (m, 1H).

MS, C₃₃H₄₇N₃O₁₄, found: M⁺710.3.

### Step 6

Two reactions were carried out in parallel as follows: Compound **1-g** (45.0 g, 60.3 mmol) and compound **1-h** (38.5 g, 54.3 mmol) were added to 270 mL of DMF. Then, DIPEA (10.1 g, 78.4 mmol, 13.6 mL) was added at 0 °C, and HOBt (8.97 g, 66.3 mmol) and EDCI (12.7 g, 66.3 mmol) were added. The mixture was stirred at 15 °C for 16 h. After the reaction was completed, the reaction solutions from the two parallel reactions were combined, diluted with 300 mL of DCM, and washed successively with saturated ammonium chloride (800 mL), saturated NaHCO₃ (800 mL), and saturated brine (800 mL). The organic phase was dried over anhydrous Na₂SO₄. After filtration, the filtrate was concentrated by evaporation under increased pressure. The residue was purified by silica gel column chromatography (petroleum ether:ethyl acetate = 5:1 to 0:1) to give a white compound **1-i** (66.0 g, 47.4 mmol, yield: 39.3%, purity: 95.1%).

¹HNMR (400 MHz, DMSO-*d*₆) δ 7.96-7.78 (m, 5H), 7.41-7.25 (m, 6H), 5.21 (d, *J* = 3.6 Hz, 2H), 5.05-4.92 (m, 4H), 4.48 (d, *J* = 8.8 Hz, 2H), 4.22-4.12 (m, 1H), 4.02 (s, 6H), 3.94-3.80 (m, 3H), 3.74-3.64 (m, 2H), 3.45-3.35 (m, 2H), 3.11-2.92 (m, 4H), 2.20-2.12 (m, 4H), 2.10 (s, 6H), 1.99 (s, 6H), 1.89 (s, 6H), 1.82-1.79 (m, 2H), 1.76 (s, 6H), 1.74-1.63 (m, 2H), 1.44 (d, *J* = 6.0 Hz, 4H), 1.37 (s, 12H), 1.24 (s, 9H).

MS: C₆₂H₉₄N₆O₂₅, found: m/z 1323.8.

### Step 7

This step was carried out through 11 reactions, each of which was carried out as follows: Compound **1-i** (5.00 g, 3.78 mmol) and toluene (300 mL) were added, and silica gel (45.0 g) was added. The mixture was stirred at 100 °C for 40 h. After the reaction was completed, the reaction mixtures from the 11 reactions were combined. The reaction mixture was distilled under reduced pressure to remove the solvent, and isopropanol and dichloromethane were added to the residue. The mixture was stirred for 20 min. Insoluble matter was removed by filtration, and the filter cake was washed with isopropanol until no product was dissolved in isopropanol. The resulting solution was concentrated to remove the solvent and dried under reduced pressure to give a light yellow compound 1-j (43.2 g, 34.0 mmol, yield: 82.0%).

¹HNMR: (400 MHz, DMSO-d6) δ 8.01 (d, J = 7.6 Hz, 1H), 7.93-7.79 (m, 2H), 7.39-7.27 (m, 3H), 5.21 (d, J = 3.2 Hz, 1H), 5.06-4.91 (m, 2H), 4.48 (d, J = 8.0 Hz, 1H), 4.07-3.97 (m, 3H), 3.94-3.82 (m, 2H), 3.73-3.65 (m, 1H), 3.45-3.36 (m, 2H), 3.10-2.94 (m, 2H), 2.15 (d, J = 7.6 Hz, 2H), 2.10 (s, 3H), 1.99 (s, 3H), 1.89 (s, 3H), 1.86-1.79 (m, 1H), 1.77 (s, 3H), 1.74-1.65 (m, 1H), 1.44 (s, 2H), 1.37 (d, J = 5.2 Hz, 2H), 1.24 (s, 4H).

MS: C₅₈H₈₆N₆O₂₅, found: m/z = 1267.8.

### Step 8

This step was carried out through two reactions in parallel: each of which was carried out as follows: compound **1-d** (11.8 g, 21.0 mmol) and compound **1-j** (21.3 g, 16.8 mmol) were added to 70 mL of DMF. Then, DIPEA (3.54 g, 27.3 mmol, 4.77 mL) was added at 0 °C, and HOBt (3.13 g, 23.1 mmol) and EDCI (4.44 g, 23.1 mmol) were added. The mixture was stirred at 15 °C for 16 h. After the reaction was completed, the reaction solutions from the two parallel reactions were combined, diluted with 500 mL of DCM, and washed successively with saturated ammonium chloride (1.5 L), saturated NaHCO₃ (1.5 mL), and saturated brine (1.5 mL). The organic phase was dried over anhydrous Na₂SO₄. After filtration, the filtrate was concentrated by evaporation under increased pressure. The residue was purified by silica gel column chromatography (dichloromethane:methanol = 50:1 to 10:1) to give a light yellow compound **1-k** (54.0 g, 31.8 mmol, yield: 75.6%).

¹HNMR (400 MHz, DMSO-*d*₆) δ 7.91 (d, J = 7.6 Hz, 1H), 7.87-7.78 (m, 5H), 7.73 (t, J = 5.2 Hz, 1H), 7.42-7.24 (m, 6H), 5.21 (d, J = 3.6 Hz, 3H), 5.06-4.92 (m, 5H), 4.48 (d, J = 8.4 Hz, 3H), 4.19-4.09 (m, 2H), 4.07-3.97 (m, 10H), 3.94-3.80 (m, 4H), 3.76-3.64 (m, 3H), 3.42-3.37 (m, 4H), 3.08-2.94 (m, 6H), 2.20-2.12 (m, 2H), 2.10 (s, 9H), 2.08-2.01 (m, 2H), 1.99 (s, 9H), 1.89 (s, 9H), 1.87-1.79 (m, 2H), 1.77 (s, 9H), 1.74-1.63 (m, 2H), 1.44 (d, J = 5.6 Hz, 6H), 1.40-1.31 (m, 6H), 1.24 (s, 13H).

MS: C₇₈H₁₁₈N₈O₃₃, found: m/z = 1696.1.

### Step 9

This step was carried out by 3 reactions in parallel, each of which was carried out as follows: Compound **1-k** (17.0 g, 10.0 mmol) and THF (100 mL) were added. Then, Pd/C (5.0 g, 10% purity) was added under argon atmosphere, and TFA(1.14 g, 10.0 mmol, 742 µL) was added. Hydrogen gas was introduced into the reaction solution, and the gas pressure was maintained at 15 Psi. The mixture was heated to 30 °C and stirred for 4 h. After the reaction was completed, the reaction mixtures from the 3 parallel reactions were combined and filtered, and the filtrate was concentrated under reduced pressure. The residue was diluted with dichloromethane and concentrated under reduced pressure; the process was repeated three times. The residue was purified by preparative liquid chromatography (C18, mobile phase A: 0.1% TFA-water, mobile phase B: 10%-40% ACN, 20 min) to give a white compound **1-l** (17.3 g, 10.2 mmol, yield: 34.0%).

¹HNMR: (400 MHz, DMSO-*d*₆) δ 8.45 (t, *J* = 5.2 Hz, 1H), 8.14 (d, *J* = 5.2 Hz, 3H), 7.97 (t, *J* = 5.2 Hz, 1H), 7.90-7.77 (m, 4H), 5.21 (d, *J* = 2.8 Hz, 3H), 4.96 (dd, *J* = 3.2, 11.6 Hz, 3H), 4.47 (d, *J* = 8.4 Hz, 3H), 4.20-4.10 (m, 1H), 4.02 (s, 8H), 3.87 (q, *J* = 9.6 Hz, 3H), 3.75-3.61 (m, 4H), 3.46-3.34 (m, 3H), 3.21-2.93 (m, 6H), 2.21 (s, 2H), 2.14-2.02 (m, 11H), 1.99 (s, 9H), 1.96-1.82 (m, 12H), 1.80-1.65 (m, 10H), 1.44 (d, *J* = 5.6 Hz, 8H), 1.36 (d, *J* = 6.4 Hz, 4H), 1.30-1.17 (m, 12H).

MS: C₇₀H₁₁₂N₈O₃₁, found: m/2z = 781.8.

### Step 10

Compound **1-m** (2 g, 12.64 mmol) was dissolved in pyridine (10 mL), and a solution of DMTrCl (4.71 g, 13.90 mmol) in pyridine (10 mL) was added dropwise at room temperature. The mixture was stirred at room temperature for 5 h. After the reaction was completed, the reaction mixture was quenched with methanol and concentrated under reduced pressure to give a crude product. The crude product was purified using a silica gel column (elution with petroleum ether:ethyl acetate = 10:1). The product eluate was collected and concentrated under reduced pressure to evaporate the solvent to give compound **1-n** (4 g).

MS m/z: C₂₉H₃₂O₅, [M+H]⁺ found: 461.3.

### Step 11

Compound **1-n** (2 g, 4.34 mmol), *N,N*-diisopropylethylamine (DIEA, 1.43 mL, 8.68 mmol), and HATU (2.47 g, 6.51 mmol) were dissolved in DMF (10 mL), and a solution of compound **1-o** in DMF (5 mL) was added at room temperature. The mixture was stirred at room temperature for 8 h. After the reaction was completed, the reaction mixture was quenched with water. The aqueous phase was extracted with ethyl acetate. The combined organic phase was washed first with water and then with saturated brine (20 mL), concentrated under reduced pressure to evaporate the solvent, purified by reversed-phase preparative HPLC (column: Boston Green ODS 150 × 30 mm × 5 µm, conditions: 25%-80% (A: water 0.075% NH₃·H₂O, B: CH₃CN), flow rate: 55 mL/min), and lyophilized to give compound **1-p** (2.4 g).

MS m/z: C₃₃H₃₉NO₇, [M+H]⁺ found: 562.4.

### Step 12

Compound **1-p** (2.4 g, 4.27 mmol) was dissolved in 15 mL of a mixed solution of methanol and water (2:1), and LiOH (0.36 g, 8.54 mmol) was added at room temperature. The mixture was stirred overnight. After the reaction was completed, the reaction mixture was concentrated under reduced pressure to evaporate the solvent, purified by reversed-phase preparative HPLC (column: Boston Green ODS 150 × 30 mm × 5 µm, conditions: 25%-75% (A: water, 0.075% NH₃·H₂O, B: CH₃CN), flow rate: 55 mL/min), and lyophilized to give compound **1-q** (2 g).

MS m/z: C₃₂H₃₇NO₇, [M+H]⁺ found: 548.6.

### Step 13

Compound **1-q** (0.37 g, 0.69 mmol), DIEA (0.19 mL, 1.15 mmol), and HATU (0.32 g, 0.86 mmol) were dissolved in 2 mL of DMF, and a solution of compound **1-l** (0.9 g, 0.69 mmol) in DMF (2 mL) was added at room temperature. The mixture was stirred at room temperature overnight. After the reaction was completed, the reaction mixture was diluted with dichloromethane (10 mL) and washed successively with saturated NaHCO₃ (20 mL) and saturated brine (20 mL). The organic phase was dried over anhydrous Na₂SO₄, filtered, and concentrated under reduced pressure. The residue was purified by reversed-phase preparative HPLC (column: Boston Green ODS 150 × 30 mm × 5 µm, conditions: 25%-65% (A: water, 0.075% NH₃·H₂O, B: CH₃CN), flow rate: 45 mL/min) and lyophilized to give compound **1-r** (0.5 g).

MS m/z: C₁₀₂H₁₄₇N₉O₃₇, [M-H]⁺ found: 2088.5.

### Step 14

Compound **1-r** (300 mg, 0.14 mmol) and succinic anhydride (28.70 mg, 0.28 mmol) were dissolved in tetrahydrofuran, and DMAP (3.50 mg, 0.028 mmol) was added to the solution. The mixture was stirred at 40 °C overnight. After the reaction was completed, methanol (18.8 mg) was added. The mixture was stirred for 10 min. Then, the reaction mixture was diluted with dichloromethane (3 mL) and washed twice with saturated NaHCO₃ (5 mL). The organic phase was concentrated to dryness under reduced pressure and purified by reversed-phase preparative HPLC (column: Boston Green ODS 150 × 30 mm × 5 µm, conditions: 25%-65% (A: water, 0.075% NH₃·H₂O, B: CH₃CN), flow rate: 35 mL/min) and lyophilized to give compound **1-s** (140 mg).

MS m/z: C₁₀₆H₁₅₁N₉O₄₀, [M-H]⁺ found: 2189.4.

### Step 15

The compound **1-r** (140 mg, 64 µmol) obtained in the previous step was added to acetonitrile (5 mL). Then, HBTU (48.7 mg, 128 µmol) was added, a solid-phase support with an amino modification on the surface (CPG-NH₂, 2.3 g) was added, and DIEA (41.5 mg, 320 µmol, 55 µL) was added. The mixture was reacted with shaking at 30 °C for 16 h. After the reaction was completed, the reaction mixture was filtered and washed successively with methanol (8 mL × 4) and dichloromethane (8 mL × 4). The solid was added to pyridine:acetic anhydride (v:v = 4:1, 10.0 mL), and the mixture was reacted with shaking at 30 °C for another 16 h. After the reaction was completed, the reaction mixture was filtered and washed successively with methanol (8 mL × 4) and dichloromethane (8 mL × 4) to give compound **1-t** linked to the solid-phase support (2.1 g).

### Example 7. Galactosamine Compound 2-e Linked to Solid-Phase Support

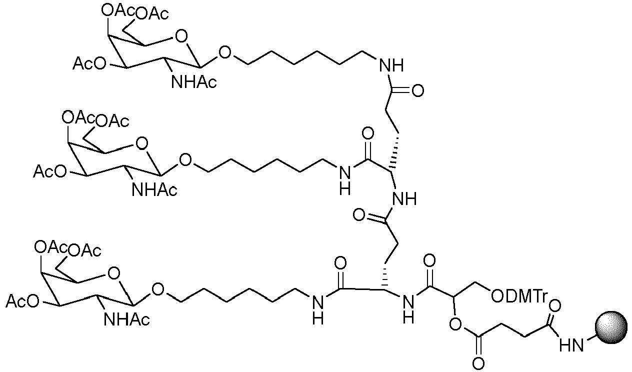

The synthetic routes were as follows:

### 1) Synthesis of compound 2-b

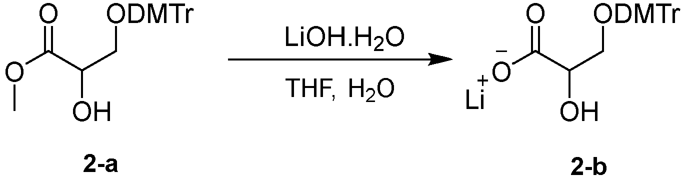

### 2) Synthesis of compound 2-e

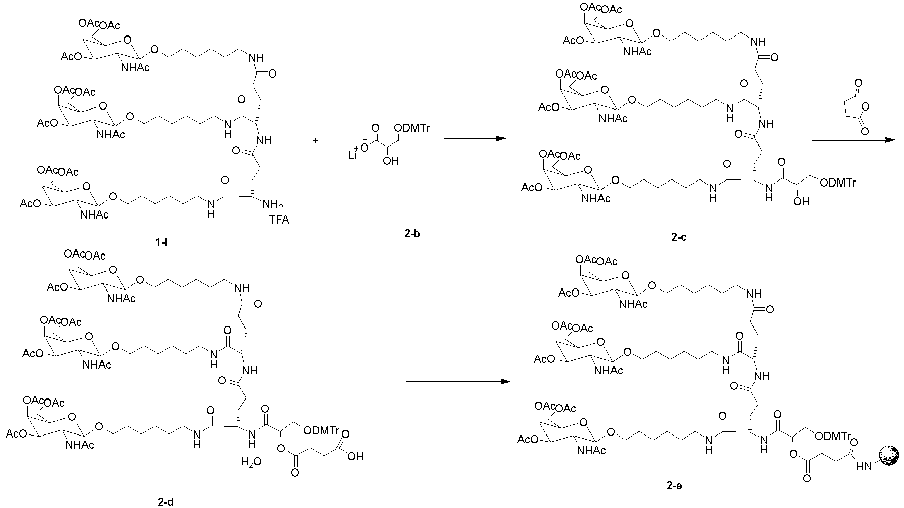

### Step 1

Compound **2-a** (1.00 g, 2.37 mmol) was added to THF (7.5 mL) and H₂O (7.5 mL), and then LiOH·H₂O (109 mg, 2.60 mmol) was added. The mixture was stirred at 16 °C for 16 h. After the reaction was completed, the reaction mixture was concentrated under reduced pressure to evaporate the solvent. The residue was lyophilized to give the target compound **2-b** (960 mg, 2.32 mmol, yield: 97.8%).

¹HNMR: (400 MHz, DMSO-d6) δ 7.44 (d, J = 8.4 Hz, 2H), 7.34-7.23 (m, 6H), 7.22-7.15 (m, 1H), 6.86 (d, J = 8.0 Hz, 4H), 3.73 (s, 6H), 3.66 (d, J = 6.4 Hz, 1H), 3.32 (d, J = 12.0 Hz, 1H), 3.11 (dd, J = 2.0, 9.2 Hz, 1H), 2.85 (t, J = 8.8 Hz, 1H).

MS m/z: C₂₄H₂₄O₆, found: m/z: 407.2.

### Step 2

Compound **1-l** (500 mg, 0.30 mmol) was added to dichloromethane (3 mL). Then, compound **2-b** (0.14 g, 0.34 mmol) was added to the mixture at 15 °C, and HBTU (142 mg, 375 µmol) and DIEA (115 mg, 895 µmol) were added at 0 °C. The mixture was reacted at 15 °C for 16 h. After the reaction was completed, the reaction mixture was diluted with dichloromethane (10 mL) and washed successively with saturated NaHCO₃ (20 mL) and saturated brine (20 mL). The organic phase was dried over anhydrous Na₂SO₄, filtered, and concentrated under reduced pressure. The residue was purified by preparative liquid chromatography (column: Welch Xtimate C18 250 × 70 mm #10 µm; mobile phase: [water-ACN]; B%: 40%-66%,18 min) to give compound **2-c.**

MS m/z: C₉₄H₁₃₄N₈O₃₆, [M-H]⁺ found: 1952.1.

### Step 3

Compound **2-c** (230 mg, 0.12 mmol) and succinic anhydride (23.5 mg, 0.26 mmol) were dissolved in a dichloromethane solution (2 mL), and DMAP (43.1 mg, 0.35 mmol) was added to the reaction mixture. The mixture was stirred at 15 °C for 16 h. After the reaction was completed, methanol (18.8 mg) was added. The mixture was stirred for 10 min. Then, the reaction mixture was diluted with dichloromethane (3 mL) and washed twice with saturated NaHCO₃. The reaction mixture was concentrated to dryness under reduced pressure to give compound **2-d** (240 mg, crude product).

MS m/z: C₁₀₆H₁₅₁N₉O₄₀, [M-H]⁺ found: m/2z: 2070.2.

### Step 4

The compound **2-d** (240 mg, 116 µmol) obtained in the previous step was added to acetonitrile (8 mL). Then, HBTU (88.7 mg, 233 µmol) was added, a solid-phase support with an amino modification on the surface (CPG-NH₂, 4 g) was added, and DIEA (75.5 mg, 584 µmol, 101 µL) was added. The mixture was reacted with shaking at 30 °C for 16 h. After the reaction was completed, the reaction mixture was filtered and washed successively with methanol (8 mL × 4) and dichloromethane (8 mL × 4). The solid was added to pyridine:acetic anhydride (v:v = 4:1, 10.0 mL), and the mixture was reacted with shaking at 30 °C for another 16 h. After the reaction was completed, the reaction mixture was filtered and washed successively with methanol (8 mL × 4) and dichloromethane (8 mL × 4) to give the target product compound **2-e** linked to the solid-phase support (3.7 g).

### Example 8. Galactosamine Compound 3-n Linked to Solid-Phase Support

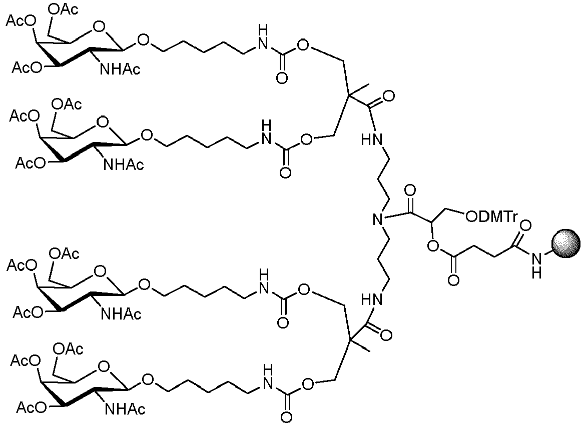

The synthetic routes were as follows:

### 1) Synthesis of compound 3-d

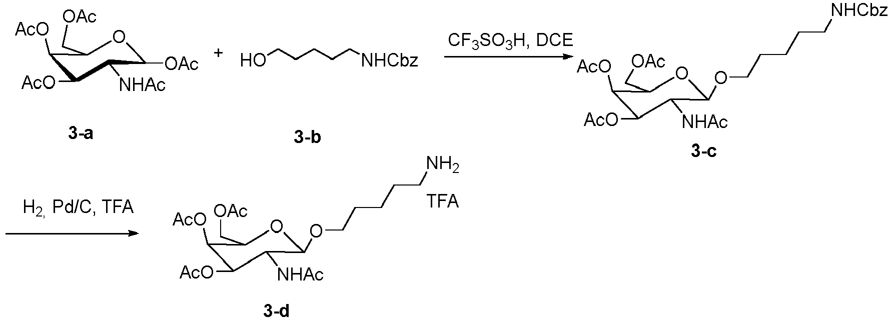

### 2) Synthesis of compound 3-g

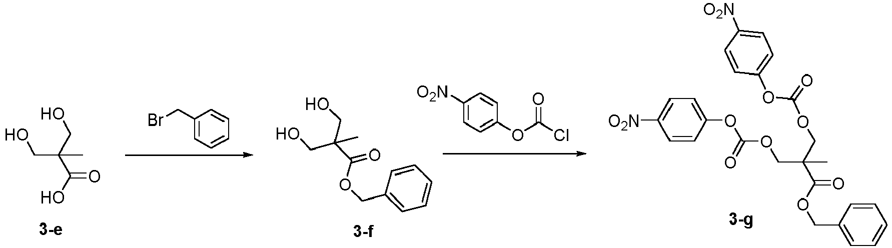

### 3) Synthesis of compound 3-n

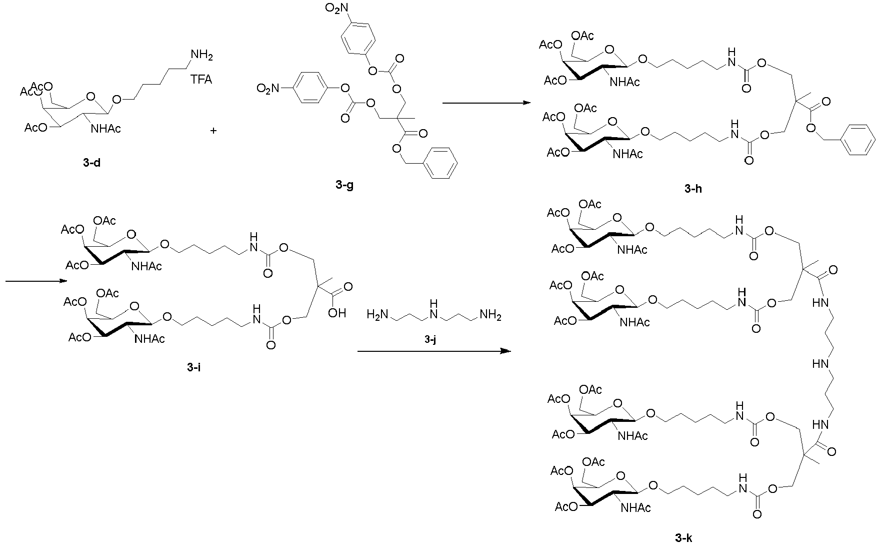

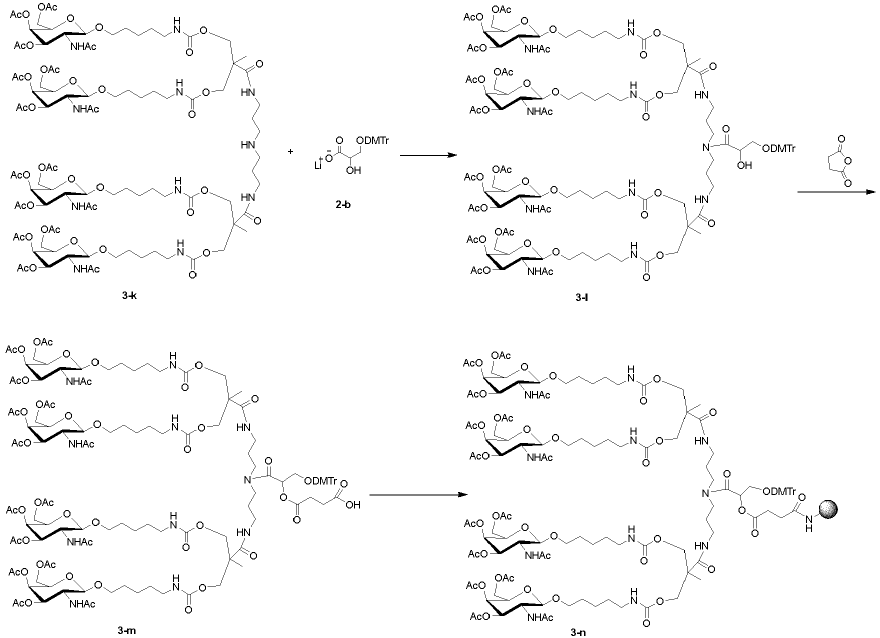

### Step 1

The starting material **3-a** (78.8 g, 202 mmol) and the starting material **3-b** (40 g, 168 mmol) were dissolved in DCE (250 mL), and CF₃SO₃H (4.15 g, 8.43 mmol) was added at 15 °C. Then, the reaction mixture was heated to 75 °C and stirred for 2 h. After the reaction was completed, 1 L of saturated NaHCO₃ was added to stop the reaction. The organic phase was isolated, washed with 1 L of saturated brine, dried over anhydrous Na₂SO₄, and filtered. The filtrate was distilled under reduced pressure and purified by silica gel column chromatography (petroleum ether:ethyl acetate = 5:1 to 0:1) to give the target product **3-c** (63.2 g, 107 mmol, yield: 63.5%).

¹HNMR: (400 MHz, CDCl₃) δ 7.35-7.26 (m, 5H), 5.88 (s, 1H), 5.34-5.25 (m, 2H), 4.65 (d, *J* = 8.4 Hz, 1H), 4.16-4.13 (m, 2H), 3.92-3.87 (m, 3H), 3.18-3.17 (m, 1H), 3.15-3.14 (m, 2H), 2.16-1.91 (m, 15H), 1.58-1.50 (m, 5H), 1.49-1.36 (m, 2H).

MS m/z: C₂₄H₄₀N₂O₁₁, found: m/z: 567.4.

### Step 2

The compound **3-c** (60.0 g, 106 mmol) obtained above was added to 360 mL of THF. Then, Pd/C (15.0 g, 10% purity) was added under argon atmosphere, and TFA (12.1 g, 106 mmol, 7.84 mL) was added. Hydrogen was introduced into the reaction mixture, and the gas pressure was maintained at 30 Psi. The mixture was heated to 30 °C and stirred for 16 h. After the reaction was completed, the reaction mixture was filtered, and the filtrate was concentrated under reduced pressure. The residue was diluted with dichloromethane and concentrated under reduced pressure; the process was repeated three times (500 mL × 3). The residue was dried under reduced pressure to give the target compound **3-d** (44 g, 102 mmol, yield: 96.1%).

### Step 3

Compound **3-e** (60.0 g, 447 mmol) was dissolved in DMF (300 mL). K₂CO₃ (92.7 g, 671 mmol) was added, and BnBr (115 g, 671 mmol, 79.7 mL) was added dropwise at 0 °C. The reaction mixture was stirred at 25 °C for 6 h. The reaction mixture was poured into crushed ice and then extracted with ethyl acetate (100 mL × 6). The organic phase was washed successively with water (100 mL × 2) and saturated brine (100 mL × 3). The organic phase was dried over anhydrous sodium sulfate and distilled under reduced pressure to remove the solvent. The residue was purified by silica gel column chromatography (petroleum ether:ethyl acetate = 2:1 to 0:1) to give the target compound **3-f** (60.3 g, 269 mmol, yield: 60.1%).

¹HNMR: (400 MHz, CDCl₃) δ 7.37-7.26 (m, 5H), 5.18 (d, J = 4.4 Hz, 2H), 3.95-3.90 (m, 2H), 3.75-3.71 (m, 2H), 1.08 (s, 1H).

MS m/z: C₁₂H₁₆O₄, found: m/z: 223.5.

### Step 4

Compound **3-f** (50.0 g, 223 mmol) was dissolved in dichloromethane (300 mL), and pyridine (73.5 g, 929 mmol, 75 mL) and a solution ofp-nitrophenyl chloroformate (180 g, 892 mmol) in dichloromethane (50 mL) were added. The mixture was stirred at 25 °C for 24 h under nitrogen atmosphere. After the reaction was completed, the reaction mixture was diluted with dichloromethane (250 mL) and washed successively with a NaHSO₄ solution (30 mL × 3) and saturated brine (30 mL × 2). The organic phase was dried over MgSO₄, filtered, and concentrated under reduced pressure to evaporate the solvent. The resulting crude product was purified by silica gel column chromatography (petroleum ether:ethyl acetate = 3:1) to give the target compound **3-g** (37.0 g, 66.7 mmol, yield: 29.9%).

MS m/z: C₂₆H₂₂N₂O₁₂, found: m/z: 553.4.

### Step 5

Compound **3-g** (22.0 g, 39.7 mmol) was added to acetonitrile (120 mL), and triethylamine (24.1 g, 238 mmol, 33.1 mL) was added under nitrogen atmosphere. The reaction mixture was cooled to 0 °C, and a solution of compound **3-d** (42.1 g, 40 mmol) in acetonitrile (120 mL) was added dropwise. The reaction mixture was heated to 25 °C and stirred for 1 h. After the reaction was completed, the mixture was concentrated under reduced pressure to remove the solvent and then purified by silica gel column chromatography (petroleum ether:ethyl acetate = 2:1) to give the target compound **3-h** (37.0 g, 12.0 mmol, yield: 30.2%).

MS m/z: C₅₂H₇₆N₄O₂₄, found: m/z: 1141.8.

### Step 6

Compound **3-h** (11.0 g, 9.64 mmol) was dissolved in ethyl acetate (60 mL), and Pd/C (2.00 g, 10% purity) was added. Hydrogen gas was introduced into the reaction mixture, and the gas pressure was maintained at 40 Psi. The mixture was stirred at 25 °C for 8 h. After the reaction was completed, the reaction mixture was filtered and concentrated to dryness by evaporation under reduced pressure to give the target compound **3-i** (10.0 g, 9.42 mmol, yield: 97.7%).

¹HNMR: (400 MHz, DMSO-*d*₆) δ 7.79 (d, J = 9.2 Hz, 2H), 7.10 (s, 2H), 5.74 (t, J = 1.6 Hz, 2H), 5.21 (d, J = 3.6 Hz, 2H), 4.98-4.95 (m, 2H), 4.48 (d, J = 8.4 Hz, 2H), 4.02 (d, J = 4.8 Hz, 11H), 3.87-3.84 (m, 2H), 3.69-3.67 (m, 2H), 3.41-3.39 (m, 2H), 2.94-2.90 (m, 4H), 2.10 (s, 5H), 1.99 (s, 7H), 1.89 (s, 6H), 1.77 (s, 6H), 1.47-1.35 (m, 8H), 1.26-1.24 (m, 4H), 1.23-1.08 (m, 3H).

MS m/z: C₄₅H₇₀N₄O₂₄, found: m/z: 1051.4.

### Step 7

Compound **3-i** (5.00 g, 4.76 mmol) was added to a mixed solvent of dichloromethane (30 mL) and DMF (30 mL). Then, compound 33 (312 mg, 2.38 mmol) was added, and HBTU (1.80 g, 4.76 mmol) and DIEA(615 mg, 4.76 mmol) were added. The mixture was stirred at 25 °C for 12 h. After the reaction was completed, the reaction mixture was poured into ethyl acetate (100 mL). Then, the mixture was washed with saturated brine, dried over anhydrous Na₂SO₄, filtered, and concentrated under reduced pressure to evaporate the solvent. The residue was purified by preparative HPLC to give the target compound **3-k** (2.1 g, 956 µmol, yield: 20.1%).

¹HNMR: (400 MHz, DMSO-*d*₆) δ 7.84-7.81 (m, 5H), 7.12-7.07 (m, 3H), 5.21 (d, J = 3.6 Hz, 4H), 4.99-4.96 (m, 4H), 4.49 (d, J = 8.4 Hz, 4H), 4.06-4.00 (m, 24H), 3.88-3.86 (m, 4H), 3.55-3.52 (m, 4H), 3.49-3.43 (m, 4H), 3.25-3.05 (m, 4H), 2.94-2.93 (m, 8H), 2.11 (s, 12H), 2.00 (s, 16H), 1.90 (s, 12H), 1.78 (s, 12H), 1.46-1.44 (m, 8H), 1.38-1.35 (m, 8H), 1.26-1.24 (m, 8H), 1.18-1.16 (m, 6H), 1.09-0.99 (m, 2H).

MS m/z: C₉₆H₁₅₃N₁₁O₄₆, found: m/z: 2197.5.

### Step 8

Compound **3-k** (100 mg, 45.5 µmol) was added to DMF (1 mL). Then, compound **2-b** (21.1 mg, 54 µmol) was added to the mixture, and HBTU (21.8 mg, 57.3 µmol) and DIEA (17.7 mg, 136 µmol) were added. The mixture was reaction at 15 °C for 16 h. After the reaction was completed, the reaction mixture was diluted with dichloromethane (10 mL) and washed successively with saturated NaHCO₃ and saturated brine. The organic phase was dried over anhydrous Na₂SO₄, filtered, and concentrated under reduced pressure. The residue was purified by preparative liquid chromatography (column: Phenomenex Gemini-NX 150 × 30 mm × 5 µm; mobile phase: [water-ACN]; B%: 35%-75%,12 min) to give compound **3-l.**

MS m/z: C₁₂₀H₁₇₅N₁₁O₅₁, found: 2586.9.

### Step 9

Compound **3-l** (14 mg, 5.4 µmol) and succinic anhydride (1.08 mg,10.8 µmol) were dissolved in a dichloromethane solution (1 mL), and DMAP (2.0 mg, 16 µmol) and TEA (1.1 mg,10.8 µmol,1.5 µL) were added to the reaction mixture. The mixture was stirred at 15 °C for 16 h. After the reaction was completed, methanol (0.9 mg) was added. The mixture was stirred for 10 min. Then, the reaction mixture was diluted with dichloromethane and washed twice with saturated NaHCO₃. The reaction mixture was concentrated to dryness under reduced pressure to give compound **3-m** (18 mg).

MS m/z: C₁₂₄H₁₇₉N₁₁O⁵⁴, found: 2687.2.

### Step 10

Compound 3-m (18 mg, 6.7 µmol) obtained in the previous step was added to acetonitrile (3 mL). Then, HBTU (5.1 mg, 13.4 µmol) was added, a solid support with an amino modification on the surface (CPG-NH₂, 200 mg) was added, and DIEA (4.3 mg, 33.5 µmol, 5.8 µL) was added. The mixture was reacted with shaking at 30 °C for 16 h. After the reaction was completed, the reaction mixture was filtered and washed successively with methanol (2 mL × 4) and dichloromethane (2 mL × 4). The solid was added to pyridine:acetic anhydride (v:v = 4:1, 2 mL), and the mixture was reacted with shaking at 30 °C for another 16 h. After the reaction was completed, the reaction mixture was filtered and washed successively with methanol and dichloromethane to give the target product compound **3-n** linked to the solid-phase support (200 mg).

### Example 9. Galactosamine Compound 4-c Linked to Solid-Phase Support

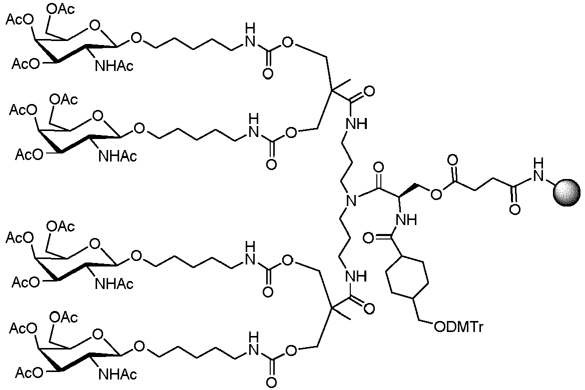

The synthetic routes were as follows:

### Synthesis of compound 4-c

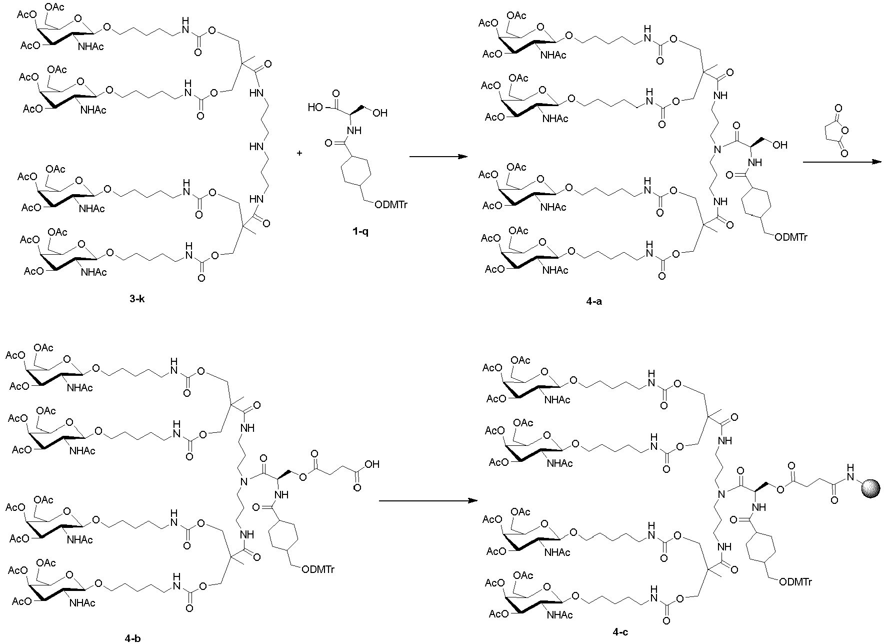

### Step 1

Compound **3-k** (149.5 mg, 68 µmol), DIEA(141.0 mg, 1.09 mmol), 3Amolecular sieves (500 mg), and DEPBT (163.4 mg, 0.55 mmol) were dissolved in 5 mL of DCM, and compound **1-q** (400 mg, 0.18 mmol) was added at room temperature. The mixture was stirred at room temperature overnight. After the reaction was completed, the molecular sieves were filtered out. The filtrate was concentrated to dryness by rotary evaporation, purified by reversed-phase preparative HPLC (column: Boston Green ODS 150 × 30 mm × 5 µm, conditions: 5%-50% (A: water, B: CH₃CN), flow rate: 45 mL/min), and lyophilized to give compound **4-a** (118 mg, 32 µmol, yield: 62.6%).

MS m/z: C₁₂₈H₁₈₈N₁₂O₅₂, found: [M+HCOO⁻] = 2770.6.

### Step 2

Compound **4-a** (110 mg, 4.0 µmol), DMAP (7.4 mg, 40 µmol), 3A molecular sieves (100 mg), and succinic anhydride (11.9 mg, 120 µmol) were dissolved in 5 mL of THF. The mixture was stirred at 40 °C for 4 h under argon atmosphere. After the reaction was completed, the molecular sieves were filtered out. The filtrate was concentrated to dryness by rotary evaporation, purified by reversed-phase preparative HPLC (column: Boston Green ODS 150 × 30 mm × 5 µm, conditions: 5%-50% (A: water, B: CH₃CN), flow rate: 45 mL/min), and lyophilized to give compound **4-b** (80 mg, 28.3 µmol, yield: 70.8%).

MS m/z: C₁₃₂H₁₉₂N₁₂O₅₅, [M-H]⁺ found: 2824.6.

### Step 3

The compound **4-b** (71mg, 25 µmol) obtained in the previous step was added to acetonitrile (5 mL). Then, HBTU (19.0 mg, 50 µmol) was added, a solid-phase support with an amino modification on the surface (CPG-NH₂, 0.86 g) was added, and DIEA(16.2 mg, 125 µmol, 21.6 µL) was added. The mixture was reacted with shaking at 30 °C for 16 h. After the reaction was completed, the reaction mixture was filtered and washed successively with methanol (5 mL × 4) and dichloromethane (5 mL × 4). The solid was added to pyridine:acetic anhydride (v:v = 4:1, 6.0 mL), and the mixture was reacted with shaking at 30 °C for another 16 h. After the reaction was completed, the reaction mixture was filtered and washed successively with methanol and dichloromethane to give compound **4-c** linked to the solid-phase support (0.74 g).

### Example 10. Preparation of Control Compound L96

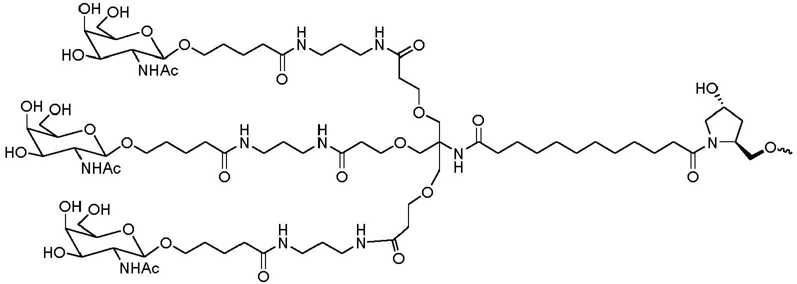

The control compound was prepared by the method described in the patent WO2014025805A1. The compound L96 was obtained by linking the compound to a CPG solid-phase support by the same method as that described above for linking to a solid-phase support.

### Example 11. Synthesis of Galactosamine Molecule Cluster-Conjugated siRNAs

An siRNA used for testing, the siRNA targeting the mRNA of the mouse TTR gene (Molecular Therapy Vol. 26 No 3 March 2018), is shown below. A galactosamine molecule cluster was linked to the 3' end of the SS strand by a covalent bond.

SS strand (5'-3'): CmsAmsGmUmGfUmUfCfUfUmGmCmUmCmUmAmUmAmAm-galactosamine molecule cluster (SEQ ID NO: 243)

AS strand (5'-3'): UmsUfsAmUmAmGfAmGmCmAmAmGmAmAfCmAfCmUmGmsUmsUm (SEQ ID NO: 244)

The synthesis of siRNAs was the same as the conventional phosphoramidite solid-phase synthesis, except that in the synthesis of the SS strand of siRNA, the conventional Universal-CPG support was replaced with the CPG support linked with the galactosamine cluster synthesized above. The synthesis process was briefly described as follows: Nucleoside phosphoramidite monomers were linked one by one according to the synthesis program on a Dr. Oligo48 synthesizer (Biolytic), starting at the synthesized CPG support linked with the galactosamine synthesized above. The nucleoside monomer starting materials 2'-F RNA, 2'-O-methyl RNA, and other nucleoside phosphoramidite monomers were purchased from Hongene, Shanghai or Genepharma, Suzhou. 5-Ethylthio-1*H*-tetrazole (ETT) was used as an activator (a 0.6 M solution in acetonitrile), a 0.22 M solution of PADS in acetonitrile and collidine in a 1:1 volume ratio (Kroma, Suzhou) was used as a sulfurizing agent, and an iodopyridine/water solution (Kroma) was used as an oxidant.

After the solid-phase synthesis was completed, oligoribonucleotides were cleaved from the solid support and soaked in a solution of 28% ammonia water and ethanol (3:1) at 50 °C for 16 h. The mixture was centrifuged, and the supernatant was transferred to another centrifuge tube. After the supernatant was concentrated to dryness by evaporation, the residue was purified by C18 reversed-phase chromatography using 0.1 M TEAA and acetonitrile as the mobile phase, and DMTr was removed using a 3% trifluoroacetic acid solution. The target oligonucleotides were collected, lyophilized, identified as the target products by LC-MS, and quantified by UV (260 nm).

The resulting single-stranded oligonucleotides were paired in an equimolar ratio in a complementary manner and annealed with the AS strand. The final double-stranded siRNA was dissolved in 1 × PBS, and the solution was adjusted to the concentration required for the experiment.

The galactosamine cluster-conjugated siRNAs were synthesized. The siRNAs used in the experiment targeted the mouse TTR mRNA.

The galactosamine molecule cluster is selected from the group consisting of:

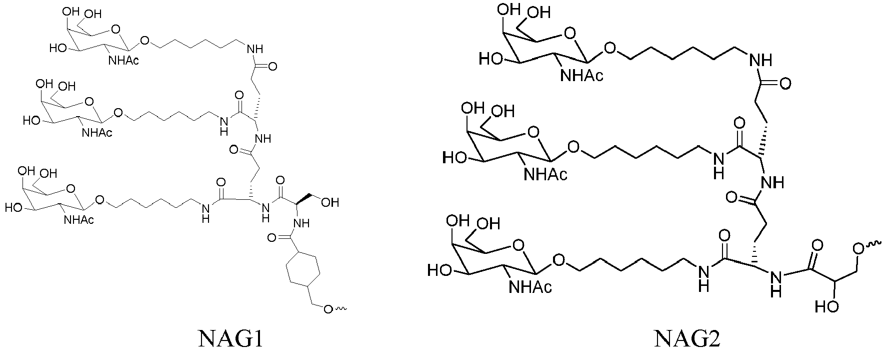

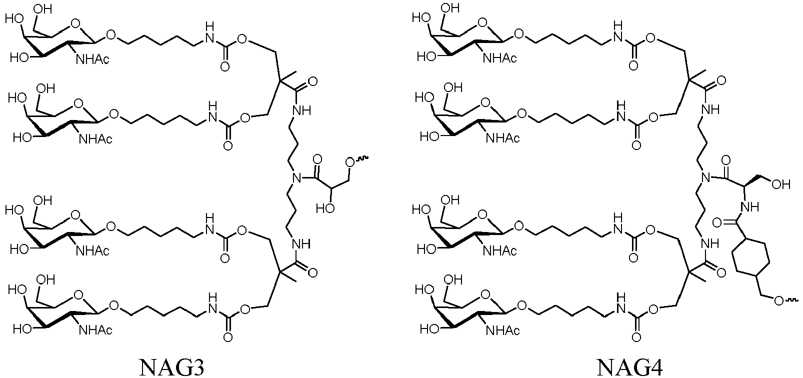

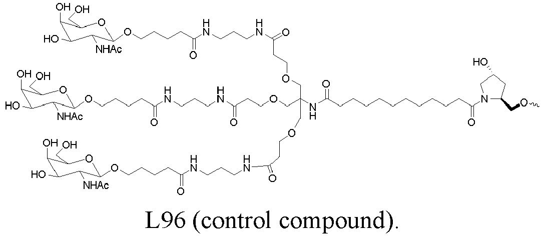

**Table 9. siRNA numbers and sequences in activity evaluation of targeting ligands**

| siRNA number | SS strand (5'-3') | AS strand (5'-3') |
|---|---|---|
| S-1 | CmsAmsGmUmGfUmUfCfUf UmGmCmUmCmUmAmUmAm Am – NAG1 (SEQ ID NO: 245) | UmsUfsAmUmAmGfAmGmCm AmAmGmAmAfCmAfCmUm GmsUmsUm (SEQ ID NO: 250) |
| S-2 | CmsAmsGmUmGfUmUfCfUf UmGmCmUmCmUmAmUmAm Am – NAG2 (SEQ ID NO: 246) | UmsUfsAmUmAmGfAmGmCm AmAmGmAmAfCmAfCmUm GmsUmsUm (SEQ ID NO: 250) |
| S-3 | CmsAmsGmUmGfUmUfCfUf UmGmCmUmCmUmAmUmAm Am – NAG3 (SEQ ID NO: 247) | UmsUfsAmUmAmGfAmGmCm AmAmGmAmAfCmAfCmUm GmsUmsUm (SEQ ID NO: 250) |
| S-4 | CmsAmsGmUmGfUmUfCfUf UmGmCmUmCmUmAmUmAm Am – NAG4 (SEQ ID NO: 248) | UmsUfsAmUmAmGfAmGmCm AmAmGmAmAfCmAfCmUm GmsUmsUm (SEQ ID NO: 250) |
| S-L96 | CmsAmsGmUmGfUmUfCfUf UmGmCmUmCmUmAmUmAm Am -L96 (SEQ ID NO: 249) | UmsUfsAmUmAmGfAmGmCm AmAmGmAmAfCmAfCmUm GmsUmsUm (SEQ ID NO: 250) |

### Example 12. Inhibition of mRNA Expression in Primary Hepatocytes by Galactosamine Molecule Cluster-Conjugated siRNAs

Fresh primary hepatocytes were isolated from mice by the method reported by Severgini et al. (Cytotechnology. 2012; 64(2): 187-195).

After being isolated, the primary hepatocytes were seeded into a 24-well plate at 100,000 cells/well. The test conjugated siRNAs were added at final concentrations of 50 nM, 10 nM, 2 nM, 0.4 nM, 0.08 nM, 0.016 nM, 0.0032 nM, and 0.00064 nM. Subsequently, the primary hepatocytes were cultured at 37 °C with 5% CO₂ for 24 h. After 24 h, the expression level of mTTR's mRNA was determined by the qPCR method.

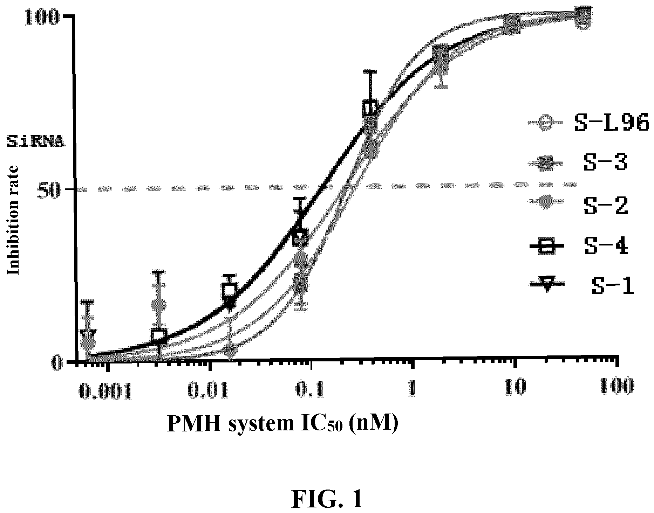

As shown in FIG. 1, S-1, S-2, S-3, and S-4 all exhibited excellent inhibition efficiency against mTTR gene expression. The IC₅₀ values of S-1 and S-4 were lower than those of the other two groups. The IC₅₀ value of the control group S-L96 was 0.280 nM, while the IC₅₀ value of S-1 was 0.131 nM and that of S-4 was 0.135 nM, indicating that S-1 and S-4 had superior efficiency of free uptake by primary hepatocytes *in vitro* than the control group, and that the S-1 and S-4 compounds were able to mediate the entry of siRNA into primary hepatocytes more efficiently.

### Example 13. In Vivo Inhibition of mRNA Expression by Galactosamine Molecule Cluster-Conjugated siRNAs

8-week-old C57BL/6 mice (Joinnbio, SPF, female) were injected subcutaneously with the conjugated siRNAs described above. On day 1, 100 µL of solution containing PBS or a dose (1 mg/kg (mpk) or 0.2 mpk) of a conjugated corresponding siRNA (S-L96, S-3, S-2, S-4, or S-1) formulated in PBS was injected subcutaneously into the loose skin on the neck and shoulder of the mice. In each group, 6 mice were given injections.

Three days after administration, the mice were sacrificed by cervical dislocation, and the expression level of mTTR's mRNA in the liver tissue of the mice were determined by qPCR.

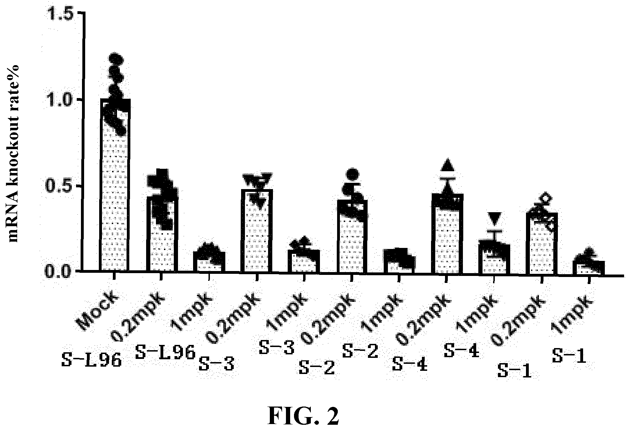

As shown in FIG. 2, S-1, S-2, S-3, and S-4 all exhibited excellent inhibition efficiency against mTTR gene expression. When administered at 1 mpk and 0.2 mpk, S-2, S-3, S-4, and the control group S-L96 showed similar activity. S-1 showed better activity than the control group S-L96 when administered at 1 mpk and 0.2 mpk.

**Table 10. Numbers of the galactosamine molecule cluster compounds**

| Numbers of the galactosamine molecule cluster compounds | Numbers of corresponding conjugated siRNAs |
|---|---|
| NAG1 | S-1 |
| NAG2 | S-2 |
| NAG4 | S-4 |
| NAG3 | S-3 |
| L96 | S-L96 |

### III. Screening and Activity Verification of siRNAs Targeting HSD17B13 and siRNA Conjugates

### Example 14. Design and Synthesis of Human HSD17B13 siRNA siRNA design

Human HSD17B13 gene (NM-178135.5) was used as the target gene to design 19/21nt siRNAs under the condition of meeting the general rules of active siRNA. The sequences of the unmodified sense strands and antisense strands are detailed in Table 11, wherein the SS strands and the AS strands of the unmodified siRNA are both unmodified.

**Table 11. Unmodified sense strands and antisense strands of human HSD siRNAs**

| SEQ ID NO: | Sense strand (5'-3') | SEQ ID NO: | Antisense strand (5'-3') |
|---|---|---|---|
| 3 | GCACCAAGGAUGAAGAGAU | 25 | AUCUCUUCAUCCUUGGUGCUG |
| 4 | CACCAAGGAUGAAGAGAUU | 26 | AAUCUCUUCAUCCUUGGUGCU |
| 5 | ACCAAGGAUGAAGAGAUUA | 27 | UAAUCUCUUCAUCCUUGGUGC |
| 6 | CCAAGGAUGAAGAGAUUAU | 28 | AUAAUCUCUUCAUCCUUGGUG |
| 7 | AGGAUGAAGAGAUUACCAA | 29 | UUGGUAAUCUCUUCAUCCUUG |
| 8 | GGGUUCACCAAAAAUCCAA | 30 | UUGGAUUUUUGGUGAACCCAG |
| 9 | GGUUCACCAAAAAUCCAAA | 31 | UUUGGAUUUUUGGUGAACCCA |
| 10 | CACCAAAAAUCCAAGCACA | 32 | UGUGCUUGGAUUUUUGGUGAA |
| 11 | CAAAAAUCCAAGCACAAGA | 33 | UCUUGUGCUUGGAUUUUUGGU |
| 12 | AAAUCCAAGCACAAGAUUA | 34 | UAAUCUUGUGCUUGGAUUUUU |
| 13 | AAUCCAAGCACAAGAUUAU | 35 | AUAAUCUUGUGCUUGGAUUUU |
| 14 | GCACAAGAUUAUGGCCUGU | 36 | ACAGGCCAUAAUCUUGUGCUU |
| 15 | CACAAGAUUAUGGCCUGUA | 37 | UACAGGCCAUAAUCUUGUGCU |
| 16 | ACAAGAUUAUGGCCUGUAU | 38 | AUACAGGCCAUAAUCUUGUGC |
| 17 | CAAGAUUAUGGCCUGUAUU | 39 | AAUACAGGCCAUAAUCUUGUG |
| 18 | CACAAAAUCAAAAUGAAAU | 40 | AUUUCAUUUUGAUUUUGUGGC |
| 19 | CAAAAUCAAAAUGAAAUGA | 41 | UCAUUUCAUUUUGAUUUUGUG |
| 20 | AAAUCAAAAUGAAAUGAAU | 42 | AUUCAUUUCAUUUUGAUUUUG |
| 21 | AUCAAAAUGAAAUGAAUAA | 43 | UUAUUCAUUUCAUUUUGAUUU |
| 22 | UCAAAAUGAAAUGAAUAAA | 44 | UUUAUUCAUUUCAUUUUGAUU |
| 23 | CAAAAUGAAAUGAAUAAAU | 45 | AUUUAUUCAUUUCAUUUUGAU |
| 24 | AAAUGAAAUGAAUAAAUAA | 46 | UUAUUUAUUCAUUUCAUUUUG |

In the synthesis of a nucleotide with a modification at position 7 of the 5' end of the AS strand, the original nucleotide of the parent sequence was replaced with the phosphoramidite monomer synthesized in Example 1. The sequences of the antisense strand with a modification at position 7 of the 5' end of the AS strand are detailed in Table 12.

The sequences of the sense strands and the antisense strands of the HSD17B13 siRNAs after being modified by 2'-fluoro, 2'-methoxy and the like are detailed in Table 13, the optical changes of the antisense strands with a modification at position 7 are detailed in Table 14, and the sequences of the sense strands and the antisense strands of the HSD17B13 siRNA conjugates are detailed in Table 15.

**Table 12. Unmodified antisense strands and corresponding antisense strands with modifications at position 7 of human HSD siRNAs**

| SEQ ID NO: | UNMODIFIED ANTISENSE STRAND (5'-3') | SEQ ID NO: | ANTISENSE STRAND WITH A MODIFICATION AT POSITION 7 (5'-3') |
|---|---|---|---|
| 25 | AUCUCUUCAUCCUUGGUGCUG | 47 | AUCUCUW'CAUCCUUGGUGCUG |
| 26 | AAUCUCUUCAUCCUUGGUGCU | 48 | AAUCUCW'UCAUCCUUGGUGCU |
| 27 | UAAUCUCUUCAUCCUUGGUGC | 49 | UAAUCUW'UUCAUCCUUGGUGC |
| 28 | AUAAUCUCUUCAUCCUUGGUG | 50 | AUAAUCW'CUUCAUCCUUGGUG |
| 29 | UUGGUAAUCUCUUCAUCCUUG | 51 | UUGGUAW'UCUCUUCAUCCUUG |
| 30 | UUGGAUUUUUGGUGAACCCAG | 52 | UUGGAUW'UUUGGUGAACCCAG |
| 31 | UUUGGAUUUUUGGUGAACCCA | 53 | UUUGGAW'UUUUGGUGAACCCA |
| 32 | UGUGCUUGGAUUUUUGGUGAA | 54 | UGUGCUW'GGAUUUUUGGUGAA |
| 33 | UCUUGUGCUUGGAUUUUUGGU | 55 | UCUUGUW'CUUGGAUUUUUGGU |
| 34 | UAAUCUUGUGCUUGGAUUUUU | 56 | UAAUCUW'GUGCUUGGAUUUUU |
| 35 | AUAAUCUUGUGCUUGGAUUUU | 57 | AUAAUCW'UGUGCUUGGAUUUU |
| 36 | ACAGGCCAUAAUCUUGUGCUU | 58 | ACAGGCW'AUAAUCUUGUGCUU |
| 37 | UACAGGCCAUAAUCUUGUGCU | 59 | UACAGGW'CAUAAUCUUGUGCU |
| 38 | AUACAGGCCAUAAUCUUGUGC | 60 | AUACAGW'CCAUAAUCUUGUGC |
| 39 | AAUACAGGCCAUAAUCUUGUG | 61 | AAUACAW'GCCAUAAUCUUGUG |
| 40 | AUUUCAUUUUGAUUUUGUGGC | 62 | AUUUCAW'UUUGAUUUUGUGGC |
| 41 | UCAUUUCAUUUUGAUUUUGUG | 63 | UCAUUUW'AUUUUGAUUUUGUG |
| 42 | AUUCAUUUCAUUUUGAUUUUG | 64 | AUUCAUW'UCAUUUUGAUUUUG |
| 43 | UUAUUCAUUUCAUUUUGAUUU | 65 | UUAUUCW'UUUCAUUUUGAUUU |
| 44 | UUUAUUCAUUUCAUUUUGAUU | 66 | UUUAUUW'AUUUCAUUUUGAUU |
| 45 | AUUUAUUCAUUUCAUUUUGAU | 67 | AUUUAUW'CAUUUCAUUUUGAU |
| 46 | UUAUUUAUUCAUUUCAUUUUG | 68 | UUAUUUW'UUCAUUUCAUUUUG |

In Table 12, W' represents a nucleotide comprising the chemical modification of formula (I) or formula (I') or the tautomeric modification thereof of the present disclosure. In some embodiments, W' is selected from the group consisting of: 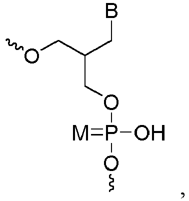 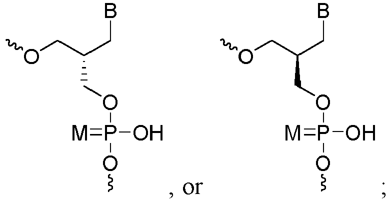 wherein: m is O or S; wherein: B is selected from the group consisting of bases at position 7 of the 5' region of SEQ ID NO: 47 to SEQ ID NO: 68 and SEQ ID NO: 25 to SEQ ID NO: 46 in Table 16, wherein, for example, SEQ ID NO: 47 corresponds to SEQ ID NO: 25, SEQ ID NO: 68 corresponds to SEQ ID NO: 46, and SEQ ID NO: 52 corresponds to SEQ ID NO: 30.

**Table 13. Modified sense strands and antisense strands of human HSD17B13 siRNAs**

| Double strand code | SEQ ID NO: | Sense strand (5'-3') | SEQ ID NO: | Antisense strand (5'-3') |
|---|---|---|---|---|
| TRD005305 | 69 | GmsCmsAm CmCfAm AfGfGf AmUmGm AmAmGm AmGmAm Um | 111 | AmsUfsCm UfCmUf UmCmAf UmCmCf UmUfGm GfUmGf CmsUmsGm |
| TRD005306 | 70 | CmsAmsCm CmAfAm GfGfAf UmGmAm AmGmAm GmAmUm Um | 112 | AmsAfsUm CfUmCf UmUmCf AmUmCf CmUfUm GfGmUf GmsCmsUm |
| TRD005307 | 71 | AmsCmsCm AmAfGm GfAfUf GmAmAm GmAmGm AmUmUm Am | 113 | UmsAfsAm UfCmUf CmUmUf CmAmUf CmCfUm UfGmGf UmsGmsCm |
| TRD005308 | 72 | CmsCmsAm AmGfGm AfUfGf AmAmGm AmGmAm UmUmAm Um | 114 | AmsUfsAm AfUmCf UmCmUf UmCmAf UmCfCm UfUmGf GmsUmsGm |
| TRD005309 | 73 | AmsGmsGm AmUfGm AfAfGf AmGmAm UmUmAm CmCmAm Am | 115 | UmsUfsGm GfUmAf AmUmCf UmCmUf UmCfAm UfCmCf UmsUmsGm |
| TRD005352 | 74 | GmsGmsGm UmUfCm AfCfCf AmAmAm AmAmUm CmCmAm Am | 116 | UmsUfsGm GfAmUf UmUmUf UmGmGf UmGfAm AfCmCf CmsAmsGm |
| TRD005353 | 75 | GmsGmsUm UmCfAm CfCfAf AmAmAm AmUmCm CmAmAm Am | 117 | UmsUfsUm GfGmAf UmUmUf UmUmGf GmUfGm AfAmCf CmsCmsAm |
| TRD005354 | 76 | CmsAmsCm CmAfAm AfAfAf UmCmCm AmAmGm CmAmCm Am | 118 | UmsGfsUm GfCmUf UmGmGf AmUmUf UmUfUm GfGmUf GmsAmsAm |
| TRD005355 | 77 | CmsAmsAm AmAfAm UfCfCf AmAmGm CmAmCm AmAmGm Am | 119 | UmsCfsUm UfGmUf GmCmUf UmGmGf AmUfUm UfUmUf GmsGmsUm |
| TRD005356 | 78 | AmsAmsAm UmCfCm AfAfGf CmAmCm AmAmGm AmUmUm Am | 120 | UmsAfsAm UfCmUf UmGmUf GmCmUf UmGfGm AfUmUf UmsUmsUm |
| TRD005357 | 79 | AmsAmsUm CmCfAm AfGfCf AmCmAm AmGmAm UmUmAm Um | 121 | AmsUfsAm AfUmCf UmUmGf UmGmCf UmUfGm GfAmUf UmsUmsUm |
| TRD005358 | 80 | GmsCmsAm CmAfAm GfAfUf UmAmUm GmGmCm CmUmGm Um | 122 | AmsCfsAm GfGmCf CmAmUf AmAmUf CmUfUm GfUmGf CmsUmsUm |
| TRD005359 | 81 | CmsAmsCm AmAfGm AfUfUf AmUmGm GmCmCm UmGmUm Am | 123 | UmsAfsCm AfGmGf CmCmAf UmAmAf UmCfUm UfGmUf GmsCmsUm |
| TRD005360 | 82 | AmsCmsAm AmGfAm UfUfAf UmGmGm CmCmUm GmUmAm Um | 124 | AmsUfsAm CfAmGf GmCmCf AmUmAf AmUfCm UfUmGf UmsGmsCm |
| TRD005361 | 83 | CmsAmsAm GmAfUm UfAfUf GmGmCm CmUmGm UmAmUm Um | 125 | AmsAfsUm AfCmAf GmGmCf CmAmUf AmAfUm CfUmUf GmsUmsGm |
| TRD005397 | 84 | CmsAmsCm AmAfAm AfUfCf AmAmAm AmUmGm AmAmAm Um | 126 | AmsUfsUm UfCmAf UmUmUf UmGmAf UmUfUm UfGmUf GmsGmsCm |
| TRD005398 | 85 | CmsAmsAm AmAfUm CfAfAf AmAmUm GmAmAm AmUmGm Am | 127 | UmsCfsAm UfUmUf CmAmUf UmUmUf GmAfUm UfUmUf GmsUmsGm |
| TRD005399 | 86 | AmsAmsAm UmCfAm AfAfAf UmGmAm AmAmUm GmAmAm Um | 128 | AmsUfsUm CfAmUf UmUmCf AmUmUf UmUfGm AfUmUf UmsUmsGm |
| TRD005400 | 87 | AmsUmsCm AmAfAm AfUfGf AmAmAm UmGmAm AmUmAm Am | 129 | UmsUfsAm UfUmCf AmUmUf UmCmAf UmUfUm UfGmAf UmsUmsUm |
| TRD005401 | 88 | UmsCmsAm AmAfAm UfGfAf AmAmUm GmAmAm UmAmAm Am | 130 | UmsUfsUm AfUmUf CmAmUf UmUmCf AmUfUm UfUmGf AmsUmsUm |
| TRD005402 | 89 | CmsAmsAm AmAfUm GfAfAf AmUmGm AmAmUm AmAmAm Um | 131 | AmsUfsUm UfAmUf UmCmAf UmUmUf CmAfUm UfUmUf GmsAmsUm |
| TRD005403 | 90 | AmsAmsAm UmGfAm AfAfUf GmAmAm UmAmAm AmUmAm Am | 132 | UmsUfsAm UfUmUf AmUmUf CmAmUf UmUfCm AfUmUf UmsUmsGm |
| | 91 | CmsAmsCm CmAfAm GfGfAf UmGmAm AmGmAm GmAmUms Um | 133 | AmsAfsUm CfUmCf(-)hmpNA(U)UmCm AfUmCf CmUfUm GfGmUf GmsCmsUm |
| | 92 | CmsCmsAm AmGfGm AfUfGf AmAmGm AmGmAm UmUmAms Um | 134 | AmsUfsAm AfUmCf(-)hmpNA(U)CmUm UfCmAf UmCfCm UfUmGf GmsUmsGm |
| | 93 | CmsAmsAm AmAfAm UfCfCf AmAmGm CmAmCm AmAmGms Am | 135 | UmsCfsUm UfGmUf(-)hmpNA(G)CmUm UfGmGf AmUfUm UfUmUf GmsGmsUm |
| | 94 | AmsAmsAm UmGfAm AfAfUf GmAmAm UmAmAm AmUmAms Am | 136 | UmsUfsAm UfUmUf(-)hmpNA(A)UmUm CfAmUf UmUfCm AfUmUf UmsUmsGm |
| | 95 | GmsCmsAm CmCfAm AfGfGf AmUmGm AmAmGm AmGmAm Um | 137 | AmsUfsCm UfCmUf(-)hmpNA(U)CmAf UmCmCf UmUfGm GfUmGf CmsUmsGm |
| | 96 | CmsAmsCm CmAfAm GfGfAf UmGmAm AmGmAm GmAmUm Um | 138 | AmsAfsUm CfUmCf(-)hmpNA(U)UmCf AmUmCf CmUfUm GfGmUf GmsCmsUm |
| | 97 | AmsCmsCm AmAfGm GfAfUf GmAmAm GmAmGm AmUmUm Am | 139 | UmsAfsAm UfCmUf(-)hmpNA(C)UmUf CmAmUf CmCfUm UfGmGf UmsGmsCm |
| | 98 | CmsCmsAm AmGfGm AfUfGf AmAmGm AmGmAm UmUmAm Um | 140 | AmsUfsAm AfUmCf(-)hmpNA(U)CmUf UmCmAf UmCfCm UfUmGf GmsUmsGm |
| | 99 | AmsGmsGm AmUfGm AfAfGf AmGmAm UmUmAm CmCmAm Am | 141 | UmsUfsGm GfUmAf(-)hmpNA(A)UmCf UmCmUf UmCfAm UfCmCf UmsUmsGm |
| | 100 | GmsGmsUm UmCfAm CfCfAf AmAmAm AmUmCm CmAmAm Am | 142 | UmsUfsUm GfGmAf(-)hmpNA(U)UmUf UmUmGf GmUfGm AfAmCf CmsCmsAm |
| | 101 | CmsAmsAm AmAfAm UfCfCf AmAmGm CmAmCm AmAmGm Am | 143 | UmsCfsUm UfGmUf(-)hmpNA(G)CmUf UmGmGf AmUfUm UfUmUf GmsGmsUm |
| | 102 | AmsAmsUm CmCfAm AfGfCf AmCmAm AmGmAm UmUmAm Um | 144 | AmsUfsAm AfUmCf(-)hmpNA(U)UmGf UmGmCf UmUfGm GfAmUf UmsUmsUm |
| | 103 | GmsCmsAm CmAfAm GfAfUf UmAmUm GmGmCm CmUmGm Um | 145 | AmsCfsAm GfGmCf(-)hmpNA(C)AmUf AmAmUf CmUfUm GfUmGf CmsUmsUm |
| | 104 | CmsAmsCm AmAfAm AfUfCf AmAmAm AmUmGm AmAmAm Um | 146 | AmsUfsUm UfCmAf(-)hmpNA(U)UmUf UmGmAf UmUfUm UfGmUf GmsGmsCm |
| | 105 | CmsAmsAm AmAfUm CfAfAf AmAmUm GmAmAm AmUmGm Am | 147 | UmsCfsAm UfUmUf(-)hmpNA(C)AmUf UmUmUf GmAfUm UfUmUf GmsUmsGm |
| | 106 | AmsAmsAm UmCfAm AfAfAf UmGmAm AmAmUm GmAmAm Um | 148 | AmsUfsUm CfAmUf(-)hmpNA(U)UmCf AmUmUf UmUfGm AfUmUf UmsUmsGm |
| | 107 | AmsUmsCm AmAfAm AfUfGf AmAmAm UmGmAm AmUmAm Am | 149 | UmsUfsAm UfUmCf(-)hmpNA(A)UmUf UmCmAf UmUfUm UfGmAf UmsUmsUm |
| | 108 | UmsCmsAm AmAfAm UfGfAf AmAmUm GmAmAm UmAmAm Am | 150 | UmsUfsUm AfUmUf(-)hmpNA(C)AmUf UmUmCf AmUfUm UfUmGf AmsUmsUm |
| | 109 | CmsAmsAm AmAfUm GfAfAf AmUmGm AmAmUm AmAmAm Um | 151 | AmsUfsUm UfAmUf(-)hmpNA(U)CmAf UmUmUf CmAfUm UfUmUf GmsAmsUm |
| | 110 | AmsAmsAm UmGfAm AfAfUf GmAmAm UmAmAm AmUmAm Am | 152 | UmsUfsAm UfUmUf(-)hmpNA(A)UmUf CmAmUf UmUfCm AfUmUf UmsUmsGm |

**Table 14. AS strands with different optical chemical modifications at position 7**

| SEQ ID NO | Antisense strand (5'-3') | SEQ ID NO | Antisense strand (5'-3') |
|---|---|---|---|
| 133 | AmsAfsUm CfUmCf(-)hmpNA(U)UmCm AfUmCf CmUfUm GfGmUf GmsCmsUm | 163 | UmsCfsUm UfGmUf(+)hmpNA(G)CmUf UmGmGf AmUfUm UfUmUf GmsGmsUm |
| 134 | AmsUfsAm AfUmCf(-)hmpNA(U)CmUmUfCmAf UmCfCm UfUmGf GmsUmsGm | 164 | AmsUfsAm AfUmCf(+)hmpNA(U)UmGf UmGmCf UmUfGm GfAmUf UmsUmsUm |
| 135 | UmsCfsUm UfGmUf(-)hmpNA(G)CmUmUfGmGf AmUfUm UfUmUf GmsGmsUm | 165 | AmsCfsAm GfGmCf(+)hmpNA(C)AmUf AmAmUf CmUfUm GfUmGf CmsUmsUm |
| 136 | UmsUfsAm UfUmUf(-)hmpNA(A)UmUm CfAmUf UmUfCm AfUmUf UmsUmsGm | 166 | AmsUfsUm UfCmAf(+)hmpNA(U)UmUf UmGmAf UmUfUm UfGmUf GmsGmsCm |
| 137 | AmsUfsCm UfCmUf(-)hmpNA(U)CmAf UmCmCf UmUfGm GfUmGf CmsUmsGm | 167 | UmsCfsAm UfUmUf(+)hmpNA(C)AmUf UmUmUf GmAfUm UfUmUf GmsUmsGm |
| 138 | AmsAfsUm CfUmCf(-)hmpNA(U)UmCf AmUmCf CmUfUm GfGmUf GmsCmsUm | 168 | AmsUfsUm CfAmUf(+)hmpNA(U)UmCf AmUmUf UmUfGm AfUmUf UmsUmsGm |
| 139 | UmsAfsAm UfCmUf(-)hmpNA(C)UmUf CmAmUf CmCfUm UfGmGf UmsGmsCm | 169 | UmsUfsAm UfUmCf(+)hmpNA(A)UmUf UmCmAf UmUfUm UfGmAf UmsUmsUm |
| 140 | AmsUfsAm AfUmCf(-)hmpNA(U)CmUf UmCmAf UmCfCm UfUmGf GmsUmsGm | 170 | UmsUfsUm AfUmUf(+)hmpNA(C)AmUf UmUmCf AmUfUm UfUmGf AmsUmsUm |
| 141 | UmsUfsGm GfUmAf(-)hmpNA(A)UmCf UmCmUf UmCfAm UfCmCf UmsUmsGm | 171 | AmsUfsUm UfAmUf(+)hmpNA(U)CmAf UmUmUf CmAfUm UfUmUf GmsAmsUm |
| 142 | UmsUfsUm GfGmAf(-)hmpNA(U)UmUf UmUmGf GmUfGm AfAmCf CmsCmsAm | 172 | UmsUfsAm UfUmUf(+)hmpNA(A)UmUf CmAmUf UmUfCm AfUmUf UmsUmsGm |
| 143 | UmsCfsUm UfGmUf(-)hmpNA(G)CmUf UmGmGf AmUfUm UfUmUf GmsGmsUm | 173 | AmsAfsUm CfUmCf hmpNA(U)UmCm AfUmCf CmUfUm GfGmUf GmsCmsUm |
| 144 | AmsUfsAm AfUmCf(-)hmpNA(U)UmGf UmGmCf UmUfGm GfAmUf UmsUmsUm | 174 | AmsUfsAm AfUmCf hmpNA(U)CmUmUfCmAf UmCfCm UfUmGf GmsUmsGm |
| 145 | AmsCfsAm GfGmCf(-)hmpNA(C)AmUf AmAmUf CmUfUm GfUmGf CmsUmsUm | 175 | UmsCfsUm UfGmUf hmpNA(G)CmUmUfGmGf AmUfUm UfUmUf GmsGmsUm |
| 146 | AmsUfsUm UfCmAf(-)hmpNA(U)UmUf UmGmAf UmUfUm UfGmUf GmsGmsCm | 176 | UmsUfsAm UfUmUf hmpNA(A)UmUm CfAmUf UmUfCm AfUmUf UmsUmsGm |
| 147 | UmsCfsAm UfUmUf(-)hmpNA(C)AmUf UmUmUf GmAfUm UfUmUf GmsUmsGm | 177 | AmsUfsCm UfCmUf hmpNA(U)CmAf UmCmCf UmUfGm GfUmGf CmsUmsGm |
| 148 | AmsUfsUm CfAmUf(-)hmpNA(U)UmCf AmUmUf UmUfGm AfUmUf UmsUmsGm | 178 | AmsAfsUm CfUmCf hmpNA(U)UmCf AmUmCf CmUfUm GfGmUf GmsCmsUm |
| 149 | UmsUfsAm UfUmCf(-)hmpNA(A)UmUf UmCmAf UmUfUm UfGmAf UmsUmsUm | 179 | UmsAfsAm UfCmUf hmpNA(C)UmUf CmAmUf CmCfUm UfGmGf UmsGmsCm |
| 150 | UmsUfsUm AfUmUf(-)hmpNA(C)AmUf UmUmCf AmUfUm UfUmGf AmsUmsUm | 180 | AmsUfsAm AfUmCf hmpNA(U)CmUf UmCmAf UmCfCm UfUmGf GmsUmsGm |
| 151 | AmsUfsUm UfAmUf(-)hmpNA(U)CmAf UmUmUf CmAfUm UfUmUf GmsAmsUm | 181 | UmsUfsGm GfUmAf hmpNA(A)UmCf UmCmUf UmCfAm UfCmCf UmsUmsGm |
| 152 | UmsUfsAm UfUmUf(-)hmpNA(A)UmUf CmAmUf UmUfCm AfUmUf UmsUmsGm | 182 | UmsUfsUm GfGmAf hmpNA(U)UmUf UmUmGf GmUfGm AfAmCf CmsCmsAm |
| 153 | AmsAfsUm CfUmCf(+)hmpNA(U)UmCm AfUmCf CmUfUm GfGmUf GmsCmsUm | 183 | UmsCfsUm UfGmUf hmpNA(G)CmUf UmGmGf AmUfUm UfUmUf GmsGmsUm |
| 154 | AmsUfsAm AfUmCf(+)hmpNA(U)CmUmUfCmAf UmCfCm UfUmGf GmsUmsGm | 184 | AmsUfsAm AfUmCf hmpNA(U)UmGf UmGmCf UmUfGm GfAmUf UmsUmsUm |
| 155 | UmsCfsUm UfGmUf(+)hmpNA(G)CmUmUfGmGf AmUfUm UfUmUf GmsGmsUm | 185 | AmsCfsAm GfGmCf hmpNA(C)AmUf AmAmUf CmUfUm GfUmGf CmsUmsUm |
| 156 | UmsUfsAm UfUmUf(+)hmpNA(A)UmUm CfAmUf UmUfCm AfUmUf UmsUmsGm | 186 | AmsUfsUm UfCmAf hmpNA(U)UmUf UmGmAf UmUfUm UfGmUf GmsGmsCm |
| 157 | AmsUfsCm UfCmUf(+)hmpNA(U)CmAf UmCmCf UmUfGm GfUmGf CmsUmsGm | 187 | UmsCfsAm UfUmUf hmpNA(C)AmUf UmUmUf GmAfUm UfUmUf GmsUmsGm |
| 158 | AmsAfsUm CfUmCf(+)hmpNA(U)UmCf AmUmCf CmUfUm GfGmUf GmsCmsUm | 188 | AmsUfsUm CfAmUf hmpNA(U)UmCf AmUmUf UmUfGm AfUmUf UmsUmsGm |
| 159 | UmsAfsAm UfCmUf(+)hmpNA(C)UmUf CmAmUf CmCfUm UfGmGf UmsGmsCm | 189 | UmsUfsAm UfUmCf hmpNA(A)UmUf UmCmAf UmUfUm UfGmAf UmsUmsUm |
| 160 | AmsUfsAm AfUmCf(+)hmpNA(U)CmUf UmCmAf UmCfCm UfUmGf GmsUmsGm | 190 | UmsUfsUm AfUmUf hmpNA(C)AmUf UmUmCf AmUfUm UfUmGf AmsUmsUm |
| 161 | UmsUfsGm GfUmAf(+)hmpNA(A)UmCf UmCmUf UmCfAm UfCmCf UmsUmsGm | 191 | AmsUfsUm UfAmUf hmpNA(U)CmAf UmUmUf CmAfUm UfUmUf GmsAmsUm |
| 162 | UmsUfsUm GfGmAf(+)hmpNA(U)UmUf UmUmGf GmUfGm AfAmCf CmsCmsAm | 192 | UmsUfsAm UfUmUf hmpNA(A)UmUf CmAmUf UmUfCm AfUmUf UmsUmsGm |

**Table 15. Modified sense strands and antisense strands of human HSD17B13 siRNA conjugates**

| Double strand code | SEQ ID NO: | Sense strand (5'-3') | SEQ ID NO: | Antisense strand (5'-3') |
|---|---|---|---|---|
| TRD006019 | 193 | GmsCmsAm CmCfAm AfGfGf AmUmGm AmAmGm AmGmAm Um-NAG1 | 137 | AmsUfsCm UfCmUf(-)hmpNA(U)CmAf UmCmCf UmUfGm GfUmGf CmsUmsGm |
| TRD006020 | 194 | CmsAmsCm CmAfAm GfGfAf UmGmAm AmGmAm GmAmUm Um-NAG1 | 138 | AmsAfsUm CfUmCf(-)hmpNA(U)UmCf AmUmCf CmUfUm GfGmUf GmsCmsUm |
| TRD006021 | 195 | AmsCmsCm AmAfGm GfAfUf GmAmAm GmAmGm AmUmUm Am-NAG1 | 139 | UmsAfsAm UfCmUf(-)hmpNA(C)UmUf CmAmUf CmCfUm UfGmGf UmsGmsCm |
| TRD006022 | 196 | CmsCmsAm AmGfGm AfUfGf AmAmGm AmGmAm UmUmAm Um-NAG1 | 140 | AmsUfsAm AfUmCf(-)hmpNA(U)CmUf UmCmAf UmCfCm UfUmGf GmsUmsGm |
| TRD006023 | 197 | AmsGmsGm AmUfGm AfAfGf AmGmAm UmUmAm CmCmAm Am-NAG1 | 141 | UmsUfsGm GfUmAf(-)hmpNA(A)UmCf UmCmUf UmCfAm UfCmCf UmsUmsGm |
| TRD006030 | 198 | GmsGmsUm UmCfAm CfCfAf AmAmAm AmUmCm CmAmAm Am-NAG1 | 142 | UmsUfsUm GfGmAf(-)hmpNA(U)UmUf UmUmGf GmUfGm AfAmCf CmsCmsAm |
| TRD006031 | 199 | CmsAmsAm AmAfAm UfCfCf AmAmGm CmAmCm AmAmGm Am-NAG1 | 143 | UmsCfsUm UfGmUf(-)hmpNA(G)CmUf UmGmGf AmUfUm UfUmUf GmsGmsUm |
| TRD006032 | 200 | AmsAmsUm CmCfAm AfGfCf AmCmAm AmGmAm UmUmAm Um-NAG1 | 144 | AmsUfsAm AfUmCf(-)hmpNA(U)UmGf UmGmCf UmUfGm GfAmUf UmsUmsUm |
| TRD006033 | 201 | GmsCmsAm CmAfAm GfAfUf UmAmUm GmGmCm CmUmGm Um-NAG1 | 145 | AmsCfsAm GfGmCf(-)hmpNA(C)AmUf AmAmUf CmUfUm GfUmGf CmsUmsUm |
| TRD006051 | 202 | CmsAmsCm AmAfAm AfUfCf AmAmAm AmUmGm AmAmAm Um-NAG1 | 146 | AmsUfsUm UfCmAf(-)hmpNA(U)UmUf UmGmAf UmUfUm UfGmUf GmsGmsCm |
| TRD006052 | 203 | CmsAmsAm AmAfUm CfAfAf AmAmUm GmAmAm AmUmGm Am-NAG1 | 147 | UmsCfsAm UfUmUf(-)hmpNA(C)AmUf UmUmUf GmAfUm UfUmUf GmsUmsGm |
| TRD006053 | 204 | AmsAmsAm UmCfAm AfAfAf UmGmAm AmAmUm GmAmAm Um-NAG1 | 148 | AmsUfsUm CfAmUf(-)hmpNA(U)UmCf AmUmUf UmUfGm AfUmUf UmsUmsGm |
| TRD006054 | 205 | AmsUmsCm AmAfAm AfUfGf AmAmAm UmGmAm AmUmAm Am-NAG1 | 149 | UmsUfsAm UfUmCf(-)hmpNA(A)UmUf UmCmAf UmUfUm UfGmAf UmsUmsUm |
| TRD006055 | 206 | UmsCmsAm AmAfAm UfGfAf AmAmUm GmAmAm UmAmAm Am-NAG1 | 150 | UmsUfsUm AfUmUf(-)hmpNA(C)AmUf UmUmCf AmUfUm UfUmGf AmsUmsUm |
| TRD006056 | 207 | CmsAmsAm AmAfUm GfAfAf AmUmGm AmAmUm AmAmAm Um-NAG1 | 151 | AmsUfsUm UfAmUf(-)hmpNA(U)CmAf UmUmUf CmAfUm UfUmUf GmsAmsUm |
| TRD006057 | 208 | AmsAmsAm UmGfAm AfAfUf GmAmAm UmAmAm AmUmAm Am-NAG1 | 152 | UmsUfsAm UfUmUf(-)hmpNA(A)UmUf CmAmUf UmUfCm AfUmUf UmsUmsGm |
| TRD006941 | 209 | CmsAmsCm CmAfAm GfGfAf UmGmAm AmGmAm GmAmUms Ums-NAG1 | 133 | AmsAfsUm CfUmCf(-)hmpNA(U)UmCm AfUmCf CmUfUm GfGmUf GmsCmsUm |
| TRD006942 | 210 | CmsCmsAm AmGfGm AfUfGf AmAmGm AmGmAm UmUmAms Ums-NAG1 | 134 | AmsUfsAm AfUmCf(-)hmpNA(U)CmUmUfCmA f UmCfCm UfUmGf GmsUmsGm |
| TRD006944 | 211 | CmsAmsAm AmAfAm UfCfCf AmAmGm CmAmCm AmAmGms Ams-NAG1 | 135 | UmsCfsUm UfGmUf(-)hmpNA(G)CmUmUfGmG f AmUfUm UfUmUf GmsGmsUm |
| TRD006947 | 212 | AmsAmsAm UmGfAm AfAfUf GmAmAm UmAmAm AmUmAms Ams-NAG1 | 136 | UmsUfsAm UfUmUf(-)hmpNA(A)UmUm CfAmUf UmUfCm AfUmUf UmsUmsGm |

In Table 12 to Table 15, the nucleotide synthesized using 2-hydroxymethyl-1,3-propanediol as the starting material was defined as hmpNA; hmpNA was a racemic structure;
(-)hmpNA(A) was obtained by solid-phase synthesis using the nucleoside phosphoramidite monomer 1-1a of example section 1.1; (+)hmpNA(A) was an optical isomer;
(-)hmpNA(G) was obtained by solid-phase synthesis using the nucleoside phosphoramidite monomer 1-6a of example section 1.6; (+)hmpNA(G) was an optical isomer;
(-)hmpNA(C) was obtained by solid-phase synthesis using the nucleoside phosphoramidite monomer 1-8a of example section 1.8; (+)hmpNA(C) was an optical isomer;
(-)hmpNA(U) was obtained by solid-phase synthesis using the nucleoside phosphoramidite monomer 1-7a of example section 1.7; (+)hmpNA(U) was an optical isomer.

The lowercase letter m indicates that the upstream nucleotide adjacent to the letter m is a 2'-methoxy-modified nucleotide; the lowercase letter f indicates that the upstream nucleotide adjacent to the letter f is a 2'-fluoro-modified nucleotide;
the lowercase letter s, when present between uppercase letters, indicates that the two nucleotides adjacent to either side of the letter s are linked by a phosphorothioate group; the lowercase letter s, when being the first at the 3' end, indicates that the upstream nucleotide adjacent to the letter s ends in a phosphorothioate group.

### Example 15. psiCHECK Screening for On-Target Activity - Inhibitory Activity at a Single Concentration Point of siRNA sequences

*In vitro* molecular level simulation of screening for on-target activity at a single concentration point (10 nM) was performed on the compounds of the present disclosure in Huh7 cells.

For the antisense strand of siRNA, an on-target plasmid GSCM, which was completely complementary with the antisense strand, was constructed and inserted into a psiCHECK plasmid containing a renilla luciferase gene and a firefly luciferase gene. The plasmid was a dual reporter gene system. The target sequence of siRNA was inserted into the 3' UTR region of the renilla luciferase gene. The activity of siRNA for the target sequence was reflected by determining the renilla luciferase expression after calibration with firefly luciferase. The determination was performed using Dual-Luciferase Reporter Assay System (Promega, E2940).

Huh7 cells were cultured in a DMEM high-glucose medium containing 10% fetal bovine serum at 37 °C with 5% CO₂. 24 h before transfection, the Huh7 cells were seeded into a 96-well plate at a density of 10,000 cells/well with 100 µL of medium each well.

The cells were co-transfected with siRNA and the corresponding plasmid using Lipofectamine2000 (ThermoFisher, 11668019) according to the instructions. 0.2 µL of Lipofectamine2000 was used for each well, the transfection amount of the plasmid was 10 ng/well, and the concentration of siRNA was 10 nM. 24 h after transfection, the on-target level was determined using Dual-Luciferase Reporter Assay System (Promega, E2940). The on-target activity of the test sequences is shown in Table 16.

**Table 16. Results for psiCHECK screening for on-target activity - inhibitory activity at a single concentration point of siRNA sequences**

| Compound No. | Residual expression level of mRNA (10 nM) | SD |
|---|---|---|
| TRD005305 | 15.4% | 2.4% |
| TRD005306 | 14.5% | 3.4% |
| TRD005307 | 23.1% | 1.6% |
| TRD005308 | 19.2% | 1.7% |
| TRD005309 | 13.8% | 2.3% |
| TRD005352 | 57.5% | 12.0% |
| TRD005353 | 20.7% | 0.2% |
| TRD005354 | 37.2% | 8.2% |
| TRD005355 | 18.4% | 3.0% |
| TRD005356 | 63.5% | 4.5% |
| TRD005357 | 13.9% | 4.5% |
| TRD005358 | 19.9% | 2.9% |
| TRD005359 | 65.3% | 3.8% |
| TRD005360 | 29.1% | 0.1% |
| TRD005361 | 38.2% | 0.4% |
| TRD005397 | 16.8% | 2.4% |
| TRD005398 | 15.3% | 0.9% |
| TRD005399 | 13.7% | 0.5% |
| TRD005400 | 17.5% | 2.2% |
| TRD005401 | 11.4% | 1.4% |
| TRD005402 | 12.0% | 1.3% |
| TRD005403 | 8.4% | 2.0% |

### Example 16. psiCHECK Screening for On-Target Activity - Inhibitory Activity at Five Concentration Points of siRNAs

*In vitro* molecular level simulation of screening for on-target activity at 5 concentration points was performed on siRNAs in HEK 293A cells.

HEK 293A cells were cultured in a DMEM high-glucose medium containing 10% fetal bovine serum at 37 °C with 5% CO₂. 24 h before transfection, the HEK 293A cells were seeded into a 96-well plate at a density of 8000 cells/well with 100 µL of medium each well.

The cells were co-transfected with siRNA and the corresponding plasmid using Lipofectamine2000 (ThermoFisher, 11668019) according to the instructions. 0.2 µL of Lipofectamine2000 was used for each well, the transfection amount of the plasmid was 10 ng/well, and a total of 5 concentration points were set for siRNA, which were obtained by 10-fold gradient dilution, with the final concentration of the highest concentration point being 10 nM. 24 h after transfection, the on-target level was determined using Dual-Luciferase Reporter Assay System (Promega, E2940).

The results in Table 17 showed that the mRNA expression level of HSD17B13 treated with an siRNA was reduced in a dose-dependent manner, and the siRNA had a high level of *in vitro* on-target inhibitory activity.

**Table 17. Results for psiCHECK screening for on-target activity - inhibitory activity at five concentration points of siRNAs**

| Compound No. | Percentage of residual expression of target gene's mRNA (mean) | | | | | IC₅₀ value (nM) |
|---|---|---|---|---|---|---|
| | 10nM | 1nM | 0.1nM | 0.01nM | 0.001nM | |
| TRD005305 | 8.4% | 7.7% | 15.0% | 47.0% | 89.1% | 0.0081 |
| TRD005306 | 6.3% | 6.2% | 7.9% | 25.7% | 69.0% | 0.0028 |
| TRD005307 | 11.6% | 9.9% | 15.8% | 51.0% | 88.3% | 0.0098 |
| TRD005308 | 9.6% | 4.6% | 6.0% | 14.9% | 51.9% | 0.0041 |
| TRD005309 | 6.4% | 6.3% | 15.2% | 54.4% | 84.8% | 0.0117 |
| TRD005353 | 10.0% | 7.3% | 10.0% | 33.1% | 78.2% | 0.0051 |
| TRD005355 | 9.5% | 7.1% | 12.9% | 60.2% | 111.1% | 0.0141 |
| TRD005357 | 4.5% | 4.4% | 8.9% | 36.1% | 92.8% | 0.0056 |
| TRD005358 | 14.9% | 8.0% | 11.1% | 39.1% | 90.7% | 0.0089 |
| TRD005397 | 8.2% | 4.3% | 6.0% | 20.6% | 62.4% | 0.0059 |
| TRD005398 | 10.6% | 4.1% | 8.0% | 45.3% | 86.6% | 0.0085 |
| TRD005399 | 6.0% | 3.8% | 4.2% | 11.3% | 40.1% | <0.001 |
| TRD005400 | 7.1% | 4.0% | 4.7% | 22.4% | 66.9% | 0.0065 |
| TRD005401 | 6.8% | 7.1% | 9.7% | 35.9% | 95.8% | 0.0056 |
| TRD005402 | 3.2% | 3.0% | 4.4% | 12.6% | 56.0% | 0.0012 |
| TRD005403 | 2.4% | 2.7% | 6.8% | 33.5% | 84.5% | 0.0049 |

### Example 17. psiCHECK Screening for On-Target Activity - Inhibitory Activity at 11 Concentration Points of Conjugated siRNAs

After modifications of the conjugated siRNAs (modifications at position 7 of the antisense strands), *in vitro* molecular level simulation of screening for on-target activity was performed in HEK 293A cells using 11 concentration points.

HEK 293A cells were cultured in a DMEM high-glucose medium containing 10% fetal bovine serum at 37 °C with 5% CO₂. 24 h before transfection, the HEK 293A cells were seeded into a 96-well plate at a density of 8000 cells/well with 100 µL of medium each well.

The cells were co-transfected with a conjugated siRNA and the corresponding plasmid using Lipofectamine2000 (ThermoFisher, 11668019) according to the instructions. 0.2 µL of Lipofectamine2000 was used for each well, the transfection amount of the plasmid was 10 ng/well, and a total of 11 concentration points were set for the conjugated siRNA, which were obtained by 3-fold gradient dilution, with the final concentration of the highest concentration point being 20 nM. 24 h after transfection, the on-target level was determined using Dual-Luciferase Reporter Assay System (Promega, E2940).

The results in Table 18 showed that the mRNA level of HSD17B13 treated with a conjugated siRNA was reduced in a dose-dependent manner, and the conjugated siRNA had a high level of *in vitro* on-target inhibitory activity.

**Table 18. Results for psiCHECK screening for on-target activity - inhibitory activity at 11 concentration points of conjugated siRNAs**

| Compound No. | Percentage of residual expression of target gene's mRNA (mean) | | | | | |
|---|---|---|---|---|---|---|
| | 20nM | 6.667nM | 2.222nM | 0.741nM | 0.247nM | 0.082nM |
| TRD006019 | 9.9% | 8.9% | 8.0% | 8.3% | 13.4% | 26.4% |
| TRD006020 | 5.9% | 5.8% | 6.8% | 6.4% | 9.2% | 15.4% |
| TRD006021 | 11.7% | 12.3% | 13.2% | 16.0% | 28.7% | 48.9% |
| TRD006022 | 8.8% | 5.1% | 4.7% | 5.1% | 7.4% | 14.6% |
| TRD006023 | 7.5% | 5.4% | 6.1% | 7.9% | 14.2% | 31.9% |
| TRD006030 | 45.5% | 26.9% | 19.1% | 15.6% | 20.5% | 38.3% |
| TRD006031 | 10.3% | 7.6% | 6.0% | 5.9% | 8.8% | 22.0% |
| TRD006032 | 5.0% | 4.9% | 5.3% | 5.3% | 7.0% | 12.2% |
| TRD006033 | 9.4% | 7.1% | 5.9% | 6.3% | 11.0% | 24.3% |
| TRD006051 | 8.2% | 6.9% | 7.1% | 8.2% | 11.8% | 21.3% |
| TRD006052 | 9.3% | 11.2% | 17.7% | 31.3% | 56.7% | 71.4% |
| TRD006053 | 31.9% | 28.5% | 27.8% | 31.8% | 44.3% | 61.6% |
| TRD006054 | 4.6% | 4.0% | 5.0% | 7.0% | 14.5% | 37.2% |
| TRD006055 | 7.3% | 8.8% | 9.6% | 14.2% | 25.1% | 48.7% |
| TRD006056 | 41.9% | 44.3% | 46.6% | 52.0% | 67.6% | 86.0% |
| TRD006057 | 3.9% | 3.6% | 3.9% | 3.6% | 7.4% | 17.9% |

| Compound No. | Percentage of residual expression of target gene's mRNA (mean) | | | | | IC₅₀ values (nM) |
|---|---|---|---|---|---|---|
| | 0.027nM | 0.009nM | 0.003nM | 0.001nM | 0.0003nM | |
| TRD006019 | 50.9% | 72.3% | 89.4% | 94.6% | 86.7% | 0.0282 |
| TRD006020 | 34.2% | 57.3% | 80.3% | 86.5% | 89.6% | 0.0133 |
| TRD006021 | 74.7% | 92.5% | 93.1% | 101.8% | 99.1% | 0.082 |
| TRD006022 | 32.3% | 58.3% | 80.1% | 88.1% | 85.7% | 0.0133 |
| TRD006023 | 60.4% | 76.9% | 88.1% | 89.4% | 89.5% | 0.0398 |
| TRD006030 | 63.3% | 81.4% | 96.1% | 97.9% | 98.5% | 0.041 |
| TRD006031 | 46.8% | 77.4% | 95.6% | 96.6% | 93.7% | 0.0251 |
| TRD006032 | 28.6% | 58.7% | 81.8% | 102.5% | 98.5% | 0.012 |
| TRD006033 | 54.6% | 79.4% | 91.1% | 93.5% | 100.3% | 0.0304 |
| TRD006051 | 48.3% | 78.0% | 90.3% | 90.0% | 96.1% | 0.0265 |
| TRD006052 | 91.9% | 97.6% | 98.0% | 94.1% | 95.1% | 0.302 |
| TRD006053 | 80.9% | 84.2% | 95.4% | 97.1% | 95.7% | 0.1549 |
| TRD006054 | 62.9% | 82.3% | 91.7% | 94.2% | 95.7% | 0.0468 |
| TRD006055 | 72.8% | 87.8% | 96.7% | 95.7% | 95.2% | 0.0764 |
| TRD006056 | 83.8% | 90.5% | 90.7% | 88.6% | 91.5% | 0.955 |
| TRD006057 | 38.5% | 64.5% | 84.9% | 95.8% | 97.0% | 0.0167 |

### Example 18. psiCHECK Off-Target Level Verification of AS Strands of Conjugated siRNAs

*In vitro* molecular level simulation of screening for off-target level was performed in HEK 293A cells using 11 concentration gradients. The experimental results are shown in Table 19.

A corresponding off-target sequence was constructed for each of siRNAs, that is, an off-target plasmid GSSM, which was completely complementary with positions 1-8 of the 5' end of the antisense strand and had completely unmatched bases at other positions, was constructed. Base mispairing should be based on the rules of A to C and G to T. In order to improve the detection sensitivity, a GSSM-5hits off-target plasmid, which was composed of 5 identical GSSM sequences connected through TTCC, was constructed and inserted into a psiCHECK plasmid containing a renilla luciferase gene and a firefly luciferase gene. The plasmid was a dual reporter gene system. The target sequence of siRNA was inserted into the 3' UTR region of the renilla luciferase gene. The activity of siRNA for the target sequence was reflected by determining the renilla luciferase expression after calibration with firefly luciferase. The determination was performed using Dual-Luciferase Reporter Assay System (Promega, E2940).

HEK 293A cells were cultured in a DMEM high-glucose medium containing 10% fetal bovine serum at 37 °C with 5% CO₂. 24 h before transfection, the HEK 293A cells were seeded into a 96-well plate at a density of 8000 cells/well with 100 µL of medium each well.

The cells were co-transfected with a conjugated siRNA and the corresponding plasmid using Lipofectamine2000 (ThermoFisher, 11668019) according to the instructions. 0.2 µL of Lipofectamine2000 was used for each well. The transfection amount of the plasmid was 10 ng/well. For the off-target plasmid, a total of 11 concentration points were set for the conjugated siRNA, which were obtained by 3-fold gradient dilution, with the final concentration of the highest concentration point being 20 nM. 24 h after transfection, the off-target level was determined using Dual-Luciferase Reporter Assay System (Promega, E2940).

**Table 19. Results for psiCHECK screening for off-target activity of seed regions of the antisense strands of the conjugated siRNAs (GSSM-5 hits)**

| Double strand code | Percentage of residual expression of target gene's mRNA (GSSM-5hits) (mean) | | | | | | | | | | | IC₅₀ value (nM) |
|---|---|---|---|---|---|---|---|---|---|---|---|---|
| | 20 nM | 6.667 nM | 2.222 nM | 0.741 nM | 0.247 nM | 0.082 nM | 0.0274 nM | 0.0091 nM | 0.003 nM | 0.001 nM | 0.0003 nM | |
| TRD006 019 | 91.2% | 91.9% | 94.6% | 93.1% | 91.6% | 99.1% | 95.4% | 97.6% | 96.3% | 97.6% | 96.6% | 155.9 |
| TRD006 020 | 50.7% | 56.0% | 67.8% | 86.8% | 102.5% | 103.9% | 102.5% | 104.4% | 102.5% | 107.3% | 109.4% | 10.6 |
| TRD006 022 | 90.9% | 88.2% | 88.1% | 92.4% | 100.7% | 107.9% | 106.7% | 106.0% | 105.3% | 106.7% | 109.0% | 127.0 |
| TRD006 031 | 117.6% | 99.8% | 101.4% | 101.1% | 98.2% | 97.2% | 96.8% | 94.6% | 95.5% | 101.5% | 102.5% | NA |
| TRD006 032 | 65.1% | 67.9% | 76.3% | 86.2% | 91.0% | 95.6% | 95.2% | 96.5% | 97.1% | 99.1% | 105.1% | 21.9 |
| TRD006 033 | 87.0% | 84.2% | 87.7% | 93.2% | 97.2% | 100.5% | 100.8% | 99.8% | 99.9% | 101.7% | 99.7% | 87.1 |
| TRD006 051 | 80.9% | 69.2% | 71.4% | 83.2% | 83.5% | 89.9% | 89.5% | 94.3% | 95.8% | 95.3% | 97.5% | 36.9 |
| TRD006 056 | 68.0% | 81.5% | 89.7% | 102.2% | 102.7% | 103.7% | 107.4% | 102.9% | 106.2% | 105.3% | 102.6% | 37.6 |
| TRD006 057 | 76.6% | 82.4% | 89.1% | 93.7% | 97.2% | 98.1% | 95.2% | 93.6% | 104.0% | 99.8% | 97.8% | 50.9 |
| NA indicates no off-target activity. | | | | | | | | | | | | |

### Example 19. Inhibition of Human HSD17B13 in Primary Human Hepatocytes (PHHs) by Conjugated siRNAs - Inhibitory Activity at 5 Concentration Points

Primary human hepatocyte (PHH) activity screening was performed on siRNAs in primary human hepatocytes (PHHs) using 5 concentration gradients.

The primary human hepatocytes (PHHs) were cryopreserved in liquid nitrogen. 24 h before transfection, the primary human hepatocytes (PHHs) were thawed and then seeded into a 96-well plate at a density of 40,000 cells/well with 100 µL of medium in each well. The siRNAs were each transfected with Lipofectamine RNAi MAX (ThermoFisher, 13778150). A total of 5 concentration points were set for the siRNA, which were obtained by 10-fold gradient dilution, with the final concentration of the highest concentration point being 10 nM. 24 h after transfection, total cellular RNA was extracted from the cells using a high-throughput cellular RNA extraction kit (FireGen, FG0417), and reverse transcription was performed using an RNA reverse transcription kit (Takara, 6210A). The mRNA expression level of human HSD17B13 was determined using a Taqman probe Q-PCR kit (ThermoFisher, 4444964). The experimental procedures were performed according to the instructions of the product. GAPDH was used as an internal reference gene in the experiment. The information on Taqman probe primers is shown in Table 20, and the working concentration of the primers is 10 µM. Corresponding Ct values were acquired according to a threshold value automatically set by a system to achieve the relative quantification of the gene expression, and data was processed using a 2-△△Ct method. The results are expressed relative to the percentage of residual expression of mRNA of human HSD17B13 in cells treated with the control conjugated siRNA. The IC₅₀ results for the inhibition rate are shown in Table 21. △△Ct = [(Ct_{target gene of the experimental group} - Ctᵢₙₜₑᵣₙₐₗ reference of the experimental group) - (Cttarget gene of the control group - Ctinternal reference of the _{control group})]. Inhibition rate (%) = (1 - residual expression amount of target gene) × 100%.

**Table 20. Information on Taqman probe primers**

| Primer name | Brand | Cat. No. |
|---|---|---|
| HSD17B13 Human probe | Thermo | Hs01068199_ml |
| Human GAPDH TaqMan Probe | Thermo | 4326317E |

**Table 21. Results for inhibitory activity at five concentration points of the conjugated siRNAs**

| against human HSD17B13 in primary human hepatocytes (PHHs) | | | | | | |
|---|---|---|---|---|---|---|
| Compound No. | Percentage of residual expression of target gene's mRNA (PHH) (mean) | | | | | IC₅₀ values (nM) |
| | 10nM | 1nM | 0.1nM | 0.01nM | 0.001nM | |
| TRD006020 | 20.1% | 16.4% | 26.8% | 50.5% | 74.9% | 0.0107 |
| TRD006022 | 14.8% | 14.0% | 26.5% | 58.8% | 71.8% | 0.0190 |
| TRD006031 | 11.1% | 17.4% | 41.8% | 50.4% | 77.1% | 0.0162 |
| TRD006032 | 11.2% | 14.8% | 34.4% | 81.4% | 132.4% | 0.0391 |
| TRD006057 | 6.7% | 10.2% | 17.5% | 49.3% | 59.9% | 0.0097 |

### Example 20. psiCHECK Off-Target Level Verification of SS Strands of Conjugated siRNAs

*In vitro* molecular level simulation of screening for off-target levels of the conjugated siRNAs in HEK 293A cells was performed using 11 concentration gradients. The experimental results are shown in Tables 22 and 23.

A corresponding off-target sequence was constructed for the SS strand of each of the siRNAs, and inserted into a psiCHECK plasmid. The plasmid contained a renilla luciferase gene and a firefly luciferase gene. The plasmid was a dual reporter gene system. The target sequence of siRNA was inserted into the 3' UTR region of the renilla luciferase gene. The activity of siRNA for the target sequence was reflected by determining the renilla luciferase expression after calibration with firefly luciferase. The determination was performed using Dual-Luciferase Reporter Assay System (Promega, E2940).

The construction rule of the target plasmid corresponding to the siRNA was as follows: An off-target plasmid PSCM, which was completely complementary with the SS strand, was constructed for the sense strand of each of the siRNAs. An off-target plasmid PSSM, which was completely complementary with positions 1-8 of 5' end of the sense strand and had completely unmatched bases at other positions, was constructed. Base mispairing should be based on the rules of A to C and G to T. In order to improve the detection sensitivity, a PSSM-5hits off-target plasmid, which was composed of 5 identical PSSM sequences connected through TTCC, was constructed.

HEK 293A cells were cultured in a DMEM high-glucose medium containing 10% fetal bovine serum at 37 °C with 5% CO₂. 24 h before transfection, the HEK 293A cells were seeded into a 96-well plate at a density of 8000 cells/well with 100 µL of medium each well.

The cells were co-transfected with a conjugated siRNA and the corresponding plasmid using Lipofectamine2000 (ThermoFisher, 11668019) according to the instructions. 0.2 µL of Lipofectamine2000 was used for each well. The transfection amount of the plasmid was 10 ng/well. For the off-target plasmid, a total of 11 concentration points were set for the conjugated siRNA, which were obtained by 3-fold gradient dilution, with the final concentration of the highest concentration point being 20 nM. 24 h after transfection, the off-target level was determined using Dual-Luciferase Reporter Assay System (Promega, E2940).

The results are shown in Tables 22 and 23, which showed that the conjugated siRNAs did not show a significant off-target effect.

**Table 22. Results for psiCHECK screening for off-target activity of the sense strands of the conjugated siRNAs (PSCM)**

| Compound No. | Percentage of residual expression of target gene's mRNA (PSCM) (mean) | | | | | |
|---|---|---|---|---|---|---|
| | 20nM | 6.667nM | 2.222nM | 0.741nM | 0.247nM | 0.082nM |
| TRD006020 | 80.6% | 89.1% | 97.4% | 98.9% | 104.4% | 107.6% |
| TRD006022 | 74.6% | 74.2% | 79.3% | 91.8% | 101.8% | 108.2% |
| TRD006031 | 99.3% | 94.5% | 96.1% | 96.2% | 105.0% | 105.1% |
| TRD006057 | 92.8% | 99.9% | 99.9% | 107.4% | 123.4% | 111.7% |

| Compound No. | Percentage of residual expression of target gene's mRNA (PSCM) (mean) | | | | | |
|---|---|---|---|---|---|---|
| | 0.027nM | 0.009nM | 0.003nM | 0.001nM | 0.0003nM | IC₅₀ value (nM) |
| TRD006020 | 103.6% | 100.6% | 103.1% | 98.8% | 96.0% | 77.27 |
| TRD006022 | 105.9% | 107.0% | 108.8% | 104.9% | 105.2% | 36.87 |
| TRD006031 | 103.0% | 100.5% | 103.4% | 105.0% | 104.2% | 695.5 |
| TRD006057 | 108.4% | 112.9% | 113.2% | 116.1% | 108.1% | 327.3 |

**Table 23. Results for psiCHECK screening for off-target activity of the sense strands of the conjugated siRNAs (PSSM-5hits)**

| Compound No. | Percentage of residual expression of target gene's mRNA (PSCM) (mean) | | | | | |
|---|---|---|---|---|---|---|
| | 20nM | 6.667nM | 2.222nM | 0.741nM | 0.247nM | 0.082nM |
| TRD006020 | 92.9% | 95.0% | 104.4% | 108.5% | 105.2% | 106.8% |
| TRD006022 | 88.9% | 75.5% | 81.8% | 89.4% | 99.9% | 101.4% |
| TRD006031 | 103.4% | 97.6% | 97.1% | 102.5% | 100.0% | 106.1% |
| TRD006057 | 111.6% | 110.5% | 108.5% | 110.4% | 99.8% | 96.1% |

| Compound No. | Percentage of residual expression of target gene's mRNA (PSCM) (mean) | | | | | |
|---|---|---|---|---|---|---|
| | 0.027nM | 0.009nM | 0.003nM | 0.001nM | 0.0003nM | IC₅₀ value (nM) |
| TRD006020 | 108.3% | 104.3% | 105.7% | 107.0% | 99.9% | 268.1 |
| TRD006022 | 104.5% | 102.8% | 106.7% | 100.6% | 96.8% | 72.99 |
| TRD006031 | 108.8% | 104.3% | 108.1% | 101.1% | 100.9% | NA |
| TRD006057 | 98.3% | 103.3% | 103.8% | 101.3% | 101.5% | NA |

In Table 23, NA indicates no significant off-target activity.

### Example 21. Inhibition of Human HSD17B13 in Primary Human Hepatocytes (PHHs) by Conjugated siRNAs - Inhibitory Activity at Multiple Concentration Points

Primary human hepatocyte (PHH) activity screening was performed on siRNAs in primary human hepatocytes (PHHs) using multiple concentration gradients.

The primary human hepatocytes (PHHs) were cryopreserved in liquid nitrogen. 24 h before transfection, the primary human hepatocytes (PHHs) were thawed and then seeded into a 96-well plate at a density of 4×10⁴ cells/well with 100 µL of medium in each well. The conjugated siRNA was transfected using Lipofectamine RNAi MAX (ThermoFisher, 13778150) according to the instructions of the product. A total of 11 or 7 concentration points were set for the siRNA, which were obtained by 3-fold or 5-fold gradient dilution, with the final concentration of the highest concentration point being 10 nM. 24 h after treatment, total cellular RNA was extracted from the cells using a high-throughput cellular RNA extraction kit, and RNA reverse transcription and quantitative real-time PCR detection were performed. The mRNA level of human HSD17B13 was determined and corrected based on the level of the GAPDH internal reference gene.

The results are expressed relative to the percentage of residual expression of mRNA of human HSD17B13 in cells treated with the control siRNA. The IC₅₀ results for the inhibition rate are shown in Tables 24 and 25.

**Table 24. Inhibitory activity at 11 concentration points of the conjugated siRNAs against human HSD17B13 in primary human hepatocytes (PHHs)**

| Compound No. | Percentage of residual expression of target gene's mRNA (PHH) (mean) | | | | | | | | | | | ICso values (nM) |
|---|---|---|---|---|---|---|---|---|---|---|---|---|
| | 10nM | 3.333nM | 1.111nM | 0.370nM | 0.123nM | 0.0411nM | 0.0137nM | 0.0046nM | 0.0015nM | 0.0005nM | 0.00017nM | |
| TRD006020 | 15.0% | 12.4% | 13.5% | 16.0% | 25.2% | 38.0% | 47.3% | 62.9% | 93.4% | 97.8% | 112.3% | 0.0129 |
| TRD006022 | 12.2% | 12.0% | 13.6% | 18.5% | 27.8% | 35.4% | 52.8% | 63.3% | 76.0% | 70.3% | 91.6% | 0.0141 |
| TRD006031 | 11.5% | 11.8% | 14.2% | 21.0% | 32.2% | 40.6% | 50.3% | 59.9% | 63.9% | 78.0% | 97.9% | 0.0100 |
| TRD006057 | 8.9% | 11.5% | 18.5% | 26.0% | 41.7% | 55.0% | 68.4% | 79.3% | 104.6% | 109.9% | 81.0% | 0.0201 |

**Table 25. Inhibitory activity at 7 concentration points of the conjugated siRNAs against human HSD17B13 in primary human hepatocytes (PHHs)**

| Compound | Percentage of residual expression of target gene's mRNA (PHH) (mean) | | | | | | | IC₅₀ |
|---|---|---|---|---|---|---|---|---|
| No. | 10nM | 2nM | 0.4nM | 0.08nM | 0.016nM | 0.0032nM | 0.00064nM | values (nM) |
| TRD006941 | 15.7% | 17.4% | 15.8% | 27.5% | 64.1% | 99.6% | 103.6% | 0.0251 |
| TRD006942 | 8.2% | 10.5% | 13.7% | 24.3% | 71.4% | 104.7% | 77.6% | 0.0324 |
| TRD006944 | 9.7% | 9.1% | 14.5% | 38.9% | 78.7% | 87.5% | 80.0% | 0.0566 |
| TRD006947 | 5.4% | 8.7% | 14.2% | 35.6% | 71.2% | 106.6% | 86.5% | 0.0438 |

Although the foregoing invention has been described in detail by way of drawings and examples for purposes of clarity of understanding, the description and examples should not be construed as limiting the scope of the present disclosure. The disclosures of all patents and scientific literature cited herein are clearly incorporated by reference in their entirety.

## Claims

1. An siRNA, comprising a sense strand and an antisense strand forming a double-stranded region, wherein:
the sense strand comprises at least 15 contiguous nucleotides and differs from any one of nucleotide sequences of SEQ ID NOs: 24, 4, 6, 11, 3, 5, 7, 8, 9, 10, 12, 13, 14, 15, 16, 17, 18, 19, 20, 21, 22, and 23 by no more than 3 nucleotides;
the antisense strand comprises at least 15 contiguous nucleotides and differs from any one of nucleotide sequences of SEQ ID NOs: 46, 26, 28, 33, 25, 27, 29, 30, 31, 32, 34, 35, 36, 37, 38, 39, 40, 41, 42, 43, 44, and 45 by no more than 3 nucleotides.

2. The siRNA according to claim 1, wherein:
the sense strand comprises at least 17 contiguous nucleotides of a nucleotide sequence selected from any one of SEQ ID NO: 3 to SEQ ID NO: 24;
the antisense strand comprises at least 19 contiguous nucleotides of a nucleotide sequence selected from any one of SEQ ID NO: 25 to SEQ ID NO: 46;
preferably, the sense strand comprises a nucleotide sequence selected from any one of SEQ ID NO: 3 to SEQ ID NO: 24;
preferably, the antisense strand comprises a nucleotide sequence selected from any one of SEQ ID NO: 25 to SEQ ID NO: 46.

3. The siRNA according to claim 1 or 2, comprising strands selected from any one of the following groups:
group 1), a sense strand set forth in SEQ ID NO: 4 and an antisense strand set forth in SEQ ID NO: 26;
group 2), a sense strand set forth in SEQ ID NO: 6 and an antisense strand set forth in SEQ ID NO: 28;
group 3), a sense strand set forth in SEQ ID NO: 11 and an antisense strand set forth in SEQ ID NO: 33; and
group 4), a sense strand set forth in SEQ ID NO: 24 and an antisense strand set forth in SEQ ID NO: 46.

4. The siRNA according to any one of the preceding claims, wherein at least one nucleotide in the sense and/or antisense strand is a modified nucleotide.

5. The siRNA according to any one of the preceding claims, wherein the antisense strand comprises a chemical modification of formula (I) or a tautomeric modification thereof in at least one nucleotide at positions 2 to 8 of the 5' region thereof: 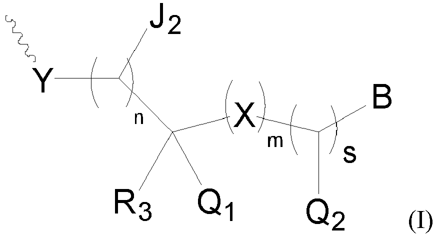 wherein: Y is selected from the group consisting of O, NH, and S;
each X is independently selected from the group consisting of CR₄(R₄'), S, NRs, and NH-CO, wherein R₄, R₄', and R₅ are each independently H or C₁-C₆ alkyl;
J₂ is H or C₁-C₆ alkyl;
n = 0, 1, or 2;
m = 0, 1, or 2;
s = 0 or 1;
R₃ is selected from the group consisting of H, OH, halogen, NH₂, C₁-C₆ alkyl, C₁-C₆ alkoxy, C₂-C₆ alkenyl, C₂-C₆ alkynyl, S-CH₃, NCH₃(CH₃), OCH₂CH₂OCH₃, -O-alkylamino, and (CH₂)ₚR₆, wherein R₆ is selected from the group consisting of OH, halogen, methoxy, ethoxy, N₃, C₂-C₆ alkenyl, and C₂-C₆ alkynyl, and p = 1, 2, or 3;
Q₁ is 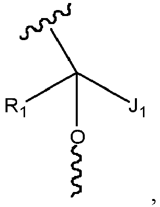 and Q₂ is R₂; or
Q₁ is R₂, and Q₂ is 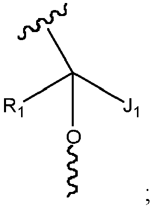 wherein:
R₁ is selected from the group consisting of H, C₁-C₆ alkyl, C₁-C₆ alkoxy, C₂-C₆ alkenyl, C₂-C₆ alkynyl, and (CH₂)_{q}R₇, wherein R₇ is selected from the group consisting of OH, halogen, methoxy, ethoxy, N₃, C₂-C₆ alkenyl, and C₂-C₆ alkynyl, and q = 1, 2, or 3;
Ji is H or C₁-C₆ alkyl;
R₂ is selected from the group consisting of H, OH, halogen, NH₂, C₁-C₆ alkyl, C₁-C₆ alkoxy, C₂-C₆ alkenyl, C₂-C₆ alkynyl, S-CH₃, NCH₃(CH₃), OCH₂CH₂OCH₃, -O-alkylamino, and (CH₂)ᵣR₈, wherein R₈ is selected from the group consisting of OH, halogen, methoxy, ethoxy, N₃, C₂-C₆ alkenyl, and C₂-C₆ alkynyl, and r = 1, 2, or 3; optionally, R₁ and R₂ are directly connected to form a ring;
B is a base;
wherein: the chemical modification of formula (I) or the tautomeric modification thereof is not 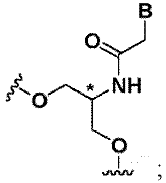
preferably, when X is NH-CO, R₁ is not H.

6. The siRNA according to any one of the preceding claims, wherein the antisense strand comprises a chemical modification of formula (I-1) or formula (I-2) or a tautomeric modification thereof in at least one of nucleotide positions 2 to 8 of the 5' region thereof:

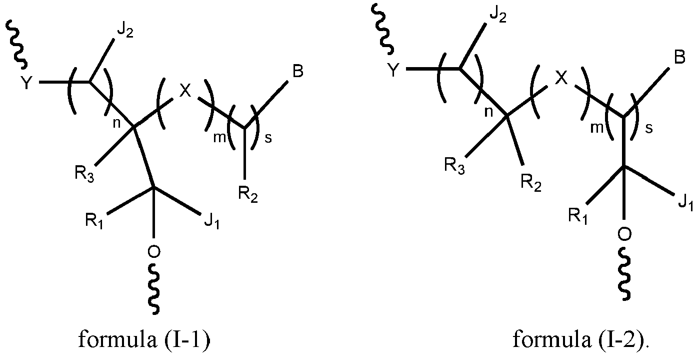

7. The siRNA according to claim 6, wherein in formula (I-1) and formula (I-2),
Y is O or NH;
each X is independently selected from the group consisting of NH-CO, CH₂, and NH;
n = 0 or 1;
m = 0 or 1;
s = 0 or 1;
J₁ and J₂ are each independently H;
R₁ is selected from the group consisting of H, methyl, and CH₂OH;
R₂ is selected from the group consisting of H, OH, NH₂, methyl, and CH₂OH;
R₃ is selected from the group consisting of H, OH, NH₂, methyl, and CH₂OH; optionally, R₁ and R₂ are directly connected to form a ring;
preferably, each B is independently selected from the group consisting of bases at nucleotide positions 2 to 8 of the 5' region of the antisense strand containing the chemical modification of formula (I-1).

8. The siRNA according to any one of claims 5 to 7, wherein the chemical modification of formula (I) is selected from the group consisting of: 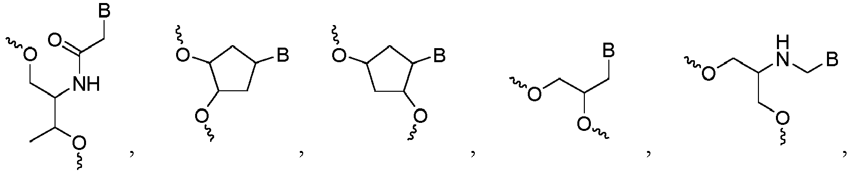 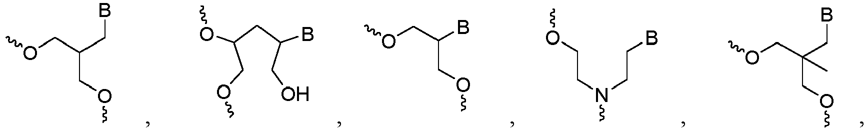 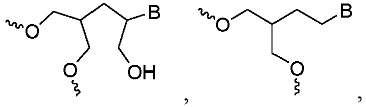 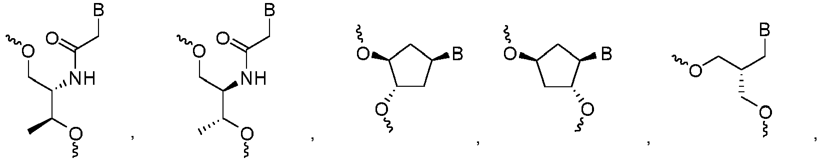 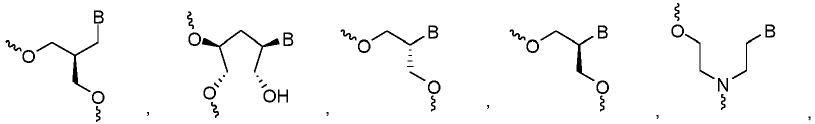 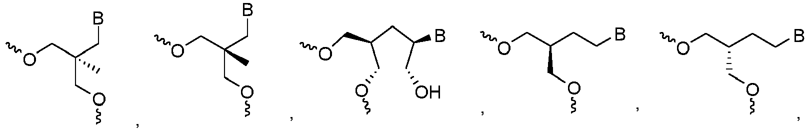 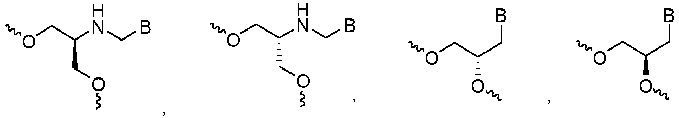 preferably, the chemical modification of formula (I) is selected from the group consisting of: 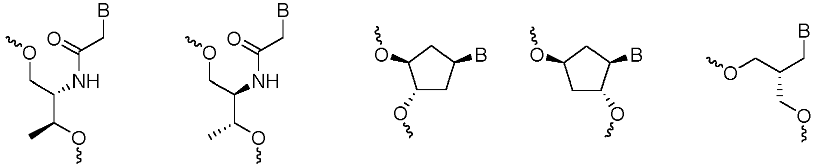 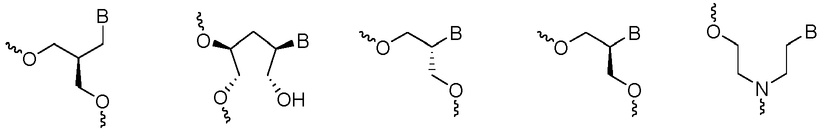 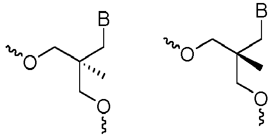 more preferably, the chemical modification of formula (I) is selected from the group consisting of: 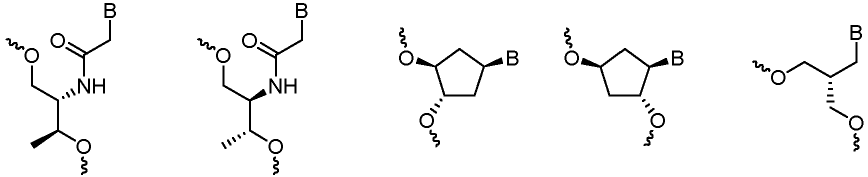 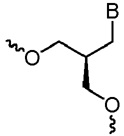 further preferably, the chemical modification of formula (I) is selected from the group consisting of: 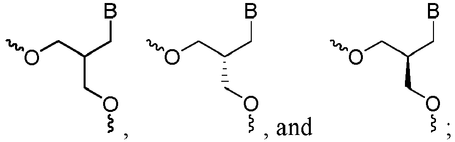 preferably, each B is independently selected from the group consisting of bases at nucleotide positions 2 to 8 of the 5' region of the antisense strand containing the chemical modification of formula (I-1).

9. The siRNA according to any one of the preceding claims, wherein the antisense strand comprises the chemical modification of formula (I) or the tautomeric modification thereof as defined in any one of claims 5 to 8 at position 5, 6, or 7 of the 5' region thereof; when the chemical modification of formula (I) or the tautomeric modification thereof is at position 5 of the 5' region, B is a base at position 5 of the 5' region of the antisense strand;
when the chemical modification of formula (I) or the tautomeric modification thereof is at position 6 of the 5' region, B is a base at position 6 of the 5' region of the antisense strand;
when the chemical modification of formula (I) or the tautomeric modification thereof is at position 7 of the 5' region, B is a base at position 7 of the 5' region of the antisense strand.

10. The siRNA according to any one of the preceding claims, wherein the nucleotide sequence of the antisense strand comprises or is: any one of SEQ ID NO: 47 to SEQ ID NO: 68,
wherein, W' represents a nucleotide comprising a chemical modification or a tautomeric modification thereof, and the chemical modification is selected from the group consisting of

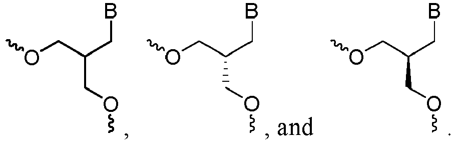

11. The siRNA according to any one of the preceding claims, wherein at least one phosphoester group in the sense strand and/or the antisense strand is a modified phosphoester group, preferably a phosphorothioate group.

12. An siRNA conjugate, comprising:
the siRNA according to any one of claims 1 to 11, and
a targeting ligand linked to the end of the siRNA;
wherein preferably, the targeting ligand is linked to the 3' end of the sense strand of the siRNA.

13. The siRNA conjugate according to claim 12, wherein:
the targeting ligand comprises at least one targeting moiety, and
the targeting moieties are each independently selected from the group consisting of: galactose, galactosamine, A-formyl-galactosamine, A-acetyl-galactosamine, *N-*propionyl-galactosamine, N n-butyryl-galactosamine, and N-isobutyryl-galactosamine; preferably, the targeting moiety is N acetyl-galactosamine;
more preferably, the targeting ligand comprises three targeting moieties.

14. A pharmaceutical composition, comprising:
the siRNA according to any one of claims 1 to 11 or the siRNA conjugate according to any one of claims 12 to 13, and
a pharmaceutically acceptable carrier.

15. A method for inhibiting expression of a 17β-hydroxysteroid dehydrogenase type 13 (HSD17B13) gene, comprising administering to a subject an effective amount or dose of the siRNA according to any one of claims 1 to 11 or the siRNA conjugate according to any one of claims 12 to 13, or the pharmaceutical composition according to claim 14.

16. A method for treating and/or preventing a disease related to HSD17B13 gene expression in a subject, comprising the step of administering to the subject an effective amount or dose of the siRNA according to any one of claims 1 to 11 or the siRNA conjugate according to any one of claims 12 to 13, or the pharmaceutical composition according to claim 14;
wherein preferably, the disease related to HSD17B13 gene expression is chronic fibro-inflammatory liver disease, and
more preferably, the chronic fibro-inflammatory liver disease is related to the accumulation and/or expansion of lipid droplets in the liver.

17. A method for treating and/or preventing a disease, comprising the step of administering to a subject an effective amount or dose of the siRNA according to any one of claims 1 to 11 or the siRNA conjugate according to any one of claims 12 to 13, or the pharmaceutical composition according to claim 14,
wherein the disease is selected from the group consisting of hepatitis, liver fibrosis, nonalcoholic steatohepatitis, nonalcoholic fatty liver disease, cirrhosis, alcoholic steatohepatitis, alcoholic fatty liver disease, HCV-associated cirrhosis, drug-induced liver injury, and hepatic necrosis.

18. A method for reducing the risk of developing chronic liver disease in an individual suffering from steatosis, and/or for inhibiting the progression of steatosis to steatohepatitis in an individual with steatosis, and/or for inhibiting the accumulation of lipid droplets in the liver, comprising the step of administering to the subject an effective amount or dose of the siRNA according to any one of claims 1 to 11 or the siRNA conjugate according to any one of claims 12 to 13, or the pharmaceutical composition according to claim 14.

19. A method for delivering siRNA to the liver *in vivo,* comprising the step of administering to a subject the siRNA according to any one of claims 1 to 11 or the siRNA conjugate according to any one of claims 12 to 13, or the pharmaceutical composition according to claim 14.

20. A method for preparing an siRNA or siRNA conjugate, comprising:
synthesizing the siRNA according to any one of claims 1 to 11 or the siRNA conjugate according to any one of claims 12 to 13.
